# EUROPEAN PATENT APPLICATION

(11) **EP 3 591 056 A1**
(43) Date of publication of application: **08.01.2020**
(21) Application number: 19175391.2
(22) Date of filing: 17.08.2012
(51) Int. Cl.: C12N 15/70, C12P 7/18, C12P 7/04, C12P 7/16, C12P 7/40, C12P 5/02

(54) **MICROORGANISMS AND METHODS FOR PRODUCING 2,4-PENTADIENOATE, BUTADIENE, PROPYLENE, 1,3-BUTANEDOL AND RELATED ALCOHOLS**

(30) Priority: 19.08.2011 US 201161525659 P; 02.09.2011 US 201161530885 P; 15.09.2011 US 201161535264 P; 10.05.2012 US 201261645509 P
(62) Divisional of application: 12826412.4
(71) Applicant: Genomatica, Inc., San Diego CA 92121 (US)
(72) Inventor: Osterhout, Robin E., San Diego, CA 92121 (US); Burgard, Anthony P., San Diego, CA 92121 (US); Burk, Mark J., San Diego, CA 92121 (US)
(74) Representative: Hoffmann Eitle

(57) **Abstract**

The invention provides non-naturally occurring microbial organisms containing 2,4-pentadienoate, butadiene, propylene, 1,3-butanediol, crotyl alcohol or 3-buten-1-ol pathways comprising at least one exogenous nucleic acid encoding a butadiene pathway enzyme expressed in a sufficient amount to produce 2,4-pentadienoate, butadiene, propylene, 1,3-butanediol, crotyl alcohol or 3-buten-1-ol. The invention additionally provides methods of using such microbial organisms to produce 2,4-pentadienoate, butadiene, propylene, 1,3-butanediol, crotyl alcohol or 3-buten-1-ol, by culturing a non-naturally occurring microbial organism containing 2,4-pentadienoate, butadiene, propylene, 1,3-butanediol, crotyl alcohol or 3-buten-1-ol pathways as described herein under conditions and for a sufficient period of time to produce 2,4-pentadienoate, butadiene, propylene, 1,3-butanediol, crotyl alcohol or 3-buten-1-ol.

SEQUENCE LISTING

<110> Genomatica, Inc.

<120> Microorganisms and methods for producing 2,4-pentadienoate, butadiene,
propylene, 1,3-butanediol and related alcohols

<130> 215191

<160> 1

<170> BiSSAP 1.3

<210> 1
<211> 20
<212> PRT
<213> Artificial Sequence

<220>
<223> sequencing result at N-terminus

<400> 1
Met Thr Tyr Lys Ala Pro Val Lys Asp Val Lys Phe Leu Leu Asp Lys
1               5                   10                  15
Val Phe Lys Val
            20

## Description

This application claims the benefit of priority of United States Provisional application serial No. 61/645,509, filed May 10, 2012, United States Provisional application serial No. 61/535,264, filed September 15, 2011, United States Provisional application serial No. 61/530,885, filed September 2, 2011, and United States Provisional application serial No. 61/525,659, filed August 19, 2011, the entire contents of which are incorporated herein by reference.

### BACKGROUND OF THE INVENTION

The present invention relates generally to biosynthetic processes, and more specifically to organisms having 2,4-pentadienoate, butadiene, propylene, 1,3-butanediol, crotyl alcohol or 3-buten-1-ol biosynthetic capability.

Over 25 billion pounds of butadiene (1,3-butadicnc, BD) arc produced annually and is applied in the manufacture of polymers such as synthetic rubbers and ABS resins, and chemicals such as hexamethylenediamine and 1,4-butanediol. Butadiene is typically produced as a by-product of the steam cracking process for conversion of petroleum feedstocks such as naphtha, liquefied petroleum gas, ethane or natural gas to ethylene and other olefins. The ability to manufacture butadiene from alternative and/or renewable feedstocks would represent a major advance in the quest for more sustainable chemical production processes

One possible way to produce butadiene renewably involves fermentation of sugars or other feedstocks to produce diols, such as 1,4-butanediol or 1,3-butanediol, which are separated, purified, and then dehydrated to butadiene in a second step involving metal-based catalysis. Direct fermentative production of butadiene from renewable feedstocks would obviate the need for dehydration steps and butadiene gas (bp -4.4°C) would be continuously emitted from the fermenter and readily condensed and collected. Developing a fermentative production process would eliminate the need for fossil-based butadiene and would allow substantial savings in cost, energy, and harmful waste and emissions relative to petrochemically-derived butadiene.

2,4-Pentadienoate is a useful substituted butadiene derivative in its own right and a valuable intermediate en route to other substituted 1,3-butadiene derivatives, including, for example, 1-carbamoyl-1,3-butadienes which are accessible via Curtius rearrangement. The resultant N-protected-1,3-butadiene derivatives can be used in Diels alder reactions for the preparation of substituted anilines. 2,4-Pentadienoate can be used in the preparation of various polymers and co-polymers.

1,3-butanediol (1,3-BDO) is a four carbon diol traditionally produced from acetylene via its hydration. The resulting acetaldehyde is then converted to 3-hydroxybutyraldehdye which is subsequently reduced to form 1,3-BDO. In more recent years, acetylene has been replaced by the less expensive ethylene as a source of acetaldehyde. 1,3-BDO is commonly used as an organic solvent for food flavoring agents. It is also used as a comonomer for polyurethane and polyester resins and is widely employed as a hypoglycaemic agent. Optically active 1,3-BDO is a useful starting material for the synthesis of biologically active compounds and liquid crystals. A commercial use of 1,3-butanediol is subsequent dehydration to afford 1,3-butadiene (Ichikawa et al., J. of Molecular Catalysis A-Chemical, 256:106-112 (2006); Ichikawa et al., J. of Molecular Catalysis A-Chemical, 231:181-189 (2005)), a 25 billion lb/yr petrochemical used to manufacture synthetic rubbers (e.g., tires), latex, and resins. The reliance on petroleum based feedstocks for either acetylene or ethylene warrants the development of a renewable feedstock based route to 1,3-butanediol and to butadiene.

3-Buten-1-ol is a raw material used in pharmaceuticals, agrochemicals, perfumes and resins. The palladium-catalyzed coupling of 3-buten-1-ol with aryl halides is a valuable process for the preparation of aryl-substituted aldehydes such as, for example, the antifolate compound Pemetrexed disodium (R. C. Larock et al., Tetrahedron Letters, 30, 6629 (1989) andU.S. Pat. No. 6,262,262). 3-Buten-1-ol is commonly prepared from propylene and formaldehyde in the presence of a catalyst at high temperature and pressure. Alternately, it is prepared from 3,4-epoxy-1-butene. Preparation of 3-buten-1-ol from renewable feedstocks has not been demonstrated to date.

Propylene is produced primarily as a by-product of petroleum refining and of ethylene production by steam cracking of hydrocarbon feedstocks. Propene is separated by fractional distillation from hydrocarbon mixtures obtained from cracking and other refining processes. Typical hydrocarbon feedstocks are from non-renewable fossil fuels, such as petroleum, natural gas and to a much lesser extent coal. Over 75 billion pounds of propylene are manufactured annually, making it the second largest fossil-based chemical produced behind ethylene. Propylene is a base chemical that is converted into a wide range of polymers, polymer intermediates and chemicals. Some of the most common derivatives of chemical and polymer grade propylene are polypropylene, acrylic acid, butanol, butanediol, acrylonitrile, propylene oxide, isopropanol and cumene. The use of the propylene derivative, polypropylene, in the production of plastics, such as injection moulding, and fibers, such as carpets, accounts for over one-third of U.S. consumption for this derivative. Propylene is also used in the production of synthetic rubber and as a propellant or component in aerosols.

The ability to manufacture propylene from alternative and/or renewable feedstocks would represent a major advance in the quest for more sustainable chemical production processes. One possible way to produce propylene renewably involves fermentation of sugars or other feedstocks to produce the alcohols 2-propanol (isopropanol) or 1-propanol, which is separated, purified, and then dehydrated to propylene in a second step involving metal-based catalysis. Direct fermentative production of propylene from renewable feedstocks would obviate the need for dehydration. During fermentative production, propylene gas would be continuously emitted from the fermenter, which could be readily collected and condensed. Developing a fermentative production process would also eliminate the need for fossil-based propylene and would allow substantial savings in cost, energy, and harmful waste and emissions relative to petrochemically-derived propylene.

Crotyl alcohol, also referred to as 2-buten-1-ol, is a valuable chemical intermediate. It serves as a precursor to crotyl halides, esters, and ethers, which in turn are chemical intermediates in the production of monomers, fine chemicals, agricultural chemicals, and pharmaceuticals. Exemplary fine chemical products include sorbic acid, trimethylhydroquinone, crotonic acid and 3-methoxybutanol. Crotyl alcohol is also a precursor to 1,3-butadiene. Crotyl alcohol is currently produced exclusively from petroleum feedstocks. For example Japanese Patent 47-013009 and U.S. Pat. Nos. 3,090,815, 3,090,816, and 3,542,883 describe a method of producing crotyl alcohol by isomerization of 1,2-epoxybutane. The ability to manufacture crotyl alcohol from alternative and/or renewable feedstocks would represent a major advance in the quest for more sustainable chemical production processes.

Thus, there exists a need for alternative methods for effectively producing commercial quantities of compounds such as 2,4-pentadienoate, butadiene, propylene, 1,3-butanediol, crotyl alcohol or 3-buten-1-ol. The present invention satisfies this need and provides related advantages as well.

### SUMMARY OF INVENTION

The invention provides non-naturally occurring microbial organisms containing 2,4-pentadienoate, butadiene, propylene, 1,3-butanediol, crotyl alcohol or 3-buten-1-ol pathways having at least one exogenous nucleic acid encoding a butadiene pathway enzyme expressed in a sufficient amount to produce 2,4-pentadienoate, butadiene, propylene, 1,3-butanediol, crotyl alcohol or 3-buten-1-ol. The invention additionally provides methods of using such microbial organisms to produce 2,4-pentadienoate, butadiene, propylene, 1,3-butanediol, crotyl alcohol or 3-buten-1-ol, by culturing a non-naturally occurring microbial organism containing 2,4-pentadienoate, butadiene, propylene, 1,3-butanediol, crotyl alcohol or 3-buten-1-ol pathways as described herein under conditions and for a sufficient period of time to produce 2,4-pentadienoate, butadiene, propylene, 1,3-butanediol, crotyl alcohol or 3-buten-1-ol.

### BRIEF DESCRIPTION OF THE DRAWINGS

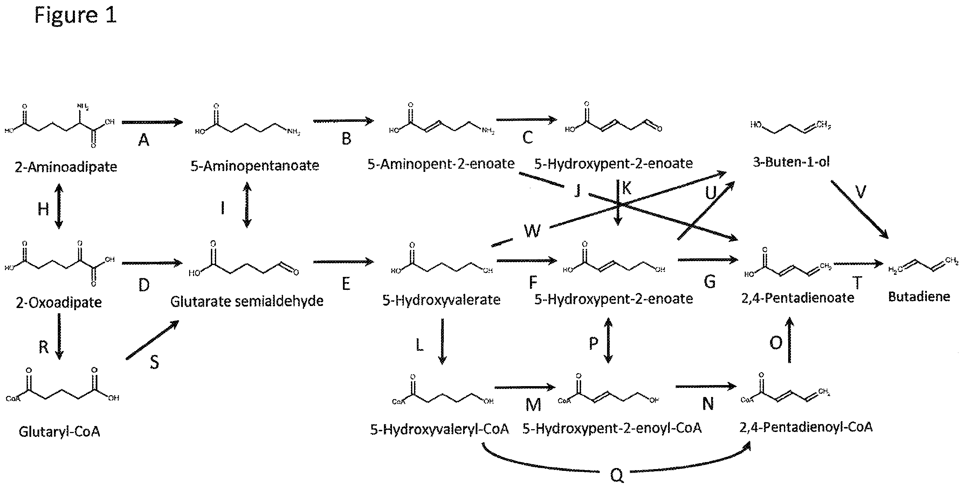

Figure 1 shows exemplary pathways to 3-buten-1-ol, 2,4-pentadienoate and butadiene from 2-oxoadipate, 2-aminoadipate, 5-aminopentanoate and glutaryl-CoA. Enzymes are: A. 2-aminoadipate decarboxylase, B. 5-aminopentanoate reductase, C. 5-aminopent-2-enoate aminotransferase, dehydrogenase or amine oxidase, D. 2-oxoadipate decarboxylase, E. glutarate semialdehyde reductase, F. 5-hydroxyvalerate dehydrogenase, G. 5-hydroxypent-2-enoate dehydratase, H. 2-aminoadipate aminotransferase, dehydrogenase or amine oxidase, I. 5-aminopentanoate aminotransferase, dehydrogenase or amine oxidase, J. 5-aminopent-2-enoate deaminase, K. 5-hydroxypent-2-enoate reductase, L. 5-hydroxyvaleryl-CoA transferase and/or synthetase, M. 5-hydroxypentanoyl-CoA dehydrogenase, N. 5-hydroxypent-2-enoyl-CoA dehydratase, O. 2,4-pentadienoyl-CoA transferase, synthetase or hydrolase, P. 5-hydroxypent-2-enoyl-CoA transferase or synthetase, Q. 5-hydroxyvaleryl-CoA dehydratase/dehydrogenase, R. 2-oxoadipate dehydrogenase, 2-oxoadipate:ferridoxin oxidoreductase or 2-oxoadipate formate lyase, S. glutaryl-CoA reductase, T. 2,4-pentadienoate decarboxylase, U. 5-hydroxypent-2-enoate decarboxylase, V. 3-buten-1-ol dehydratase or chemical conversion, W. 5-hydroxyvalerate decarboxylase.

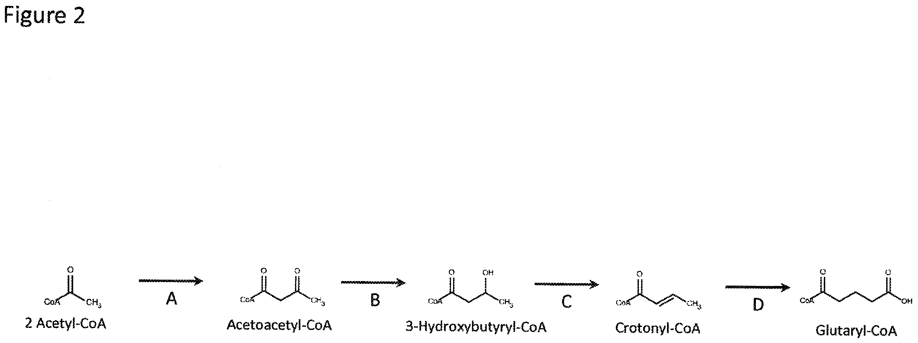

Figure 2 shows an exemplary carbon-efficient pathway from acetyl-CoA to the 2,4-pentadienoate precursor glutaryl-CoA. Enzymes are: A. acetoacetyl-CoA thiolase or synthase, B. acetoacetyl-CoA reductase, C. 3-hydroxybutyryl-CoA dehydratase, D. glutaryl-CoA dehydrogenase.

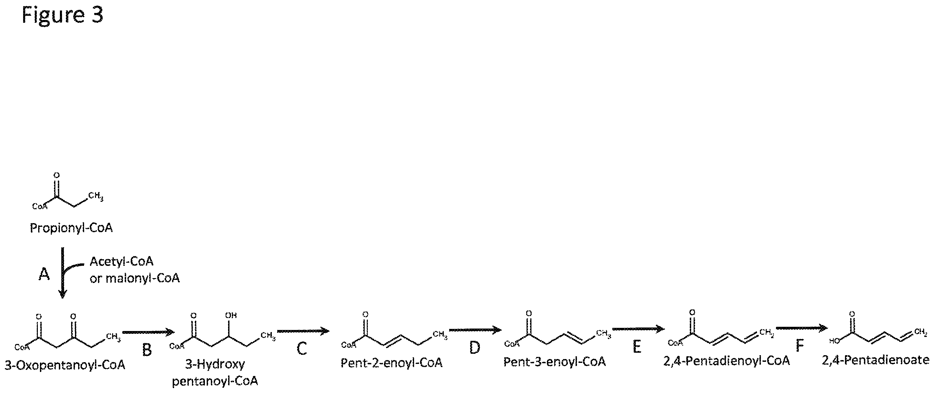

Figure 3 shows exemplary pathways for conversion of propionyl-CoA to 2,4-pentadienoate. Enzymes are: A. 3-oxopentanoyl-CoA thiolase or synthase, B. 3-oxopentanoyl-CoA reductase, C. 3-hydroxypentanoyl-CoA dehydratase, D. pent-2-enoyl-CoA isomerase, E. pent-3-enoyl-CoA dehydrogenase, F. 2,4-pentadienoyl-CoA hydrolase, transferase or synthetase, G. pent-2-enoyl-CoA dehydrogenase.

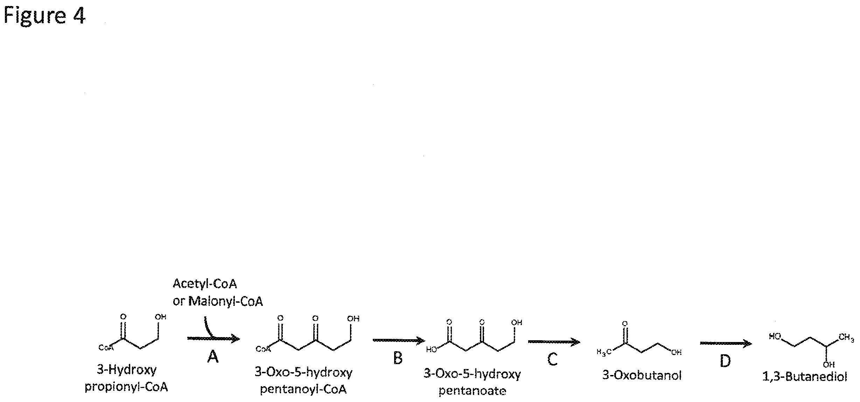

Figure 4 shows an exemplary pathway for 1,3-butanediol formation from 3-hydroxypropionyl-CoA and acetyl-CoA. Enzymes are: A. 3-oxo-5-hydroxypentanoyl-CoA thiolase or synthase, B. 3-oxo-5-hydroxypentanoyl-CoA hydrolase, transferase or synthetase, C. 3-oxo-5-hydroxypentanoate decarboxylase and D. 3-oxobutanol reductase.

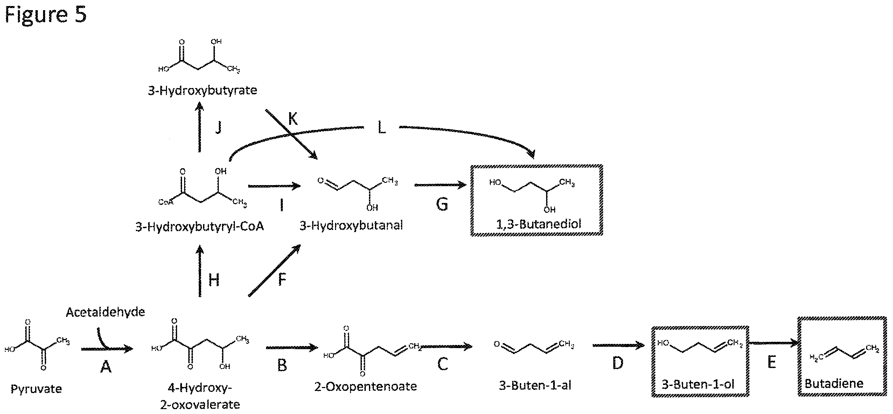

Figure 5 shows exemplary pathways to 1,3-butanediol (13-BDO), 3-buten-1-ol and butadiene from pyruvate and acetaldehyde. Enzymes are: A. 4-hydroxy-2-oxovalerate aldolase, B. 4-hydroxy-2-oxovalerate dehydratase, C. 2-oxopentenoate decarboxylase, D. 3-buten-1-al reductase, E. 3-buten-1-ol dehydratase, F. 4-hydroxy-2-oxovalerate decarboxylase, G. 3-hydroxybutanal reductase, H. 4-hydroxy-2-oxopentanoate dehydrogenase, 4-hydroxy-2-oxopentanoate:ferredoxin oxidoreductase or 4-hydroxy-2-oxopentanoate formate lyase, I. 3-hydroxybutyryl-CoA reductase (aldehyde forming), J. 3-hydroxybutyryl-CoA hydrolase, transferase or synthetase, K. 3-hydroxybutyrate reductase, L. 3-hydroxybutyryl-CoA reductase (alcohol forming). Step E can also be catalyzed via chemical dehydration.

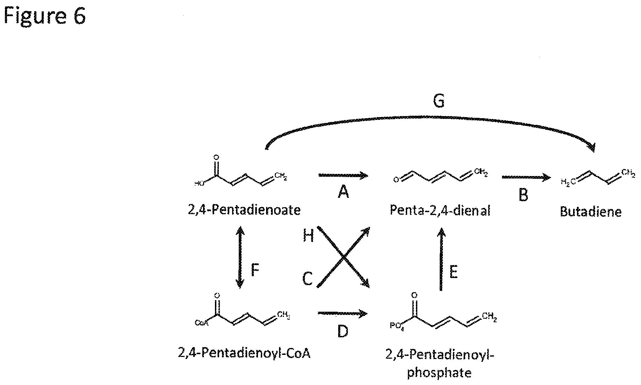

Figure 6 shows exemplary pathways to butadiene from 2,4-pentadienoate and 2,4-pentadienoyl-CoA. Enzymes are: A. 2,4-pentadienoate reductase (acid reducing), B. penta-2,4-dienal decarbonylase, C. 2,4-pentadienoyl-CoA reductase (acid reducing), D. 2,4-pentadienoyl-CoA phosphotransferase, E. 2,4-pentadienoyl-phosphate reductase, F. 2,4-pentadienoyl-CoA hydrolase, transferase or synthetase, G. 2,4-pentadienoate decarboxylase, H. 2,4-pentadienoate kinase.

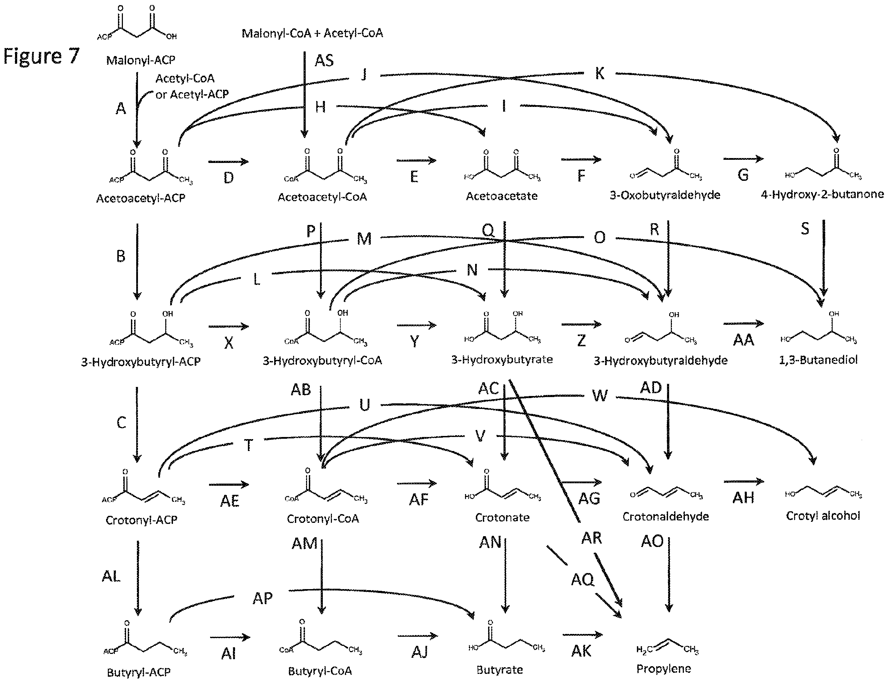

Figure 7 shows exemplary pathways for formation of 1,3-butanediol, crotyl alcohol and propylene from malonyl-ACP. Enyzmes are: A. 3-ketoacyl-ACP synthase, B. Acetoacetyl-ACP reductase, C. 3-hydroxybutyryl-ACP dehydratase, D. acetoacetyl-CoA:ACP transferase, E. acetoacetyl-CoA hydrolase, transferase or synthetase, F. acetoacetate reductase (acid reducing), G. 3-oxobutyraldehyde reductase (aldehyde reducing), H. acetoacetyl-ACP thioesterase, I. acetoacetyl-CoA reductase (CoA-dependent, aldehyde forming), J. acetoacetyl-ACP reductase (aldehyde forming), K. acetoacetyl-CoA reductase (alcohol forming), L. 3-hydroxybutyryl-ACP thioesterase, M. 3-hydroxybutyryl-ACP reductase (aldehyde forming), N. 3-hydroxybutyryl-CoA reductase (aldehyde forming), O. 3-hydroxybutyryl-CoA reductase (alcohol forming), P. acetoacetyl-CoA reductase (ketone reducing), Q. acetoacetate reductase (ketone reducing), R. 3-oxobutyraldehyde reductase (ketone reducing), S. 4-hydroxy-2-butanone reductase, T. crotonyl-ACP thioesterase, U. crotonyl-ACP reductase (aldehyde forming), V. crotonyl-CoA reductase (aldehyde forming), W. crotonyl-CoA (alcohol forming), X. 3-hydroxybutyryl-CoA:ACP transferase, Y. 3-hydroxybutyryl-CoA hydrolase, transferase or synthetase, Z. 3-hydroxybutyrate reductase, AA. 3-hydroxybutyraldehyde reductase, AB. 3-hydroxybutyryl-CoA dehydratase, AC. 3-hydroxybutyrate dehydratase, AD. 3-hydroxybutyraldehyde dehydratase, AE. crotonyl-CoA:ACP transferase, AF. crotonyl-CoA hydrolase, transferase or synthetase, AG. crotonate reductase, AH. crotonaldehyde reductase, AI. Butryl-CoA:ACP transferase, AJ. Butyryl-CoA transferase, hydrolase or synthetase, AK. Butyrate decarboxylase, AL. crotonyl-ACP reductase, AM. crotonyl-CoA reductase, AN. crotonate reductase, AO. crotonaldehyde decarbonylase, AP. butyryl-ACP thioesterase, AQ. crotonate decarboxylase, AR. 3-hydroxybutyrate decarboxylase, AS. acetoacetyl-CoA synthase. ACP is acyl carrier protein.

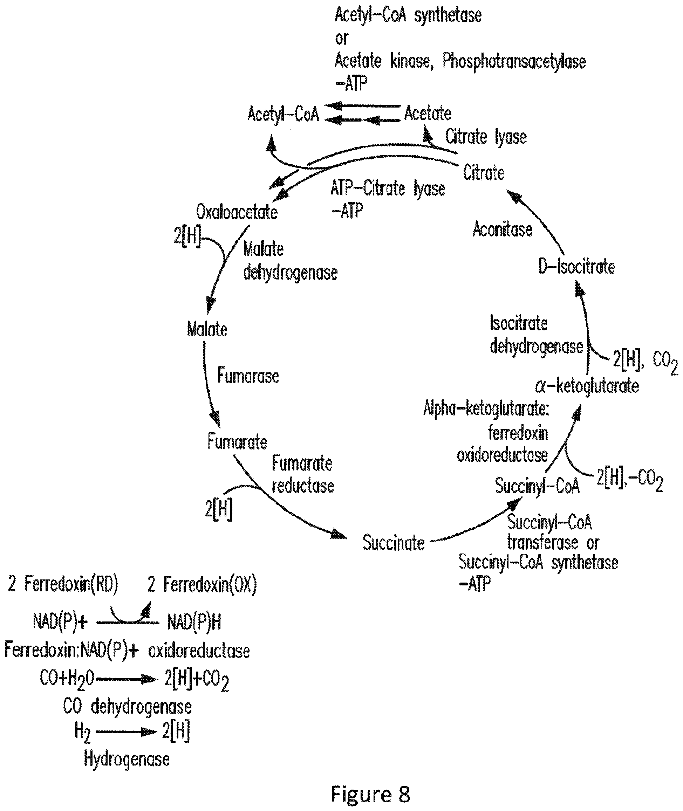

Figure 8 shows the reverse TCA cycle for fixation of CO₂ on carbohydrates as substrates. The enzymatic transformations are carried out by the enzymes as shown.

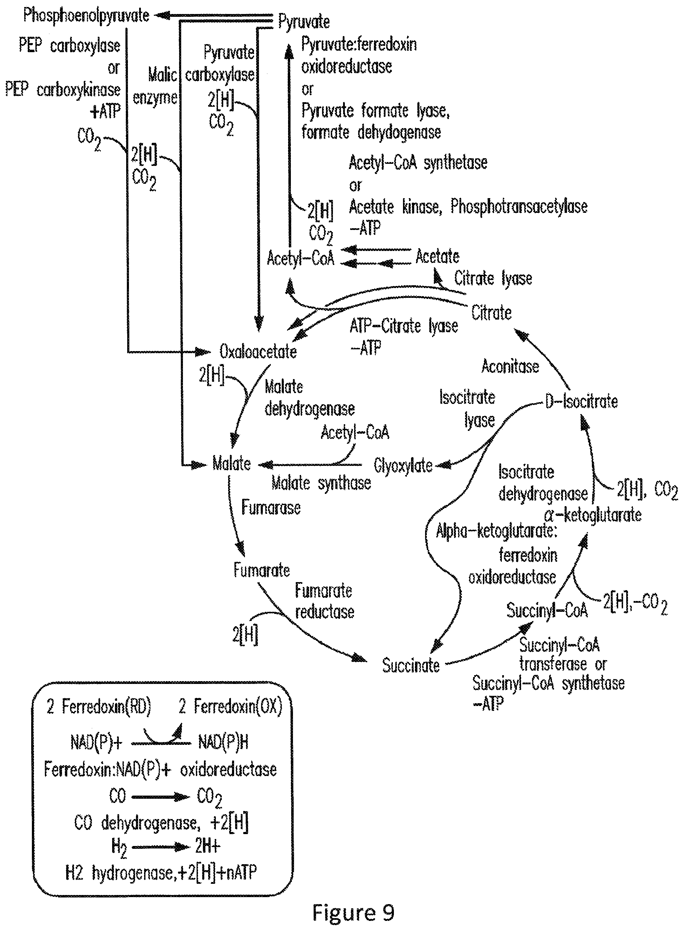

Figure 9 shows the pathway for the reverse TCA cycle coupled with carbon monoxide dehydrogenase and hydrogenase for the conversion of syngas to acetyl-CoA.

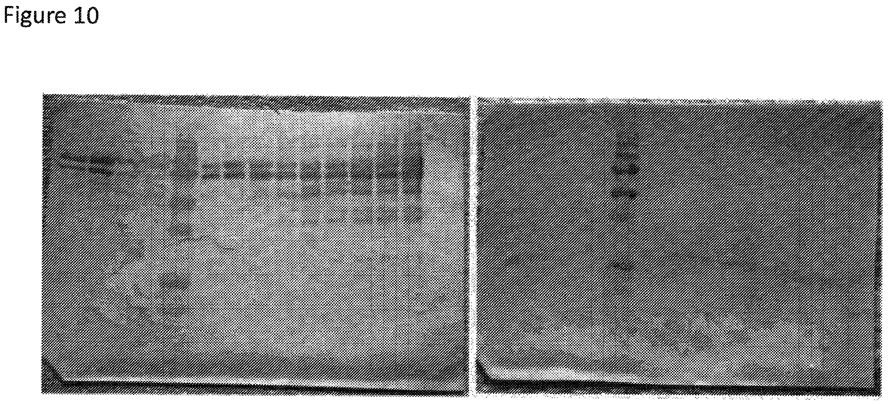

Figure 10 shows Western blots of 10 micrograms ACS90 (lane 1), ACS91 (lane2), Mta98/99 (lanes 3 and 4) cell extracts with size standards (lane 5) and controls of M. thermoacetica CODH (Moth_1202/1203) or Mtr (Moth_1197) proteins (50, 150, 250, 350, 450, 500, 750, 900, and 1000 ng).

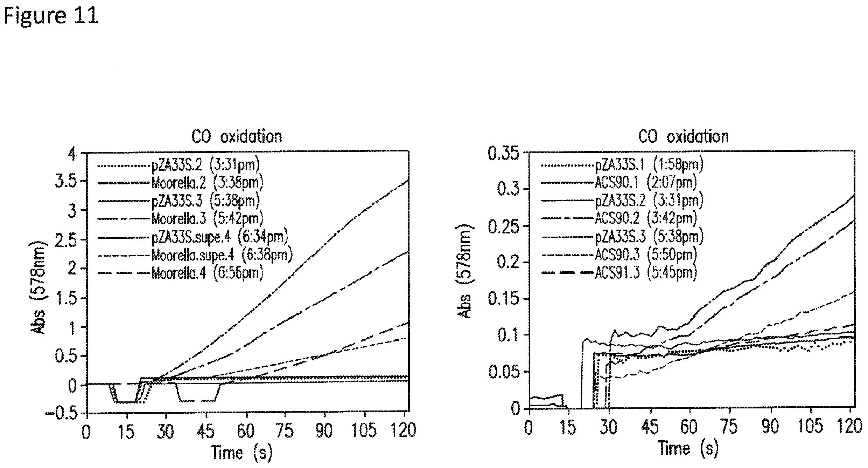

Figure 11 shows CO oxidation assay results. Cells (*M. thermoacetica* or *E. coli* with the CODH/ACS operon; ACS90 or ACS91 or empty vector: pZA33S) were grown and extracts prepared. Assays were performed at 55°C at various times on the day the extracts were prepared. Reduction of methylviologen was followed at 578 nm over a 120 sec time course.

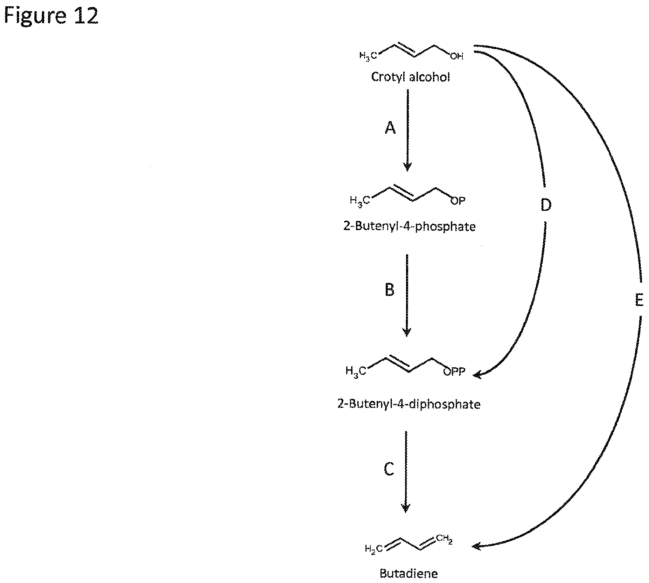

Figure 12 shows pathways for conversion of crotyl alcohol to butadiene. Enzymes are: A. crotyl alcohol kinase, B. 2-butenyl-4-phosphate kinase, C. butadiene synthase, and D. crotyl alcohol diphosphokinase. Step E is catalyzed non-enzymatically.

### DETAILED DESCRIPTION OF THE INVENTION

The present invention is directed to the design and production of cells and organisms having biosynthetic production capabilities for 2,4-pentadienoate, butadiene, propylene, 1,3-butanediol, crotyl alcohol or 3-buten-1-ol. The invention, in particular, relates to the design of microbial organisms capable of producing 2,4-pentadienoate, butadiene, propylene, 1,3-butanediol, crotyl alcohol or 3-buten-1-ol by introducing one or more nucleic acids encoding a 2,4-pentadienoate, butadiene, propylene, 1,3-butanediol, crotyl alcohol or 3-buten-1-ol pathway enzyme.

In one embodiment, the invention utilizes *in silico* stoichiometric models of *Escherichia coli* metabolism that identify metabolic designs for biosynthetic production of 2,4-pentadienoate, butadiene, propylene, 1,3-butanediol, crotyl alcohol or 3-buten-1-ol. The results described herein indicate that metabolic pathways can be designed and recombinantly engineered to achieve the biosynthesis of 2,4-pentadienoate, butadiene, propylene, 1,3-butanediol, crotyl alcohol or 3-buten-1-ol in *Escherichia coli* and other cells or organisms. Biosynthetic production of 2,4-pentadienoate, butadiene, propylene, 1,3-butanediol, crotyl alcohol or 3-buten-1-ol, for example, for the *in silico* designs can be confirmed by construction of strains having the designed metabolic genotype. These metabolically engineered cells or organisms also can be subjected to adaptive evolution to further augment butadiene biosynthesis, including under conditions approaching theoretical maximum growth.

In certain embodiments, the 2,4-pentadienoate, butadiene, propylene, 1,3-butanediol, crotyl alcohol or 3-buten-1-ol biosynthesis characteristics of the designed strains make them genetically stable and particularly useful in continuous bioprocesses. Separate strain design strategies were identified with incorporation of different non-native or heterologous reaction capabilities into *E. coli* or other host organisms leading to 2,4-pentadienoate, butadiene, propylene, 1,3-butanediol, crotyl alcohol or 3-buten-1-ol producing metabolic pathways from 2-aminoadipate, 5-aminopentanoate, 2-oxoadipate, glutaryl-CoA, acetyl-CoA, propionyl-CoA, 3-hydroxypropionyl-CoA or pyruvate. *In silico* metabolic designs were identified that resulted in the biosynthesis of 2,4-pentadienoate, butadiene, propylene, 1,3-butanediol, crotyl alcohol or 3-buten-1-ol in microorganisms from each of these substrates or metabolic intermediates.

Strains identified via the computational component of the platform can be put into actual production by genetically engineering any of the predicted metabolic alterations, which lead to the biosynthetic production of 2,4-pentadienoate, butadiene, propylene, 1,3-butanediol, crotyl alcohol or 3-buten-1-ol or other intermediate and/or downstream products. In yet a further embodiment, strains exhibiting biosynthetic production of these compounds can be further subjected to adaptive evolution to further augment product biosynthesis. The levels of product biosynthesis yield following adaptive evolution also can be predicted by the computational component of the system.

The maximum theoretical 2,4-pentadienoate yield from glucose is 1.09 mol/mol (0.59 g/g).

11C₆H₁₂O₆ = 12C₅H₆O₂ + 6CO₂ + 30H₂O

The pathways presented in Figure 1 achieve a yield of 0.85 moles 2,4-pentadienoate per mole of glucose utilized. Increasing product yields is possible if cells are capable of fixing CO₂ through pathways such as the reductive (or reverse) TCA cycle or the Wood-Ljungdahl pathway. Organisms engineered to possess the pathway depicted in Figure 1 are also capable of reaching near theoretical maximum yields of 2,4-pentadienoate.

The maximum theoretical butadiene yield from glucose is 1.09 mol/mol (0.327g/g).

11C₆H₁₂O₆ = 12C₄H₆ - 18CO₂ + 30H₂O

The pathways presented in Figure 1 achieves a yield of 0.85 moles butadiene per mole of glucose utilized. Increasing product yields to near theoretical maximum values is possible if cells are capable of fixing CO₂ through pathways such as the reductive (or reverse) TCA cycle or the Wood-Ljungdahl pathway. Organisms engineered to possess a pathway depicted in Figures 5, 6 or Figure 1 in combination with a pathways depicted in Figure 12 are also capable of reaching near theoretical maximum yields of butadiene.

The maximum theoretical 1,3-butanediol yield from glucose is 1.09 mol/mol (0.54 g/g).

11C₆H₁₂O₆ = 12C₄H₁₀O₂ + 18CO₂ + 6H₂O

The pathways presented in Figure 5 achieve a yield of 1 moles 1,3-butanediol per mole of glucose utilized. Increasing product yields to theoretical maximum value is possible if cells are capable of fixing CO₂ through pathways such as the reductive (or reverse) TCA cycle or the Wood-Ljungdahl pathway. Organisms engineered to possess the pathways depicted in Figure 7 are also capable of reaching theoretical maximum yields of 1,3-butanediol.

The maximum theoretical 3-buten-1-ol yield from glucose is 1.09 mol/mol (0.437 g/g).

11C₆H₁₂O₆ = 12C₄H₈O + 18CO₂ + 18H₂O

The pathways presented in Figure 1 achieve a yield of 0.85 moles 3-buten-1-ol per mole of glucose utilized. Increasing product yields to nearly the theoretical maximum is possible if cells are capable of fixing CO₂ through pathways such as the reductive (or reverse) TCA cycle or the Wood-Ljungdahl pathway. Organisms engineered to possess the pathway depicted in Figure 5 are also capable of reaching near theoretical maximum yields of butadiene.

The maximum theoretical crotyl alcohol yield from glucose is 1.09 mol/mol (0.436 g/g).

11C₆H₁₂O₆ = 12C₄H₈O + 18CO₂ + 18H₂O

The pathways presented in Figure 7 achieve a yield of 1.08 moles crotyl alcohol per mole of glucose utilized. Increasing product yields to the theoretical maximum is possible if cells are capable of fixing CO₂ through pathways such as the reductive (or reverse) TCA cycle or the Wood-Ljungdahl pathway.

The maximum theoretical propylene yield from glucose is 1.33 mol/mol (0.31 g/g).

3C₆H₁₂O₆ = 4C₄H₈O + 6CO₂ + 6H₂O

The pathways presented in Figure 7 achieve a yield of 1.2 moles propylene per mole of glucose utilized. Increasing product yields to nearly the theoretical maximum is possible if cells are capable of fixing CO₂ through pathways such as the reductive (or reverse) TCA cycle or the Wood-Ljungdahl pathway.

As used herein, the term "non-naturally occurring" when used in reference to a microbial organism or microorganism of the invention is intended to mean that the microbial organism has at least one genetic alteration not normally found in a naturally occurring strain of the referenced species, including wild-type strains of the referenced species. Genetic alterations include, for example, modifications introducing expressible nucleic acids encoding metabolic polypeptides, other nucleic acid additions, nucleic acid deletions and/or other functional disruption of the microbial organism's genetic material. Such modifications include, for example, coding regions and functional fragments thereof, for heterologous, homologous or both heterologous and homologous polypeptides for the referenced species. Additional modifications include, for example, non-coding regulatory regions in which the modifications alter expression of a gene or operon. Exemplary metabolic polypeptides include enzymes or proteins within a 2,4-pentadienoate, butadiene, propylene, 1,3-butanediol, crotyl alcohol or 3-buten-1-ol biosynthetic pathway.

A metabolic modification refers to a biochemical reaction that is altered from its naturally occurring state. Therefore, non-naturally occurring microorganisms can have genetic modifications to nucleic acids encoding metabolic polypeptides, or functional fragments thereof. Exemplary metabolic modifications are disclosed herein.

As used herein, the term "isolated" when used in reference to a microbial organism is intended to mean an organism that is substantially free of at least one component as the referenced microbial organism is found in nature. The term includes a microbial organism that is removed from some or all components as it is found in its natural environment. The term also includes a microbial organism that is removed from some or all components as the microbial organism is found in non-naturally occurring environments. Therefore, an isolated microbial organism is partly or completely separated from other substances as it is found in nature or as it is grown, stored or subsisted in non-naturally occurring environments. Specific examples of isolated microbial organisms include partially pure microbes, substantially pure microbes and microbes cultured in a medium that is non-naturally occurring.

As used herein, the terms "microbial," "microbial organism" or "microorganism" are intended to mean any organism that exists as a microscopic cell that is included within the domains of archaea, bacteria or eukarya. Therefore, the term is intended to encompass prokaryotic or eukaryotic cells or organisms having a microscopic size and includes bacteria, archaca and cubacteria of all species as well as eukaryotic microorganisms such as yeast and fungi. The term also includes cell cultures of any species that can be cultured for the production of a biochemical.

As used herein, the term "CoA" or "coenzyme A" is intended to mean an organic cofactor or prosthetic group (nonprotein portion of an enzyme) whose presence is required for the activity of many enzymes (the apoenzyme) to form an active enzyme system. Coenzyme A functions in certain condensing enzymes, acts in acetyl or other acyl group transfer and in fatty acid synthesis and oxidation, pyruvate oxidation and in other acetylation.

As used herein, the term "ACP" or "acyl carrier protein" refers to any of the relatively small acidic proteins that are associated with the fatty acid synthase system of many organisms, from bacteria to plants. ACPs can contain one 4'-phosphopantetheine prosthetic group bound covalently by a phosphate ester bond to the hydroxyl group of a serine residue. The sulfhydryl group of the 4'-phosphopantetheine moiety serves as an anchor to which acyl intermediates are (thio)esterified during fatty-acid synthesis. An example of an ACP is *Escherichia coli* ACP, a separate single protein, containing 77 amino-acid residues (8.85 kDa), wherein the phosphopantetheine group is linked to serine 36.

As used herein, the term "butadiene," having the molecular formula C₄H₆ and a molecular mass of 54.09 g/mol (see Figures 1, 5, 6 and 12) (IUPAC name Buta-1,3-diene) is used interchangeably throughout with 1,3-butadiene, biethylene, erythrene, divinyl, vinylethylene. Butadiene is a colorless, non corrosive liquefied gas with a mild aromatic or gasoline-like odor. Butadiene is both explosive and flammable because of its low flash point.

As used herein, the term "substantially anaerobic" when used in reference to a culture or growth condition is intended to mean that the amount of oxygen is less than about 10% of saturation for dissolved oxygen in liquid media. The term also is intended to include sealed chambers of liquid or solid medium maintained with an atmosphere of less than about 1% oxygen.

"Exogenous" as it is used herein is intended to mean that the referenced molecule or the referenced activity is introduced into the host microbial organism. The molecule can be introduced, for example, by introduction of an encoding nucleic acid into the host genetic material such as by integration into a host chromosome or as non-chromosomal genetic material such as a plasmid. Therefore, the term as it is used in reference to expression of an encoding nucleic acid refers to introduction of the encoding nucleic acid in an expressible form into the microbial organism. When used in reference to a biosynthetic activity, the term refers to an activity that is introduced into the host reference organism. The source can be, for example, a homologous or heterologous encoding nucleic acid that expresses the referenced activity following introduction into the host microbial organism. Therefore, the term "endogenous" refers to a referenced molecule or activity that is present in the host. Similarly, the term when used in reference to expression of an encoding nucleic acid refers to expression of an encoding nucleic acid contained within the microbial organism. The term "heterologous" refers to a molecule or activity derived from a source other than the referenced species whereas "homologous" refers to a molecule or activity derived from the host microbial organism. Accordingly, exogenous expression of an encoding nucleic acid of the invention can utilize either or both a heterologous or homologous encoding nucleic acid.

It is understood that when more than one exogenous nucleic acid is included in a microbial organism that the more than one exogenous nucleic acids refers to the referenced encoding nucleic acid or biosynthetic activity, as discussed above. It is further understood, as disclosed herein, that such more than one exogenous nucleic acids can be introduced into the host microbial organism on separate nucleic acid molecules, on polycistronic nucleic acid molecules, or a combination thereof, and still be considered as more than one exogenous nucleic acid. For example, as disclosed herein a microbial organism can be engineered to express two or more exogenous nucleic acids encoding a desired pathway enzyme or protein. In the case where two exogenous nucleic acids encoding a desired activity are introduced into a host microbial organism, it is understood that the two exogenous nucleic acids can be introduced as a single nucleic acid, for example, on a single plasmid, on separate plasmids, can be integrated into the host chromosome at a single site or multiple sites, and still be considered as two exogenous nucleic acids. Similarly, it is understood that more than two exogenous nucleic acids can be introduced into a host organism in any desired combination, for example, on a single plasmid, on separate plasmids, can be integrated into the host chromosome at a single site or multiple sites, and still be considered as two or more exogenous nucleic acids, for example three exogenous nucleic acids. Thus, the number of referenced exogenous nucleic acids or biosynthetic activities refers to the number of encoding nucleic acids or the number of biosynthetic activities, not the number of separate nucleic acids introduced into the host organism.

The non-naturally occurring microbal organisms of the invention can contain stable genetic alterations, which refers to microorganisms that can be cultured for greater than five generations without loss of the alteration. Generally, stable genetic alterations include modifications that persist greater than 10 generations, particularly stable modifications will persist more than about 25 generations, and more particularly, stable genetic modifications will be greater than 50 generations, including indefinitely.

Those skilled in the art will understand that the genetic alterations, including metabolic modifications exemplified herein, are described with reference to a suitable host organism such as *E. coli* and their corresponding metabolic reactions or a suitable source organism for desired genetic material such as genes for a desired metabolic pathway. However, given the complete genome sequencing of a wide variety of organisms and the high level of skill in the area of genomics, those skilled in the art will readily be able to apply the teachings and guidance provided herein to essentially all other organisms. For example, the *E. coli* metabolic alterations exemplified herein can readily be applied to other species by incorporating the same or analogous encoding nucleic acid from species other than the referenced species. Such genetic alterations include, for example, genetic alterations of species homologs, in general, and in particular, orthologs, paralogs or nonorthologous gene displacements.

An ortholog is a gene or genes that are related by vertical descent and are responsible for substantially the same or identical functions in different organisms. For example, mouse epoxide hydrolase and human epoxide hydrolase can be considered orthologs for the biological function of hydrolysis of epoxides. Genes are related by vertical descent when, for example, they share sequence similarity of sufficient amount to indicate they are homologous, or related by evolution from a common ancestor. Genes can also be considered orthologs if they share three-dimensional structure but not necessarily sequence similarity, of a sufficient amount to indicate that they have evolved from a common ancestor to the extent that the primary sequence similarity is not identifiable. Genes that are orthologous can encode proteins with sequence similarity of about 25% to 100% amino acid sequence identity. Genes encoding proteins sharing an amino acid similarity less that 25% can also be considered to have arisen by vertical descent if their three-dimensional structure also shows similarities. Members of the serine protease family of enzymes, including tissue plasminogen activator and elastase, are considered to have arisen by vertical descent from a common ancestor.

Orthologs include genes or their encoded gene products that through, for example, evolution, have diverged in structure or overall activity. For example, where one species encodes a gene product exhibiting two functions and where such functions have been separated into distinct genes in a second species, the three genes and their corresponding products are considered to be orthologs. For the production of a biochemical product, those skilled in the art will understand that the orthologous gene harboring the metabolic activity to be introduced or disrupted is to be chosen for construction of the non-naturally occurring microorganism. An example of orthologs exhibiting separable activities is where distinct activities have been separated into distinct gene products between two or more species or within a single species. A specific example is the separation of elastase proteolysis and plasminogen proteolysis, two types of serine protease activity, into distinct molecules as plasminogen activator and elastase. A second example is the separation of mycoplasma 5'-3' exonuclease and *Drosophila* DNA polymerase III activity. The DNA polymerase from the first species can be considered an ortholog to either or both of the exonuclease or the polymerase from the second species and vice versa.

In contrast, paralogs are homologs related by, for example, duplication followed by evolutionary divergence and have similar or common, but not identical functions. Paralogs can originate or derive from, for example, the same species or from a different species. For example, microsomal epoxide hydrolase (epoxide hydrolase I) and soluble epoxide hydrolase (epoxide hydrolase II) can be considered paralogs because they represent two distinct enzymes, co-evolved from a common ancestor, that catalyze distinct reactions and have distinct functions in the same species. Paralogs are proteins from the same species with significant sequence similarity to each other suggesting that they are homologous, or related through co-evolution from a common ancestor. Groups of paralogous protein families include HipA homologs, luciferase genes, peptidases, and others.

A nonorthologous gene displacement is a nonorthologous gene from one species that can substitute for a referenced gene function in a different species. Substitution includes, for example, being able to perform substantially the same or a similar function in the species of origin compared to the referenced function in the different species. Although generally, a nonorthologous gene displacement will be identifiable as structurally related to a known gene encoding the referenced function, less structurally related but functionally similar genes and their corresponding gene products nevertheless will still fall within the meaning of the term as it is used herein. Functional similarity requires, for example, at least some structural similarity in the active site or binding region of a nonorthologous gene product compared to a gene encoding the function sought to be substituted. Therefore, a nonorthologous gene includes, for example, a paralog or an unrelated gene.

Therefore, in identifying and constructing the non-naturally occurring microbial organisms of the invention having 2,4-pentadienoate, butadiene, propylene, 1,3-butanediol, crotyl alcohol or 3-buten-1-ol biosynthetic capability, those skilled in the art will understand with applying the teaching and guidance provided herein to a particular species that the identification of metabolic modifications can include identification and inclusion or inactivation of orthologs. To the extent that paralogs and/or nonorthologous gene displacements are present in the referenced microorganism that encode an enzyme catalyzing a similar or substantially similar metabolic reaction, those skilled in the art also can utilize these evolutionally related genes.

Orthologs, paralogs and nonorthologous gene displacements can be determined by methods well known to those skilled in the art. For example, inspection of nucleic acid or amino acid sequences for two polypeptides will reveal sequence identity and similarities between the compared sequences. Based on such similarities, one skilled in the art can determine if the similarity is sufficiently high to indicate the proteins are related through evolution from a common ancestor. Algorithms well known to those skilled in the art, such as Align, BLAST, Clustal W and others compare and determine a raw sequence similarity or identity, and also determine the presence or significance of gaps in the sequence which can be assigned a weight or score. Such algorithms also are known in the art and are similarly applicable for determining nucleotide sequence similarity or identity. Parameters for sufficient similarity to determine relatedness are computed based on well known methods for calculating statistical similarity, or the chance of finding a similar match in a random polypeptide, and the significance of the match determined. A computer comparison of two or more sequences can, if desired, also be optimized visually by those skilled in the art. Related gene products or proteins can be expected to have a high similarity, for example, 25% to 100% sequence identity. Proteins that are unrelated can have an identity which is essentially the same as would be expected to occur by chance, if a database of sufficient size is scanned (about 5%). Sequences between 5% and 24% may or may not represent sufficient homology to conclude that the compared sequences are related. Additional statistical analysis to determine the significance of such matches given the size of the data set can be carried out to determine the relevance of these sequences.

Exemplary parameters for determining relatedness of two or more sequences using the BLAST algorithm, for example, can be as set forth below. Briefly, amino acid sequence alignments can be performed using BLASTP version 2.0.8 (Jan-05-1999) and the following parameters: Matrix: 0 BLOSUM62; gap open: 11; gap extension: 1; x_dropoff: 50; expect: 10.0; wordsize: 3; filter: on. Nucleic acid sequence alignments can be performed using BLASTN version 2.0.6 (Sept-16-1998) and the following parameters: Match: 1; mismatch: -2; gap open: 5; gap extension: 2; x_dropoff: 50; expect: 10.0; wordsize: 11; filter: off. Those skilled in the art will know what modifications can be made to the above parameters to either increase or decrease the stringency of the comparison, for example, and determine the relatedness of two or more sequences.

In some embodiments, the invention provides a non-naturally occurring microbial organism, having a microbial organism having a 2,4-pentadienoate pathway having at least one exogenous nucleic acid encoding a 2,4-pentadienoate pathway enzyme expressed in a sufficient amount to produce 2,4-pentadienoate, wherein the 2,4-pentadienoate pathway includes a pathway shown in Figures 1 and/or 3 selected from: (1) 1D, 1I, 1B, 1C, 1K and 1G; (2) 1D, IE, 1F and 1G; (3) 1D, IE, 1L, 1M, 1P and 1G; (4) 1D, 1I, 1B and 1J; (5) 1D, 1I, 1B, 1C, 1K, 1P, IN and 1O; (6) 1D, IE, 1F, 1P, IN and 1O; (7) 1D, IE, 1L, 1M, IN and 10; (8) 1D, IE, 1L, 1Q and 1O; (9) IS, 1I, 1B, 1C, 1K and 1G; (10) IS, IE, 1F and 1G; (11) IS, 1I, 1B and 1J; (12) IS, 1I, 1B, 1C, 1K, 1P, IN and 1O; (13) IS, IE, 1F, 1P, IN and 1O; (14) IS, IE, 1L, 1M, IN and 1O; (15) IS, IE, 1L, 1Q and 1O; (16) 1B, 1C, 1K and 1G; (17) 1I, IE, 1F and 1G; (18) 1I, IE, 1L, 1M, 1P and 1G; (19) 1B and 1J; (20) 1I, IE, 1F, 1P, IN and 10; (21) 1I, IE, 1L, 1M, IN and 10; (22) 1I, IE, 1L, 1Q and 10; (23) 3A, 3B, 3C, 3D, 3E and 3F; and (24) 3A, 3B, 3C, 3G and 3F, wherein 1B is a 5-aminopentanoate reductase, wherein 1C is a 5-aminopent-2-enoate aminotransferase, a 5-aminopent-2-enoate dehydrogenase or an amine oxidase, wherein 1D is a 2-oxoadipate decarboxylase, wherein 1E is a glutarate semialdehyde reductase, wherein 1F is a 5-hydroxyvalerate dehydrogenase, wherein 1G is a 5-hydroxypent-2-enoate dehydratase, wherein 1I is a 5-aminopentanoate aminotransferase, a 5-aminopentanoate dehydrogenase or an amine oxidase, wherein 1J is a 5-aminopent-2-enoate deaminase, wherin 1K is a 5-hydroxypent-2-enoate reductase, wherein 1L is a 5-hydroxyvaleryl-CoA transferase or a 5-hydroxyvaleryl-CoA synthetase, wherein 1M is a 5-hydroxypentanoyl-CoA dehydrogenase, wherein IN is a 5-hydroxypent-2-enoyl-CoA dehydratase, wherein 10 is a 2,4-pentadienoyl-CoA transferase, a 2,4-pentadienoyl-CoA synthetase or a 2,4-pentadienoyl-CoA hydrolase, wherein 1P is a 5-hydroxypent-2-enoyl-CoA transferase or a 5-hydroxypent-2-enoyl-CoA synthetase, wherein 1Q is a 5-hydroxyvaleryl-CoA dehydratase/dehydrogenase, wherein 1S a glutaryl-CoA reductase, wherein 3A is a 3-oxopentanoyl-CoA thiolase or 3-oxopentanoyl-CoA synthase, wherein 3B is a 3-oxopentanoyl-CoA reductase, wherein 3C is a 3-hydroxypentanoyl-CoA dehydratase, wherein 3D is a pent-2-enoyl-CoA isomerase, wherein 3E is a pent-3-enoyl-CoA dehydrogenase, wherein 3F is a 2,4-pentadienoyl-CoA hydrolase, a 2,4-pentadienoyl-CoA transferase or a 2,4-pentadienoyl-CoA synthetase, wherein 3G is a pent-2-enoyl-CoA dehydrogenase.

In some aspects of the invention, the microbial organism can include two, three, four, five, six, seven, eight, nine or ten exogenous nucleic acids each encoding a 2,4-pentadienoate pathway enzyme. In some aspects of the invention, the microbial organism can include exogenous nucleic acids encoding each of the enzymes of at least one of the pathways selected from (1)-(24) as described above. In some aspects, the at least one exogenous nucleic acid is a heterologous nucleic acid. In some aspects, the non-naturally occurring microbial organism is in a substantially anaerobic culture medium.

In some embodiments, the invention provides a non-naturally occurring microbial organism as described herein, wherein the non-naturally occuring microbial organism having a 2,4-pentadienoate pathway selected from (9)-(15) as described above further includes a glutaryl-CoA pathway having at least one exogenous nucleic acid encoding a glutaryl-CoA pathway enzyme expressed in a sufficient amount to produce glutaryl-CoA, the glutaryl-CoA pathway having a pathway selected from: an acetoacetyl-CoA thiolase or an acetoacetyl-CoA synthase; an acetoacetyl-CoA reductase; a 3-hydroxybutyryl-CoA dehydratase; and a glutaryl-CoA dehydrogenase; or a 2-aminoadipate aminotransferase, a 2-aminoadipate dehydrogenase or a 2-aminoadipate amine oxidase; and a 2-oxoadipate dehydrogenase, a 2-oxoadipate:ferridoxin oxidoreductase or a 2-oxoadipate formate lyase.

In some embodiments, the invention provides a non-naturally occurring microbial organism as described herein, wherein the non-naturally occuring microbial organism having a 2,4-pentadienoate pathway selected from (16)-(22) as described above further includes a 5-aminopentanoate pathway having at least one exogenous nucleic acid encoding a 5-aminopcntanoatc pathway enzyme expressed in a sufficient amount to produce 5-aminopentanoate, the 5-aminopentanoate pathway having a 2-aminoadipate decarboxylase; or a 2-aminoadipate decarboxylase and a 2-aminoadipate aminotransferase, a 2-aminoadipate dehydrogenase or a 2-aminoadipate amine oxidase.

In some embodiments, the invention provides a non-naturally occurring microbial organism as described herein, wherein the non-naturally occuring microbial organism having a 2,4-pentadienoate pathway selected from (1)-(8) as described above further includes a 2-oxoadipate pathway having an exogenous nucleic acid encoding a 2-oxoadipate pathway enzyme expressed in a sufficient amount to produce a 2-oxoadipate, the 2-oxoadipate pathway having a 2-aminoadipate aminotransferase, a 2-aminoadipate dehydrogenase or a 2-aminoadipate amine oxidase.

In some embodiments, the invention provides a non-naturally occurring microbial organism having a 2,4-pentadienoate pathway having at least one exogenous nucleic acid encoding a 2,4-pentadienoate pathway enzyme expressed in a sufficient amount to produce 2,4-pentadienoate, wherein the 2,4-pentadienoate pathway includes a pathway as described above, further having: (i) a reductive TCA pathway having at least one exogenous nucleic acid encoding a reductive TCA pathway enzyme, wherein the at least one exogenous nucleic acid is selected from an ATP-citrate lyase, citrate lyase, a fumarate reductase, and an alpha-ketoglutarate:ferredoxin oxidoreductase; (ii) a reductive TCA pathway having at least one exogenous nucleic acid encoding a reductive TCA pathway enzyme, wherein the at least one exogenous nucleic acid is selected from a pyruvate:ferredoxin oxidoreductase, a phosphoenolpyruvate carboxylase, a phosphoenolpyruvate carboxykinase, a CO dehydrogenase, and an H2 hydrogenase; or (iii) at least one exogenous nucleic acid encodes an enzyme selected from a CO dehydrogenase, an H2 hydrogenase, and combinations thereof.

In some aspects, the microbial organism having (i) as described above further includes an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof. In some aspects, the microbial organism having (ii) further includes an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof.

In some aspects, the microbial organism having (i) as described above includes four exogenous nucleic acids encoding an ATP-citrate lyase, citrate lyase, a fumarate reductase, and an alpha-ketoglutarate:ferredoxin oxidoreductase; wherein the microbial organism having (ii) as described above includes five exogenous nucleic acids encoding a pyruvate:ferredoxin oxidoreductase, a phosphoenolpyruvate carboxylase, a phosphoenolpyruvate carboxykinase, a CO dehydrogenase, and an H2 hydrogenase; or wherein the microbial organism having (iii) as described above includes two exogenous nucleic acids encoding a CO dehydrogenase and an H2 hydrogenase.

In some embodiments, the invention provides a method for producing 2,4-pentadienoate, having culturing the non-naturally occurring microbial organism as described herein under conditions and for a sufficient period of time to produce 2,4-pentadienoate.

In some embodiments, the invention provides a non-naturally occurring microbial organism, having a microbial organism having a butadiene pathway having at least one exogenous nucleic acid encoding a butadiene pathway enzyme expressed in a sufficient amount to produce butadiene, wherein the butadiene pathway includes a pathway shown in Figures 1, 3, 5, 6 and/or 12 selected from: (1) 1D, 1I, 1B, 1C, 1K, 1G and 1T; (2) 1D, 1E, 1F, 1G and 1T; (3) 1D, IE, 1L, 1M, 1P, 1G and 1T; (4) 1D, 1I, 1B, 1J and 1T; (5) 1D, 1I, 1B, 1C, 1K, 1P, IN, 10 and 1T; (6) 1D, IE, 1F, 1P, IN, 10 and 1T; (7) 1D, IE, 1L, 1M, IN, 10 and 1T; (8) 1D, 1E, 1L, 1Q, 10 and 1T; (9) 1D, IE, 1F, 1U and 1V; (10) 1D, 1I, 1B, 1C, 1K, 1U and 1V; (11) 1D, IE, 1L, 1M, 1P, 1U and 1V; (12) 1D, 1E, 1W and 1V; (13) 1D, 1I, 1B, 1C, 1K, 1G, 6A and 6B; (14) 1D, IE, 1F, 1G, 6A and 6B; (15) 1D, 1E, 1L, 1M, 1P, 1G, 6A and 6B; (16) 1D, 1I, 1B, 1J, 6A and 6B; (17) 1D, 1I, 1B, 1C, 1K, 1P, 1N, 1O, 6A and 6B; (18) 1D, 1E, 1F, 1P, 1N, 1O, 6A and 6B; (19) 1D, 1E, 1L, 1M, 1N, 1O, 6A and 6B; (20) 1D, 1E, 1L, 1Q, 10, 6A and 6B; (21) 1D, 1I, 1B, 1C, 1K, 1G, 6H, 6E and 6B; (22) 1D, 1E, 1F, 1G, 6H, 6E and 6B; (23) 1D, 1E, 1L, 1M, 1P, 1G, 6H, 6E and 6B; (24) 1D, 11, 1B, 1J, 6H, 6E and 6B; (25) 1D, 1I, 1B, 1C, 1K, 1P, 1N, 1O, 6H, 6E and 6B; (26) 1D, IE, 1F, 1P, IN, 1O, 6H, 6E and 6B; (27) 1D, 1E, 1L, 1M, 1N, 1O, 6H, 6E and 6B; (28) 1D, IE, 1L, 1Q, 1O, 6H, 6E and 6B; (29) 1D, 1I, 1B, 1C, 1K, 1P, 1N, 6C and 6B; (30) 1D, 1E, 1F, 1P, 1N, 6C and 6B; (31) 1D, 1E, 1L, 1M, 1N, 6C and 6B; (32) 1D, 1E, 1L, 1Q, 6C and 6B; (33) 1D, 1I, 1B, 1C, 1K, 1P, IN, 6D, 6E and 6B; (34) 1D, 1E, 1F, 1P, IN, 6D, 6E and 6B; (35) 1D, IE, 1L, 1M, 1N, 6D, 6E and 6B; (36) 1D, IE, 1L, 1Q, 6D, 6E and 6B; (37) 1D, 1I, 1B, 1C, 1K, 1G, 6F, 6C and 6B; (38) 1D, 1E, 1F, 1G, 6F, 6C and 6B; (39) 1D, IE, 1L, 1M, 1P, 1G, 6F, 6C and 6B; (40) 1D, 1I, 1B, 1C, 1K, 1G, 6F, 6D, 6E and 6B; (41) 1D, IE, 1F, 1G, 6F, 6D, 6E and 6B; (42) 1D, IE, 1L, 1M, 1P, 1G, 6F, 6D, 6E and 6B; (43) 1S, 1I, 1B, 1C, 1K, 1G and 1T; (44) IS, IE, 1F, 1G and 1T; (45) IS, 1I, 1B, 1J and 1T; (46) 1S, 1I, 1B, 1C, 1K, 1P, IN, 10 and 1T; (47) IS, IE, 1F, 1P, 1N, 10 and 1T; (48) IS, IE, 1L, 1M, IN, 10 and 1T; (49) IS, IE, 1L, 1Q, 10 and 1T; (50) IS, IE, 1F, 1U and 1V; (51) IS, 1I, 1B, 1C, 1K, 1U and 1V; (52) IS, IE, 1L, 1M, 1P, 1U and 1V; (53) IS, IE, 1W and 1V; (54) IS, 1I, 1B, 1C, 1K, 1G, 6A and 6B; (55) 1S, 1E, 1F, 1G, 6A and 6B; (56) IS, 1I, 1B, 1J, 6A and 6B; (57) IS, 1I, 1B, 1C, 1K, 1P, IN, 10, 6A and 6B; (58) 1S, 1E, 1F, 1P, 1N, 10, 6A and 6B; (59) IS, IE, 1L, 1M, 1N, 10, 6A and 6B; (60) 1S, 1E, 1L, 1Q, 10, 6A and 6B; (61) 1S, 1I, 1B, 1C, 1K, 1G, 6H, 6E and 6B; (62) 1S, IE, 1F, 1G, 6H, 6E and 6B; (63) 1S, 1I, 1B, 1J, 6H, 6E and 6B; (64) 1S, 1I, 1B, 1C, 1K, 1P, IN, 1O, 6H, 6E and 6B; (65) 1S, 1E, 1F, 1P, 1N, 1O, 6H, 6E and 6B; (66) IS, IE, 1L, 1M, IN, 1O, 6H, 6E and 6B; (67) 1S, 1E, 1L, 1Q, 1O, 6H, 6E and 6B; (68) IS, 1I, 1B, 1C, 1K, 1P, 1N, 6C and 6B; (69) 1S, 1E, 1F, 1P, IN, 6C and 6B; (70) 1S, 1E, 1L, 1M, IN, 6C and 6B; (71) IS, IE, 1L, 1Q, 6C and 6B; (72) IS, 1I, 1B, 1C, 1K, 1P, IN, 6D, 6E and 6B; (73) 1S, IE, 1F, 1P, IN, 6D, 6E and 6B; (74) 1S, 1E, 1L, 1M, IN, 6D, 6E and 6B; (75) 1S, IE, 1L, 1Q, 6D, 6E and 6B; (76) 1S, 1I, 1B, 1C, 1K, 1G, 6F, 6C and 6B; (77) 1S, 1E, 1F, 1G, 6F, 6C and 6B; (78) 1S, 11, 1B, 1C, 1K, 1G, 6F, 6D, 6E and 6B; (79) 1S, 1E, 1F, 1G, 6F, 6D, 6E and 6B; (80) 1B, 1C, 1K, 1G and 1T; (81) 1I, 1E, 1F, 1G and 1T; (82) 1I, IE, 1L, 1M, 1P, 1G and 1T; (83) 1B, 1J and 1T; (84) 1I, IE, 1F, 1P, IN, 10 and 1T; (85) 1I, 1E, 1L, 1M, IN, 10 and 1T; (86) 1I, 1E, 1L, 1Q, 10 and 1T; (87) 1B, 1C, 1K, 1U and 1V; (88) 1I, IE, 1F, 1U and 1V; (89) 1I, 1E, 1L, 1M, 1P, 1U and 1V; (90) 1I, IE, 1W and 1V; (91) 1B, 1C, 1K, 1G, 6A and 6B; (92) 1I, IE, 1F, 1G, 6A and 6B; (93) 1I, IE, 1L, 1M, 1P, 1G, 6A and 6B; (94) 1B, 1J, 6A and 6B; (95) 1I, 1E, 1F, 1P, IN, 10, 6A and 6B; (96) 1I, IE, 1L, 1M, IN, 1O, 6A and 6B; (97) 1I, IE, 1L, 1Q, 1O, 6A and 6B; (98) 1B, 1C, 1K, 1G, 6H, 6E and 6B; (99) 1I, IE, 1F, 1G, 6H, 6E and 6B; (100) 1I, IE, 1L, 1M, 1P, 1G, 6H, 6E and 6B; (101) 1B, 1J, 6H, 6E and 6B; (102) 1I, IE, 1F, 1P, IN, 1O, 6H, 6E and 6B; (103) 1I, IE, 1L, 1M, IN, 1O, 6H, 6E and 6B; (104) 1I, IE, 1L, 1Q, 1O, 6H, 6E and 6B; (105) 1I, IE, 1F, 1P, IN, 6C and 6B; (106) 1I, IE, 1L, 1M, IN, 6C and 6B; (107) 1I, IE, 1L, 1Q, 6C and 6B; (108) 1I, IE, 1F, 1P, IN, 6D, 6E and 6B; (109) 1I, IE, 1L, 1M, IN, 6D, 6E and 6B; (110) 1I, IE, 1L, 1Q, 6D, 6E and 6B; (111) 1B, 1C, 1K, 1G, 6F, 6C and 6B; (112) 1I, IE, 1F, 1G, 6F, 6C and 6B; (113) 1I, IE, 1L, 1M, 1P, 1G, 6F, 6C and 6B; (114) 1B, 1C, 1K, 1G, 6F, 6D, 6E and 6B; (115) 1I, 1E, 1F, 1G, 6F, 6D, 6E and 6B; (116) 1I, 1E, 1L, 1M, 1P, 1G, 6F, 6D, 6E and 6B; (117) 3A, 3B, 3C, 3D, 3E, 3F and 1T; (118) 3A, 3B, 3C, 3D, 3E, 3F, 6A and 6B; (119) 3A, 3B, 3C, 3D, 3E, 3F, 6H, 6E and 6B; (120) 3A, 3B, 3C, 3D, 3E, 6C and 6B; (121) 3A, 3B, 3C, 3D, 3E, 6D, 6E and 6B; and (122) 3A, 3B, 3C, 3G, 3F and 1T; (123) 3A, 3B, 3C, 3G, 3F, 6A and 6B; (124) 3A, 3B, 3C, 3G, 3F, 6H, 6E and 6B; (125) 3A, 3B, 3C, 3G, 6C and 6B; (126) 3A, 3B, 3C, 3G, 6D, 6E and 6B; (127) 5A, 5B, 5C, 5D and 5E; (128) 7A, 7J, 7R, 7AD, 7AH, 12A, 12B and 12C; (129) 7A, 7H, 7F, 7R, 7AD, 7AH, 12A, 12B and 12C; (130) 7A, 7H, 7Q, 7Z, 7AD, 7AH, 12A, 12B and 12C; (131) 7A, 7H, 7Q, 7AC, 7AG, 7AH, 12A, 12B and 12C; (132) 7A, 7D, 7I, 7R, 7AD, 7AH, 12A, 12B and 12C; (133) 7A, 7D, 7E, 7F, 7R, 7AD, 7AH, 12A, 12B and 12C; (134) 7A, 7D, 7E, 7Q, 7Z, 7AD, 7AH, 12A, 12B and 12C; (135) 7A, 7D, 7E, 7Q, 7AC, 7AG, 7AH, 12A, 12B and 12C; (136) 7A, 7D, 7P, 7N, 7AD, 7AH, 12A, 12B and 12C; (137) 7A, 7D, 7P, 7Y, 7Z, 7AD, 7AH, 12A, 12B and 12C; (138) 7A, 7D, 7P, 7Y, 7AC, 7AG, 7AH, 12A, 12B and 12C; (139) 7A, 7D, 7P, 7AB, 7V, 7AH, 12A, 12B and 12C; (140) 7A, 7D, 7P, 7AB, 7AF, 7AG, 7AH, 12A, 12B and 12C; (141) 7A, 7B, 7M, 7AD, 7AH, 12A, 12B and 12C; (142) 7A, 7B, 7L, 7Z, 7AD, 7AH, 12A, 12B and 12C; (143) 7A, 7B, 7L, 7AC, 7AG, 7AH, 12A, 12B and 12C; (144) 7A, 7B, 7X, 7Y, 7Z, 7AD, 7AH, 12A, 12B and 12C; (145) 7A, 7B, 7X, 7Y, 7AC, 7AG, 7AH, 12A, 12B and 12C; (146) 7A, 7B, 7X, 7AB, 7V, 7AH, 12A, 12B and 12C; (147) 7A, 7B, 7X, 7AB, 7AF, 7AG, 7AH, 12A, 12B and 12C; (148) 7A, 7B, 7C, 7U, 7AH, 12A, 12B and 12C; (149) 7A, 7B, 7C, 7T, 7AG, 7AH, 12A, 12B and 12C; (150) 7A, 7B, 7C, 7AE, 7AF, 7AG, 7AH, 12A, 12B and 12C; (151) 7A, 7D, 7P, 7AB, 7W, 12A, 12B and 12C; (152) 7A, 7B, 7X, 7AB, 7W, 12A, 12B and 12C; (153) 7A, 7B, 7C, 7AE, 7W, 12A, 12B and 12C; (154) 7A, 7B, 7C, 7AE, 7V, 7AH;, 12A, 12B and 12C (155) 7A, 7J, 7R, 7AD, 7AH, 12D and 12C; (156) 7A, 7H, 7F, 7R, 7AD, 7AH, 12D and 12C; (157) 7A, 7H, 7Q, 7Z, 7AD, 7AH, 12D and 12C; (158) 7A, 7H, 7Q, 7AC, 7AG, 7AH, 12D and 12C; (159) 7A, 7D, 7I, 7R, 7AD, 7AH, 12D and 12C; (160) 7A, 7D, 7E, 7F, 7R, 7AD, 7AH, 12D and 12C; (161) 7A, 7D, 7E, 7Q, 7Z, 7AD, 7AH, 12D and 12C; (164) 7A, 7D, 7E, 7Q, 7AC, 7AG, 7AH, 12D and 12C; (163) 7A, 7D, 7P, 7N, 7AD, 7AH, 12D and 12C; (164) 7A, 7D, 7P, 7Y, 7Z, 7AD, 7AH, 12D and 12C; (165) 7A, 7D, 7P, 7Y, 7AC, 7AG, 7AH, 12D and 12C; (166) 7A, 7D, 7P, 7AB, 7V, 7AH, 12D and 12C; (167) 7A, 7D, 7P, 7AB, 7AF, 7AG, 7AH, 12D and 12C; (168) 7A, 7B, 7M, 7AD, 7AH, 12D and 12C; (169) 7A, 7B, 7L, 7Z, 7AD, 7AH, 12D and 12C; (170) 7A, 7B, 7L, 7AC, 7AG, 7AH, 12D and 12C; (171) 7A, 7B, 7X, 7Y, 7Z, 7AD, 7AH, 12D and 12C; (172) 7A, 7B, 7X, 7Y, 7AC, 7AG, 7AH, 12D and 12C; (173) 7A, 7B, 7X, 7AB, 7V, 7AH, 12D and 12C; (174) 7A, 7B, 7X, 7AB, 7AF, 7AG, 7AH, 12D and 12C; (175) 7A, 7B, 7C, 7U, 7AH, 12D and 12C; (176) 7A, 7B, 7C, 7T, 7AG, 7AH, 12D and 12C; (177) 7A, 7B, 7C, 7AE, 7AF, 7AG, 7AH, 12D and 12C; (178) 7A, 7D, 7P, 7AB, 7W, 12D and 12C; (179) 7A, 7B, 7X, 7AB, 7W, 12D and 12C; (180) 7A, 7B, 7C, 7AE, 7W, 12D and 12C; (181) 7A, 7B, 7C, 7AE, 7V, 7AH, 12D and 12C; (182) 7I, 7R, 7AD, 7AH, 12A, 12B and 12C; (183) 7E, 7F, 7R, 7AD, 7AH, 12A, 12B and 12C; (184) 7E, 7Q, 7Z, 7AD, 7AH, 12A, 12B and 12C; (185) 7E, 7Q, 7AC, 7AG, 7AH, 12A, 12B and 12C; (186) 7P, 7N, 7AD, 7AH, 12A, 12B and 12C; (187) 7P, 7Y, 7Z, 7AD, 7AH, 12A, 12B and 12C; (188) 7P, 7Y, 7AC, 7AG, 7AH, 12A, 12B and 12C; (189) 7P, 7AB, 7V, 7AH, 12A, 12B and 12C; (190) 7P, 7AB, 7AF, 7AG, 7AH, 12A, 12B and 12C; (191) 7P, 7AB, 7W, 12A, 12B and 12C; (192) 7I, 7R, 7AD, 7AH, 12D and 12C; (193) 7E, 7F, 7R, 7AD, 7AH, 12D and 12C; (194) 7E, 7Q, 7Z, 7AD, 7AH, 12D and 12C; (195) 7E, 7Q, 7AC, 7AG, 7AH, 12D and 12C; (196) 7P, 7N, 7AD, 7AH, 12D and 12C; (197) 7P, 7Y, 7Z, 7AD, 7AH, 12D and 12C; (198) 7P, 7Y, 7AC, 7AG, 7AH, 12D and 12C; (199) 7P, 7AB, 7V, 7AH, 12D and 12C; (200) 7P, 7AB, 7AF, 7AG, 7AH, 12D and 12C; (201) 7P, 7AB, 7W, 12D and 12C, (202) 7AS, 7I, 7R, 7AD, 7AH, 12A, 12B and 12C; (203) 7AS, 7E, 7F, 7R, 7AD, 7AH, 12A, 12B and 12C; (204) 7AS, 7E, 7Q, 7Z, 7AD, 7AH, 12A, 12B and 12C; (205) 7AS, 7E, 7Q, 7AC, 7AG, 7AH, 12A, 12B and 12C; (206) 7AS, 7P, 7N, 7AD, 7AH, 12A, 12B and 12C; (207) 7AS, 7P, 7Y, 7Z, 7AD, 7AH, 12A, 12B and 12C; (208) 7AS, 7P, 7Y, 7AC, 7AG, 7AH, 12A, 12B and 12C; (209) 7AS, 7P, 7AB, 7V, 7AH, 12A, 12B and 12C; (210) 7AS, 7P, 7AB, 7AF, 7AG, 7AH, 12A, 12B and 12C; (211) 7AS, 7P, 7AB, 7W, 12A, 12B and 12C; (212) 7AS, 7I, 7R, 7AD, 7AH, 12D and 12C; (213) 7AS, 7E, 7F, 7R, 7AD, 7AH, 12D and 12C; (214) 7AS, 7E, 7Q, 7Z, 7AD, 7AH, 12D and 12C; (215) 7AS, 7E, 7Q, 7AC, 7AG, 7AH, 12D and 12C; (216) 7AS, 7P, 7N, 7AD, 7AH, 12D and 12C; (217) 7AS, 7P, 7Y, 7Z, 7AD, 7AH, 12D and 12C; (218) 7AS, 7P, 7Y, 7AC, 7AG, 7AH, 12D and 12C; (219) 7AS, 7P, 7AB, 7V, 7AH, 12D and 12C; (220) 7AS, 7P, 7AB, 7AF, 7AG, 7AH, 12D and 12C; and (221) 7AS, 7P, 7AB, 7W, 12D and 12C, wherein 1B is a 5-aminopentanoate reductase, a 5-aminopent-2-enoate aminotransferase, a 5-aminopent-2-enoate dehydrogenase or 5-aminopent-2-enoate amine oxidase, wherein 1D is a 2-oxoadipate decarboxylase, wherein 1E is a glutarate semialdehyde reductase, wherein 1F is a 5-hydroxyvalerate reductase, wherein 1G is a 5-hydroxypent-2-enoate dehydratase, wherein 1I is a 5-aminopentanoate aminotransferase, a 5-aminopentanoate dehydrogenase or a 5-aminopentanoate amine oxidase, wherein 1J is a 5-aminopent-4-enoate deaminase, wherein 1K is a 5-hydroxypent-2-enoate reductase, wherein 1L is a 5-hydroxyvaleryl-CoA transferase or a 5-hydroxyvaleryl-CoA synthetase, wherein 1M is a 5-hydroxypentanoyl-CoA dehydrogenase, wherin IN is a 5-hydroxypent-2-enoyl-CoA dehydratase, wherein 10 is a 2,4-pentadienoyl-CoA transferase, a 2,4-pentadienoyl-CoA synthetase or a 2,4-pentadienoyl-CoA hydrolase, wherein 1P is a 5-hydroxypent-2-enoyl-CoA transferase or a 5-hydroxypent-2-enoyl-CoA synthetase, wherein in 1Q is a 5-hydroxyvaleryl-CoA dehydratase/dehydrogenase, wherein 1S is a glutaryl-CoA reductase, wherein 1T is a 2,4-pentadienoate decarboxylase, wherein 1U is a 5-hydroxypent-2-enoate decarboxylase, wherein 1V is a 3-buten-1-ol dehydratase, wherein 1W is a 5-hydroxyvalerate decarboxylase, wherein 3A is a 3-oxopentanoyl-CoA thiolase or a 3-oxopentanoyl-CoA synthase, wherein 3B is a 3-oxopentanoyl-CoA reductase, wherein 3C is a 3-hydroxypentanoyl-CoA dehydratase, wherein 3D is a pent-2-enoyl-CoA isomerase, wherein 3E is a pent-3-enoyl-CoA dehydrogenase, wherein 3F is a 2,4-pentadienoyl-CoA hydrolase, a 2,4-pentadienoyl-CoA transferase or a 2,4-pentadienoyl-CoA synthetase, wherein 3G is a pent-2-enoyl-CoA dehydrogenase, wherein 5A is a 4-hydroxy-2-oxovalerate aldolase, wherein 5B is a 4-hydroxy-2-oxovalerate dehydratase, wherein 5C is a 2-oxopentenoate decarboxylase, wherein 5D is a 3-buten-1-al reductase, wherein 5E is a 3-buten-1-ol dehydratase, wherein 6A is a 2,4-pentadienoate reductase (acid reducing), wherein 6B is a penta-2,4-dienal decarbonylase, wherein 6C is a 2,4-pentadienoyl-CoA reductase (acid reducing), wherein 6D is a 2,4-pentadienoyl-CoA phosphotransferase, wherein 6E is a 2,4-pentadienoyl-phosphate reductase, wherein 6F is a 2,4-pentadienoyl-CoA hydrolase, a 2,4-pentadienoyl-CoA transferase or a 2,4-pentadienoyl-CoA synthetase, wherein 6H is a 2,4-pentadienoate kinase, wherein 7A is a 3-ketoacyl-ACP synthase, wherein 7B is an acetoacetyl-ACP reductase, wherein 7C is a 3-hydroxybutyryl-ACP dehydratase, wherein 7D is an acetoacetyl-CoA:ACP transferase, wherein 7E is an acetoacetyl-CoA hydrolase, an acetoacetyl-CoA transferase or an acetoacetyl-CoA synthetase, wherein 7F is an acetoacetate reductase (acid reducing), wherein 7H is an acetoacetyl-ACP thioesterase, wherein 7I is an acetoacetyl-CoA reductase (CoA-dependent, aldehyde forming), wherein 7J is an acetoacetyl-ACP reductase (aldehyde forming), wherein 7K is an acetoacetyl-CoA reductase (alcohol forming), wherein 7L is an 3-hydroxybutyryl-ACP thioesterase, wherein 7M is an 3-hydroxybutyryl-ACP reductase (aldehyde forming), wherein 7N is an 3-hydroxybutyryl-CoA reductase (aldehyde forming), wherein 7O is an 3-hydroxybutyryl-CoA reductase (alcohol forming), wherein 7P is an acetoacetyl-CoA reductase (ketone reducing), wherein 7Q is an acetoacetate reductase (ketone reducing), wherein 7R is a 3-oxobutyraldehyde reductase (ketone reducing), wherein 7T is a crotonyl-ACP thioesterase, wherein 7U is a crotonyl-ACP reductase (aldehyde forming), wherein 7V is a crotonyl-CoA reductase (aldehyde forming), wherein 7W is a crotonyl-CoA (alcohol forming), wherein 7X is a 3-hydroxybutyryl-CoA:ACP transferase, wherein 7Y is a 3-hydroxybutyryl-CoA hydrolase, a 3-hydroxybutyryl-CoA transferase or a 3-hydroxybutyryl-CoA synthetase, wherein 7Z is a 3-hydroxybutyrate reductase, wherein 7AB is a 3-hydroxybutyryl-CoA dehydratase, wherein 7AC is a 3-hydroxybutyrate dehydratase, wherein 7AD is a 3-hydroxybutyraldehyde dehydratase, wherein 7AE is a crotonyl-CoA:ACP transferase, wherein 7AF is a crotonyl-CoA hydrolase, a crotonyl-CoA transferase or a crotonyl-CoA synthetase, wherein 7AG is a crotonate reductase, wherein 7AH is a crotonaldehyde reductase, wherein 7AS is an acetoacetyl-CoA synthase, wherein 12A is a crotyl alcohol kinase, wherein 12B is a 2-butenyl-4-phosphate kinase, wherein 12C is a butadiene synthase, and wherein 12D is a crotyl alcohol diphosphokinase.

In some aspects of the invention, the microbial organism can include two, three, four, five, six, seven, eight, nine, ten or eleven exogenous nucleic acids each encoding a butadiene pathway enzyme. In some aspects, the microbial organism includes exogenous nucleic acids encoding each of the enzymes of at least one of the pathways selected from (1)-(221). In some aspects, the at least one exogenous nucleic acid is a heterologous nucleic acid. In some aspects, the non-naturally occurring microbial organism is in a substantially anaerobic culture medium.

In some embodiments, the invention provides a non-naturally occurring microbial organism as described herein, wherein the non-naturally occuring microbial organism having a butadiene pathway selected from (43)-(79) as described above further includes a glutaryl-CoA pathway having at least one exogenous nucleic acid encoding a glutaryl-CoA pathway enzyme expressed in a sufficient amount to produce glutaryl-CoA, the glutaryl-CoA pathway having a pathway selected from: an acetoacetyl-CoA thiolase or acetoacetyl-CoA synthase; an acetoacetyl-CoA reductase; a 3-hydroxybutyryl-CoA dehydratase; and a glutaryl-CoA dehydrogenase; or a 2-aminoadipate aminotransferase, a 2-aminoadipate dehydrogenase or a 2-aminoadipate amine oxidase; and a 2-oxoadipate dehydrogenase, a 2-oxoadipate:ferridoxin oxidoreductase or a 2-oxoadipate formate lyase.

In some embodiments, the invention provides a non-naturally occurring microbial organism as described herein, wherein the non-naturally occuring microbial organism having a butadiene pathway selected from (80)-(116) as described above further includes a 5-aminopentanoate pathway having at least one exogenous nucleic acid encoding a 5-aminopentanoate pathway enzyme expressed in a sufficient amount to produce 5-aminopcntanoatc, the 5-aminopentanoate pathway having a 2-aminoadipate decarboxylase; or a 2-aminoadipate decarboxylase and a 2-aminoadipate aminotransferase, a 2-aminoadipate dehydrogenase or a 2-aminoadipate amine oxidase.

In some embodiments, the invention provides a non-naturally occurring microbial organism as described herein, wherein the non-naturally occuring microbial organism having a butadiene pathway selected from (1)-(42) as described above further includes a 2-oxoadipate pathway having an exogenous nucleic acid encoding a 2-oxoadipate pathway enzyme expressed in a sufficient amount to produce a 2-oxoadipate, the 2-oxoadipate pathway having a 2-aminoadipate aminotransferase, a 2-aminoadipate dehydrogenase or a 2-aminoadipate amine oxidase.

In some embodiments, the invention provides a non-naturally occurring microbial organism having a butadiene pathway having at least one exogenous nucleic acid encoding a butadiene pathway enzyme expressed in a sufficient amount to produce butadiene, wherein the butadiene pathway includes a pathway as described above, further having: (i) a reductive TCA pathway having at least one exogenous nucleic acid encoding a reductive TCA pathway enzyme, wherein the at least one exogenous nucleic acid is selected from an ATP-citrate lyase, citrate lyase, a fumarate reductase, and an alpha-ketoglutarate:ferredoxin oxidoreductase; (ii) a reductive TCA pathway having at least one exogenous nucleic acid encoding a reductive TCA pathway enzyme, wherein the at least one exogenous nucleic acid is selected from a pyruvate:ferredoxin oxidoreductase, a phosphoenolpyruvate carboxylase, a phosphoenolpyruvate carboxykinase, a CO dehydrogenase, and an H2 hydrogenase; or (iii) at least one exogenous nucleic acid encodes an enzyme selected from a CO dehydrogenase, an H2 hydrogenase, and combinations thereof.

In some aspects, the microbial organism having (i) as described above further includes an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof. In some aspect, the microbial organism having (ii) as described above further includes an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof. In some aspects, the microbial organism having (i) as described above includes four exogenous nucleic acids encoding an ATP-citrate lyase, citrate lyase, a fumarate reductase, and an alpha-ketoglutarate:ferredoxin oxidoreductase; wherein the microbial organism having (ii) as described above includes five exogenous nucleic acids encoding a pyruvate:ferredoxin oxidoreductase, a phosphoenolpyruvate carboxylase, a phosphoenolpyruvate carboxykinase, a CO dehydrogenase, and an H2 hydrogenase; or wherein the microbial organism having (iii) as described above includes two exogenous nucleic acids encoding a CO dehydrogenase and an H2 hydrogenase.

In some embodiments, the invention provides a method for producing butadiene, having culturing the non-naturally occurring microbial organism as described herein under conditions and for a sufficient period of time to produce butadiene.

In some embodiments, the invention provides a non-naturally occurring microbial organism, having a microbial organism having a 1,3-butanediol pathway having at least one exogenous nucleic acid encoding a 1,3-butanediol pathway enzyme expressed in a sufficient amount to produce 1,3-butanediol, wherein the 1,3-butanediol pathway includes a pathway shown in Figures 4, 5 and/or 7 selected from: (1) 4A, 4B, 4C and 4D; (2) 5A, 5H, 5J, 5K and 5G; (3) 5A, 5H, 5I and 5G; (4) 5A, 5H and 5L; (5) 5A, 5F and 5G; (6) 7A, 7D, 7E, 7F, 7G and 7S; (7) 7A, 7D, 7I, 7G and 7S; (8) 7A, 7D, 7K, and 7S; (9) 7A, 7H, 7F, 7G and 7S; (10) 7A, 7J, 7G and 7S; (11) 7A, 7J, 7R and 7AA; (12) 7A, 7H, 7F, 7R and 7AA; (13) 7A, 7H, 7Q, 7Z and 7AA; (14) 7A, 7D, 7I, 7R and 7AA; (15) 7A, 7D, 7E, 7F, 7R and 7AA; (16) 7A, 7D, 7E, 7Q, 7Z and 7AA; (17) 7A, 7D, 7P, 7N and 7AA; (18) 7A, 7D, 7P, 7Y, 7Z and 7AA; (19) 7A, 7B, 7M and 7AA; (20) 7A, 7B, 7L, 7Z and 7AA; (21) 7A, 7B, 7X, 7N and 7AA; (22) 7A, 7B, 7X, 7Y, 7Z and 7AA; (23) 7A, 7D, 7P and 7O; (24) 7A, 7B, 7X and 7O; (25) 7A, 7D, 7E, 7F, 7R, 7AA; (26) 7A, 7D, 7E, 7F, 7G, 7S, (27) 7AS, 7E, 7F, 7G and 7S; (28) 7AS, 7I, 7G and 7S; (29) 7AS, 7K, and 7S; (30) 7AS, 7I, 7R and 7AA; (31) 7AS, 7E, 7F, 7R and 7AA; (32) 7AS, 7E, 7Q, 7Z and 7AA; (33) 7AS, 7P, 7N and 7AA; (34) 7AS, 7P, 7Y, 7Z and 7AA; (35) 7AS, 7P and 7O; (36) 7AS, 7E, 7F, 7R, and 7AA; and (37) 7AS, 7E, 7F, 7G, and 7S, wherein 4A is a 3-oxo-5-hydroxypentanoyl-CoA thiolase or a 3-oxo-5-hydroxypentanoyl-CoA synthase, wherein 4B is a 3-oxo-5-hydroxypentanoyl-CoA hydrolase, 3-oxo-5-hydroxypentanoyl-CoA transferase or 3-oxo-5-hydroxypentanoyl-CoA synthetase, wherein 4C is a 3-oxo-5-hydroxypentanoate decarboxylase, wherein 4D is a 3-oxobutanol reductase, wherein in 5A is a 4-hydroxy-2-oxovalerate aldolase, wherein 5F is a 4-hydroxy-2-oxovalerate decarboxylase, wherin 5G is a 3-hydroxybutanal reductase, wherein 5H is a 4-hydroxy-2-oxopentanoate dehydrogenase, a 4-hydroxy-2-oxopentanoate:ferredoxin oxidoreductase or a 4-hydroxy-2-oxopentanoate formate lyase, wherein 5I is a 3-hydroxybutyryl-CoA reductase (aldehyde forming), wherein 5J is a 3-hydroxybutyryl-CoA hydrolase, a 3-hydroxybutyryl-CoA transferase or a 3-hydroxybutyryl-CoA synthetase, wherein 5K is a 3-hydroxybutyrate reductase, wherein 5L is a 3-hydroxybutyryl-CoA reductase (alcohol forming), wherein 7A is a 3-ketoacyl-ACP synthase, wherein 7B is an acetoacetyl-ACP reductase, wherein 7D is an acetoacetyl-CoA:ACP transferase, wherein 7E is an acetoacetyl-CoA hydrolase, acetoacetyl-CoA transferase or acetoacetyl-CoA synthetase, wherein 7F is an acetoacetate reductase (acid reducing), wherein 7G is a 3-oxobutyraldehyde reductase (aldehyde reducing), wherein 7H is an acetoacetyl-ACP thioesterase, wherein 7I is an acetoacetyl-CoA reductase (CoA-dependent, aldehyde forming), wherein 7J is an acetoacetyl-ACP reductase (aldehyde forming), wherein 7K is an acetoacetyl-CoA reductase (alcohol forming), wherein 7L is a 3-hydroxybutyryl-ACP thioesterase, wherein 7M is a 3-hydroxybutyryl-ACP reductase (aldehyde forming), wherein 7N is a 3-hydroxybutyryl-CoA reductase (aldehyde forming), wherein 7O is a 3-hydroxybutyryl-CoA reductase (alcohol forming), wherein 7P is an acetoacetyl-CoA reductase (ketone reducing), wherein 7Q is an acetoacetate reductase (ketone reducing), wherein 7R is a 3-oxobutyraldehyde reductase (ketone reducing), wherein 7S is a 4-hydroxy-2-butanone reductase, wherein 7X is a 3-hydroxybutyryl-CoA:ACP transferase, wherein 7Y is a 3-hydroxybutyryl-CoA hydrolase, a 3-hydroxybutyryl-CoA transferase or a 3-hydroxybutyryl-CoA synthetase, wherein 7Z is a 3-hydroxybutyrate reductase, wherein 7AA is a 3-hydroxybutyraldehyde reductase and wherein 7AS is an acetoacetyl-CoA synthase.

In some aspects, the microbial organism includes two, three, four or five exogenous nucleic acids each encoding a 1,3-butanediol pathway enzyme. In some aspects, the microbial organism includes exogenous nucleic acids encoding each of the enzymes of at least one of the pathways selected from (1)-(37) as described above. In some aspects, the at least one exogenous nucleic acid is a heterologous nucleic acid. In some aspects, the non-naturally occurring microbial organism is in a substantially anaerobic culture medium.

In some embodiments, the invention provides a non-naturally occurring microbial organism having a 1,3-butanediol pathway having at least one exogenous nucleic acid encoding a 1,3-butanediol pathway enzyme expressed in a sufficient amount to produce 1,3-butanediol, wherein the 1,3-butanediol pathway includes a pathway as described above, further having: (i) a reductive TCA pathway having at least one exogenous nucleic acid encoding a reductive TCA pathway enzyme, wherein the at least one exogenous nucleic acid is selected from an ATP-citrate lyase, citrate lyase, a fumarate reductase, and an alpha-ketoglutarate:ferredoxin oxidoreductase; (ii) a reductive TCA pathway having at least one exogenous nucleic acid encoding a reductive TCA pathway enzyme, wherein the at least one exogenous nucleic acid is selected from a pyruvate:ferredoxin oxidoreductase, a phosphoenolpyruvate carboxylase, a phosphoenolpyruvate carboxykinase, a CO dehydrogenase, and an H2 hydrogenase; or (iii) at least one exogenous nucleic acid encodes an enzyme selected from a CO dehydrogenase, an H2 hydrogenase, and combinations thereof.

In some aspects, the microbial organism having (i) as described above further includes an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof. In some aspects, the microbial organism having (ii) as described above further includes an exogenous nucleic acid encoding an enzyme selected from an aconitase, an isocitratc dehydrogenase, a succinyl-CoA synthetase, a succinyl-CoA transferase, a fumarase, a malate dehydrogenase, and combinations thereof. In some aspects, the microbial organism having (i) as described herein includes four exogenous nucleic acids encoding an ATP-citratc lyase, citrate lyase, a fumarate reductase, and an alpha-ketoglutarate:ferredoxin oxidoreductase; wherein the microbial organism having (ii) includes five exogenous nucleic acids encoding a pyruvate:ferredoxin oxidoreductase, a phosphoenolpyruvate carboxylase, a phosphoenolpyruvate carboxykinase, a CO dehydrogenase, and an H2 hydrogenase; or wherein the microbial organism having (iii) includes two exogenous nucleic acids encoding a CO dehydrogenase and an H2 hydrogenase.

In some embodiments, the invention provides a method for producing 1,3-butanediol, having culturing the non-naturally occurring microbial organism as described herein under conditions and for a sufficient period of time to produce 1,3-butanediol.

In some embodiments, the invention provides a non-naturally occurring microbial organism, having a microbial organism having a 3-buten-1-ol pathway having at least one exogenous nucleic acid encoding a 3-buten-1-ol pathway enzyme expressed in a sufficient amount to produce 3-buten-1-ol, wherein the 3-buten-1-ol pathway includes a pathway shown in Figures 1 and/or 5 selected from: (1) 1D, IE, 1F and 1U; (2) 1D, 1I, 1B, 1C, 1K and 1U; (3) 1D, IE, 1L, 1M, 1P and 1U; (4) 1D, 1E and 1W; (5) IS, IE, 1F and 1U; (6) IS, 1I, 1B, 1C, 1K and 1U; (7) IS, IE, 1L, 1M, 1P and 1U; (8) IS, 1E and 1W; (9) 1B, 1C, 1K and 1U; (10) 1I, IE, 1F and 1U; (11) 1I, IE, 1L, 1M, 1P and 1U; (12) 1I, 1E and 1W; and (13) 5A, 5B, 5C and 5D, wherein 1B is a 5-aminopentanoate reductase, wherein 1C is a 5-aminopent-2-enoate aminotransferase, a 5-aminopent-2-enoate dehydrogenase or an amine oxidase, wherein 1D is a 2-oxoadipate decarboxylase, wherein 1E is a glutarate semialdehyde reductase, wherein 1F is a 5-hydroxyvalerate dehydrogenase, wherein 1I is a 5-aminopentanoate aminotransferase, a 5-aminopentanoate dehydrogenase or a 5-aminopentanoate amine oxidase, wherein 1K is a 5-hydroxypent-2-enoate reductase, wherein 1L is a 5-hydroxyvaleryl-CoA transferase or a 5-hydroxyvaleryl-CoA synthetase, wherein 1M is a 5-hydroxypentanoyl-CoA dehydrogenase, wherein 1P is a 5-hydroxypent-2-enoyl-CoA transferase or a 5-hydroxypent-2-enoyl-CoA synthetase, wherein 1S is a glutaryl-CoA reductase, wherein 1U is a 5-hydroxypent-2-enoate decarboxylase, wherein 1W is a 5-hydroxyvalerate decarboxylase, wherein 5A is a 4-hydroxy-2-oxovalerate aldolase, wherein 5B is a 4-hydroxy-2-oxovalerate dehydratase, wherein 5C is a 2-oxopentenoate decarboxylase, wherein 5D is a 3-buten-1-al reductase.

In some aspects, the microbial organism includes two, three, four, five or six exogenous nucleic acids each encoding a 3-buten-1-ol pathway enzyme. In some aspects, the microbial organism includes exogenous nucleic acids encoding each of the enzymes of at least one of the pathways selected from (1)-(13) as described above. In some aspects, the at least one exogenous nucleic acid is a heterologous nucleic acid. In some aspects, the non-naturally occurring microbial organism is in a substantially anaerobic culture medium.

In some embodiments, the invention provides a non-naturally occurring microbial organism having a 3-buten-1-ol pathway having at least one exogenous nucleic acid encoding a 3-buten-1-ol pathway enzyme expressed in a sufficient amount to produce 3-buten-1-ol, wherein the 3-buten-1-ol pathway includes a pathway as described above, further having: (i) a reductive TCA pathway having at least one exogenous nucleic acid encoding a reductive TCA pathway enzyme, wherein the at least one exogenous nucleic acid is selected from an ATP-citrate lyase, citrate lyase, a fumarate reductase, and an alpha-ketoglutarate:ferredoxin oxidoreductase; (ii) a reductive TCA pathway having at least one exogenous nucleic acid encoding a reductive TCA pathway enzyme, wherein the at least one exogenous nucleic acid is selected from a pyruvate:ferredoxin oxidoreductase, a phosphoenolpyruvate carboxylase, a phosphoenolpyruvate carboxykinase, a CO dehydrogenase, and an H2 hydrogenase; or (iii) at least one exogenous nucleic acid encodes an enzyme selected from a CO dehydrogenase, an H2 hydrogenase, and combinations thereof.

In some aspects, the microbial organism having (i) as described above further includes an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof. In some aspects, the microbial organism having (ii) as described above further includes an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof. In some aspects the microbial organism having (i) as described above includes four exogenous nucleic acids encoding an ATP-citrate lyase, citrate lyase, a fumarate reductase, and an alpha-ketoglutarate:ferredoxin oxidoreductase; wherein the microbial organism having (ii) as described above includes five exogenous nucleic acids encoding a pyruvate:ferredoxin oxidoreductase, a phosphoenolpyruvate carboxylase, a phosphoenolpyruvate carboxykinase, a CO dehydrogenase, and an H2 hydrogenase; or wherein the microbial organism having (iii) as described above includes two exogenous nucleic acids encoding a CO dehydrogenase and an H2 hydrogenase.

In some embodiments, the invention provides a method for producing 3-buten-1-ol, having culturing the non-naturally occurring microbial organism as described above under conditions and for a sufficient period of time to produce 3-buten-1-ol.

In some embodiments, the invention provides a method for producing butadiene, having culturing the non-naturally occurring microbial organism as described above under conditions and for a sufficient to produce 3-buten-1-ol, and chemically dehydrating the 3-buten-1-ol to produce butadiene.

In some embodiments, the invention provides a non-naturally occurring microbial organism, including a microbial organism having a crotyl alcohol pathway including at least one exogenous nucleic acid encoding a crotyl alcohol pathway enzyme expressed in a sufficient amount to produce crotyl alcohol, wherein the crotyl alcohol pathway includes a pathway shown in Figure 7 selected from: (1) 7A, 7J, 7R, 7AD and 7AH; (2) 7A, 7H, 7F, 7R, 7AD and 7AH; (3) 7A, 7H, 7Q, 7Z, 7AD and 7AH; (4) 7A, 7H, 7Q, 7AC, 7AG and 7AH; (5) 7A, 7D, 7I, 7R, 7AD and 7AH; (6) 7A, 7D, 7E, 7F, 7R, 7AD and 7AH; (7) 7A, 7D, 7E, 7Q, 7Z, 7AD and 7AH; (8) 7A, 7D, 7E, 7Q, 7AC, 7AG and 7AH; (9) 7A, 7D, 7P, 7N, 7AD and 7AH; (10) 7A, 7D, 7P, 7Y, 7Z, 7AD and 7AH; (11) 7A, 7D, 7P, 7Y, 7AC, 7AG and 7AH; (12) 7A, 7D, 7P, 7AB, 7V and 7AH; (13) 7A, 7D, 7P, 7AB, 7AF, 7AG AND 7AH (14) 7A, 7B, 7M, 7AD and 7AH; (15) 7A, 7B, 7L, 7Z, 7AD and 7AH; (16) 7A, 7B, 7L, 7AC, 7AG and 7AH; (17) 7A, 7B, 7X, 7Y, 7Z, 7AD and 7AH; (18) 7A, 7B, 7X, 7Y, 7AC, 7AG and 7AH; (19) 7A, 7B, 7X, 7AB, 7V and 7AH; (20) 7A, 7B, 7X, 7AB, 7AF, 7AG and 7AH; (21) 7A, 7B, 7C, 7U and 7AH; (22) 7A, 7B, 7C, 7T, 7AG and 7AH; (23) 7A, 7B, 7C, 7AE, 7AF, 7AG and 7AH; (24) 7A, 7D, 7P, 7AB and 7W; (25) 7A, 7B, 7X, 7AB and 7W; (26) 7A, 7B, 7C, 7AE and 7W; (27) 7A, 7B, 7C, 7AE, 7V and 7AH; (28) 7I, 7R, 7AD and 7AH; (29) 7E, 7F, 7R, 7AD and 7AH; (30) 7E, 7Q, 7Z, 7AD and 7AH; (31) 7E, 7Q, 7AC, 7AG and 7AH; (32) 7P, 7N, 7AD and 7AH; (33) 7P, 7Y, 7Z, 7AD and 7AH; (34) 7P, 7Y, 7AC, 7AG and 7AH; (35) 7P, 7AB, 7V and 7AH; (36) 7P, 7AB, 7AF, 7AG and 7AH; (37) 7P, 7AB and 7W, (38) 7AS, 71, 7R, 7AD and 7AH; (39) 7AS, 7E, 7F, 7R, 7AD and 7AH; (40) 7AS, 7E, 7Q, 7Z, 7AD and 7AH; (41) 7AS, 7E, 7Q, 7AC, 7AG and 7AH; (42) 7AS, 7P, 7N, 7AD and 7AH; (43) 7AS, 7P, 7Y, 7Z, 7AD and 7AH; (44) 7AS, 7P, 7Y, 7AC, 7AG and 7AH; (45) 7AS, 7P, 7AB, 7V and 7AH; (46) 7AS, 7P, 7AB, 7AF, 7AG and 7AH; and (47) 7AS, 7P, 7AB and 7W, wherein 7A is a 3-ketoacyl-ACP synthase, wherein 7B is an acetoacetyl-ACP reductase, wherein 7C is a 3-hydroxybutyryl-ACP dehydratase, wherein 7D is an acetoacetyl-CoA:ACP transferase, wherein 7E is an acetoacetyl-CoA hydrolase, an acetoacetyl-CoA transferase or an acetoacetyl-CoA synthetase, wherein 7F is an acetoacetate reductase (acid reducing), wherein 7H is an acetoacetyl-ACP thioesterase, wherein 7I is an acetoacetyl-CoA reductase (CoA-dependent, aldehyde forming), wherein 7J is an acetoacetyl-ACP reductase (aldehyde forming), wherein 7K is an acetoacetyl-CoA reductase (alcohol forming), wherein 7L is an 3-hydroxybutyryl-ACP thioesterase, wherein 7M is an 3-hydroxybutyryl-ACP reductase (aldehyde forming), wherein 7N is an 3-hydroxybutyryl-CoA reductase (aldehyde forming), wherein 7O is an 3-hydroxybutyryl-CoA reductase (alcohol forming), wherein 7P is an acetoacetyl-CoA reductase (ketone reducing), wherein 7Q is an acetoacetate reductase (ketone reducing), wherein 7R is a 3-oxobutyraldehyde reductase (ketone reducing), wherein 7T is a crotonyl-ACP thioesterase, wherein 7U is a crotonyl-ACP reductase (aldehyde forming), wherein 7V is a crotonyl-CoA reductase (aldehyde forming), wherein 7W is a crotonyl-CoA (alcohol forming), wherein 7X is a 3-hydroxybutyryl-CoA:ACP transferase, wherein 7Y is a 3-hydroxybutyryl-CoA hydrolase, a 3-hydroxybutyryl-CoA transferase or a 3-hydroxybutyryl-CoA synthetase, wherein 7Z is a 3-hydroxybutyrate reductase, wherein 7AB is a 3-hydroxybutyryl-CoA dehydratase, wherein 7AC is a 3-hydroxybutyrate dehydratase, wherein 7AD is a 3-hydroxybutyraldehyde dehydratase, wherein 7AE is a crotonyl-CoA:ACP transferase, wherein 7AF is a crotonyl-CoA hydrolase, a crotonyl-CoA transferase or a crotonyl-CoA synthetase, wherein 7AG is a crotonate reductase, wherein 7AH is a crotonaldehyde reductase and wherein 7AS is an acetoacetyl-CoA synthase.

In some aspects, the invention provides that the microbial organism having a crotyl alcohol pathway as described above, wherein the microbial organism includes two, three, four, five, six or seven exogenous nucleic acids each encoding a crotyl alcohol pathway enzyme. In some aspects, the microbial organism includes exogenous nucleic acids encoding each of the enzymes of at least one of the crotyl alcohol pathways selected from (1)-(47) as described above. In some aspects, the at least one exogenous nucleic acid is a heterologous nucleic acid. In som aspects, the non-naturally occurring microbial organism is in a substantially anaerobic culture medium.

In some embodiments, the invention provides a non-naturally occurring microbial organism having a crotyl alcohol pathway having at least one exogenous nucleic acid encoding a crotyl alcohol pathway enzyme expressed in a sufficient amount to produce crotyl alcohol, wherein the crotyl alcohol pathway includes a pathway as described above, and further includes: (i) a reductive TCA pathway including at least one exogenous nucleic acid encoding a reductive TCA pathway enzyme, wherein the at least one exogenous nucleic acid is selected from an ATP-citrate lyase, citrate lyase, a fumarate reductase, and an alpha-ketoglutarate:ferredoxin oxidoreductase; (ii) a reductive TCA pathway including at least one exogenous nucleic acid encoding a reductive TCA pathway enzyme, wherein the at least one exogenous nucleic acid is selected from a pyruvate:ferredoxin oxidoreductase, a phosphoenolpyruvate carboxylase, a phosphoenolpyruvate carboxykinase, a CO dehydrogenase, and an H2 hydrogenase; or (iii) at least one exogenous nucleic acid encodes an enzyme selected from a CO dehydrogenase, an H2 hydrogenase, and combinations thereof.

In some aspects, the microbial organism including (i) as described above further includes an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof. In some aspects, the microbial organism including (ii) as described above further includes an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof. In some aspects, the microbial organism including (i) as described above includes four exogenous nucleic acids encoding an ATP-citrate lyase, citrate lyase, a fumarate reductase, and an alpha-ketoglutarate:ferredoxin oxidoreductase; wherein the microbial organism including (ii) includes five exogenous nucleic acids encoding a pyruvate:ferredoxin oxidoreductase, a phosphoenolpyruvate carboxylase, a phosphoenolpyruvate carboxykinase, a CO dehydrogenase, and an H2 hydrogenase; or wherein the microbial organism including (iii) includes two exogenous nucleic acids encoding a CO dehydrogenase and an H2 hydrogenase.

In some embodiments, the invention provides a method for producing crotyl alcohol, including culturing the non-naturally occurring microbial organism as described above under conditions and for a sufficient period of time to produce crotyl alcohol.

In some embodiments, access to butadiene can be accomplished by biosynthetic production of crotyl alcohol and subsequent chemical dehydration to butadiene. In some embodiments, the invention provides a process for the production of butadiene that includes (a) culturing by fermentation in a sufficient amount of nutrients and media a non-naturally occurring microbial organism as described herein that produces crotyl alcohol; and (b) converting crotyl alcohol produced by culturing the non-naturally occurring microbial organism to butadiene.

The dehydration of alcohols are known in the art and can include various thermal processes, both catalyzed and non-catalyzed. In some embodiments, a catalyzed thermal dehydration employs a metal oxide catalyst or silica. In some embodiments, step (b) of the process is performed by chemical dehydration in the presence of a catalyst. For example, it has been indicated that crotyl alcohol can be dehydrated over bismuth molybdatc (Adams, C.R. J. Catal. 10:355-361, 1968) to afford 1,3-butadiene.

Dehydration can be achieved via activation of the alcohol group and subsequent elimination by standard elimination mechanisms such as E1 or E2 elimination. Activation can be achieved by way of conversion of the alcohol group to a halogen such as iodide, chloride, or bromide. Activation can also be accomplished by way of a sulfonyl, phosphate or other activating functionality that convert the alcohol into a good leaving group. In some embodiments, the activating group is a sulfate or sulfate ester selected from a tosylate, a mesylate, a nosylate, a brosylate, and a triflate. In some embodiments, the leaving group is a phosphate or phosphate ester. In some such embodiments, the dehydrating agent is phosphorus pentoxide.

In some embodiments, the invention provides a non-naturally occurring microbial organism, including a microbial organism having a propylene pathway including at least one exogenous nucleic acid encoding a propylene pathway enzyme expressed in a sufficient amount to produce propylene, wherein the propylene pathway includes a pathway shown in Figure 7 selected from: (1) 7A, 7J, 7R, 7AD and 7AO; (2) 7A, 7H, 7F, 7R, 7AD and 7AO; (3) 7A, 7D, 7I, 7R, 7AD and 7AO; (4) 7A, 7D, 7E, 7F, 7R, 7AD and 7AO; (5) 7A, 7H, 7Q, 7Z, 7AD and 7AO; (6) 7A, 7D, 7E, 7Q, 7AD and 7AO; (7) 7A, 7D, 7P, 7Y, 7Z, 7AD and 7AO; (8) 7A, 7D, 7P, 7N, 7AD and 7AO; (9) 7A, 7B, 7X, 7N, 7AD and 7AO; (10) 7A, 7B, 7X, 7Y, 7Z, 7AD and 7AO; (11) 7A, 7H, 7Q, 7V, 7AG and 7AO; (12) 7A, 7D, 7E, 7Q, 7AC, 7AG and 7AO; (13) 7A, 7D, 7P, 7Y, 7AC, 7AG and 7AO; (14) 7A, 7D, 7P, 7AB, 7AF, 7AG and AO; (15) 7A, 7P, 7AB, 7V and 7AO; (16) 7A, 7B, 7M, 7AD and 7AO; (17) 7A, 7B, 7L, 7Z, 7AD and 7AO; (18) 7A, 7B, 7X, 7N, 7AD and 7AO; (19) 7A, 7B, 7X, 7Y, 7Z, 7AD and 7AO; (20) 7A, 7B, 7C, 7U and 7AO; (21) 7A, 7B, 7C, 7T, 7AG and 7AO; (22) 7A, 7B, 7C, 7AE, 7V and 7AO; (23) 7A, 7B, 7C, 7AE, 7AF, 7AG and 7AO; (24) 7A, 7H, 7Q and 7AR; (25) 7A, 7D, 7E, 7Q and 7AR; (26) 7A, 7D, 7P, 7Y and 7AR; (27) 7A, 7B, 7X, 7Y and 7AR; (28) 7A, 7B, 7L and 7AR; (29) 7A, 7H, 7Q, 7AC and 7AQ; (30) 7A, 7D, 7E, 7Q, 7AC and 7AQ;(31) 7A, 7D, 7P, 7Y, 7AC and 7AQ; (32) 7A, 7D, 7P, 7AB, 7AF and 7AQ; (33) 7A, 7B, 7L, 7AC and 7AQ; (34) 7A, 7B, 7X, 7Y, 7AC and 7AQ; (35) 7A, 7B, 7X, 7AB, 7AF and 7AQ; (36) 7A, 7B, 7C, 7AE, 7AF and 7AQ; (37) 7A, 7B, 7C, 7T and 7AQ; (38) 7A, 7H, 7Q, 7AC, 7AN and 7AK; (39) 7A, 7D, 7E, 7Q, 7AC, 7AN and 7AK; (40) 7A, 7D, 7P, 7Y, 7AC, 7AN and 7AK; (41) 7A, 7D, 7P, 7AB, 7AF, 7AN and 7AK; (42) 7A, 7D, 7P, 7AB, 7AM, 7AJ and 7AK; (43) 7A, 7B, 7L, 7AC, 7AN and 7AK; (44) 7A, 7B, 7X, 7Y, 7AC, 7AN and 7AK; (45) 7A, 7B, 7X, 7AB, 7AF, 7AN and 7AK; (46) 7A, 7B, 7X, 7AB, 7AM, 7AJ and 7AK; (47) 7A, 7B, 7C, 7T, 7AN and 7AK; (48) 7A, 7B, 7C, 7AE, 7AF, 7AN and 7AK; (49) 7A, 7B, 7C, 7AE, 7AM, 7AJ and 7AK; (50) 7A, 7B, 7C, 7AL, 7AP and 7AK; (51) 7A, 7B, 7C, 7AL, 7AI, 7AJ and 7AK; (52) 7A, 7B, 7X, 7AB, 7V and 7AO; (53) 7A 7B, 7L, 7AC, 7AG and 7AO; (54) 7A, 7B, 7X, 7Y, 7AC, 7AC, 7AG and 7AO; (55) 7A, 7B, 7X, 7AB, 7AF, 7AG and 7AO; and (56) 7A, 7H, 7Q, 7AC, 7AG and 7AO; (57) 7I, 7R, 7AD and 7AO; (58) 7E, 7F, 7R, 7AD and 7AO; (59) 7E, 7Q, 7Z, 7AD and 7AO; (60) 7P, 7Y, 7Z, 7AD and 7AO; (61) 7P, 7N, 7AD and 7AO; (62) 7E, 7Q, 7AC, 7AG and 7AO; (63) 7P, 7Y, 7AC, 7AG and 7AO; (64) 7P, 7AB, 7AF, 7AG and 7AO; (65) 7P, 7AB, 7V and 7AO; (66) 7E, 7Q and 7AR; (67) 7P, 7Y and 7AR; (68) 7E, 7Q, 7AC and 7AQ; (69) 7P, 7Y, 7AC and 7AQ; (70) 7P, 7AB, 7AF and 7AQ; (71) 7E, 7Q, 7AC, 7AN and 7AK; (72) 7P, 7Y, 7AC, 7AN and 7AK; (73) 7P, 7AB, 7AF, 7AN and 7AK; (74) 7P, 7AB, 7AM, 7AJ and 7AK, (75) 7AS, 7I, 7R, 7AD and 7AO; (76) 7AS, 7E, 7F, 7R, 7AD and 7AO; (77) 7AS, 7E, 7Q, 7AD and 7AO; (78) 7AS, 7P, 7Y, 7Z, 7AD and 7AO; (79) 7AS, 7P, 7N, 7AD and 7AO; (80) 7AS, 7E, 7Q, 7AC, 7AG and 7AO; (81) 7AS, 7P, 7Y, 7AC, 7AG and 7AO; (82) 7AS, 7P, 7AB, 7AF, 7AG and 7AO; (83) 7AS, 7E, 7Q and 7AR; (84) 7AS, 7P, 7Y and 7AR; (85) 7AS, 7E, 7Q, 7AC and 7AQ; (86) 7AS, 7P, 7Y, 7AC and 7AQ; (87) 7AS, 7P, 7AB, 7AF and 7AQ; (88) 7AS, 7E, 7Q, 7AC, 7AN and 7AK; (89) 7AS, 7P, 7Y, 7AC, 7AN and 7AK;(90) 7AS, 7P, 7AB, 7AF, 7AN and 7AK; and (91) 7AS, 7P, 7AB, 7AM, 7AJ and 7AK, wherein 7A is a 3-ketoacyl-ACP synthase, wherein 7B is an acetoacetyl-ACP reductase, wherein 7C is a 3-hydroxybutyryl-ACP dehydratase, wherein 7D is an acetoacetyl-CoA:ACP transferase, wherein 7E is an acetoacetyl-CoA hydrolase, an acetoacetyl-CoA transferase or an acetoacetyl-CoA synthetase, wherein 7F is an acetoacetate reductase (acid reducing), wherein 7H is an acetoacetyl-ACP thioesterase, wherein 7I is an acetoacetyl-CoA reductase (CoA-dependent, aldehyde forming), wherein 7J is an acetoacetyl-ACP reductase (aldehyde forming), wherein 7L is a 3-hydroxybutyryl-ACP thioesterase, wherein 7M is a 3-hydroxybutyryl-ACP reductase (aldehyde forming), wherein 7N is a 3-hydroxybutyryl-CoA reductase (aldehyde forming), wherein 7P is an acetoacetyl-CoA reductase (ketone reducing), wherein 7Q is an acetoacetate reductase (ketone reducing), wherein 7R is a 3-oxobutyraldehyde reductase (ketone reducing), wherein 7S is a 4-hydroxy-2-butanone reductase, wherein 7T is a crotonyl-ACP thioesterase, wherein 7U is a crotonyl-ACP reductase (aldehyde forming), wherein 7V is a crotonyl-CoA reductase (aldehyde forming), wherein 7X is a 3-hydroxybutyryl-CoA:ACP transferase, wherein 7Y is a 3-hydroxybutyryl-CoA hydrolase, a 3-hydroxybutyryl-CoA transferase or a 3-hydroxybutyryl-CoA synthetase, wherein 7Z is a 3-hydroxybutyrate reductase, wherein 7AB is a 3-hydroxybutyryl-CoA dehydratase, wherein 7AC is a 3-hydroxybutyrate dehydratase, wherein 7AD is a 3-hydroxybutyraldehyde dehydratase, wherein 7AE is a crotonyl-CoA:ACP transferase, wherein 7AF is a crotonyl-CoA hydrolase, a crotonyl-CoA transferase or a crotonyl-CoA synthetase, wherein 7AG is a crotonate reductase, wherein 7AI is a butryl-CoA:ACP transferase, wherein 7AJ is a butyryl-CoA transferase, a butyryl-CoA hydrolase or a butyryl-CoA synthetase, wherein 7AK is a butyrate decarboxylase, wherein 7AL is a crotonyl-ACP reductase, wherein 7AM is a crotonyl-CoA reductase, wherein 7AN is a crotonate reductase, wherein 7AO is a crotonaldehyde decarbonylase, wherein 7AP is a butyryl-ACP thioesterase, wherein 7AQ is a crotonate decarboxylase, wherein 7AR is a 3-hydroxybutyrate decarboxylase and wherein 7AS is an acetoacetyl-CoA synthase.

In some aspects, the invention provides that the microbial organism having a propylene pathway as described above, wherein the microbial organism includes two, three, four, five, six, seven or eight exogenous nucleic acids each encoding a propylene pathway enzyme. In some aspects, the microbial organism includes exogenous nucleic acids encoding each of the enzymes of at least one of the pathways selected from (1)-(91) as described above. In some aspects, the at least one exogenous nucleic acid is a heterologous nucleic acid. In some aspects, the non-naturally occurring microbial organism is in a substantially anaerobic culture medium.

In some embodiments, the invention provides a non-naturally occurring microbial organism having a propylene pathway having at least one exogenous nucleic acid encoding a propylene pathway enzyme expressed in a sufficient amount to produce propylene, wherein the propylene pathway includes a pathway as described above, and further includes: (i) a reductive TCA pathway including at least one exogenous nucleic acid encoding a reductive TCA pathway enzyme, wherein the at least one exogenous nucleic acid is selected from an ATP-citrate lyase, citrate lyase, a fumarate reductase, and an alpha-ketoglutarate:ferredoxin oxidoreductase; (ii) a reductive TCA pathway including at least one exogenous nucleic acid encoding a reductive TCA pathway enzyme, wherein the at least one exogenous nucleic acid is selected from a pyruvate:ferredoxin oxidoreductase, a phosphoenolpyruvate carboxylase, a phosphoenolpyruvate carboxykinase, a CO dehydrogenase, and an H2 hydrogenase; or (iii) at least one exogenous nucleic acid encodes an enzyme selected from a CO dehydrogenase, an H2 hydrogenase, and combinations thereof.

In some aspects, the microbial organism including (i) as described above further includes an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof. In some aspects, the microbial organism including (ii) as described above further includes an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof. In some aspects, the microbial organism including (i) as described above includes four exogenous nucleic acids encoding an ATP-citrate lyase, citrate lyase, a fumarate reductase, and an alpha-ketoglutarate:ferredoxin oxidoreductase; wherein the microbial organism including (ii) as described above includes five exogenous nucleic acids encoding a pyruvate:ferredoxin oxidoreductase, a phosphoenolpyruvate carboxylase, a phosphoenolpyruvate carboxykinase, a CO dehydrogenase, and an H2 hydrogenase; or wherein the microbial organism including (iii) as described above includes two exogenous nucleic acids encoding a CO dehydrogenase and an H2 hydrogenase.

In some embodiments, the invention provides a method for producing propylene, including culturing the non-naturally occurring microbial organism of as described above under conditions and for a sufficient period of time to produce propylene.

In an additional embodiment, the invention provides a non-naturally occurring microbial organism having a 2,4-pentadienoate, butadiene, propylene, 1,3-butanediol, crotyl alcohol or 3-buten-1-ol pathway, wherein the non-naturally occurring microbial organism includes at least one exogenous nucleic acid encoding an enzyme or protein that converts a substrate to a product selected from the group consisting of 2-aminoadipate to 5-aminopentanoate, 2-aminoadipate to 2-oxoadipate, 5-aminopentanoate to glutarate semialdehyde, 2-oxoadipate to glutarate semialdehyde, 2-oxoadipate to glutaryl-CoA, glutaryl-CoA to glutarate semialdehyde, glutarate semialdehyde to 5-hydroxyvalerate, 5-aminopentanoate to 5-aminopent-2enoate, 5-aminopent-2enoate to 5-hydroxypent-2-enoate, 5-hydroxypent-2-enoate to 5-hydroxypent-2-enoate, 5-hydroxyvalerate to 5-hydroxypent-2-enoate, 5-hydroxyvalerate to 5-hydroxyvaleryl-CoA, 5-hydroxyvalerate to 3-buten-1-ol, 5-aminopent-2-enoate to 2,4-pentadienoate, 5-hydroxypent-2-enoate to 3-buten-1-ol, 5-hydroxypent-2-enoate to 2,4-pentadienoate, 5-hydroxypent-2-enoate to 5-hydroxypent-2-enoyl-CoA, 5-hydroxypent-2-enoyl-CoA to 5-hydroxypent-2-enoate, glutarate semialdehyde to 5-aminopentanoate, 2-oxoadipate to 2-aminoadipate, 5-hydroxyvaleryl-CoA to 5-hydroxypent-2-enoyl-CoA, 5-hydroxyvaleryl-CoA to 2,4-pentadienoyl-CoA, 5-hydroxypent-2-enoyl-CoA to 2,4-pentadienoyl-CoA, 2,4-pentadienoyl-CoA to 2,4-pentadienoate, 2,4-pendienoate to butadiene, 3-buten-1-ol to butadiene, acetyl-CoA to acetoacetyl-CoA, acetoacetyl-CoA to 3-hydroxybutyryl-CoA, 3-hydroxybutyryl-CoA to crotonyl-CoA, crotonyl-CoA, glutaryl-CoA, propionyl-CoA and acetyl-CoA to 3-oxopentanoyl-CoA, propionyl-CoA and malonyl-CoA to 3-oxopentanoyl-CoA, 3-oxopentanoyl-CoA to 3-hydroxypentanoyl-CoA, 3-hydroxypentanoyl-CoA to pent-2-onoyl-CoA, pent-2-onoyl-CoA to pent-3-enoyl-CoA, pent-3-enoyl-CoA to 2,4-pentadienoyl-CoA, 3-hydroxypropionyl-CoA and acetyl-CoA to 3-oxo-5-hydroxypentanoyl-CoA, 3-oxo-5-hydroxypentanoyl-CoA to 3-oxo-5-hydroxypentanoate, 3-oxo-5-hydroxypentanoate to 3-oxobutanol, 3-oxobutanol to 1,3-butanediol, pyruvate and acetaldehyde to 4-hydroxy-2-oxovalerate, 4-hydroxy-2-oxovalerate to 2-oxopentenoate, 2-oxopentenoate to 3-buten-1-al, 3-buten-1-al to 3-buten-1-ol, 4-hydroxy-2-oxovalerate to 3-hydroxybutyryl-CoA, 4-hydroxy-2-oxovalerate to 3-hydroxybutanal, 3-hydroxybutyryl-CoA to 3-hydroxybutanal, 3-hydroxybutanal to 1,3-butanediol, 3-hydroxybutyryl-CoA to 3-hydroxybutyrate, 3-hydroxybutyrate to 3-hydroxybutyrate to 3-hydroxybutanal, 3-hydroxybutyryl-CoA to 1,3-butanediol, 2,4-pentadienoate to 2,4-pentadienoyl-CoA, 2,4-pentadienoate to penta-2,4-dienal, penta-2,4-dienal to butadiene, 2,4-pentadienoate to 2,4-pentadienoyl-phosphate, 2,4-pentadienoyl-phosphate to penta-2,4-dienal, 2,4-pentadienoyl-CoA to 2,4-pentadienoyl-phosphate, 2,4-pentadienoyl-CoA to penta-2,4-dienal, malonyl-ACP and acetyl-CoA or acetyl-ACP to acetoacetyl-ACP, acetoacetyl-ACP to 3-hydroxybutyryl-ACP, 3-hydroxybutyryl-ACP to crotonyl-ACP, acetoacetyl-ACP to acetoacetyl-CoA, malonyl-CoA and acetyl-CoA to acetoacetyl-CoA, acetoacetyl-CoA to acetoacetate, acetoacetate to 3-oxobutyraldehyde, 3-oxobutyraldehyde to 4-hydroxy-2-butanone, acetoacetyl-ACP to acetoacetate, acetoacetyl-CoA to 3-oxobutyraldehyde, acetoacetyl-ACP to 3-oxobutyraldehyde, acetoacetyl-CoA to 4-hydroxy-2-butanone, 3-hydroxybutyryl-ACP to 3-hydroxybutyrate, 3-hydroxybutyryl-ACP to 3-hydroxybutyraldehyde, 3-hydroxybutyryl-CoA to 3-hydroxybutyraldehyde, 3-hydroxybutyryl-CoA to 1,3-butanediol, acetoacetyl-CoA to 3-hydroxybutyryl-CoA, acetoacetate to 3-hydroxybutyrate, 3-oxobutyraldehyde to 3-hydroxybutyraldehyde, 4-hydroxy-2-butanone to 1,3-butanediol, crotonyl-ACP to crotonate, crotonyl-ACP to crotonaldehyde, crotonyl-CoA to crotonaldehyde, crotonyl-CoA to crotyl alcohol, 3-hydroxybutyryl-ACP to 3-hydroxybutyryl-CoA, 3-hydroxybutyryl-CoA to 3-hydroxybutyrate, 3-hydroxybutyrate to 3-hydroxybutyraldehyde, 3-hydroxybutyraldehyde to 1,3-butanediol, 3-hydroxybutyryl-CoA to crotonyl-CoA, 3-hydroxybutyrate to crotonate, 3-hydroxybutyraldehyde to crotonaldehyde, crotonyl-ACP to crotonyl-CoA, crotonyl-CoA to crotonate, crotonate to crotonaldehyde, crotonaldehyde to crotyl alcohol, butyryl-ACP to butyryl-CoA, butyryl-CoA to butyrate, butyrate to propylene, crotonyl-ACP to butyryl-ACP, crotonyl-CoA to butyryl-CoA, crotonate to butyrate, crotonaldehyde to propylene, butyryl-ACP to butyrate, crotonate to propylene, 3-hydroxybutyrate to propylene, crotyl alcohol to 2-butenyl-4-phosphate, 2-butenyl-4-phosphate to 2-butenyl-4-diphosphate, crotyl alcohol to 2-butenyl-4-diphosphate and 2-butenyl-4-diphosphate to butadiene. One skilled in the art will understand that these are merely exemplary and that any of the substrate-product pairs disclosed herein suitable to produce a desired product and for which an appropriate activity is available for the conversion of the substrate to the product can be readily determined by one skilled in the art based on the teachings herein. Thus, the invention provides a non-naturally occurring microbial organism containing at least one exogenous nucleic acid encoding an enzyme or protein, where the enzyme or protein converts the substrates and products of a 2,4-pentadienoate, butadiene, propylene, 1,3-butanediol, crotyl alcohol or 3-buten-1-ol pathway, such as that shown in Figures 1-7 and 12.

While generally described herein as a microbial organism that contains a 2,4-pcntadicnoatc, butadiene, propylene, 1,3-butanediol, crotyl alcohol or 3-buten-1-ol pathway, it is understood that the invention additionally provides a non-naturally occurring microbial organism having at least one exogenous nucleic acid encoding a 2,4-pentadienoate, butadiene, propylene, 1,3-butanediol, crotyl alcohol or 3-buten-1-ol pathway enzyme expressed in a sufficient amount to produce an intermediate of a 2,4-pentadienoate, butadiene, propylene, 1,3-butanediol, crotyl alcohol or 3-buten-1-ol pathway. For example, as disclosed herein, a 2,4-pentadienoate, butadiene, propylene, 1,3-butanediol, crotyl alcohol or 3-buten-1-ol pathway is exemplified in Figures 1-7 and 12. Therefore, in addition to a microbial organism containing a 2,4-pentadienoate, butadiene, propylene, 1,3-butanediol, crotyl alcohol or 3-buten-1-ol pathway that produces 2,4-pentadienoate, butadiene, propylene, 1,3-butanediol, crotyl alcohol or 3-buten-1-ol, the invention additionally provides a non-naturally occurring microbial organism having at least one exogenous nucleic acid encoding a 2,4-pentadienoate, butadiene, propylene, 1,3-butanediol, crotyl alcohol or 3-buten-1-ol pathway enzyme, where the microbial organism produces a 2,4-pentadienoate, butadiene, propylene, 1,3-butanediol, crotyl alcohol or 3-buten-1-ol pathway intermediate, for example, 5-aminopent-2-enoate, glutarate semialdehyde, 5-hydroxyvalerate, 5-hydroxyvaleryl-CoA, 5-hydroxypent-2-enoyl-CoA, 2,4-pentadienoyl-CoA, 5-hydroxypent-2-enoate, 5-hydroxypent-2-enoate, acetoacetyl-CoA, 3-hydroxybutyryl-CoA, crotoyl-CoA, glutaryl-CoA, 3-oxopentanoyl-CoA, 3-hydroxypentanoyl-CoA, pent-2-enoyl-CoA, pent-3-enoyl-CoA, 3-oxo-5-hydroxypentanoyl-CoA, 3-oxo-5-hydroxypentanoate, 3-oxobutanol, 4-hydroxy-2-oxovalerate, 2-oxopentenoate, 3-buten-1-al, 3-hydroxybutyryl-CoA, 3-hydroxybutyrate, 3-hydroxybutanal, 2,4-pentadienoyl-phosphate, penta-2,4-dienal, acetoacetyl-ACP, acetoacetyl-CoA, acetoacetate, 3-oxobutyraldehyde, 4-hydroxy-2-butanone, 3-hydroxybutyryl-ACP, 3-hydroxybutyryl-CoA, 3-hydroxybutyrate, 3-hydroxybutyraldehyde, crotonyl-ACP, crotonyl-CoA, crotonate, crotonaldehyde, butyryl-ACP, butyryl-CoA, butyrate, 2-butenyl-4-phosphate, or 2-butenyl-4-diphosphate.

It is understood that any of the pathways disclosed herein, as described in the Examples and exemplified in the Figures, including the pathways of Figures 1-9 and 12, can be utilized to generate a non-naturally occurring microbial organism that produces any pathway intermediate or product, as desired. As disclosed herein, such a microbial organism that produces an intermediate can be used in combination with another microbial organism expressing downstream pathway enzymes to produce a desired product. However, it is understood that a non-naturally occurring microbial organism that produces a 2,4-pentadienoate, butadiene, propylene, 1,3-butancdiol, crotyl alcohol or 3-buten-1-ol pathway intermediate can be utilized to produce the intermediate as a desired product.

The invention is described herein with general reference to the metabolic reaction, reactant or product thereof, or with specific reference to one or more nucleic acids or genes encoding an enzyme associated with or catalyzing, or a protein associated with, the referenced metabolic reaction, reactant or product. Unless otherwise expressly stated herein, those skilled in the art will understand that reference to a reaction also constitutes reference to the reactants and products of the reaction. Similarly, unless otherwise expressly stated herein, reference to a reactant or product also references the reaction, and reference to any of these metabolic constituents also references the gene or genes encoding the enzymes that catalyze or proteins involved in the referenced reaction, reactant or product. Likewise, given the well known fields of metabolic biochemistry, enzymology and genomics, reference herein to a gene or encoding nucleic acid also constitutes a reference to the corresponding encoded enzyme and the reaction it catalyzes or a protein associated with the reaction as well as the reactants and products of the reaction.

As disclosed herein, the product 2,4-pentadienoate and intermediates 5-aminopentanoate, 5-aminopent-2-enoate, 5-hydroxypent-2-enoate, 5-hydroxyvalerate, 5-hydroxypent-2-enoate, 3-oxo-5-hydroxypentanoate, 3-hydroxybutyrate, 4-hydroxy-2-exovalerate, 2-oxopentenoate, acetoacetate, crotonate, butyrate, as well as other intermediates, are carboxylic acids, which can occur in various ionized forms, including fully protonated, partially protonated, and fully deprotonated forms. Accordingly, the suffix "-ate," or the acid form, can be used interchangeably to describe both the free acid form as well as any deprotonated form, in particular since the ionized form is known to depend on the pH in which the compound is found. It is understood that carboxylate products or intermediates includes ester forms of carboxylate products or pathway intermediates, such as O-carboxylate and S-carboxylate esters. O- and S-carboxylates can include lower alkyl, that is C1 to C6, branched or straight chain carboxylates. Some such O- or S-carboxylates include, without limitation, methyl, ethyl, n-propyl, n-butyl, i-propyl, sec-butyl, and tert-butyl, pentyl, hexyl O- or S-carboxylates, any of which can further possess an unsaturation, providing for example, propenyl, butenyl, pentyl, and hexenyl O- or S-carboxylatcs. O-carboxylates can be the product of a biosynthetic pathway. Exemplary O-carboxylates accessed via biosynthetic pathways can include, without limitation, methyl 2,4-pentadienoate, ethyl 2,4-pentadienoate, and n-propyl 2,4-pentadienoate. Other biosynthetically accessible O-carboxylates can include medium to long chain groups, that is C7-C22, O-carboxylate esters derived from fatty alcohols, such heptyl, octyl, nonyl, decyl, undecyl, lauryl, tridecyl, myristyl, pentadecyl, cetyl, palmitolyl, heptadecyl, stearyl, nonadecyl, arachidyl, heneicosyl, and behenyl alcohols, any one of which can be optionally branched and/or contain unsaturations. O-carboxylate esters can also be accessed via a biochemical or chemical process, such as esterification of a free carboxylic acid product or transesterification of an O- or S-carboxylate. S-carboxylates are exemplified by CoA S-esters, cysteinyl S-esters, alkylthioesters, and various aryl and heteroaryl thioesters. The non-naturally occurring microbial organisms of the invention can be produced by introducing expressible nucleic acids encoding one or more of the enzymes or proteins participating in one or more 2,4-pentadienoate, butadiene, propylene, 1,3-butanediol, crotyl alcohol or 3-buten-1-ol biosynthetic pathways. Depending on the host microbial organism chosen for biosynthesis, nucleic acids for some or all of a particular 2,4-pentadienoate, butadiene, propylene, 1,3-butanediol, crotyl alcohol or 3-buten-1-ol biosynthetic pathway can be expressed. For example, if a chosen host is deficient in one or more enzymes or proteins for a desired biosynthetic pathway, then expressible nucleic acids for the deficient enzyme(s) or protein(s) are introduced into the host for subsequent exogenous expression. Alternatively, if the chosen host exhibits endogenous expression of some pathway genes, but is deficient in others, then an encoding nucleic acid is needed for the deficient enzyme(s) or protein(s) to achieve 2,4-pentadienoate, butadiene, propylene, 1,3-butanediol, crotyl alcohol or 3-buten-1-ol biosynthesis. Thus, a non-naturally occurring microbial organism of the invention can be produced by introducing exogenous enzyme or protein activities to obtain a desired biosynthetic pathway or a desired biosynthetic pathway can be obtained by introducing one or more exogenous enzyme or protein activities that, together with one or more endogenous enzymes or proteins, produces a desired product such as 2,4-pentadienoate, butadiene, propylene, 1,3-butanediol, crotyl alcohol or 3-buten-1-ol.

Host microbial organisms can be selected from, and the non-naturally occurring microbial organisms generated in, for example, bacteria, yeast, fungus or any of a variety of other microorganisms applicable to fermentation processes. Exemplary bacteria include species selected from *Escherichia coli, Klebsiella oxytoca,* Anaerobiospirillum *succiniciproducens, Actinobacillus succinogenes, Mannheimia succiniciproducens, Rhizobium etli, Bacillus subtilis, Corynebacterium glutamicum, Cupriavidus necator, Gluconobacter oxydans, Zymomonas mobilis, Lactococcus lactis, Lactobacillus plantarum, Streptomyces coelicolor, Clostridium acetobutylicum, Pseudomonas fluorescens,* and *Pseudomonas putida.* Exemplary yeasts or fungi include species selected from *Saccharomyces cerevisiae, Schizosaccharomyces pombe, Kluyveromyces lactis, Kluyveromyces marxianus, Aspergillus terreus, Aspergillus niger, Pichia pastoris, Rhizopus arrhizus, Rhizobus oryzae, Yarrowia lipolytica, Candida albicans* and the like. *E. coli* is a particularly useful host organism since it is a well characterized microbial organism suitable for genetic engineering. Other particularly useful host organisms include yeast such as *Saccharomyces cerevisiae.* It is understood that any suitable microbial host organism can be used to introduce metabolic and/or genetic modifications to produce a desired product.

Depending on the 2,4-pentadienoate, butadiene, propylene, 1,3-butanediol, crotyl alcohol or 3-buten-1-ol biosynthetic pathway constituents of a selected host microbial organism, the non-naturally occurring microbial organisms of the invention will include at least one exogenously expressed 2,4-pentadienoate, butadiene, propylene, 1,3-butanediol, crotyl alcohol or 3-buten-1-ol pathway-encoding nucleic acid and up to all encoding nucleic acids for one or more 2,4-pentadienoate, butadiene, propylene, 1,3-butanediol, crotyl alcohol or 3-buten-1-ol biosynthetic pathways. For example, 2,4-pentadienoate, butadiene, propylene, 1,3-butanediol, crotyl alcohol or 3-buten-1-ol biosynthesis can be established in a host deficient in a pathway enzyme or protein through exogenous expression of the corresponding encoding nucleic acid. In a host deficient in all enzymes or proteins of a 2,4-pentadienoate, butadiene, propylene, 1,3-butanediol, crotyl alcohol or 3-buten-1-ol pathway, exogenous expression of all enzymes or proteins in the pathway can be included, although it is understood that all enzymes or proteins of a pathway can be expressed even if the host contains at least one of the pathway enzymes or proteins. For example, exogenous expression of all enzymes or proteins in a pathway for production of butadiene can be included, such as, a glutaryl-CoA reductase, aglutarate semialdehyde reductase, a 5-hydroxyvaleryl-CoA transferase and/or synthetase, a 5-hydroxyvaleryl-CoA dehydratase/dehydrogenase, a 2,4-pentadienoyl-CoA transferase, synthetase or hydrolase and a 2,4-pentadienoate decarboxylase. As another example, exogenous expression of all enzymes or proteins in a pathway for production of 1,3-butanediol can be included, such as, a 3-ketoacyl-ACP synthase, an acetoacetyl-ACP reductase, a 3-hydroxybutyryl-CoA:ACP transferase, an 3-hydroxybutyryl-CoA hydrolase, transferase or synthetase, a 3-hydroxybutyrate reductase, and a 3-hydroxybutyraldehyde reductase. As yet another example, exogenous expression of all enzymes or proteins in a pathway for production of crotyl-alcohol can be included, such as, a 3-ketoacyl-ACP synthase, an acetoacetyl-ACP reductase, a 3-hydroxybutyryl-ACP dehydratase, a crotonyl-CoA:ACP transferase, a crotonyl-CoA hydrolase, transferase or synthetase, a crotonate reductase and a crotonaldehyde reductase.

Given the teachings and guidance provided herein, those skilled in the art will understand that the number of encoding nucleic acids to introduce in an expressible form will, at least, parallel the 2,4-pentadienoate, butadiene, propylene, 1,3-butanediol, crotyl alcohol or 3-buten-1-ol pathway deficiencies of the selected host microbial organism. Therefore, a non-naturally occurring microbial organism of the invention can have one, two, three, four, five, six, seven, eight, nine, ten or eleven, up to all nucleic acids encoding the enzymes or proteins constituting a 2,4-pentadienoate, butadiene, propylene, 1,3-butanediol, crotyl alcohol or 3-buten-1-ol biosynthetic pathway disclosed herein. In some embodiments, the non-naturally occurring microbial organisms also can include other genetic modifications that facilitate or optimize 2,4-pentadienoate, butadiene, propylene, 1,3-butanediol, crotyl alcohol or 3-buten-1-ol biosynthesis or that confer other useful functions onto the host microbial organism. One such other functionality can include, for example, augmentation of the synthesis of one or more of the 2,4-pentadienoate, butadiene, propylene, 1,3-butanediol, crotyl alcohol or 3-buten-1-ol pathway precursors such as 2-aminoadipate, 5-aminopentanoate, 2-oxoadipate, glutaryl-CoA, propionyl-CoA, acetyl-CoA, malonyl-CoA, 3-hydroxypropionyl-CoA, malonyl-ACP or pyruvate.

Generally, a host microbial organism is selected such that it produces the precursor of a 2,4-pentadienoate, butadiene, propylene, 1,3-butanediol, crotyl alcohol or 3-buten-1-ol pathway, either as a naturally produced molecule or as an engineered product that either provides de novo production of a desired precursor or increased production of a precursor naturally produced by the host microbial organism. For example, 2-aminoadipate, 5-aminopentanoate, 2-oxoadipate, glutaryl-CoA, propionyl-CoA, acetyl-CoA, malonyl-CoA, 3-hydroxypropionyl-CoA, pyruvate or malonyl-ACP is produced naturally in a host organism such as E. coli. A host organism can be engineered to increase production of a precursor, as disclosed herein. In addition, a microbial organism that has been engineered to produce a desired precursor can be used as a host organism and further engineered to express enzymes or proteins of a 2,4-pentadienoate, butadiene, propylene, 1,3-butanediol, crotyl alcohol or 3-buten-1-ol pathway.

In some embodiments, a non-naturally occurring microbial organism of the invention is generated from a host that contains the enzymatic capability to synthesize 2,4-pentadienoate, butadiene, propylene, 1,3-butanediol, crotyl alcohol or 3-buten-1-ol. In this specific embodiment it can be useful to increase the synthesis or accumulation of a 2,4-pentadienoate, butadiene, propylene, 1,3-butanediol, crotyl alcohol or 3-buten-1-ol pathway product to, for example, drive 2,4-pentadienoate, butadiene, propylene, 1,3-butanediol, crotyl alcohol or 3-buten-1-ol pathway reactions toward 2,4-pentadienoate, butadiene, propylene, 1,3-butanediol, crotyl alcohol or 3-buten-1-ol production. Increased synthesis or accumulation can be accomplished by, for example, overexpression of nucleic acids encoding one or more of the above-described 2,4-pentadienoate, butadiene, propylene, 1,3-butanediol, crotyl alcohol or 3-buten-1-ol pathway enzymes or proteins. Overexpression of the enzyme or enzymes and/or protein or proteins of the 2,4-pentadienoate, butadiene, propylene, 1,3-butanediol, crotyl alcohol or 3-buten-1-ol pathway can occur, for example, through exogenous expression of the endogenous gene or genes, or through exogenous expression of the heterologous gene or genes. Therefore, naturally occurring organisms can be readily generated to be non-naturally occurring microbial organisms of the invention, for example, producing 2,4-pentadienoate, butadiene, propylene, 1,3-butanediol, crotyl alcohol or 3-buten-1-ol, through overexpression of one, two, three, four, five, six, seven, eight, nine, ten or eleven, that is, up to all nucleic acids encoding 2,4-pentadienoate, butadiene, propylene, 1,3-butanediol, crotyl alcohol or 3-buten-1-ol biosynthetic pathway enzymes or proteins. In addition, a non-naturally occurring organism can be generated by mutagenesis of an endogenous gene that results in an increase in activity of an enzyme in the 2,4-pentadienoate, butadiene, propylene, 1,3-butanediol, crotyl alcohol or 3-buten-1-ol biosynthetic pathway.

In particularly useful embodiments, exogenous expression of the encoding nucleic acids is employed. Exogenous expression confers the ability to custom tailor the expression and/or regulatory elements to the host and application to achieve a desired expression level that is controlled by the user. However, endogenous expression also can be utilized in other embodiments such as by removing a negative regulatory effector or induction of the gene's promoter when linked to an inducible promoter or other regulatory element. Thus, an endogenous gene having a naturally occurring inducible promoter can be up-regulated by providing the appropriate inducing agent, or the regulatory region of an endogenous gene can be engineered to incorporate an inducible regulatory element, thereby allowing the regulation of increased expression of an endogenous gene at a desired time. Similarly, an inducible promoter can be included as a regulatory element for an exogenous gene introduced into a non-naturally occurring microbial organism.

It is understood that, in methods of the invention, any of the one or more exogenous nucleic acids can be introduced into a microbial organism to produce a non-naturally occurring microbial organism of the invention. The nucleic acids can be introduced so as to confer, for example, a 2,4-pentadienoate, butadiene, propylene, 1,3-butanediol, crotyl alcohol or 3-buten-1-ol biosynthetic pathway onto the microbial organism. Alternatively, encoding nucleic acids can be introduced to produce an intermediate microbial organism having the biosynthetic capability to catalyze some of the required reactions to confer 2,4-pentadienoate, butadiene, propylene, 1,3-butanediol, crotyl alcohol or 3-buten-1-ol biosynthetic capability. For example, a non-naturally occurring microbial organism having a 2,4-pentadienoate, butadiene, propylene, 1,3-butanediol, crotyl alcohol or 3-buten-1-ol biosynthetic pathway can comprise at least two exogenous nucleic acids encoding desired enzymes or proteins, such as the combination of a 5-hydroxypentanoyl-CoA dehydrogenase and a 2,4-pentadienoyl-CoA transferase, or alternatively a 5-aminopentanoate reductase and a 5-aminopent-2-enoate deaminase, or alternatively a 2,4-pentadienoate decarboxylase and a 3-hydroxybutyryl-CoA dehydratase, or alternatively a 3-oxopentanoyl-CoA reductase and a 2,4-pentadienoyl-CoA hydrolase, or alternatively a 4-hydroxy-2-oxopentanoate dehydrogenase and a 3-hydroxybutyryl-CoA reductase (alcohol forming), or alternatively a 3-hydroxybutyrate reductase and a 3-hydroxybutyraldehyde reductase, and the like. Thus, it is understood that any combination of two or more enzymes or proteins of a biosynthetic pathway can be included in a non-naturally occurring microbial organism of the invention. Similarly, it is understood that any combination of three or more enzymes or proteins of a biosynthetic pathway can be included in a non-naturally occurring microbial organism of the invention, for example, a 5-hydroxyvalerate dehydrogenase, a 5-hydroxypent-2-enoyl-CoA transferase and a 5-hydroxypent-2-enoyl-CoA dehydratase, or alternavely a penta-2,4-dienal decarbonylase, a 2,4-pentadienoyl-CoA reductase (acid reducing) and a 5-hydroxyvaleryl-CoA dehydratase/dehydrogenase, or alternatively a 2-oxopentenoate decarboxylase, a 3-buten-1-al reductase and a 3-buten-1-ol dehydratase, or alternatively a crotonaldehyde reductase, a crotonate reductase and a crotonyl-CoA hydrolase, and so forth, as desired, so long as the combination of enzymes and/or proteins of the desired biosynthetic pathway results in production of the corresponding desired product. Similarly, any combination of four, five, six, seven, eight, nine, ten, eleven or more enzymes or proteins of a biosynthetic pathway as disclosed herein can be included in a non-naturally occurring microbial organism of the invention, as desired, so long as the combination of enzymes and/or proteins of the desired biosynthetic pathway results in production of the corresponding desired product.

In addition to the biosynthesis of 2,4-pentadienoate, butadiene, propylene, 1,3-butanediol, crotyl alcohol or 3-buten-1-ol as described herein, the non-naturally occurring microbial organisms and methods of the invention also can be utilized in various combinations with each other and with other microbial organisms and methods well known in the art to achieve product biosynthesis by other routes. For example, one alternative to produce 2,4-pentadienoate, butadiene, propylene, 1,3-butanediol, crotyl alcohol or 3-buten-1-ol other than use of the 2,4-pentadienoate, butadiene, propylene, 1,3-butanediol, crotyl alcohol or 3-buten-1-ol producers is through addition of another microbial organism capable of converting a 2,4-pentadienoate, butadiene, propylene, 1,3-butanediol, crotyl alcohol or 3-buten-1-ol pathway intermediate to 2,4-pentadienoate, butadiene, propylene, 1,3-butanediol, crotyl alcohol or 3-buten-1-ol. One such procedure includes, for example, the fermentation of a microbial organism that produces a 2,4-pentadienoate, butadiene, propylene, 1,3-butanediol, crotyl alcohol or 3-buten-1-ol pathway intermediate. The 2,4-pentadienoate, butadiene, propylene, 1,3-butanediol, crotyl alcohol or 3-buten-1-ol pathway intermediate can then be used as a substrate for a second microbial organism that converts the 2,4-pentadienoate, butadiene, propylene, 1,3-butanediol, crotyl alcohol or 3-buten-1-ol pathway intermediate to 2,4-pentadienoate, butadiene, propylene, 1,3-butanediol, crotyl alcohol or 3-buten-1-ol. The 2,4-pentadienoate, butadiene, propylene, 1,3-butanediol, crotyl alcohol or 3-buten-1-ol pathway intermediate can be added directly to another culture of the second organism or the original culture of the 2,4-pentadienoate, butadiene, propylene, 1,3-butanediol, crotyl alcohol or 3-buten-1-ol pathway intermediate producers can be depleted of these microbial organisms by, for example, cell separation, and then subsequent addition of the second organism to the fermentation broth can be utilized to produce the final product without intermediate purification steps.

In other embodiments, the non-naturally occurring microbial organisms and methods of the invention can be assembled in a wide variety of subpathways to achieve biosynthesis of, for example, 2,4-pentadienoate, butadiene, propylene, 1,3-butanediol, crotyl alcohol or 3-buten-1-ol. In these embodiments, biosynthetic pathways for a desired product of the invention can be segregated into different microbial organisms, and the different microbial organisms can be co-cultured to produce the final product. In such a biosynthetic scheme, the product of one microbial organism is the substrate for a second microbial organism until the final product is synthesized. For example, the biosynthesis of 2,4-pentadienoate, butadiene, propylene, 1,3-butanediol, crotyl alcohol or 3-buten-1-ol can be accomplished by constructing a microbial organism that contains biosynthetic pathways for conversion of one pathway intermediate to another pathway intermediate or the product. Alternatively, 2,4-pentadienoate, butadiene, propylene, 1,3-butanediol, crotyl alcohol or 3-buten-1-ol also can be biosynthetically produced from microbial organisms through co-culture or co-fermentation using two organisms in the same vessel, where the first microbial organism produces a 2,4-pentadienoate, butadiene, propylene, 1,3-butanediol, crotyl alcohol or 3-buten-1-ol intermediate and the second microbial organism converts the intermediate to 2,4-pentadienoate, butadiene, propylene, 1,3-butanediol, crotyl alcohol or 3-buten-1-ol.

Given the teachings and guidance provided herein, those skilled in the art will understand that a wide variety of combinations and permutations exist for the non-naturally occurring microbial organisms and methods of the invention together with other microbial organisms, with the co-culture of other non-naturally occurring microbial organisms having subpathways and with combinations of other chemical and/or biochemical procedures well known in the art to produce 2,4-pentadienoate, butadiene, propylene, 1,3-butanediol, crotyl alcohol or 3-buten-1-ol.

Sources of encoding nucleic acids for a 2,4-pentadienoate, butadiene, propylene, 1,3-butanediol, crotyl alcohol or 3-buten-1-ol pathway enzyme or protein can include, for example, any species where the encoded gene product is capable of catalyzing the referenced reaction. Such species include both prokaryotic and eukaryotic organisms including, but not limited to, bacteria, including archaea and eubacteria, and eukaryotes, including yeast, plant, insect, animal, and mammal, including human. Exemplary species for such sources include, for example, *Escherichia coli, Acetobacter aceti, Acetobacter pasteurians, Achromobacter denitrificans, Acidaminococcusfermentans, Acinetobacter baumanii, Acinetobacter baylyi, Acinetobacter calcoaceticus, Acinetobacter sp. ADP1, Acinetobacter sp. Strain M-1, Actinobacillus succinogenes, Acyrthosiphon pisum, Aeropyrum pernix, Agrobacterium tumefaciens, Allochromatium vinosum DSM 180, Anabaena variabilis, Anaerobiospirillum succiniciproducens, Anaerostipes caccae DSM 14662, Anaerotruncus colihominis, Antheraea yamamai, Aquifex aeolicus, Arabidopsis thaliana, Archaeglubus fulgidus,* Archacoglobus *fulgidus*, *Aromatoleum aromaticum EbN1, Ascaris suum, Ascarius suum, Aspergillus nidulans, Aspergillus niger, Aspergillus oryzae, Aspergillus terreus, Aspergillus terreus NIH2624, Azoarcus sp. CIB, Azoarcus sp. T, Azotobacter vinelandii DJ, Anabaena variabilis, Bacillus anthracis, Bacillus amyloliquefaciens, Bacillus cereus, Bacillus coahuilensis, Bacillus megaterium, Bacillus pseudofirmus, Bacillus pumilus, Bacillus sphaericus, Bacillus subtilis ,Bacteroides capillosus, Balnearium lithotrophicum, Bos taurus, Bradyrhizobium japonicum, Bradyrhizobium japonicum USDA110, Brassica napsus, Burkholderia ambifaria AMMD, Burkholderia phymatum, Burkholderia xenovorans, butyrate-producing bacterium L2-50, butyrate-producing bacterium L2-50, butyrate-producing bacterium SS3*/*4, Campylobacter curvus 525.92, Campylobacter jejuni, Candida albicans, Candida parapsilosis ,Candida tropicalis, Carboxydothermus hydrogenoformans, Chlamydomonas reinhardtii, Chlorobium limicola, Chlorobium phaeobacteroides DSM 266, Chlorobium tepidum, Chlorobium tepidum, Chloroflexus aurantiacus, Citrobacter amalonaticus, Citrobacter youngae ATCC 29220, Clostridium acetobutylicum, Clostridium aminobutyricum, Clostridium beijerinckii, Clostridium beijerinckii NRRL B593, Clostridium botulinum, Clostridium botulinum A3 str, Clostridium botulinum C str. Eklund, Clostridium carboxidivorans P7, Clostridium carboxidivorans P7, Clostridium cellulolyticum H10, Clostridium kluyveri, Clostridium kluyveri DSM 555, Clostridium novyi NT, Clostridium pasteurianum, Clostridium propionicum, Clostridium saccharoperbutylacetonicum, Clostridium sp. SS2*/*1, Clostridium tetani, Clostridium tetanomorphum, Clostridium tyrobutyricum, Comamonas sp. CNB-1, Corynebacterium glutamicum, Corynebacterium glutamicum ATCC 13032, Corynebacterium glutanicum, Cucumis sativus, Cupriavidus necator, Cupriavidus taiwanensis, Cyanobium PCC7001, Desulfovibrio africanus, DesulfoVibrio desulfuricans G20, Desulfovibrio desulfuricans subsp. desulfuricans str. ATCC 27774, Desulfovibrio fructosovorans JJ, Desuljovibrio vulgaris str. Hildenborough, Dictyostelium discoideum AX4, Drosophila melanogaster, Elizabethkingia meningoseptica, Erythrobacter sp. NAP1, Escherichia coli C, Escherichia coli K12, Escherichia coli K12 subsp. MG1655, Escherichia coli O157:H7 str. Sakai, Escherichia coli str. K-12 substr. MG1655, Escherichia coli W, Eubacterium barkeri, Eubacterium rectale ATCC 33656, Eubacterium yurii, Euglena gracilis, Flavobacterium lutescens, Fusobacterium gonidiaformans, Fusobacterium nucleatum, Geobacillus stearothermophilus, Geobacillus thermoglucosidasius* , *Geobacter metallireducens GS-15, Geobacter sulfurreducens, Gibberella zeae, Haemophilus influenza, Haloarcula marismortui, Halobacillus dabanensis, Halobacterium salinarum, Haloferax mediterranei, Helicobacter pylori, Helicobacter pylori 26695, Helicoverpa zea, Heliobacter pylori, Homo sapiens, Hydrogenobacter thermophilus, Jeotgalicoccus sp. ATCC8456, Klebsiella oxytoca, Klebsiella pneumonia, Klebsiella pneumonia, Kluyveromyces lactis, Kocuria rosea, Lactobacillus plantarum, Lactobacillus sp. 30a, Lactococcus lactis, Leuconostoc mesenteroides, Macrococcus caseolyticus, Mannheimia succiniciproducens, marine gamma proteobacterium HTCC2080, Marinococcus halophilus, Marinomonas mediterranea, Medicago truncatula, Mesorhizobium loti, Metallosphaera sedula, Methanocaldococcus jannaschii, Methanosarcina thermophila, Methanothermobacter thermautotrophicus, Methylobacterium extorquens, Moorella thermoacetica, Mus musculus, Musca domestica, Mycobacterium avium, Mycobacterium avium subsp. paratuberculosis K-10, Mycobacterium avium subsp. Pratuberculosis, Mycobacterium bovis BCG, Mycobacterium marinum M, Mycobacterium smegmatis, Mycobacterium smegmatis MC2 155, Mycobacterium tuberculosis, Natranaerobius thermophilus, Neosartorya fischeri, Nicotiana glutinosa, Nicotiana tabacum, Nocardia farcinica IFM 10152, Nocardia iowensis, Nostoc sp. PCC 7120, Oryctolagus cuniculus, Oryza sativa, Paracoccus denitrificans, Pedicoccus pentosaceus, Pelobacter carbinolicus DSM 2380, Pelotomaculum thermopropionicum, Penicillium chrysogenum, Peptoniphilus harei, Pichia stipitis, Porphyromonas gingivalis, Pseudoalteromonas tunicate, Pseudomonas aeruginosa, Pseudomonas aeruginosa PAO1, Pseudomonas fluorescens, Pseudomonas fluorescens KU-7, Pseudomonas fluorescens Pf-5, Pseudomonas knackmussii (E13), Pseudomonas mendocina, Pseudomonas putida, Pseudomonas putida KT2440, Pseudomonas reinekei MT1, Pseudomonas sp, Pseudomonas sp. CF600, Pseudomonas sp. CF600, Pseudomonas sp. CF600, Pseudomonas sp. strain E13, Pseudomonas stutzeri, Pseudoramibacter alactolyticus, Psychroflexus torquis A TCC 700755, Pyrobaculum aerophilum str. IM2, Pyrococcus furiosus, Ralstonia eutropha, Ralstonia eutropha H16, Ralstonia eutropha JMP134, Ralstonia metallidurans, Ralstonia pickettii, Rattus norvegicus, Rhizobium leguminosarum, Rhodobacter capsulates, Rhodobacter capsulatus, Rhodobacter sphaeroides, Rhodococcus opacus, Rhodococcus ruber, Rhodopseudomonas palustris, Rhodopseiidomonas palustris CGA009, Rhodospirillum rubrum, Roseburia intestinalis L1-82, Roseburia inulinivorans, Roseburia sp. A2-183, Roseiflexus castenholzii, Saccharomyces cerevisae, Salinispora arenicola, Salmonella enteric, Salmonella enterica subsp. arizonae serovar, Salmonella typhimurium, Salmonella typhimurium LT2, Schizosaccharomyces pombe, Selenomonas ruminantium, Serratia marcescens, Simmondsia chinensis, Solibacillus silvestris, Sordaria macrospora, Sporosarcina newyorkensis, Staphylococcus pseudintermedius, Streptococcus mutans, Streptococcus oligofermentans, Streptococcus pyogenes ATCC 10782, Streptomyces clavuligenus, Streptomyces coelicolor, Streptomyces griseus, Streptomyces griseus subsp. griseus NBRC 13350, Sulfolobus acidocalarius, Sulfolobus sp. strain 7, Sulfolobus tokodaii, Sulfolobus tokodaii 7, Sulfurihydrogenibium subterraneum, Sulfurimonas denitrificans, Sus scrofa, Synechocystis str. PCC 6803, Syntrophus aciditrophicus, Thauera aromatic, Thauera aromatic, Thermoanaerobacter brockii HTD4, Thermocrinis albus, Thermoproteus neutrophilus, Thermotoga maritime, Thermus thermophilus, Thiobacillus denitrificans, Treponema denticola, Trichomonas vaginalis G3, Trypanosoma brucei, Tsukamurella paurometabola DSM 20162, Vibrio cholera, Vibrio parahaemolyticus* , *Vibrio vulnificus, Vitis vinifera, Yarrowia lipolytica, Yersinia intermedia ATCC 29909, Zea mays, Zoogloea ramigera, Zymomonas mobilis, Carthamus tinctorius, Cuphea hookeriana, Cuphea palustris, Cyanothece sp. PCC 7425, Elizabethkingia meningoseptica, Lyngbya sp. PCC 8106, Nodularia spumigena CCY9414, Nostoc azollae, Plasmodium falciparum, Prochlorococcus marinus, Streptococcus pneumoniae, Streptococcus pyogenes ATCC 10782, Streptomyces avermitillis, Synechococcus elongatus, Synechococcus elongatus PCC7942, Thermomyces lanuginosus, Umbellularia californica, Arabidopsis thaliana col, Enterococcus faecalis, Mycoplasma pneumoniae M129, Populus alba, Populus tremula, Pueraria montana, Staphylococcus aureus, Streptomyces sp. ACT-1, Thermotoga maritime MSB8, Streptomyces sp CL190, Streptomyces sp. KO-3988, Streptomyces cinnamonensis, Streptomyces anulatus, Nocardia brasiliensis* as well as other exemplary species disclosed herein are available as source organisms for corresponding genes. However, with the complete genome sequence available for now more than 550 species (with more than half of these available on public databases such as the NCBI), including 395 microorganism genomes and a variety of yeast, fungi, plant, and mammalian genomes, the identification of genes encoding the requisite 2,4-pentadienoate, butadiene, propylene, 1,3-butanediol, crotyl alcohol or 3-buten-1-ol biosynthetic activity for one or more genes in related or distant species, including for example, homologues, orthologs, paralogs and nonorthologous gene displacements of known genes, and the interchange of genetic alterations between organisms is routine and well known in the art. Accordingly, the metabolic alterations allowing biosynthesis of 2,4-pentadienoate, butadiene, propylene, 1,3-butanediol, crotyl alcohol or 3-buten-1-ol described herein with reference to a particular organism such as *E. coli* can be readily applied to other microorganisms, including prokaryotic and eukaryotic organisms alike. Given the teachings and guidance provided herein, those skilled in the art will know that a metabolic alteration exemplified in one organism can be applied equally to other organisms.

In some instances, such as when an alternative 2,4-pentadienoate, butadiene, propylene, 1,3-butanediol, crotyl alcohol or 3-buten-1-ol biosynthetic pathway exists in an unrelated species, 2,4-pentadienoate, butadiene, propylene, 1,3-butanediol, crotyl alcohol or 3-buten-1-ol biosynthesis can be conferred onto the host species by, for example, exogenous expression of a paralog or paralogs from the unrelated species that catalyzes a similar, yet non-identical metabolic reaction to replace the referenced reaction. Because certain differences among metabolic networks exist between different organisms, those skilled in the art will understand that the actual gene usage between different organisms may differ. However, given the teachings and guidance provided herein, those skilled in the art also will understand that the teachings and methods of the invention can be applied to all microbial organisms using the cognate metabolic alterations to those exemplified herein to construct a microbial organism in a species of interest that will synthesize 2,4-pentadienoate, butadiene, propylene, 1,3-butanediol, crotyl alcohol or 3-buten-1-ol.

Methods for constructing and testing the expression levels of a non-naturally occurring 2,4-pentadienoate, butadiene, propylene, 1,3-butanediol, crotyl alcohol or 3-buten-1-ol-producing host can be performed, for example, by recombinant and detection methods well known in the art. Such methods can be found described in, for example, Sambrook et al., Molecular Cloning: A Laboratory Manual, Third Ed., Cold Spring Harbor Laboratory, New York (2001); and Ausubel et al., Current Protocols in Molecular Biology, John Wiley and Sons, Baltimore, MD (1999).

Exogenous nucleic acid sequences involved in a pathway for production of 2,4-pentadienoate, butadiene, propylene, 1,3-butanediol, crotyl alcohol or 3-buten-1-ol can be introduced stably or transiently into a host cell using techniques well known in the art including, but not limited to, conjugation, electroporation, chemical transformation, transduction, transfection, and ultrasound transformation. For exogenous expression in *E. co*li or other prokaryotic cells, some nucleic acid sequences in the genes or cDNAs of eukaryotic nucleic acids can encode targeting signals such as an N-terminal mitochondrial or other targeting signal, which can be removed before transformation into prokaryotic host cells, if desired. For example, removal of a mitochondrial leader sequence led to increased expression in *E. coli* (Hoffmeister et al., J. Biol. Chem. 280:4329-4338 (2005)). For exogenous expression in yeast or other eukaryotic cells, genes can be expressed in the cytosol without the addition of leader sequence, or can be targeted to mitochondrion or other organelles, or targeted for secretion, by the addition of a suitable targeting sequence such as a mitochondrial targeting or secretion signal suitable for the host cells. Thus, it is understood that appropriate modifications to a nucleic acid sequence to remove or include a targeting sequence can be incorporated into an exogenous nucleic acid sequence to impart desirable properties. Furthermore, genes can be subjected to codon optimization with techniques well known in the art to achieve optimized expression of the proteins.

An expression vector or vectors can be constructed to include one or more 2,4-pentadienoate, butadiene, propylene, 1,3-butanediol, crotyl alcohol or 3-buten-1-ol biosynthetic pathway encoding nucleic acids as exemplified herein operably linked to expression control sequences functional in the host organism. Expression vectors applicable for use in the microbial host organisms of the invention include, for example, plasmids, phage vectors, viral vectors, episomes and artificial chromosomes, including vectors and selection sequences or markers operable for stable integration into a host chromosome. Additionally, the expression vectors can include one or more selectable marker genes and appropriate expression control sequences. Selectable marker genes also can be included that, for example, provide resistance to antibiotics or toxins, complement auxotrophic deficiencies, or supply critical nutrients not in the culture media. Expression control sequences can include constitutive and inducible promoters, transcription enhancers, transcription terminators, and the like which are well known in the art. When two or more exogenous encoding nucleic acids are to be co-expressed, both nucleic acids can be inserted, for example, into a single expression vector or in separate expression vectors. For single vector expression, the encoding nucleic acids can be operationally linked to one common expression control sequence or linked to different expression control sequences, such as one inducible promoter and one constitutive promoter. The transformation of exogenous nucleic acid sequences involved in a metabolic or synthetic pathway can be confirmed using methods well known in the art. Such methods include, for example, nucleic acid analysis such as Northern blots or polymerase chain reaction (PCR) amplification of mRNA, or immunoblotting for expression of gene products, or other suitable analytical methods to test the expression of an introduced nucleic acid sequence or its corresponding gene product. It is understood by those skilled in the art that the exogenous nucleic acid is expressed in a sufficient amount to produce the desired product, and it is further understood that expression levels can be optimized to obtain sufficient expression using methods well known in the art and as disclosed herein.

Suitable purification and/or assays to test for the production of 2,4-pentadienoate, butadiene, propylene, 1,3-butanediol, crotyl alcohol or 3-buten-1-ol can be performed using well known methods. Suitable replicates such as triplicate cultures can be grown for each engineered strain to be tested. For example, product and byproduct formation in the engineered production host can be monitored. The final product and intermediates, and other organic compounds, can be analyzed by methods such as HPLC (High Performance Liquid Chromatography), GC-MS (Gas Chromatography-Mass Spectroscopy) and LC-MS (Liquid Chromatography-Mass Spectroscopy) or other suitable analytical methods using routine procedures well known in the art. The release of product in the fermentation broth can also be tested with the culture supernatant. Byproducts and residual glucose can be quantified by HPLC using, for example, a refractive index detector for glucose and alcohols, and a UV detector for organic acids (Lin et al., Biotechnol. Bioeng. 90:775-779 (2005)), or other suitable assay and detection methods well known in the art. The individual enzyme or protein activities from the exogenous DNA sequences can also be assayed using methods well known in the art.

The 2,4-pentadienoate, butadiene, propylene, 1,3-butanediol, crotyl alcohol or 3-buten-1-ol can be separated from other components in the culture using a variety of methods well known in the art. Such separation methods include, for example, extraction procedures as well as methods that include continuous liquid-liquid extraction, pervaporation, membrane filtration, membrane separation, reverse osmosis, electrodialysis, distillation, crystallization, centrifugation, extractive filtration, ion exchange chromatography, size exclusion chromatography, adsorption chromatography, and ultrafiltration. All of the above methods are well known in the art.

Any of the non-naturally occurring microbial organisms described herein can be cultured to produce and/or secrete the biosynthetic products of the invention. For example, the 2,4-pentadienoate, butadiene, propylene, 1,3-butanediol, crotyl alcohol or 3-buten-1-ol producers can be cultured for the biosynthetic production of 2,4-pentadienoate, butadiene, propylene, 1,3-butanediol, crotyl alcohol or 3-buten-1-ol.

For the production of 2,4-pentadienoate, butadiene, propylene, 1,3-butanediol, crotyl alcohol or 3-buten-1-ol, the recombinant strains are cultured in a medium with carbon source and other essential nutrients. It is sometimes desirable and can be highly desirable to maintain anaerobic conditions in the fermenter to reduce the cost of the overall process. Such conditions can be obtained, for example, by first sparging the medium with nitrogen and then sealing the flasks with a septum and crimp-cap. For strains where growth is not observed anaerobically, microaerobic or substantially anaerobic conditions can be applied by perforating the septum with a small hole for limited aeration. Exemplary anaerobic conditions have been described previously and are well-known in the art. Exemplary aerobic and anaerobic conditions are described, for example, in United State publication 2009/0047719, filed August 10, 2007. Fermentations can be performed in a batch, fed-batch or continuous manner, as disclosed herein. Fermentations can also be conducted in two phases, if desired. The first phase can be aerobic to allow for high growth and therefore high productivity, followed by an anaerobic phase of high 2,4-pentadienoate, butadiene, propylene, 1,3-butanediol, crotyl alcohol or 3-buten-1-ol yields.

If desired, the pH of the medium can be maintained at a desired pH, in particular neutral pH, such as a pH of around 7 by addition of a base, such as NaOH or other bases, or acid, as needed to maintain the culture medium at a desirable pH. The growth rate can be determined by measuring optical density using a spectrophotometer (600 nm), and the glucose uptake rate by monitoring carbon source depletion over time.

The growth medium can include, for example, any carbohydrate source which can supply a source of carbon to the non-naturally occurring microorganism. Such sources include, for example, sugars such as glucose, xylose, arabinose, galactose, mannose, fructose, sucrose and starch. Other sources of carbohydrate include, for example, renewable feedstocks and biomass. Exemplary types of biomasses that can be used as feedstocks in the methods of the invention include cellulosic biomass, hemicellulosic biomass and lignin feedstocks or portions of feedstocks. Such biomass feedstocks contain, for example, carbohydrate substrates useful as carbon sources such as glucose, xylose, arabinose, galactose, mannose, fructose and starch. Given the teachings and guidance provided herein, those skilled in the art will understand that renewable feedstocks and biomass other than those exemplified above also can be used for culturing the microbial organisms of the invention for the production of 2,4-pentadienoate, butadiene, propylene, 1,3-butanediol, crotyl alcohol or 3-buten-1-ol.

In addition to renewable feedstocks such as those exemplified above, the 2,4-pentadienoate, butadiene, propylene, 1,3-butanediol, crotyl alcohol or 3-buten-1-ol microbial organisms of the invention also can be modified for growth on syngas as its source of carbon. In this specific embodiment, one or more proteins or enzymes are expressed in the 2,4-pentadienoate, butadiene, propylene, 1,3-butanediol, crotyl alcohol or 3-buten-1-ol producing organisms to provide a metabolic pathway for utilization of syngas or other gaseous carbon source.

Synthesis gas, also known as syngas or producer gas, is the major product of gasification of coal and of carbonaceous materials such as biomass materials, including agricultural crops and residues. Syngas is a mixture primarily of H₂ and CO and can be obtained from the gasification of any organic feedstock, including but not limited to coal, coal oil, natural gas, biomass, and waste organic matter. Gasification is generally carried out under a high fuel to oxygen ratio. Although largely H₂ and CO, syngas can also include CO₂ and other gases in smaller quantities. Thus, synthesis gas provides a cost effective source of gaseous carbon such as CO and, additionally, CO₂.

The Wood-Ljungdahl pathway catalyzes the conversion of CO and H₂ to acetyl-CoA and other products such as acetate. Organisms capable of utilizing CO and syngas also generally have the capability of utilizing CO₂ and CO₂/H₂ mixtures through the same basic set of enzymes and transformations encompassed by the Wood-Ljungdahl pathway. H₂-dependent conversion of CO₂ to acetate by microorganisms was recognized long before it was revealed that CO also could be used by the same organisms and that the same pathways were involved. Many acetogens have been shown to grow in the presence of CO₂ and produce compounds such as acetate as long as hydrogen is present to supply the necessary reducing equivalents (see for example, Drake, Acetogenesis, pp. 3-60 Chapman and Hall, New York, (1994)). This can be summarized by the following equation:

2CO₂ + 4H₂ + nADP + nPi → CH₃COOH + 2H₂O + nATP

Hence, non-naturally occurring microorganisms possessing the Wood-Ljungdahl pathway can utilize CO₂ and H₂ mixtures as well for the production of acetyl-CoA and other desired products.

The Wood-Ljungdahl pathway is well known in the art and consists of 12 reactions which can be separated into two branches: (1) methyl branch and (2) carbonyl branch. The methyl branch converts syngas to methyl-tetrahydrofolate (methyl-THF) whereas the carbonyl branch converts methyl-THF to acetyl-CoA. The reactions in the methyl branch are catalyzed in order by the following enzymes or proteins: ferredoxin oxidoreductase, formate dehydrogenase, formyltetrahydrofolate synthetase, methenyltetrahydrofolate cyclodehydratase, methylenetetrahydrofolate dehydrogenase and methylenetetrahydrofolate reductase. The reactions in the carbonyl branch are catalyzed in order by the following enzymes or proteins: methyltetrahydrofolate:corrinoid protein methyltransferase (for example, AcsE), corrinoid iron-sulfur protein, nickel-protein assembly protein (for example, AcsF), ferredoxin, acetyl-CoA synthase, carbon monoxide dehydrogenase and nickel-protein assembly protein (for example, CooC). Following the teachings and guidance provided herein for introducing a sufficient number of encoding nucleic acids to generate a 2,4-pentadienoate, butadiene, propylene, 1,3-butanediol, crotyl alcohol or 3-buten-1-ol pathway, those skilled in the art will understand that the same engineering design also can be performed with respect to introducing at least the nucleic acids encoding the Wood-Ljungdahl enzymes or proteins absent in the host organism. Therefore, introduction of one or more encoding nucleic acids into the microbial organisms of the invention such that the modified organism contains the complete Wood-Ljungdahl pathway will confer syngas utilization ability.

Additionally, the reductive (reverse) tricarboxylic acid cycle coupled with carbon monoxide dehydrogenase and/or hydrogenase activities can also be used for the conversion of CO, CO₂ and/or H₂ to acetyl-CoA and other products such as acetate. Organisms capable of fixing carbon via the reductive TCA pathway can utilize one or more of the following enzymes: ATP citrate-lyase, citrate lyase, aconitase, isocitrate dehydrogenase, alpha-ketoglutarate:ferredoxin oxidoreductase, succinyl-CoA synthetase, succinyl-CoA transferase, fumarate reductase, fumarase, malate dehydrogenase, NAD(P)H:ferredoxin oxidoreductase, carbon monoxide dehydrogenase, and hydrogenase. Specifically, the reducing equivalents extracted from CO and/or H₂ by carbon monoxide dehydrogenase and hydrogenase are utilized to fix CO₂ via the reductive TCA cycle into acetyl-CoA or acetate. Acetate can be converted to acetyl-CoA by enzymes such as acetyl-CoA transferase, acetate kinase/phosphotransacetylase, and acetyl-CoA synthetase. Acetyl-CoA can be converted to the 2,4-pentadienoate, butadiene, propylene, 1,3-butanediol, crotyl alcohol or 3-buten-1-ol precursors, glyceraldehyde-3-phosphate, phosphoenolpyruvate, and pyruvate, by pyruvate:ferredoxin oxidoreductase and the enzymes of gluconeogencsis. Following the teachings and guidance provided herein for introducing a sufficient number of encoding nucleic acids to generate a 2,4-pentadienoate, butadiene, propylene, 1,3-butanediol, crotyl alcohol or 3-buten-1-ol pathway, those skilled in the art will understand that the same engineering design also can be performed with respect to introducing at least the nucleic acids encoding the reductive TCA pathway enzymes or proteins absent in the host organism. Therefore, introduction of one or more encoding nucleic acids into the microbial organisms of the invention such that the modified organism contains a reductive TCA pathway can confer syngas utilization ability.

Thus, this invention is also directed, in part to engineered biosynthetic pathways to improve carbon flux through a central metabolism intermediate en route to 2,4-pentadienoate, butadiene, propylene, 1,3-butanediol, crotyl alcohol or 3-buten-1-ol. The present invention provides non-naturally occurring microbial organisms having one or more exogenous genes encoding enzymes that can catalyze various enzymatic transformations en route to 2,4-pentadienoate, butadiene, propylene, 1,3-butanediol, crotyl alcohol or 3-buten-1-ol. In some embodiments, these enzymatic transformations are part of the reductive tricarboxylic acid (RTCA) cycle and are used to improve product yields, including but not limited to, from carbohydrate-based carbon feedstock.

In numerous engineered pathways, realization of maximum product yields based on carbohydrate feedstock is hampered by insufficient reducing equivalents or by loss of reducing equivalents and/or carbon to byproducts. In accordance with some embodiments, the present invention increases the yields of 2,4-pentadienoate, butadiene, propylene, 1,3-butanediol, crotyl alcohol or 3-buten-1-ol by (i) enhancing carbon fixation via the reductive TCA cycle, and/or (ii) accessing additional reducing equivalents from gaseous carbon sources and/or syngas components such as CO, CO₂, and/or H₂. In addition to syngas, other sources of such gases include, but are not limted to, the atmosphere, either as found in nature or generated.

The CO₂-fixing reductive tricarboxylic acid (RTCA) cycle is an endergenic anabolic pathway of CO₂ assimilation which uses reducing equivalents and ATP (Figure 8). One turn of the RTCA cycle assimilates two moles of CO₂ into one mole of acetyl-CoA, or four moles of CO₂ into one mole of oxaloacetate. This additional availability of acetyl-CoA improves the maximum theoretical yield of product molecules derived from carbohydrate-based carbon feedstock. Exemplary carbohydrates include but are not limited to glucose, sucrose, xylose, arabinose and glycerol.

In some embodiments, the reductive TCA cycle, coupled with carbon monoxide dehydrogenase and/or hydrogenase enzymes, can be employed to allow syngas, CO₂, CO, H₂, and/or other gaseous carbon source utilization by microorganisms. Synthesis gas (syngas), in particular is a mixture of primarily H₂ and CO, sometimes including some amounts of CO₂, that can be obtained via gasification of any organic feedstock, such as coal, coal oil, natural gas, biomass, or waste organic matter. Numerous gasification processes have been developed, and most designs are based on partial oxidation, where limiting oxygen avoids full combustion, of organic materials at high temperatures (500-1500°C) to provide syngas as a 0.5:1-3:1 H₂/CO mixture. In addition to coal, biomass of many types has been used for syngas production and represents an inexpensive and flexible feedstock for the biological production of renewable chemicals and fuels. Carbon dioxide can be provided from the atmosphere or in condensed from, for example, from a tank cylinder, or via sublimation of solid CO₂. Similarly, CO and hydrogen gas can be provided in reagent form and/or mixed in any desired ratio. Other gaseous carbon forms can include, for example, methanol or similar volatile organic solvents.

The components of synthesis gas and/or other carbon sources can provide sufficient CO₂, reducing equivalents, and ATP for the reductive TCA cycle to operate. One turn of the RTCA cycle assimilates two moles of CO₂ into one mole of acetyl-CoA and requires 2 ATP and 4 reducing equivalents. CO and/or H₂ can provide reducing equivalents by means of carbon monoxide dehydrogenase and hydrogenase enzymes, respectively. Reducing equivalents can come in the form of NADH, NADPH, FADH, reduced quinones, reduced ferredoxins, reduced flavodoxins and thioredoxins. The reducing equivalents, particularly NADH, NADPH, and reduced ferredoxin, can serve as cofactors for the RTCA cycle enzymes, for example, malate dehydrogenase, fumarate reductase, alpha-ketoglutarate:ferredoxin oxidoreductase (alternatively known as 2-oxoglutarate:ferredoxin oxidoreductase, alpha-ketoglutarate synthase, or 2-oxoglutarate synthase), pyruvate:ferredoxin oxidoreductase and isocitrate dehydrogenase. The electrons from these reducing equivalents can alternatively pass through an ion-gradient producing electron transport chain where they are passed to an acceptor such as oxygen, nitrate, oxidized metal ions, protons, or an electrode. The ion-gradient can then be used for ATP generation via an ATP synthase or similar enzyme.

The reductive TCA cycle was first reported in the green sulfur photosynthetic bacterium *Chlorobium limicola* (Evans ct al., Proc. Natl. Acad. Sci. U.S.A. 55:928-934 (1966)). Similar pathways have been characterized in some prokaryotes (proteobacteria, green sulfur bacteria and thermophillic Knallgas bacteria) and sulfur-dependent archaea (Hugler ct al., J. Bacteriol. 187:3020-3027 (2005; Hugler ct al., Environ. Microbiol. 9:81-92 (2007). In some cases, reductive and oxidative (Krebs) TCA cycles are present in the same organism (Hugler et al., *supra* (2007); Siebers et al., J. Bacteriol. 186:2179-2194 (2004)). Some methanogens and obligate anaerobes possess incomplete oxidative or reductive TCA cycles that may function to synthesize biosynthetic intermediates (Ekiel et al., J. Bacteriol. 162:905-908 (1985); Wood et al., FEMS Microbiol. Rev. 28:335-352 (2004)).

The key carbon-fixing enzymes of the reductive TCA cycle are alpha-ketoglutarate:ferredoxin oxidoreductase, pyruvate:ferredoxin oxidoreductase and isocitrate dehydrogenase. Additional carbon may be fixed during the conversion of phosphoenolpyruvate to oxaloacetate by phosphoenolpyruvate carboxylase or phosphoenolpyruvate carboxykinase or by conversion of pyruvate to malate by malic enzyme.

Many of the enzymes in the TCA cycle are reversible and can catalyze reactions in the reductive and oxidative directions. However, some TCA cycle reactions are irreversible *in vivo* and thus different enzymes are used to catalyze these reactions in the directions required for the reverse TCA cycle. These reactions are: (1) conversion of citrate to oxaloacetate and acetyl-CoA, (2) conversion of fumarate to succinate, and (3) conversion of succinyl-CoA to alpha-ketoglutarate. In the TCA cycle, citrate is formed from the condensation of oxaloacetate and acetyl-CoA. The reverse reaction, cleavage of citrate to oxaloacetate and acetyl-CoA, is ATP-dependent and catalyzed by ATP-citrate lyase, or citryl-CoA synthetase and citryl-CoA lyase. Alternatively, citrate lyase can be coupled to acetyl-CoA synthetase, an acetyl-CoA transferase, or phosphotransacetylase and acetate kinase to form acetyl-CoA and oxaloacetate from citrate. The conversion of succinate to fumarate is catalyzed by succinate dehydrogenase while the reverse reaction is catalyzed by fumarate reductase. In the TCA cycle succinyl-CoA is formed from the NAD(P)⁺ dependent decarboxylation of alpha-ketoglutarate by the alpha-ketoglutarate dehydrogenase complex. The reverse reaction is catalyzed by alpha-ketoglutarate:ferredoxin oxidoreductase.

An organism capable of utilizing the reverse tricarboxylic acid cycle to enable production of acetyl-CoA-derived products on 1) CO, 2) CO₂ and H₂, 3) CO and CO₂, 4) synthesis gas comprising CO and H₂, and 5) synthesis gas or other gaseous carbon sources comprising CO, CO₂, and H₂ can include any of the following enzyme activities: ATP-citratc lyase, citrate lyase, aconitasc, isocitratc dehydrogenase, alpha-ketoglutarate:ferredoxin oxidoreductase, succinyl-CoA synthetase, succinyl-CoA transferase, fumarate reductase, fumarase, malate dehydrogenase, acetate kinase, phosphotransacetylase, acetyl-CoA synthetase, acetyl-CoA transferase, pyruvate:ferredoxin oxidoreductase, NAD(P)H:ferredoxin oxidoreductase, carbon monoxide dehydrogenase, hydrogenase, and ferredoxin (see Figure 9). Enzymes and the corresponding genes required for these activities are described herein above.

Carbon from syngas or other gaseous carbon sources can be fixed via the reverse TCA cycle and components thereof. Specifically, the combination of certain carbon gas-utilization pathway components with the pathways for formation of 2,4-pentadienoate, butadiene, propylene, 1,3-butanediol, crotyl alcohol or 3-buten-1-ol from acetyl-CoA results in high yields of these products by providing an efficient mechanism for fixing the carbon present in carbon dioxide, fed exogenously or produced endogenously from CO, into acetyl-CoA.

In some embodiments, a 2,4-pentadienoate, butadiene, propylene, 1,3-butanediol, crotyl alcohol or 3-buten-1-ol pathway in a non-naturally occurring microbial organism of the invention can utilize any combination of (1) CO, (2) CO₂, (3) H₂, or mixtures thereof to enhance the yields of biosynthetic steps involving reduction, including addition to driving the reductive TCA cycle.

In some embodiments a non-naturally occurring microbial organism having an 2,4-pentadienoate, butadiene, propylene, 1,3-butanediol, crotyl alcohol or 3-buten-1-ol pathway includes at least one exogenous nucleic acid encoding a reductive TCA pathway enzyme. The at least one exogenous nucleic acid is selected from an ATP-citrate lyase, citrate lyase, a fumarate reductase, isocitrate dehydrogenase, aconitase, and an alpha-ketoglutarate:ferredoxin oxidoreductase; and at least one exogenous enzyme selected from a carbon monoxide dehydrogenase, a hydrogenase, a NAD(P)H:ferredoxin oxidoreductase, and a ferredoxin, expressed in a sufficient amount to allow the utilization of (1) CO, (2) CO₂, (3) H₂, (4) CO₂ and H₂, (5) CO and CO₂, (6) CO and H₂, or (7) CO, CO₂, and H₂.

In some embodiments a method includes culturing a non-naturally occurring microbial organism having a 2,4-pentadienoate, butadiene, propylene, 1,3-butanediol, crotyl alcohol or 3-buten-1-ol pathway also comprising at least one exogenous nucleic acid encoding a reductive TCA pathway enzyme. The at least one exogenous nucleic acid is selected from an ATP-citrate lyase, citrate lyase, a fumarate reductase, isocitrate dehydrogenase, aconitase, and an alpha-ketoglutarate:ferredoxin oxidoreductase. Additionally, such an organism can also include at least one exogenous enzyme selected from a carbon monoxide dehydrogenase, a hydrogenase, a NAD(P)H:ferredoxin oxidoreductase, and a ferredoxin, expressed in a sufficient amount to allow the utilization of (1) CO, (2) CO₂, (3) H₂, (4) CO₂ and H₂, (5) CO and CO₂, (6) CO and H₂, or (7) CO, CO₂, and H₂ to produce a product.

In some embodiments a non-naturally occurring microbial organism having an 2,4-pentadienoate, butadiene, propylene, 1,3-butanediol, crotyl alcohol or 3-buten-1-ol pathway further includes at least one exogenous nucleic acid encoding a reductive TCA pathway enzyme expressed in a sufficient amount to enhance carbon flux through acetyl-CoA. The at least one exogenous nucleic acid is selected from an ATP-citrate lyase, citrate lyase, a fumarate reductase, a pyruvate:ferredoxin oxidoreductase, isocitrate dehydrogenase, aconitase and an alpha-ketoglutarate:ferredoxin oxidoreductase.

In some embodiments a non-naturally occurring microbial organism having an 2,4-pentadienoate, butadiene, propylene, 1,3-butanediol, crotyl alcohol or 3-buten-1-ol pathway includes at least one exogenous nucleic acid encoding an enzyme expressed in a sufficient amount to enhance the availability of reducing equivalents in the presence of carbon monoxide and/or hydrogen, thereby increasing the yield of redox-limited products via carbohydrate-based carbon feedstock. The at least one exogenous nucleic acid is selected from a carbon monoxide dehydrogenase, a hydrogenase, an NAD(P)H:ferredoxin oxidoreductase, and a ferredoxin. In some embodiments, the present invention provides a method for enhancing the availability of reducing equivalents in the presence of carbon monoxide or hydrogen thereby increasing the yield of redox-limited products via carbohydrate-based carbon feedstock, such as sugars or gaseous carbon sources, the method includes culturing this non-naturally occurring microbial organism under conditions and for a sufficient period of time to produce 2,4-pentadienoate, butadiene, propylene, 1,3-butanediol, crotyl alcohol or 3-buten-1-ol.

In some embodiments, the non-naturally occurring microbial organism having an 2,4-pentadienoate, butadiene, propylene, 1,3-butanediol, crotyl alcohol or 3-buten-1-ol pathway includes two exogenous nucleic acids, each encoding a reductive TCA pathway enzyme. In some embodiments, the non-naturally occurring microbial organism having an 2,4-pentadienoate, butadiene, propylene, 1,3-butanediol, crotyl alcohol or 3-buten-1-ol pathway includes three exogenous nucleic acids each encoding a reductive TCA pathway enzyme. In some embodiments, the non-naturally occurring microbial organism includes three exogenous nucleic acids encoding an ATP-citrate lyase, a fumarate reductase, and an alpha-ketoglutarate:ferredoxin oxidoreductase. In some embodiments, the non-naturally occurring microbial organism includes three exogenous nucleic acids encoding a citrate lyase, a fumarate reductase, and an alpha-ketoglutarate:ferredoxin oxidoreductase. In some embodiments, the non-naturally occurring microbial organism includes four exogenous nucleic acids encoding a pyruvate:ferredoxin oxidoreductase; a phosphoenolpyruvate carboxylase or a phosphoenolpyruvate carboxykinase, a CO dehydrogenase; and an H₂ hydrogenase. In some embodiments, the non-naturally occurring microbial organism includes two exogenous nucleic acids encoding a CO dehydrogenase and an H₂ hydrogenase.

In some embodiments, the non-naturally occurring microbial organisms having an 2,4-pentadienoate, butadiene, propylene, 1,3-butanediol, crotyl alcohol or 3-buten-1-ol pathway further include an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, and combinations thereof.

In some embodiments, the non-naturally occurring microbial organism having an 2,4-pentadienoate, butadiene, propylene, 1,3-butanediol, crotyl alcohol or 3-buten-1-ol pathway further includes an exogenous nucleic acid encoding an enzyme selected from carbon monoxide dehydrogenase, acetyl-CoA synthase, ferredoxin, NAD(P)H:ferredoxin oxidoreductase and combinations thereof.

In some embodiments, the non-naturally occurring microbial organism having an 2,4-pentadienoate, butadiene, propylene, 1,3-butanediol, crotyl alcohol or 3-buten-1-ol pathway utilizes a carbon feedstock selected from (1) CO, (2) CO₂, (3) CO₂ and H₂, (4) CO and H₂, or (5) CO, CO₂, and H₂. In some embodiments, the non-naturally occurring microbial organism having an 2,4-pentadienoate, butadiene, propylene, 1,3-butanediol, crotyl alcohol or 3-buten-1-ol pathway utilizes hydrogen for reducing equivalents. In some embodiments, the non-naturally occurring microbial organism having an 2,4-pentadienoate, butadiene, propylene, 1,3-butanediol, crotyl alcohol or 3-buten-1-ol pathway utilizes CO for reducing equivalents. In some embodiments, the non-naturally occurring microbial organism having an 2,4-pentadienoate, butadiene, propylene, 1,3-butanediol, crotyl alcohol or 3-buten-1-ol pathway utilizes combinations of CO and hydrogen for reducing equivalents.

In some embodiments, the non-naturally occurring microbial organism having an 2,4-pentadienoate, butadiene, propylene, 1,3-butanediol, crotyl alcohol or 3-buten-1-ol pathway further includes one or more nucleic acids encoding an enzyme selected from a phosphoenolpyruvate carboxylase, a phosphoenolpyruvate carboxykinase, a pyruvate carboxylase, and a malic enzyme.

In some embodiments, the non-naturally occurring microbial organism having an 2,4-pentadienoate, butadiene, propylene, 1,3-butanediol, crotyl alcohol or 3-buten-1-ol pathway further includes one or more nucleic acids encoding an enzyme selected from a malate dehydrogenase, a fumarase, a fumarate reductase, a succinyl-CoA synthetase, and a succinyl-CoA transferase.

In some embodiments, the non-naturally occurring microbial organism having an 2,4-pentadienoate, butadiene, propylene, 1,3-butanediol, crotyl alcohol or 3-buten-1-ol pathway further includes at least one exogenous nucleic acid encoding a citrate lyase, an ATP-citrate lyase, a citryl-CoA synthetase, a citryl-CoA lyase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, and a ferredoxin.

Accordingly, given the teachings and guidance provided herein, those skilled in the art will understand that a non-naturally occurring microbial organism can be produced that secretes the biosynthesized compounds of the invention when grown on a carbon source such as a carbohydrate. Such compounds include, for example, 2,4-pentadienoate, butadiene, propylene, 1,3-butanediol, crotyl alcohol or 3-buten-1-ol and any of the intermediate metabolites in the 2,4-pentadienoate, butadiene, propylene, 1,3-butanediol, crotyl alcohol or 3-buten-1-ol pathway. All that is required is to engineer in one or more of the required enzyme or protein activities to achieve biosynthesis of the desired compound or intermediate including, for example, inclusion of some or all of the 2,4-pentadienoate, butadiene, propylene, 1,3-butanediol, crotyl alcohol or 3-buten-1-ol biosynthetic pathways. Accordingly, the invention provides a non-naturally occurring microbial organism that produces and/or secretes 2,4-pentadienoate, butadiene, propylene, 1,3-butanediol, crotyl alcohol or 3-buten-1-ol when grown on a carbohydrate or other carbon source and produces and/or secretes any of the intermediate metabolites shown in the 2,4-pentadienoate, butadiene, propylene, 1,3-butanediol, crotyl alcohol or 3-buten-1-ol pathway when grown on a carbohydrate or other carbon source. The 2,4-pentadienoate, butadiene, propylene, 1,3-butanediol, crotyl alcohol or 3-buten-1-ol producing microbial organisms of the invention can initiate synthesis from an intermediate, for example, 5-aminopent-2-enoate, glutarate semialdehyde, 5-hydroxyvalerate, 5-hydroxyvaleryl-CoA, 5-hydroxypent-2-enoyl-CoA, 2,4-pentadienoyl-CoA, 5-hydroxypent-2-enoate, 5-hydroxypent-2-enoate, acetoacetyl-CoA, 3-hydroxybutyryl-CoA, crotoyl-CoA, glutaryl-CoA, 3-oxopentanoyl-CoA, 3-hydroxypentanoyl-CoA, pent-2-enoyl-CoA, pent-3-enoyl-CoA, 3-oxo-5-hydroxypentanoyl-CoA, 3-oxo-5-hydroxypentanoate, 3-oxobutanol, 4-hydroxy-2-oxovalerate, 2-oxopentenoate, 3-buten-1-al, 3-hydroxybutyryl-CoA, 3-hydroxybutyrate, 3-hydroxybutanal, 2,4-pentadienoyl-phosphate, or penta-2,4-dienal.

The non-naturally occurring microbial organisms of the invention are constructed using methods well known in the art as exemplified herein to exogenously express at least one nucleic acid encoding a 2,4-pentadienoate, butadiene, propylene, 1,3-butanediol, crotyl alcohol or 3-buten-1-ol pathway enzyme or protein in sufficient amounts to produce 2,4-pentadienoate, butadiene, propylene, 1,3-butanediol, crotyl alcohol or 3-buten-1-ol. It is understood that the microbial organisms of the invention are cultured under conditions sufficient to produce 2,4-pentadienoate, butadiene, propylene, 1,3-butanediol, crotyl alcohol or 3-buten-1-ol. Following the teachings and guidance provided herein, the non-naturally occurring microbial organisms of the invention can achieve biosynthesis of 2,4-pentadienoate, butadiene, propylene, 1,3-butanediol, crotyl alcohol or 3-buten-1-ol resulting in intracellular concentrations between about 0.1-200 mM or more. Generally, the intracellular concentration of 2,4-pentadienoate, butadiene, propylene, 1,3-butanediol, crotyl alcohol or 3-buten-1-ol is between about 3-150 mM, particularly between about 5-125 mM and more particularly between about 8-100 mM, including about 10 mM, 20 mM, 50 mM, 80 mM, or more. Intracellular concentrations between and above each of these exemplary ranges also can be achieved from the non-naturally occurring microbial organisms of the invention.

In some embodiments, culture conditions include anaerobic or substantially anaerobic growth or maintenance conditions. Exemplary anaerobic conditions have been described previously and are well known in the art. Exemplary anaerobic conditions for fermentation processes are described herein and are described, for example, in U.S. publication 2009/0047719, filed August 10, 2007. Any of these conditions can be employed with the non-naturally occurring microbial organisms as well as other anaerobic conditions well known in the art. Under such anaerobic or substantially anaerobic conditions, the 2,4-pentadienoate, butadiene, propylene, 1,3-butanediol, crotyl alcohol or 3-buten-1-ol producers can synthesize 2,4-pentadienoate, butadiene, propylene, 1,3-butanediol, crotyl alcohol or 3-buten-1-ol at intracellular concentrations of 5-10 mM or more as well as all other concentrations exemplified herein. It is understood that, even though the above description refers to intracellular concentrations, 2,4-pentadienoate, butadiene, propylene, 1,3-butanediol, crotyl alcohol or 3-buten-1-ol producing microbial organisms can produce 2,4-pentadienoate, butadiene, propylene, 1,3-butanediol, crotyl alcohol or 3-buten-1-ol intracellularly and/or secrete the product into the culture medium.

In addition to the culturing and fermentation conditions disclosed herein, growth condition for achieving biosynthesis of 2,4-pentadienoate, butadiene, propylene, 1,3-butanediol, crotyl alcohol or 3-buten-1-ol can include the addition of an osmoprotectant to the culturing conditions. In certain embodiments, the non-naturally occurring microbial organisms of the invention can be sustained, cultured or fermented as described herein in the presence of an osmoprotectant. Briefly, an osmoprotectant refers to a compound that acts as an osmolyte and helps a microbial organism as described herein survive osmotic stress. Osmoprotectants include, but are not limited to, betaines, amino acids, and the sugar trehalose. Non-limiting examples of such are glycine betaine, praline betaine, dimethylthetin, dimethylslfonioproprionate, 3-dimethylsulfonio-2-methylproprionate, pipecolic acid, dimethylsulfonioacetate, choline, L-carnitine and ectoine. In one aspect, the osmoprotectant is glycine betaine. It is understood to one of ordinary skill in the art that the amount and type of osmoprotectant suitable for protecting a microbial organism described herein from osmotic stress will depend on the microbial organism used. The amount of osmoprotectant in the culturing conditions can be, for example, no more than about 0.1 mM, no more than about 0.5 mM, no more than about 1.0 mM, no more than about 1.5 mM, no more than about 2.0 mM, no more than about 2.5 mM, no more than about 3.0 mM, no more than about 5.0 mM, no more than about 7.0 mM, no more than about 10mM, no more than about 50mM, no more than about 100mM or no more than about 500mM.

In some embodiments, the carbon feedstock and other cellular uptake sources such as phosphate, ammonia, sulfate, chloride and other halogens can be chosen to alter the isotopic distribution of the atoms present in 2,4-pentadienoate, butadiene, propylene, 1,3-butanediol, crotyl alcohol or 3-buten-1-ol or any 2,4-pentadienoate, butadiene, propylene, 1,3-butanediol, crotyl alcohol or 3-buten-1-ol pathway intermediate. The various carbon feedstock and other uptake sources enumerated above will be referred to herein, collectively, as "uptake sources." Uptake sources can provide isotopic enrichment for any atom present in the product 2,4-pentadienoate, butadiene, propylene, 1,3-butanediol, crotyl alcohol or 3-buten-1-ol or 2,4-pentadienoate, butadiene, propylene, 1,3-butanediol, crotyl alcohol or 3-buten-1-ol pathway intermediate, or for side products generated in reactions diverging away from a 2,4-pentadienoate, butadiene, propylene, 1,3-butanediol, crotyl alcohol or 3-buten-1-ol pathway. Isotopic enrichment can be achieved for any target atom including, for example, carbon, hydrogen, oxygen, nitrogen, sulfur, phosphorus, chloride or other halogens.

In some embodiments, the uptake sources can be selected to alter the carbon-12, carbon-13, and carbon-14 ratios. In some embodiments, the uptake sources can be selected to alter the oxygen-16, oxygen-17, and oxygen-18 ratios. In some embodiments, the uptake sources can be selected to alter the hydrogen, deuterium, and tritium ratios. In some embodiments, the uptake sources can be selected to alter the nitrogen-14 and nitrogen-15 ratios. In some embodiments, the uptake sources can be selected to alter the sulfur-32, sulfur-33, sulfur-34, and sulfur-35 ratios. In some embodiments, the uptake sources can be selected to alter the phosphorus-31, phosphorus-32, and phosphorus-33 ratios. In some embodiments, the uptake sources can be selected to alter the chlorine-35, chlorine-36, and chlorine-37 ratios.

In some embodiments, the isotopic ratio of a target atom can be varied to a desired ratio by selecting one or more uptake sources. An uptake source can be derived from a natural source, as found in nature, or from a man-made source, and one skilled in the art can select a natural source, a man-made source, or a combination thereof, to achieve a desired isotopic ratio of a target atom. An example of a man-made uptake source includes, for example, an uptake source that is at least partially derived from a chemical synthetic reaction. Such isotopically enriched uptake sources can be purchased commercially or prepared in the laboratory and/or optionally mixed with a natural source of the uptake source to achieve a desired isotopic ratio. In some embodiments, a target atom isotopic ratio of an uptake source can be achieved by selecting a desired origin of the uptake source as found in nature. For example, as discussed herein, a natural source can be a biobased derived from or synthesized by a biological organism or a source such as petroleum-based products or the atmosphere. In some such embodiments, a source of carbon, for example, can be selected from a fossil fuel-derived carbon source, which can be relatively depleted of carbon-14, or an environmental or atmospheric carbon source, such as CO₂, which can possess a larger amount of carbon-14 than its petroleum-derived counterpart.

The unstable carbon isotope carbon-14 or radiocarbon makes up for roughly 1 in 10¹² carbon atoms in the earth's atmosphere and has a half-life of about 5700 years. The stock of carbon is replenished in the upper atmosphere by a nuclear reaction involving cosmic rays and ordinary nitrogen (¹⁴N). Fossil fuels contain no carbon-14, as it decayed long ago. Burning of fossil fuels lowers the atmospheric carbon-14 fraction, the so-called "Suess effect".

Methods of determining the isotopic ratios of atoms in a compound are well known to those skilled in the art. Isotopic enrichment is readily assessed by mass spectrometry using techniques known in the art such as accelerated mass spectrometry (AMS), Stable Isotope Ratio Mass Spectrometry (SIRMS) and Site-Specific Natural Isotopic Fractionation by Nuclear Magnetic Resonance (SNIF-NMR). Such mass spectral techniques can be integrated with separation techniques such as liquid chromatography (LC), high performance liquid chromatography (HPLC) and/or gas chromatography, and the like.

In the case of carbon, ASTM D6866 was developed in the United States as a standardized analytical method for determining the biobased content of solid, liquid, and gaseous samples using radiocarbon dating by the American Society for Testing and Materials (ASTM) International. The standard is based on the use of radiocarbon dating for the determination of a product's biobased content. ASTM D6866 was first published in 2004, and the current active version of the standard is ASTM D6866-11 (effective April 1, 2011). Radiocarbon dating techniques are well known to those skilled in the art, including those described herein.

The biobased content of a compound is estimated by the ratio of carbon-14 (¹⁴C) to carbon-12 (¹²C). Specifically, the Fraction Modern (Fm) is computed from the expression: Fm = (S-B)/(M-B), where B, S and M represent the ¹⁴C/¹²C ratios of the blank, the sample and the modern reference, respectively. Fraction Modern is a measurement of the deviation of the ¹⁴C/¹²C ratio of a sample from "Modern." Modern is defined as 95% of the radiocarbon concentration (in AD 1950) of National Bureau of Standards (NBS) Oxalic Acid 1 (i.e., standard reference materials (SRM) 4990b) normalized to δ¹³C_{VPDB}=-19 per mil (Olsson, The use of Oxalic acid as a Standard. in, Radiocarbon Variations and Absolute Chronology, Nobel Symposium, 12th Proc., John Wiley & Sons, New York (1970)). Mass spectrometry results, for example, measured by ASM, are calculated using the internationally agreed upon definition of 0.95 times the specific activity of NBS Oxalic Acid I (SRM 4990b) normalized to δ¹³C_{VPDB}=-19 per mil. This is equivalent to an absolute (AD 1950) ¹⁴C/¹²C ratio of 1.176 ± 0.010 x 10⁻¹² (Karlen et al., Arkiv Geofysik, 4:465-471 (1968)). The standard calculations take into account the differential uptake of one istope with respect to another, for example, the preferential uptake in biological systems of C¹² over C¹³ over C¹⁴, and these corrections are reflected as a Fm corrected for δ¹³.

An oxalic acid standard (SRM 4990b or HOx 1) was made from a crop of 1955 sugar beet. Although there were 1000 lbs made, this oxalic acid standard is no longer commercially available. The Oxalic Acid II standard (HOx 2; N.I.S.T designation SRM 4990 C) was made from a crop of 1977 French beet molasses. In the early 1980's, a group of 12 laboratories measured the ratios of the two standards. The ratio of the activity of Oxalic acid II to 1 is 1.2933±0.001 (the weighted mean). The isotopic ratio of HOx II is -17.8 per mille. ASTM D6866-11 suggests use of the available Oxalic Acid II standard SRM 4990 C (Hox2) for the modern standard (see discussion of original vs. currently available oxalic acid standards in Mann, Radiocarbon, 25(2):519-527 (1983)). A Fm = 0% represents the entire lack of carbon-14 atoms in a material, thus indicating a fossil (for example, petroleum based) carbon source. A Fm = 100%, after correction for the post-1950 injection of carbon-14 into the atmosphere from nuclear bomb testing, indicates an entirely modern carbon source. As described herein, such a "modern" source includes biobased sources.

As described in ASTM D6866, the percent modern carbon (pMC) can be greater than 100% because of the continuing but diminishing effects of the 1950s nuclear testing programs, which resulted in a considerable enrichment of carbon-14 in the atmosphere as described in ASTM D6866-11. Because all sample carbon-14 activities are referenced to a "pre-bomb" standard, and because nearly all new biobased products are produced in a post-bomb environment, all pMC values (after correction for isotopic fraction) must be multiplied by 0.95 (as of 2010) to better reflect the true biobased content of the sample. A biobased content that is greater than 103% suggests that either an analytical error has occurred, or that the source of biobased carbon is more than several years old.

ASTM D6866 quantifies the biobased content relative to the material's total organic content and does not consider the inorganic carbon and other non-carbon containing substances present. For example, a product that is 50% starch-based material and 50% water would be considered to have a Biobased Content = 100% (50% organic content that is 100% biobased) based on ASTM D6866. In another example, a product that is 50% starch-based material, 25% petroleum-based, and 25% water would have a Biobased Content = 66.7% (75% organic content but only 50% of the product is biobased). In another example, a product that is 50% organic carbon and is a petroleum-based product would be considered to have a Biobased Content = 0% (50% organic carbon but from fossil sources). Thus, based on the well known methods and known standards for determining the biobased content of a compound or material, one skilled in the art can readily determine the biobased content and/or prepared downstream products that utilize of the invention having a desired biobased content.

Applications of carbon-14 dating techniques to quantify bio-based content of materials are known in the art (Currie et al., Nuclear Instruments and Methods in Physics Research B, 172:281-287 (2000)). For example, carbon-14 dating has been used to quantify bio-based content in terephthalate-containing materials (Colonna et al., Green Chemistry, 13:2543-2548 (2011)). Notably, polypropylene terephthalate (PPT) polymers derived from renewable 1,3-propanediol and petroleum-derived terephthalic acid resulted in Fm values near 30% (i.e., since 3/11 of the polymeric carbon derives from renewable 1,3-propanediol and 8/11 from the fossil end member terephthalic acid) (Currie et al., *supra,* 2000). In contrast, polybutylene terephthalate polymer derived from both renewable 1,4-butanediol and renewable terephthalic acid resulted in bio-based content exceeding 90% (Colonna et al., *supra,* 2011).

Accordingly, in some embodiments, the present invention provides 2,4-pentadienoate, butadiene, propylene, 1,3-butanediol, crotyl alcohol or 3-buten-1-ol or a 2,4-pentadienoate, butadiene, propylene, 1,3-butanediol, crotyl alcohol or 3-buten-1-ol pathway intermediate that has a carbon-12, carbon-13, and carbon-14 ratio that reflects an atmospheric carbon, also referred to as environmental carbon, uptake source. For example, in some aspects the 2,4-pentadienoate, butadiene, propylene, 1,3-butanediol, crotyl alcohol or 3-buten-1-ol or a 2,4-pentadienoate, butadiene, propylene, 1,3-butanediol, crotyl alcohol or 3-buten-1-ol pathway intermediate can have an Fm value of at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 98% or as much as 100%. In some such embodiments, the uptake source is CO₂. In some embodiments, the present invention provides 2,4-pentadienoate, butadiene, propylene, 1,3-butanediol, crotyl alcohol or 3-buten-1-ol or a 2,4-pentadienoate, butadiene, propylene, 1,3-butanediol, crotyl alcohol or 3-buten-1-ol intermediate that has a carbon-12, carbon-13, and carbon-14 ratio that reflects petroleum-based carbon uptake source. In this aspect, the 2,4-pentadienoate, butadiene, propylene, 1,3-butanediol, crotyl alcohol or 3-buten-1-ol or a 2,4-pentadienoate, butadiene, propylene, 1,3-butanediol, crotyl alcohol or 3-buten-1-ol pathway intermediate can have an Fm value of less than 95%, less than 90%, less than 85%, less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, less than 2% or less than 1%. In some embodiments, the present invention provides 2,4-pentadienoate, butadiene, propylene, 1,3-butanediol, crotyl alcohol or 3-buten-1-ol or a 2,4-pentadienoate, butadiene, propylene, 1,3-butanediol, crotyl alcohol or 3-buten-1-ol intermediate that has a carbon-12, carbon-13, and carbon-14 ratio that is obtained by a combination of an atmospheric carbon uptake source with a petroleum-based uptake source. Using such a combination of uptake sources is one way by which the carbon-12, carbon-13, and carbon-14 ratio can be varied, and the respective ratios would reflect the proportions of the uptake sources.

Further, the present invention relates to the biologically produced 2,4-pentadienoate, butadiene, propylene, 1,3-butanediol, crotyl alcohol or 3-buten-1-ol or 2,4-pentadienoate, butadiene, propylene, 1,3-butanediol, crotyl alcohol or 3-buten-1-ol pathway intermediate as disclosed herein, and to the products derived therefrom, wherein the 2,4-pentadienoate, butadiene, propylene, 1,3-butanediol, crotyl alcohol or 3-buten-1-ol or a 2,4-pentadienoate, butadiene, propylene, 1,3-butanediol, crotyl alcohol or 3-buten-1-ol pathway intermediate has a carbon-12, carbon-13, and carbon-14 isotope ratio of about the same value as the CO₂ that occurs in the environment. For example, in some aspects the invention provides: bioderived 2,4-pentadienoate, butadiene, propylene, 1,3-butanediol, crotyl alcohol or 3-buten-1-ol or a bioderived 2,4-pentadienoate, butadiene, propylene, 1,3-butanediol, crotyl alcohol or 3-buten-1-ol pathway intermediate having a carbon-12 versus carbon-13 versus carbon-14 isotope ratio of about the same value as the CO₂ that occurs in the environment, or any of the other ratios disclosed herein. It is understood, as disclosed herein, that a product can have a carbon-12 versus carbon-13 versus carbon-14 isotope ratio of about the same value as the CO₂ that occurs in the environment, or any of the ratios disclosed herein, wherein the product is generated from bioderived 2,4-pentadienoate, butadiene, propylene, 1,3-butanediol, crotyl alcohol or 3-buten-1-ol or a bioderived 2,4-pentadienoate, butadiene, propylene, 1,3-butanediol, crotyl alcohol or 3-buten-1-ol intermediate as disclosed herein, wherein the bioderived product is chemically modified to generate a final product. Methods of chemically modifying a bioderived product of 2,4-pentadienoate, butadiene, propylene, 1,3-butanediol, crotyl alcohol or 3-buten-1-ol, or an intermediate thereof, to generate a desired product are well known to those skilled in the art, as described herein. The invention further provides polyurethane, polymer, co-polymer, synthetic rubber, resin, chemical, polymer intermediate, organic solvent, hypoglycaemic agent, polyester resin, latex, monomer, fine chemical, agricultural chemical, pharmaceutical, or perfume having a carbon-12 versus carbon-13 versus carbon-14 isotope ratio of about the same value as the CO₂ that occurs in the environment, wherein the polyurethane, polymer, co-polymer, synthetic rubber, resin, chemical, polymer intermediate, organic solvent, hypoglycaemic agent, polyester resin, latex, monomer, fine chemical, agricultural chemical, pharmaceutical, or perfume is generated directly from or in combination with bioderived 2,4-pentadienoate, butadiene, propylene, 1,3-butanediol, crotyl alcohol or 3-buten-1-ol or a bioderived 2,4-pentadienoate, butadiene, propylene, 1,3-butanediol, crotyl alcohol or 3-buten-1-ol intermediate as disclosed herein.

2,4-Pentadienoate is a useful substituted butadiene derivative and a valuable intermediate en route to other substituted 1,3-butadiene derivatives, including, for example, 1-carbamoyl-1,3-butadienes. Non-limiting examples of applications of 2,4-pentadienoate include production of N-protected-1,3-butadiene derivatives that can be used in the preparation of anilines, a precursor to many inductrial chemicals, such as polyurethane and production of various polymers and co-polymers. Accordingly, in some embodiments, the invention provides a biobascd polyurethane, polymer or co-polymer comprising one or more bioderived 2,4-pentadienoate or bioderived 2,4-pentadienoate intermediate produced by a non-naturally occurring microorganism of the invention or produced using a method disclosed herein.

Butadiene is a chemical commonly used in many commercial and industrial applications. Non-limiting examples of such applications include production of polymers, such as synthetic rubbers and ABS resins, and chemicals, such as hexamethylenediamine and 1,4-butanediol. Accordingly, in some embodiments, the invention provides a biobased polymer, synthetic rubber, resin, or chemical comprising one or more bioderived butadiene or bioderived butadiene intermediate produced by a non-naturally occurring microorganism of the invention or produced using a method disclosed herein.

Propylene is a chemical commonly used in many commercial and industrial applications. Non-limiting examples of such applications include production of polymers, polymer intermediates and chemicals, such as polypropylene, acrylic acid, butanol, butanediol, acrylonitrile, propylene oxide, isopropanol and cumene. Moreover, these propylene derivatives, such as polypropylene, are used in the production of a wide range of products including plastics, such as injection moulding, and fibers, such as carpets. Accordingly, in some embodiments, the invention provides a biobased polymer, polymer intermediate, or chemical comprising one or more bioderived propylene or bioderived propylene intermediate produced by a non-naturally occurring microorganism of the invention or produced using a method disclosed herein.

1,3-Butanediol is a chemical commonly used in many commercial and industrial applications. Non-limiting examples of such applications include its use as an organic solvent for food flavoring agents or as a hypoglycaemic agent and its use in the production of polyurethane and polyester resins . Moreover, optically active 1,3-butanediol is also used in the synthesis of biologically active compounds and liquid crystals. Still further, 1,3-butanediol can be used in commercial production of 1,3-butadiene, a compound used in the manufacture of synthetic rubbers (e.g., tires), latex, and resins. Accordingly, in some embodiments, the invention provides a biobased organic solvent, hypoglycaemic agent, polyurethane, polyester resin, synthetic rubber, latex, or resin comprising one or more bioderived 1,3-butanediol or bioderived 1,3-butanediolintermediate produced by a non-naturally occurring microorganism of the invention or produced using a method disclosed herein.

Crotyl alcohol is a chemical commonly used in many commercial and industrial applications. Non-limiting examples of such applications include production of crotyl halides, esters, and ethers, which in turn are chemical are chemical intermediates in the production of monomers, fine chemicals, such as sorbic acid, trimethylhydroquinone, crotonic acid and 3-methoxybutanol, agricultural chemicals, and pharmaceuticals . Crotyl alcohol can also be used as a precursor in the production of 1,3-butadiene. Accordingly, in some embodiments, the invention provides a biobased monomer, fine chemical, agricultural chemical, or pharmaceutical comprising one or more bioderived crotyl alcohol or bioderived crotyl alcohol intermediate produced by a non-naturally occurring microorganism of the invention or produced using a method disclosed herein.

3-Buten-1-ol is a chemical commonly used in many commercial and industrial applications. Non-limiting examples of such applications include production of pharmaceuticals, agrochcmicals, perfumes and resins. Accordingly, in some embodiments, the invention provides a biobased pharmaceutical, agrochemical, perfume or resin comprising one or more bioderived 3-buten-1-ol or bioderived 3-buten-1-ol intermediate produced by a non-naturally occurring microorganism of the invention or produced using a method disclosed herein.

As used herein, the term "bioderived" means derived from or synthesized by a biological organism and can be considered a renewable resource since it can be generated by a biological organism. Such a biological organism, in particular the microbial organisms of the invention disclosed herein, can utilize feedstock or biomass, such as, sugars or carbohydrates obtained from an agricultural, plant, bacterial, or animal source. Alternatively, the biological organism can utilize atmospheric carbon. As used herein, the term "biobased" means a product as described above that is composed, in whole or in part, of a bioderived compound of the invention. A biobased or bioderived product is in contrast to a petroleum derived product, wherein such a product is derived from or synthesized from petroleum or a petrochemical feedstock.

In some embodiments, the invention provides polyurethane, polymer or co-polymer comprising bioderived 2,4-pentadienoate or bioderived 2,4-pentadienoate pathway intermediate, wherein the bioderived 2,4-pentadienoate or bioderived 2,4-pentadienoate pathway intermediate includes all or part of the 2,4-pentadienoate or 2,4-pentadienoate pathway intermediate used in the production of polyurethane, polymer or co-polymer. Thus, in some aspects, the invention provides a biobased polyurethane, polymer or co-polymer comprising at least 2%, at least 3%, at least 5%, at least 10%, at least 15%, at least 20%, at least 25%, at least 30%, at least 35%, at least 40%, at least 50%, at least 60%, at least 70%, at least 80%, at least 90%, at least 95%, at least 98% or 100% bioderived 2,4-pentadienoate or bioderived 2,4-pentadienoate pathway intermediate as disclosed herein. Additionally, in some aspects, the invention provides a biobased polyurethane, polymer or co-polymer wherein the 2,4-pentadienoate or 2,4-pentadienoate pathway intermediate used in its production is a combination of bioderived and petroleum derived 2,4-pentadienoate or 2,4-pentadienoate pathway intermediate. For example, a biobased polyurethane, polymer or co-polymer can be produced using 50% bioderived 2,4-pentadienoate and 50% petroleum derived 2,4-pentadienoate or other desired ratios such as 60%/40%, 70%/30%, 80%/20%, 90%/10%, 95%/5%, 100%/0%, 40%/60%, 30%/70%, 20%/80%, 10%/90% of bioderived/petroleum derived precursors, so long as at least a portion of the product comprises a bioderived product produced by the microbial organisms disclosed herein. It is understood that methods for producing polyurethane, polymer or co-polymer using the bioderived 2,4-pentadienoate or bioderived 2,4-pentadienoate pathway intermediate of the invention are well known in the art.

In some embodiments, the invention provides polymer, synthetic rubber, resin, or chemical comprising bioderived butadiene or bioderived butadiene pathway intermediate, wherein the bioderived butadiene or bioderived butadiene pathway intermediate includes all or part of the butadiene or butadiene pathway intermediate used in the production of polymer, synthetic rubber, resin, or chemical. Thus, in some aspects, the invention provides a biobased polymer, synthetic rubber, resin, or chemical comprising at least 2%, at least 3%, at least 5%, at least 10%, at least 15%, at least 20%, at least 25%, at least 30%, at least 35%, at least 40%, at least 50%, at least 60%, at least 70%, at least 80%, at least 90%, at least 95%, at least 98% or 100% bioderived butadiene or bioderived butadiene pathway intermediate as disclosed herein. Additionally, in some aspects, the invention provides a biobased polymer, synthetic rubber, resin, or chemical wherein the butadiene or butadiene pathway intermediate used in its production is a combination of bioderived and petroleum derived butadiene or butadiene pathway intermediate. For example, a biobased polymer, synthetic rubber, resin, or chemical can be produced using 50% bioderived butadiene and 50% petroleum derived butadiene or other desired ratios such as 60%/40%, 70%/30%, 80%/20%, 90%/10%, 95%/5%, 100%/0%, 40%/60%, 30%/70%, 20%/80%, 10%/90% of bioderived/petroleum derived precursors, so long as at least a portion of the product comprises a bioderived product produced by the microbial organisms disclosed herein. It is understood that methods for producing polymer, synthetic rubber, resin, or chemical using the bioderived butadiene or bioderived butadiene pathway intermediate of the invention are well known in the art.

In some embodiments, the invention provides polymer, polymer intermediate, or chemical comprising bioderived propylene or bioderived propylene pathway intermediate, wherein the bioderived propylene or bioderived propylene pathway intermediate includes all or part of the propylene or propylene pathway intermediate used in the production of polymer, polymer intermediate, or chemical. Thus, in some aspects, the invention provides a biobased polymer, polymer intermediate, or chemical comprising at least 2%, at least 3%, at least 5%, at least 10%, at least 15%, at least 20%, at least 25%, at least 30%, at least 35%, at least 40%, at least 50%, at least 60%, at least 70%, at least 80%, at least 90%, at least 95%, at least 98% or 100% bioderived propylene or bioderived propylene pathway intermediate as disclosed herein. Additionally, in some aspects, the invention provides a biobased polymer, polymer intermediate, or chemical wherein the propylene or propylene pathway intermediate used in its production is a combination of bioderived and petroleum derived propylene or propylene pathway intermediate. For example, a biobased polymer, polymer intermediate, or chemical can be produced using 50% bioderived propylene and 50% petroleum derived propylene or other desired ratios such as 60%/40%, 70%/30%, 80%/20%, 90%/10%, 95%/5%, 100%/0%, 40%/60%, 30%/70%, 20%/80%, 10%/90% of bioderived/petroleum derived precursors, so long as at least a portion of the product comprises a bioderived product produced by the microbial organisms disclosed herein. It is understood that methods for producing polymer, polymer intermediate, or chemical using the bioderived propylene or bioderived propylene pathway intermediate of the invention are well known in the art.

In some embodiments, the invention provides organic solvent, hypoglycaemic agent, polyurethane, polyester resin, synthetic rubber, latex, or resin comprising bioderived 1,3-butanediol or bioderived 1,3-butanediol pathway intermediate, wherein the bioderived 1,3-butanediol or bioderived 1,3-butanediol pathway intermediate includes all or part of the 1,3-butanediol or 1,3-butanediol pathway intermediate used in the production of organic solvent, hypoglycaemic agent, polyurethane, polyester resin, synthetic rubber, latex, or resin. Thus, in some aspects, the invention provides a biobased organic solvent, hypoglycaemic agent, polyurethane, polyester resin, synthetic rubber, latex, or resin comprising at least 2%, at least 3%, at least 5%, at least 10%, at least 15%, at least 20%, at least 25%, at least 30%, at least 35%, at least 40%, at least 50%, at least 60%, at least 70%, at least 80%, at least 90%, at least 95%, at least 98% or 100% bioderived 1,3-butanediol or bioderived 1,3-butanediol pathway intermediate as disclosed herein. Additionally, in some aspects, the invention provides a biobased organic solvent, hypoglycaemic agent, polyurethane, polyester resin, synthetic rubber, latex, or resin wherein the 1,3-butanediol or 1,3-butanediol pathway intermediate used in its production is a combination of bioderived and petroleum derived 1,3-butanediol or 1,3-butanediol pathway intermediate. For example, a biobased organic solvent, hypoglycaemic agent, polyurethane, polyester resin, synthetic rubber, latex, or resin can be produced using 50% bioderived 1,3-butanediol and 50% petroleum derived 1,3-butanediol or other desired ratios such as 60%/40%, 70%/30%, 80%/20%, 90%/10%, 95%/5%, 100%/0%, 40%/60%, 30%/70%, 20%/80%, 10%/90% of bioderived/petroleum derived precursors, so long as at least a portion of the product comprises a bioderived product produced by the microbial organisms disclosed herein. It is understood that methods for producing organic solvent, hypoglycaemic agent, polyurethane, polyester resin, synthetic rubber, latex, or resin using the bioderived 1,3-butanediol or bioderived 1,3-butanediol pathway intermediate of the invention are well known in the art.

In some embodiments, the invention provides monomer, fine chemical, agricultural chemical, or pharmaceutical comprising bioderived crotyl alcohol or bioderived crotyl alcohol pathway intermediate, wherein the bioderived crotyl alcohol or bioderived crotyl alcohol pathway intermediate includes all or part of the crotyl alcohol or crotyl alcohol pathway intermediate used in the production of monomer, fine chemical, agricultural chemical, or pharmaceutical. Thus, in some aspects, the invention provides a biobased monomer, fine chemical, agricultural chemical, or pharmaceutical comprising at least 2%, at least 3%, at least 5%, at least 10%, at least 15%, at least 20%, at least 25%, at least 30%, at least 35%, at least 40%, at least 50%, at least 60%, at least 70%, at least 80%, at least 90%, at least 95%, at least 98% or 100% bioderived crotyl alcohol or bioderived crotyl alcohol pathway intermediate as disclosed herein. Additionally, in some aspects, the invention provides a biobased monomer, fine chemical, agricultural chemical, or pharmaceutical wherein the crotyl alcohol or crotyl alcohol pathway intermediate used in its production is a combination of bioderived and petroleum derived crotyl alcohol or crotyl alcohol pathway intermediate. For example, a biobased monomer, fine chemical, agricultural chemical, or pharmaceutical can be produced using 50% bioderived crotyl alcohol and 50% petroleum derived crotyl alcohol or other desired ratios such as 60%/40%, 70%/30%, 80%/20%, 90%/10%, 95%/5%, 100%/0%, 40%/60%, 30%/70%, 20%/80%, 10%/90% of bioderived/petroleum derived precursors, so long as at least a portion of the product comprises a bioderived product produced by the microbial organisms disclosed herein. It is understood that methods for producing monomer, fine chemical, agricultural chemical, or pharmaceutical using the bioderived crotyl alcohol or bioderived crotyl alcohol pathway intermediate of the invention are well known in the art.

In some embodiments, the invention provides pharmaceutical, agrochemical, perfume, or resin comprising bioderived 3-buten-1-ol or bioderived 3-buten-1-ol pathway intermediate, wherein the bioderived 3-buten-1-ol or bioderived 3-buten-1-ol pathway intermediate includes all or part of the 3-buten-1-ol or 3-buten-1-ol pathway intermediate used in the production of pharmaceutical, agrochemical, perfume, or resin. Thus, in some aspects, the invention provides a biobased pharmaceutical, agrochemical, perfume, or resin comprising at least 2%, at least 3%, at least 5%, at least 10%, at least 15%, at least 20%, at least 25%, at least 30%, at least 35%, at least 40%, at least 50%, at least 60%, at least 70%, at least 80%, at least 90%, at least 95%, at least 98% or 100% bioderived 3-buten-1-ol or bioderived 3-buten-1-ol pathway intermediate as disclosed herein. Additionally, in some aspects, the invention provides a biobased pharmaceutical, agrochemical, perfume, or resin wherein the 3-buten-1-ol or 3-buten-1-ol pathway intermediate used in its production is a combination of bioderived and petroleum derived 3-buten-1-ol or 3-buten-1-ol pathway intermediate. For example, a biobased pharmaceutical, agrochemical, perfume, or resin can be produced using 50% bioderived 3-buten-1-ol and 50% petroleum derived 3-buten-1-ol or other desired ratios such as 60%/40%, 70%/30%, 80%/20%, 90%/10%, 95%/5%, 100%/0%, 40%/60%, 30%/70%, 20%/80%, 10%/90% of bioderived/petroleum derived precursors, so long as at least a portion of the product comprises a bioderived product produced by the microbial organisms disclosed herein. It is understood that methods for producing pharmaceutical, agrochemical, perfume, or resin using the bioderived 3-buten-1-ol or bioderived 3-buten-1-ol pathway intermediate of the invention are well known in the art.

The culture conditions can include, for example, liquid culture procedures as well as fermentation and other large scale culture procedures. As described herein, particularly useful yields of the biosynthetic products of the invention can be obtained under anaerobic or substantially anaerobic culture conditions.

As described herein, one exemplary growth condition for achieving biosynthesis of 2,4-pentadienoate, butadiene, propylene, 1,3-butanediol, crotyl alcohol or 3-buten-1-ol includes anaerobic culture or fermentation conditions. In certain embodiments, the non-naturally occurring microbial organisms of the invention can be sustained, cultured or fermented under anaerobic or substantially anaerobic conditions. Briefly, anaerobic conditions refers to an environment devoid of oxygen. Substantially anaerobic conditions include, for example, a culture, batch fermentation or continuous fermentation such that the dissolved oxygen concentration in the medium remains between 0 and 10% of saturation. Substantially anaerobic conditions also includes growing or resting cells in liquid medium or on solid agar inside a sealed chamber maintained with an atmosphere of less than 1% oxygen. The percent of oxygen can be maintained by, for example, sparging the culture with an N₂/CO₂ mixture or other suitable non-oxygen gas or gases.

The culture conditions described herein can be scaled up and grown continuously for manufacturing of 2,4-pentadienoate, butadiene, propylene, 1,3-butanediol, crotyl alcohol or 3-buten-1-ol.Exemplary growth procedures include, for example, fed-batch fermentation and batch separation; fed-batch fermentation and continuous separation, or continuous fermentation and continuous separation. All of these processes are well known in the art. Fermentation procedures are particularly useful for the biosynthetic production of commercial quantities of 2,4-pentadienoate, butadiene, propylene, 1,3-butanediol, crotyl alcohol or 3-buten-1-ol. Generally, and as with non-continuous culture procedures, the continuous and/or near-continuous production of 2,4-pentadienoate, butadiene, propylene, 1,3-butanediol, crotyl alcohol or 3-buten-1-ol will include culturing a non-naturally occurring 2,4-pentadienoate, butadiene, propylene, 1,3-butanediol, crotyl alcohol or 3-buten-1-ol producing organism of the invention in sufficient nutrients and medium to sustain and/or nearly sustain growth in an exponential phase. Continuous culture under such conditions can include, for example, growth for 1 day, 2, 3, 4, 5, 6 or 7 days or more. Additionally, continuous culture can include longer time periods of 1 week, 2, 3, 4 or 5 or more weeks and up to several months. Alternatively, organisms of the invention can be cultured for hours, if suitable for a particular application. It is to be understood that the continuous and/or near-continuous culture conditions also can include all time intervals in between these exemplary periods. It is further understood that the time of culturing the microbial organism of the invention is for a sufficient period of time to produce a sufficient amount of product for a desired purpose.

Fermentation procedures are well known in the art. Briefly, fermentation for the biosynthetic production of 2,4-pentadienoate, butadiene, propylene, 1,3-butanediol, crotyl alcohol or 3-buten-1-ol can be utilized in, for example, fed-batch fermentation and batch separation; fed-batch fermentation and continuous separation, or continuous fermentation and continuous separation. Examples of batch and continuous fermentation procedures are well known in the art.

In addition to the above fermentation procedures using the 2,4-pentadienoate, butadiene, propylene, 1,3-butanediol, crotyl alcohol or 3-buten-1-ol producers of the invention for continuous production of substantial quantities of 2,4-pentadienoate, butadiene, propylene, 1,3-butanediol, crotyl alcohol or 3-buten-1-ol, the 2,4-pentadienoate, butadiene, propylene, 1,3-butanediol, crotyl alcohol or 3-buten-1-ol producers also can be, for example, simultaneously subjected to chemical synthesis procedures to convert the product to other compounds or the product can be separated from the fermentation culture and sequentially subjected to chemical or enzymatic conversion to convert the product to other compounds, if desired.

To generate better producers, metabolic modeling can be utilized to optimize growth conditions. Modeling can also be used to design gene knockouts that additionally optimize utilization of the pathway (see, for example, U.S. patent publications US 2002/0012939, US 2003/0224363, US 2004/0029149, US 2004/0072723, US 2003/0059792, US 2002/0168654 and US 2004/0009466, and U.S. Patent No. 7,127,379). Modeling analysis allows reliable predictions of the effects on cell growth of shifting the metabolism towards more efficient production of 2,4-pentadienoate, butadiene, propylene, 1,3-butanediol, crotyl alcohol or 3-buten-1-ol.

One computational method for identifying and designing metabolic alterations favoring biosynthesis of a desired product is the OptKnock computational framework (Burgard et al., Biotechnol. Bioeng. 84:647-657 (2003)). OptKnock is a metabolic modeling and simulation program that suggests gene deletion or disruption strategies that result in genetically stable microorganisms which overproduce the target product. Specifically, the framework examines the complete metabolic and/or biochemical network of a microorganism in order to suggest genetic manipulations that force the desired biochemical to become an obligatory byproduct of cell growth. By coupling biochemical production with cell growth through strategically placed gene deletions or other functional gene disruption, the growth selection pressures imposed on the engineered strains after long periods of time in a bioreactor lead to improvements in performance as a result of the compulsory growth-coupled biochemical production. Lastly, when gene deletions are constructed there is a negligible possibility of the designed strains reverting to their wild-type states because the genes selected by OptKnock are to be completely removed from the genome. Therefore, this computational methodology can be used to either identify alternative pathways that lead to biosynthesis of a desired product or used in connection with the non-naturally occurring microbial organisms for further optimization of biosynthesis of a desired product.

Briefly, OptKnock is a term used herein to refer to a computational method and system for modeling cellular metabolism. The OptKnock program relates to a framework of models and methods that incorporate particular constraints into flux balance analysis (FBA) models. These constraints include, for example, qualitative kinetic information, qualitative regulatory information, and/or DNA microarray experimental data. OptKnock also computes solutions to various metabolic problems by, for example, tightening the flux boundaries derived through flux balance models and subsequently probing the performance limits of metabolic networks in the presence of gene additions or deletions. OptKnock computational framework allows the construction of model formulations that allow an effective query of the performance limits of metabolic networks and provides methods for solving the resulting mixed-integer linear programming problems. The metabolic modeling and simulation methods referred to herein as OptKnock are described in, for example, U.S. publication 2002/0168654, filed January 10, 2002, in International Patent No. PCT/US02/00660, filed January 10, 2002, and U.S. publication 2009/0047719, filed August 10, 2007.

Another computational method for identifying and designing metabolic alterations favoring biosynthetic production of a product is a metabolic modeling and simulation system termed SimPheny®. This computational method and system is described in, for example, U.S. publication 2003/0233218, filed June 14, 2002, and in International Patent Application No. PCT/US03/18838, filed June 13, 2003. SimPheny® is a computational system that can be used to produce a network model *in silico* and to simulate the flux of mass, energy or chargc through the chemical reactions of a biological system to define a solution space that contains any and all possible functionalities of the chemical reactions in the system, thereby determining a range of allowed activities for the biological system. This approach is referred to as constraints-bascd modeling because the solution space is defined by constraints such as the known stoichiometry of the included reactions as well as reaction thermodynamic and capacity constraints associated with maximum fluxes through reactions. The space defined by these constraints can be interrogated to determine the phenotypic capabilities and behavior of the biological system or of its biochemical components.

These computational approaches are consistent with biological realities because biological systems are flexible and can reach the same result in many different ways. Biological systems are designed through evolutionary mechanisms that have been restricted by fundamental constraints that all living systems must face. Therefore, constraints-based modeling strategy embraces these general realities. Further, the ability to continuously impose further restrictions on a network model via the tightening of constraints results in a reduction in the size of the solution space, thereby enhancing the precision with which physiological performance or phenotype can be predicted.

Given the teachings and guidance provided herein, those skilled in the art will be able to apply various computational frameworks for metabolic modeling and simulation to design and implement biosynthesis of a desired compound in host microbial organisms. Such metabolic modeling and simulation methods include, for example, the computational systems exemplified above as SimPheny® and OptKnock. For illustration of the invention, some methods are described herein with reference to the OptKnock computation framework for modeling and simulation. Those skilled in the art will know how to apply the identification, design and implementation of the metabolic alterations using OptKnock to any of such other metabolic modeling and simulation computational frameworks and methods well known in the art.

The methods described above will provide one set of metabolic reactions to disrupt. Elimination of each reaction within the set or metabolic modification can result in a desired product as an obligatory product during the growth phase of the organism. Because the reactions are known, a solution to the bilevel OptKnock problem also will provide the associated gene or genes encoding one or more enzymes that catalyze each reaction within the set of reactions. Identification of a set of reactions and their corresponding genes encoding the enzymes participating in each reaction is generally an automated process, accomplished through correlation of the reactions with a reaction database having a relationship between enzymes and encoding genes.

Once identified, the set of reactions that are to be disrupted in order to achieve production of a desired product are implemented in the target cell or organism by functional disruption of at least one gene encoding each metabolic reaction within the set. One particularly useful means to achieve functional disruption of the reaction set is by deletion of each encoding gene. However, in some instances, it can be beneficial to disrupt the reaction by other genetic aberrations including, for example, mutation, deletion of regulatory regions such as promoters or cis binding sites for regulatory factors, or by truncation of the coding sequence at any of a number of locations. These latter aberrations, resulting in less than total deletion of the gene set can be useful, for example, when rapid assessments of the coupling of a product are desired or when genetic reversion is less likely to occur.

To identify additional productive solutions to the above described bilevel OptKnock problem which lead to further sets of reactions to disrupt or metabolic modifications that can result in the biosynthesis, including growth-coupled biosynthesis of a desired product, an optimization method, termed integer cuts, can be implemented. This method proceeds by iteratively solving the OptKnock problem exemplified above with the incorporation of an additional constraint referred to as an integer cut at each iteration. Integer cut constraints effectively prevent the solution procedure from choosing the exact same set of reactions identified in any previous iteration that obligatorily couples product biosynthesis to growth. For example, if a previously identified growth-coupled metabolic modification specifies reactions 1, 2, and 3 for disruption, then the following constraint prevents the same reactions from being simultaneously considered in subsequent solutions. The integer cut method is well known in the art and can be found described in, for example, Burgard et al., Biotechnol. Prog. 17:791-797 (2001). As with all methods described herein with reference to their use in combination with the OptKnock computational framework for metabolic modeling and simulation, the integer cut method of reducing redundancy in iterative computational analysis also can be applied with other computational frameworks well known in the art including, for example, SimPheny®.

The methods exemplified herein allow the construction of cells and organisms that biosynthetically produce a desired product, including the obligatory coupling of production of a target biochemical product to growth of the cell or organism engineered to harbor the identified genetic alterations. Therefore, the computational methods described herein allow the identification and implementation of metabolic modifications that are identified by an *in silico* method selected from OptKnock or SimPheny®. The set of metabolic modifications can include, for example, addition of one or more biosynthetic pathway enzymes and/or functional disruption of one or more metabolic reactions including, for example, disruption by gene deletion.

As discussed above, the OptKnock methodology was developed on the premise that mutant microbial networks can be evolved towards their computationally predicted maximum-growth phenotypes when subjected to long periods of growth selection. In other words, the approach leverages an organism's ability to self-optimize under selective pressures. The OptKnock framework allows for the exhaustive enumeration of gene deletion combinations that force a coupling between biochemical production and cell growth based on network stoichiometry. The identification of optimal gene/reaction knockouts requires the solution of a bilevel optimization problem that chooses the set of active reactions such that an optimal growth solution for the resulting network overproduces the biochemical of interest (Burgard et al., Biotechnol. Bioeng. 84:647-657 (2003)).

An *in silico* stoichiometric model of *E. coli* metabolism can be employed to identify essential genes for metabolic pathways as exemplified previously and described in, for example, U.S. patent publications US 2002/0012939, US 2003/0224363, US 2004/0029149, US 2004/0072723, US 2003/0059792, US 2002/0168654 and US 2004/0009466, and in U.S. Patent No. 7,127,379. As disclosed herein, the OptKnock mathematical framework can be applied to pinpoint gene deletions leading to the growth-coupled production of a desired product. Further, the solution of the bilevel OptKnock problem provides only one set of deletions. To enumerate all meaningful solutions, that is, all sets of knockouts leading to growth-coupled production formation, an optimization technique, termed integer cuts, can be implemented. This entails iteratively solving the OptKnock problem with the incorporation of an additional constraint referred to as an integer cut at each iteration, as discussed above.

As disclosed herein, a nucleic acid encoding a desired activity of a 2,4-pentadienoate, butadiene, propylene, 1,3-butanediol, crotyl alcohol or 3-buten-1-ol pathway can be introduced into a host organism. In some cases, it can be desirable to modify an activity of a 2,4-pentadienoate, butadiene, propylene, 1,3-butanediol, crotyl alcohol or 3-buten-1-ol pathway enzyme or protein to increase production of 2,4-pentadienoate, butadiene, propylene, 1,3-butanediol, crotyl alcohol or 3-buten-1-ol.For example, known mutations that increase the activity of a protein or enzyme can be introduced into an encoding nucleic acid molecule. Additionally, optimization methods can be applied to increase the activity of an enzyme or protein and/or decrease an inhibitory activity, for example, decrease the activity of a negative regulator.

One such optimization method is directed evolution. Directed evolution is a powerful approach that involves the introduction of mutations targeted to a specific gene in order to improve and/or alter the properties of an enzyme. Improved and/or altered enzymes can be identified through the development and implementation of sensitive high-throughput screening assays that allow the automated screening of many enzyme variants (for example, >10⁴). Iterative rounds of mutagenesis and screening typically are performed to afford an enzyme with optimized properties. Computational algorithms that can help to identify areas of the gene for mutagenesis also have been developed and can significantly reduce the number of enzyme variants that need to be generated and screened. Numerous directed evolution technologies have been developed (for reviews, see Hibbert et al., Biomol.Eng 22:11-19 (2005); Huisman and Lalonde, In Biocatalysis in the pharmaceutical and biotechnology industries pgs. 717-742 (2007), Patel (ed.), CRC Press; Otten and Quax. Biomol.Eng 22:1-9 (2005).; and Sen et al., Appl Biochem.Biotechnol 143:212-223 (2007)) to be effective at creating diverse variant libraries, and these methods have been successfully applied to the improvement of a wide range of properties across many enzyme classes. Enzyme characteristics that have been improved and/or altered by directed evolution technologies include, for example: selectivity/specificity, for conversion of non-natural substrates; temperature stability, for robust high temperature processing; pH stability, for bioprocessing under lower or higher pH conditions; substrate or product tolerance, so that high product titers can be achieved; binding (Kₘ), including broadening substrate binding to include non-natural substrates; inhibition (Kᵢ), to remove inhibition by products, substrates, or key intermediates; activity (kcat), to increases enzymatic reaction rates to achieve desired flux; expression levels, to increase protein yields and overall pathway flux; oxygen stability, for operation of air sensitive enzymes under aerobic conditions; and anaerobic activity, for operation of an aerobic enzyme in the absence of oxygen.

A number of exemplary methods have been developed for the mutagenesis and diversification of genes to target desired properties of specific enzymes. Such methods arc well known to those skilled in the art. Any of these can be used to alter and/or optimize the activity of a 2,4-pentadienoate, butadiene, propylene, 1,3-butanediol, crotyl alcohol or 3-buten-1-ol pathway enzyme or protein. Such methods include, but are not limited to EpPCR, which introduces random point mutations by reducing the fidelity of DNA polymerase in PCR reactions (Pritchard et al., J Theor.Biol. 234:497-509 (2005)); Error-prone Rolling Circle Amplification (epRCA), which is similar to epPCR except a whole circular plasmid is used as the template and random 6-mers with exonuclease resistant thiophosphate linkages on the last 2 nucleotides are used to amplify the plasmid followed by transformation into cells in which the plasmid is re-circularized at tandem repeats (Fujii et al., Nucleic Acids Res. 32:e145 (2004); and Fujii et al., Nat. Protoc. 1:2493-2497 (2006)); DNA or Family Shuffling, which typically involves digestion of two or more variant genes with nucleases such as Dnase I or EndoV to generate a pool of random fragments that are reassembled by cycles of annealing and extension in the presence of DNA polymerase to create a library of chimeric genes (Stemmer, Proc Natl Acad Sci USA 91:10747-10751 (1994); and Stemmer, Nature 370:389-391 (1994)); Staggered Extension (StEP), which entails template priming followed by repeated cycles of 2 step PCR with denaturation and very short duration of annealing/extension (as short as 5 sec) (Zhao et al., Nat. Biotechnol. 16:258-261 (1998)); Random Priming Recombination (RPR), in which random sequence primers are used to generate many short DNA fragments complementary to different segments of the template (Shao et al., Nucleic Acids Res 26:681-683 (1998)).

Additional methods include Heteroduplex Recombination, in which linearized plasmid DNA is used to form heteroduplexes that are repaired by mismatch repair (Volkov et al, Nucleic Acids Res. 27:e18 (1999); and Volkov et al., Methods Enzymol. 328:456-463 (2000)); Random Chimeragenesis on Transient Templates (RACHITT), which employs Dnase I fragmentation and size fractionation of single stranded DNA (ssDNA) (Coco et al., Nat. Biotechnol. 19:354-359 (2001)); Recombined Extension on Truncated templates (RETT), which entails template switching of unidirectionally growing strands from primers in the presence of unidirectional ssDNA fragments used as a pool of templates (Lee et al., J. Molec. Catalysis 26:119-129 (2003)); Degenerate Oligonucleotide Gene Shuffling (DOGS), in which degenerate primers are used to control recombination between molecules; (Bcrgquist and Gibbs, Methods Mol.Biol 352:191-204 (2007); Bergquist et al., Biomol.Eng 22:63-72 (2005); Gibbs et al., Gene 271:13-20 (2001)); Incremental Truncation for the Creation of Hybrid Enzymes (ITCHY), which creates a combinatorial library with 1 base pair deletions of a gene or gene fragment of interest (Ostermeier et al., Proc. Natl. Acad. Sci. USA 96:3562-3567 (1999); and Ostermeier et al., Nat. Biotechnol. 17:1205-1209 (1999)); Thio-Incremental Truncation for the Creation of Hybrid Enzymes (THIO-ITCHY), which is similar to ITCHY except that phosphothioate dNTPs are used to generate truncations (Lutz et al., Nucleic Acids Res 29:E16 (2001)); SCRATCHY, which combines two methods for recombining genes, ITCHY and DNA shuffling (Lutz et al., Proc. Natl. Acad. Sci. USA 98:11248-11253 (2001)); Random Drift Mutagenesis (RNDM), in which mutations made via epPCR are followed by screening/selection for those retaining usable activity (Bergquist et al., Biomol. Eng. 22:63-72 (2005)); Sequence Saturation Mutagenesis (SeSaM), a random mutagenesis method that generates a pool of random length fragments using random incorporation of a phosphothioate nucleotide and cleavage, which is used as a template to extend in the presence of "universal" bases such as inosine, and replication of an inosine-containing complement gives random base incorporation and, consequently, mutagenesis (Wong et al., Biotechnol. J. 3:74-82 (2008); Wong et al., Nucleic Acids Res. 32:e26 (2004); and Wong et al., Anal. Biochem. 341:187-189 (2005)); Synthetic Shuffling, which uses overlapping oligonucleotides designed to encode "all genetic diversity in targets" and allows a very high diversity for the shuffled progeny (Ness et al., Nat. Biotechnol. 20:1251-1255 (2002)); Nucleotide Exchange and Excision Technology NexT, which exploits a combination of dUTP incorporation followed by treatment with uracil DNA glycosylase and then piperidine to perform endpoint DNA fragmentation (Muller et al., Nucleic Acids Res. 33:e117 (2005)).

Further methods include Sequence Homology-Independent Protein Recombination (SHIPREC), in which a linker is used to facilitate fusion between two distantly related or unrelated genes, and a range of chimeras is generated between the two genes, resulting in libraries of single-crossover hybrids (Sieber et al., Nat. Biotechnol. 19:456-460 (2001)); Gene Site Saturation Mutagenesis™ (GSSM™), in which the starting materials include a supercoiled double stranded DNA (dsDNA) plasmid containing an insert and two primers which are degenerate at the desired site of mutations (Kretz et al., Methods Enzymol. 388:3-11 (2004)); Combinatorial Cassette Mutagenesis (CCM), which involves the use of short oligonucleotide cassettes to replace limited regions with a large number of possible amino acid sequence alterations (Reidhaar-Olson et al. Methods Enzymol. 208:564-586 (1991); and Reidhaar-Olson et al. Science 241:53-57 (1988)); Combinatorial Multiple Cassette Mutagenesis (CMCM), which is essentially similar to CCM and uses epPCR at high mutation rate to identify hot spots and hot regions and then extension by CMCM to cover a defined region of protein sequence space (Reetz et al., Angew. Chem. Int. Ed Engl. 40:3589-3591 (2001)); the Mutator Strains technique, in which conditional *ts* mutator plasmids, utilizing the *mutD5* gene, which encodes a mutant subunit of DNA polymerase III, to allow increases of 20 to 4000-X in random and natural mutation frequency during selection and block accumulation of deleterious mutations when selection is not required (Selifonova et al., Appl. Environ. Microbiol. 67:3645-3649 (2001)); Low et al., J. Mol. Biol. 260:359-3680 (1996)).

Additional exemplary methods include Look-Through Mutagenesis (LTM), which is a multidimensional mutagenesis method that assesses and optimizes combinatorial mutations of selected amino acids (Rajpal et al., Proc. Natl. Acad. Sci. USA 102:8466-8471 (2005)); Gene Reassembly, which is a DNA shuffling method that can be applied to multiple genes at one time or to create a large library of chimeras (multiple mutations) of a single gene (Tunable GeneReassembly™ (TGR™) Technology supplied by Verenium Corporation), *in Silico* Protein Design Automation (PDA), which is an optimization algorithm that anchors the structurally defined protein backbone possessing a particular fold, and searches sequence space for amino acid substitutions that can stabilize the fold and overall protein energetics, and generally works most effectively on proteins with known three-dimensional structures (Hayes et al., Proc. Natl. Acad. Sci. USA 99:15926-15931 (2002)); and Iterative Saturation Mutagenesis (ISM), which involves using knowledge of structure/function to choose a likely site for enzyme improvement, performing saturation mutagenesis at chosen site using a mutagenesis method such as Stratagene QuikChange (Stratagene; San Diego CA), screening/selecting for desired properties, and, using improved clone(s), starting over at another site and continue repeating until a desired activity is achieved (Reetz et al., Nat. Protoc. 2:891-903 (2007); and Reetz et al., Angew. Chem. Int. Ed Engl. 45:7745-7751 (2006)).

Any of the aforementioned methods for mutagenesis can be used alone or in any combination. Additionally, any one or combination of the directed evolution methods can be used in conjunction with adaptive evolution techniques, as described herein.

It is understood that modifications which do not substantially affect the activity of the various embodiments of this invention are also provided within the definition of the invention provided herein. Accordingly, the following examples are intended to illustrate but not limit the present invention.

### EXAMPLE I

### Pathways for producing 2,4-pentadienoate, 3-buten-1-ol and butadiene from 2-aminopentanoate, 2-oxoadipate and glutaryl-CoA

Several routes to 2,4-pentadienoate, 3-buten-1-ol and butadiene, are depicted in Figure 1. Starting metabolites include 2-oxoadipate, glutaryl-CoA, and 5-aminopentanoate. These routes are catalyzed by one or more of the following enzymes: 2-aminoadipate decarboxylase, 5-aminopentanoate reductase, 5-aminopent-2-enoate aminotransferase, dehydrogenase or amine oxidase, 2-oxoadipate decarboxylase, glutarate semialdehyde reductase, 5-hydroxyvalerate dehydrogenase, 5-hydroxypent-2-enoate dehydratase, 2-aminoadipate aminotransferase, dehydrogenase or amine oxidase, 5-aminopentanoate aminotransferase, dehydrogenase or amine oxidase, 5-aminopent-2-enoate deaminase, 5-hydroxypent-2-enoate reductase, 5-hydroxyvaleryl-CoA transferase and/or synthetase, 5-hydroxypentanoyl-CoA dehydrogenase, 5-hydroxypent-2-enoyl-CoA dehydratase, 2,4-pentadienoyl-CoA transferase, synthetase or hydrolase, 5-hydroxypent-2-enoyl-CoA transferase or synthetase, 5-hydroxyvaleryl-CoA dehydratase/dehydrogenase, 2-oxoadipate dehydrogenase, 2-oxoadipate:ferridoxin oxidoreductase, 2-oxoadipate formate lyase, glutaryl-CoA reductase, 2,4-pentadienoate decarboxylase, 5-hydroxypent-2-enoate decarboxylase, 3-buten-1-ol dehydratase and 5-hydroxyvalerate decarboxylase.

Glutaryl-CoA is an intermediate in the degradation of numerous metabolites including benzoyl-CoA, lysine and tryptophan. Glutaryl-CoA can also be biosynthesized by means of, for example, the pathway shown in Figure 2. Glutaryl-CoA can be converted to 2,4-pentadienoate in five or more enzymatic steps. In the first step, glutaryl-CoA is reduced to glutarate semialdehyde by glutaryl-CoA reductase (step S). Further reduct ion to 5-hydroxyvalerte is catalyzed by an aldehyde reductase enzyme (step E). 5-Hydroxyvalerate is subsequently activated to 5-hydroxyvaleryl-CoA by a CoA transferase or synthetase in step L. The conversion of 5-hydroxyvaleryl-CoA to 2,4-pentadienoyl-CoA is catalyzed by a bifunctional enzyme with dehydratase and dehydrogenase activity (step Q). Alternately, the reaction is catalyzed in two steps by separate enzymes (step M, N). 2,4-Pentadienoate is formed by removal of the CoA moiety by a CoA transferase, synthetase or hydrolase (step O). 2,4-Pentadienoate or 2,4-pcntadicnoyl-CoA can be further converted to butadiene by a number of pathways shown in Figure 6. Alternate pathways for converting 5-hydroxyvalerate to 2,4-pentadienoate and butadiene are also shown. The 5-hydroxyvalcratc intermediate can also be converted to 3-buten-1-ol in one or more enzymatic steps. Direct conversion of 5-hydroxyvalerate to 3-buten-1-ol is catalyzed by an alkene-forming decarboxylase (step W). Indirect conversion entails oxidation of 5-hydroxyvalerate to 5-hydroxypent-2-enoate, followed by decarboxylation to 3-buten-1-ol (steps F and U). The 3-buten-1-ol can be isolated as a product, or further dehydrated to form butadiene. The dehydration proceeds via an enzymatic or catalytic reaction.

Another starting metabolite for the pathways shown in Figure 1 is 5-aminopentanoate. 5-Aminopentanoate is an intermediate formed during lysine, ornithine and proline degradation. An aminotransferase, dehydrogenase or amine oxidase is required to convert 5-aminopentanoate to glutarate semialdehyde. Glutarate semialdehyde is then converted to 2,4-pentadienoate, 3-buten-1-ol or butadiene as described above. Alternately, 5-aminopentanoate is oxidized to 5-aminopent-2-enoate by an enoic acid reductase (step B). Deamination of 5-aminopent-2-enoate yields 2,4-pentadienoate. In yet another embodiment, 5-aminopent-2-enoate is first converted to its corresponding aldehyde, 5-hydroxypent-2-enoate by an aminotransferase, dehydrogenase or amine oxidase. 5-Hydroxypent-2-enoate is then dehydrated to 2,4-pentadienoate directly (step G) or via a CoA intermediate (steps P, N, Q).

2-Aminoadipate and 2-oxoadipate (also called alpha-ketoadipate) are intermediates of lysine metabolism in organisms such as *Saccharomyces cerevisiae.* 2-Oxoadipate is also an intermediate of coenzyme B biosynethesis, where it is formed from alpha-ketoglutarate and acetyl-CoA by the enzymes homocitrate synthase, homoaconitase, and homoisocitrate dehydrogenase. 2-Oxoadipate and 2-aminoadipate are interconverted by aminotransferase, dehydrogenase or amine oxidase enzymes. Decarboxylation of 2-oxoadipate by a ketoacid decarboxylase yields glutarate semialdehyde (step D). Alternately, an acylating decarboxylase with alpha-ketoadipate dehydrogenase activity forms glutaryl-CoA from 2-oxoadipate (step R). Decarboxylation of 2-aminoadipate by an amino acid decarboxylase yields 5-aminopentanoate. Further transformation of the glutaryl-CoA, glutarate semialdehyde or 5-aminopentanoate intermediates to 2,4-pentadienoate, 3-buten-1-ol or butadiene proceeds as shown in Figure 1 and described previously.

Enzyme candidates for the reactions shown in Figure 1 are described in Example VII

### Example II.

### Pathway for producing glutaryl-CoA from acetyl-CoA

Figure 2 shows a carbon efficient pathway for converting two molecules of acetyl-CoA to glutaryl-CoA. In the first step, acetoacetyl-CoA is formed by the condensation of two molecules of acetyl-CoA by acetoacetyl-CoA thiolase, a beta-ketothiolase enzyme. Acetoacetyl-CoA can alternately be formed from malonyl-CoA and acetyl-CoA by acetoacetyl-CoA synthase. The 3-keto group of acetoacetyl-CoA is then reduced and dehydrated to form crotonyl-CoA. Glutaryl-CoA is formed from the reductive carboxylation of crotonyl-CoA. Enzymes and gene candidates for converting acetoacetyl-CoA to glutaryl-CoA are described in further detail in Example VII.

### Example III.

### Pathway for producing 2,4-pentadienoate from propionyl-CoA

This example describes a pathway for converting propionyl-CoA to 2,4-pentadienoate, shown in Figure 3. Enzymes include: 3-oxopentanoyl-CoA thiolase or synthase, 3-oxopentanoyl-CoA reductase, 3-hydroxypentanoyl-CoA dehydratase, pent-2-enoyl-CoA isomerase, pent-3-enoyl-CoA dehydrogenase, one or more of 2,4-pentadienoyl-CoA hydrolase, transferase or synthetase and pent-2-enoyl-CoA dehydrogenase.

Propionyl-CoA is formed as a metabolic intermediate in numerous biological pathways including the 3-hydroxypropionate/4-hydroxybutyrate and 3-hydroxypropionate cycles of CO₂ fixation, conversion of succinate or pyruvate to propionate, glyoxylate assimilation and amino acid degradation. In the pathways of Figure 3, propionyl-CoA is further converted to 2,4-pentadienoate. In the first step of the pathway, propionyl-CoA and acetyl-CoA are condensed to 3-oxopentanoyl-CoA by 3-oxopentanoyl-CoA thiolase. Alternately, propionyl-CoA and malonyl-CoA are condensed by an enzyme with 3-oxopentanoyl-CoA synthase activity. Alternately, the 3-oxopentanoyl-CoA intermediate can be formed in two steps by first converting propionyl-CoA and malonyl-ACP to 3-oxopentanoyl-ACP, then converting the ACP to the CoA. 3-Oxopentanoyl-CoA is then reduced to 3-hydroxypentanoyl-CoA, and subsequently dehydrated to pent-2-enoyl-CoA by a 3-oxoacyl-CoA reductase and 3-hydroxyacyl-CoA dehydratase, resepectively (steps B, C). A delta-isomerase shifts the double bond from the 2- to the 3- position, forming pent-3-enoyl-CoA, the substrate for pent-3-enoyl-CoA dehydrogenase (steps D and E). Together the enzymes catalyzing steps B, C, D and E participate in the reverse direction in 5-aminovalerate utilizing organisms such as *Clostridium aminovalericum.* Alternately the pent-2-enoyl-CoA intermediate is oxidized to 2,4-pentadienoyl-CoA by a pent-2-enoyl-CoA dehydrogenase. In the final step of the pathway, 2,4-pentadienoyl-CoA is converted to its corresponding acid by a CoA hydrolase, transferse or synthetase (step F). 2,4-Pentadiene can be isolated as a product, or 2,4-Pentadienoate or 2,4-pentadienoyl-CoA can be further converted to butadiene as depicted in Figure 6. Enzymes and gene candidates for converting propionyl-CoA to 2,4-pentadienoate are described in further detail in Example VII.

### Example IV.

### Pathway for synthesizing 1,3-butanediol from 3-hydroxypropionyl-CoA.

This example describes a pathway for converting 3-hydroxypropionyl-CoA to 1,3-butanediol, shown in Figure 4. Enzymes include: 3-oxo-5-hydroxypentanoyl-CoA thiolase or a 3-oxo-5-hydroxypentanoyl-CoA synthase, 3-oxo-5-hydroxypentanoate decarboxylase, 3-oxobutanol reductase and one or more of 3-oxo-5-hydroxypentanoyl-CoA hydrolase, transferase or synthetase.

3-Hydroxypropionyl-CoA is an intermediate of the 3-hydroxypropionate/4-hydroxybutyrate CO₂ fixation cycle of autotrophs and a related 3-hydroxypropionate cycle discovered in phototrophic bacteria (Berg et al, Science 318(5857):1782-6 (2007); Strauss and Fuchs, Eur J Biochem 215(3):633-43 (1993)). In the pathway to 1,3-butanediol, 3-hydroxypropionyl-CoA and acetyl-CoA are condensed by a 3-oxo-5-hydroxypentanoyl-CoA thiolase to form 3-oxo-5-hydroxypentanoyl-CoA (step A). Alternately, the 3-oxo-5-hydroxypentanoyl-CoA intermediate is formed from 3-HP-CoA and malonyl-CoA by a 3-oxo-5-hydroxypentanoyl-CoA synthase. Removal of the CoA moiety by a CoA synthetase, transferase or hydrolase yields 3-oxo-5-hydroxypentanoate (step B). Decarboxylation of 3-oxo-5-hydroxypentanoate to 3-oxobutanol is catalyzed by a ketoacid decarboxylase (step C). In the final step of the pathway 3-oxobutanol is reduced to 1,3-butanol by an alcohol dehydrogenase or ketone reductase. Enzymes and gene candidates are described in further detail in Example VII.

### Example V.

### Pathways for the formation of 1,3-butanediol, 3-buten-1-ol and butadiene from pyruvate and acetaldehyde.

This example describes pathways for converting pyruvate and acetaldehyde to 1,3-butanediol, 3-buten-1-ol and butadiene. The pathways are shown in Figure 5. Relevant enzymes include: 4-hydroxy-2-oxovalerate aldolase, 4-hydroxy-2-oxovalerate dehydratase, 2-oxopentenoate decarboxylase, 3-buten-1-al reductase, 3-buten-1-ol dehydratase, 4-hydroxy-2-oxovalerate decarboxylase, 3-hydroxybutanal reductase, 4-hydroxy-2-oxopentanoate dehydrogenase, 4-hydroxy-2-oxopentanoate:ferredoxin oxidoreductase, 3-hydroxybutyryl-CoA reductase (aldehyde forming), 3-hydroxybutyryl-CoA hydrolase, 3-hydroxybutyryl-CoA transferase or 3-hydroxybutyryl-CoA synthetase, 3-hydroxybutyrate reductase and 3-hydroxybutyryl-CoA reductase (alcohol forming). Step E can also be catalyzed via chemical dehydration.

The conversion of pyruvate and acetaldehyde to 3-buten-1-ol is accomplished in four enzymatic steps. Pyruvate and acetaldehyde are first condensed to 4-hydroxy-2-oxovalerate by 4-hydroxy-2-ketovalerate aldolase (Step A of Figure 5). The 4-hydroxy-2-oxovalerate product is subsequently dehydrated to 2-oxopentenoate (Step B of Figure 5). Decarboxylation of 2-oxopentenoate yields 3-buten-1-al (step C), which is further reduced to 3-buten-1-ol by an alcohol dehydrogenase (Step D). Further dehydration of the 3-buten-1-ol product to butadiene is performed by an enzyme or chemical catalyst.

The 4-hydroxy-2-oxovalerate intermediate can also be converted to 1,3-butanediol in two or more enzymatic steps. In one embodiment, 4-hydroxy-2-oxovalerate is decarboxylated to 3-hydroxybutanal (step F) and reduced to form 1,3-butanediol (step G). Alternately, 4-hydroxy-2-oxovalerate is converted to 3-hydroxybutyryl-CoA by an acylating and decarboxylating oxidoreductase or formate lyase (step H). The 3-hydroxybutyryl-CoA intermediate is further reduced to 3-hydroxybutanal in one or two enzymatic steps, by either an aldehyde-forming acyl-CoA reductase (step I) or the combined reaction of a 3-hydroxybutyryl-CoA hydrolase, transferase or synthetase and a 3-hydroxybutyrate reductase (steps J, K). 3-Hydroxybutanal is further reduced to 1,3-butanediol by 3-hydroxybutanal reductase (step G). In another embodiment, the 3-hydroxybutyryl-CoA intermediate is directly converted to 1,3-butanediol by an alcohol-forming bifunctional aldehyde/alcohol dehydrogenase (step L). Enzymes and gene candidates are described in further detail in Example VII.

### Example VI.

### Pathways for converting 2,4-pentadienoate or 2,4-pentadienoyl-CoA to butadiene.

Figures 1 and 3 show pathways for forming 2,4-pentadienoate or 2,4-pentadienoyl-CoA from common metabolic precursors. Figure 6 shows pathways for further converting 2,4-pcntadicnoatc or 2,4-pentadienoyl-CoA to butadiene. 2,4-Pentadienoate is converted to butadiene by several alternate pathways. One route is direct decarboxylation, shown in step G. Alternately, the acid moiety is reduced to an aldehyde by a carboxylic acid reductase enzyme (step A). Dccarbonylation of the pcnta-2,4-dicnal intermediate forms butadiene (step B). Steps H and E depict an alternate pathway wherein 2,4-pentadienoate is first activated to 2,4-pentadienoyl-phosphate by a kinase, and subsequently reduced to penta-2,4-dienal by a phosphate reductase. 2,4-Pentadienoate and 2,4-pentadienoyl-CoA are interconverted by a CoA transferase, hydrolase or synthetase. Reduction of 2,4-pentadienoyl-CoA to its corresponding aldehyde is catalyzed by an acylating aldehyde dehydrogenase (step C). Alternately, the CoA moiety is exchanged for a phosphate by a 2,4-pentadienoyl-CoA phosphotransferase (step D). The 2,4-pentadienoyl-phosphate or penta-2,4-dienal intermediates are further converted to butadiene as described previously. Enzymes and gene candidates for the reactions shown in Figure 6 are described in further detail in Example VII.

### Example VII.

### Enzyme candidates for the reactions shown in Figures 1-6

| **Label** | **Function** | **Step** |
|---|---|---|
| 1.1.1.a | Oxidoreductase (oxo to alcohol) | 1E,1K;2B; 3B; 4D;5D, 5G |
| 1.1.1.c | Oxidoreductase (acyl-CoA to alcohol) | 5L |
| 1.2.1.b | Oxidoreductase (acyl-CoA to aldehyde) | IS, 5I, 6C |
| 1.2.1.c | Oxidoreductase (2-oxo acid to acyl-CoA) | 1R, 5H |
| 1.2.1.d | Oxidoreductase (dephosphorylating) | 6E |
| 1.2.1.e | Oxidoreductase (acid to aldehyde) | 5K, 6A |
| 1.3.1.a | Oxidoreducatse (alkane to alkene) | 1B,1F,1M; 3E, 3G |
| 1.4.1.a | Oxidoreductase (amine to oxo) | 1C,1H,1I |
| 1.4.3.a | Amine oxidase | 1C,1H,1I |
| 2.3.1.a | Acyltransferase (transferring phosphate group to CoA; phosphotransacylase) | 6D |
| 2.3.1.b | Beta-ketothiolase | 2A,3A,4A |
| 2.3.1.d | Formate C-acyltransferase | 1R, 5H |
| 2.3.1.e | Synthase | 2A, 3A, 4A |
| 2.6.1.a | Aminotransferase | 1C, 1H,1I |
| 2.7.2.a | Phosphotransferase (kinase) | 6H |
| 2.8.3.a | CoA transferase | 1L,1P,1O; 3F; 4B; 5J; 6F |
| 3.1.2.a | CoA hydrolase | 1O; 3F; 4B; 5J; 6F |
| 4.1.1.a | Decarboxylase | 1A,1D,1T, 1U; 2D; 4C; 5C, 5F; 6G |
| 4.1.1.b | Decarboxylase, alkene forming | 1W |
| 4.1.99.a | Decarbonylase | 6B |
| 4.1.3.a | Lyase | 5A |
| 4.2.1.a | Hydro-lyase | 1G,1N, 1V; 2C, 3C; 5B, 5E |
| 4.3.1.a | Ammonia-lyase | 1J |
| 5.3.3.a | Delta-isomerase | 3D |
| 6.2.1.a | CoA synthetase | 1L,1P,1O; 3F; 4B; 5J; 6F |
| N/A | Bifunctional dehydratase/dehydrogenase | 1Q |

### 1.1.1.a Oxidoreductase (oxo to alcohol)

Several reactions shown in Figures 1-5 are catalyzed by alcohol dehydrogenase enzymes. These reactions include Steps E and K of Figure 1, Step B of Figure 2, Step B of Figure 3, Step D of Figure 4 and Steps D and G of Figure 5. Exemplary alcohol dehydrogenase enzymes are described in further detail below.

The reduction of glutarate semialdehyde to 5-hydroxyvalerate by glutarate semialdehyde reductase entails reduction of an aldehyde to its corresponding alcohol. Enzymes with glutarate semialdehyde reductase activity include the ATEG_00539 gene product of *Aspergillus terreus* and 4-hydroxybutyrate dehydrogenase of *Arabidopsis thaliana,* encoded by *4hbd* (WO 2010/068953A2). The *A. thaliana* enzyme was cloned and characterized in yeast (Breitkreuz et al., J.Biol.Chem. 278:41552-41556 (2003)).

| PROTEIN | GENBANK ID | GI NUMBER | ORGANISM |
|---|---|---|---|
| *ATEG_00539* | XP_001210625.1 | 115491995 | *Aspergillus terreus NIH2624* |
| *4hbd* | AAK94781.1 | 15375068 | *Arabidopsis thaliana* |

Additional genes encoding enzymes that catalyze the reduction of an aldehyde to alcohol (i.e., alcohol dehydrogenase or equivalently aldehyde reductase) include *alr A* encoding a medium-chain alcohol dehydrogenase for C2-C14 (Tani et al., Appl.Environ.Microbiol. 66:5231-5235 (2000)), *yqhD* and *fucO* from *E*. *coli* (Sulzenbacher et al., 342:489-502 (2004)), and *bdh* I and *bdh* II from *C. acetobutylicum* which converts butyryaldehyde into butanol (Walter et al., 174:7149-7158 (1992)). YqhD catalyzes the reduction of a wide range of aldehydes using NADPH as the cofactor, with a preference for chain lengths longer than C(3) (Sulzenbacher et al., 342:489-502 (2004);Perez et al., J Biol.Chem. 283:7346-7353 (2008)). The *adhA* gene product from *Zymomonas mobilisE* has been demonstrated to have activity on a number of aldehydes including formaldehyde, acetaldehyde, propionaldehyde, butyraldehyde, and acrolein (Kinoshita et al., Appl Microbiol Biotechnol 22:249-254 (1985)). Additional aldehyde reductase candidates are encoded by *bdh* in *C. saccharoperbutylacetonicum* and *Cbei_1722, Cbei_2181* and *Cbei_2421* in *C. Beijerinckii.* Additional aldehyde reductase gene candidates in *Saccharomyces cerevisiae* include the aldehyde reductases GRE3, ALD2-6 and HFD1, glyoxylate reductases GOR1 and YPL113C and glycerol dehydrogenase GCY1 (WO 2011/022651A1; Atsumi et al., Nature 451:86-89 (2008)). The enzyme candidates described previously for catalyzing the reduction of methylglyoxal to acetol or lactaldehyde are also suitable lactaldehyde reductase enzyme candidates.

| Protein | GENBANK ID | GI NUMBER | ORGANISM |
|---|---|---|---|
| *alrA* | BAB12273.1 | 9967138 | *Acinetobacter* sp. strain M-1 |
| *ADH2* | NP_014032.1 | 6323961 | *Saccharomyces cerevisiae* |
| *yqhD* | NP_417484.1 | 16130909 | *Escherichia coli* |
| *fucO* | NP_417279.1 | 16130706 | *Escherichia coli* |
| *bdh* I | NP_349892.1 | 15896543 | *Clostridium acetobutylicum* |
| *bdh* II | NP_349891.1 | 15896542 | *Clostridium acetobutylicum* |
| *adhA* | YP_162971.1 | 56552132 | *Zymomonas mobilis* |
| *bdh* | BAF45463.1 | 124221917 | *Clostridium saccharoperbutylacetonicum* |
| *Cbei_1722* | YP_001308850 | 150016596 | *Clostridium beijerinckii* |
| *Cbei_2181* | YP_001309304 | 150017050 | *Clostridium beijerinckii* |
| *Cbei_2421* | YP_001309535 | 150017281 | *Clostridium beijerinckii* |
| *GRE3* | P38715.1 | 731691 | *Saccharomyces cerevisiae* |
| *ALD2* | CAA89806.1 | 825575 | *Saccharomyces cerevisiae* |
| *ALD3* | NP_013892.1 | 6323821 | *Saccharomyces cerevisiae* |
| *ALD4* | NP_015019.1 | 6324950 | *Saccharomyces cerevisiae* |
| *ALD5* | NP_010996.2 | 330443526 | *Saccharomyces cerevisiae* |
| *ALD6* | ABX39192.1 | 160415767 | *Saccharomyces cerevisiae* |
| *HFD1* | Q04458.1 | 2494079 | *Saccharomyces cerevisiae* |
| *GOR1* | NP_014125.1 | 6324055 | *Saccharomyces cerevisiae* |
| *YPL113C* | AAB68248.1 | 1163100 | *Saccharomyces cerevisiae* |
| *GCY1* | CAA99318.1 | 1420317 | *Saccharomyces cerevisiae* |

Enzymes exhibiting 4-hydroxybutyrate dehydrogenase activity (EC 1.1.1.61) also fall into this category. Such enzymes have been characterized in *Ralstonia eutropha* (Bravo et al., J Forens Sci, 49:379-387 (2004)) and *Clostridium kluyveri* (Wolff ct al., Protein Expr.Purif. 6:206-212 (1995)). Yet another gene is the alcohol dehydrogenase *adhI* from *Geobacillus thermoglucosidasius* (Jeon et al., J Biotechnol 135:127-133 (2008)).

| PROTEIN | GENBANK ID | GI NUMBER | ORGANISM |
|---|---|---|---|
| *4hbd* | YP_726053.1 | 113867564 | *Ralstonia eutropha* H16 |
| *4hbd* | L21902.1 | 146348486 | *Clostridium kluyveri* DSM 555 |
| *adhI* | AAR91477.1 | 40795502 | *Geobacillus thermoglucosidasius* |

Another exemplary aldehyde reductase is methylmalonate semialdehyde reductase, also known as 3-hydroxyisobutyrate dehydrogenase (EC 1.1.1.31). This enzyme participates in valine, leucine and isoleucine degradation and has been identified in bacteria, eukaryotes, and mammals. The enzyme encoded by *P84067* from *Thermus thermophilus* HB8 has been structurally characterized (Lokanath et al., J Mol Biol, 352:905-17 (2005)). The reversibility of the human 3-hydroxyisobutyrate dehydrogenase was demonstrated using isotopically-labeled substrate (Manning et al., Biochem J, 231:481-4 (1985)). Additional genes encoding this enzyme include *3hidh* in *Homo sapiens* (Hawes et al., Methods Enzymol, 324:218-228 (2000)) and *Oryctolagus cuniculus* (Hawes ct al., *supra;* Chowdhury et al., Biosci.Biotechnol Biochem. 60:2043-2047 (1996)), *mmsB* in *Pseudomonas aeruginosa* and *Pseudomonas putida,* and *dhat* in *Pseudomonas putida* (Aberhart et al., J Chem.Soc.[Perkin 1] 6:1404-1406 (1979); Chowdhury et al., Biosci.Biotechnol Biochem. 60:2043-2047 (1996); Chowdhury et al., Biosci.Biotechnol Biochem. 67:438-441 (2003)). Several 3-hydroxyisobutyrate dehydrogenase enzymes have been characterized in the reductive direction, including *mmsB* from *Pseudomonas aeruginosa* (Gokarn et al., US Patent 739676, (2008)) and *mmsB* from *Pseudomonas putida.*

| PROTEIN | GENBANK ID | GI NUMBER | ORGANISM |
|---|---|---|---|
| *P84067* | P84067 | 75345323 | *Thermus thermophilus* |
| *3hidh* | P31937.2 | 12643395 | *Homo sapiens* |
| *3hidh* | P32185.1 | 416872 | *Oryctolagus cuniculus* |
| *mmsB* | NP_746775.1 | 26991350 | *Pseudomonas putida* |
| *mmsB* | P28811.1 | 127211 | *Pseudomonas aeruginosa* |
| *dhat* | Q59477.1 | 2842618 | *Pseudomonas putida* |

There exist several exemplary alcohol dehydrogenases that convert a ketone to a hydroxyl functional group. Two such enzymes from *E. coli* are encoded by malate dehydrogenase (*mdh*) and lactate dehydrogenase (*ldhA*)*.* In addition, lactate dehydrogenase from *Ralstonia eutropha* has been shown to demonstrate high activities on 2-ketoacids of various chain lengths includings lactate, 2-oxobutyrate, 2-oxopentanoate and 2-oxoglutarate (Steinbuchel et al., Eur.J.Biochem. 130:329-334 (1983)). Conversion of alpha-ketoadipate into alpha-hydroxyadipate can be catalyzed by 2-ketoadipate reductase, an enzyme reported to be found in rat and in human placenta (Suda et al., Arch.Biochem.Biophys. 176:610-620 (1976); Suda et al., Biochem.Biophys.Res.Commun. 77:586-591 (1977)). An additional oxidoreductase is the mitochondrial 3-hydroxybutyrate dehydrogenase (*bdh*) from the human heart which has been cloned and characterized (Marks et al., J.Biol.Chem. 267:15459-15463 (1992)). Alcohol dehydrogenase enzymes of *C. beijerinckii* (Ismaiel et al., J.Bacteriol. 175:5097-5105 (1993)) and *T. brockii* (Lamed et al., Biochem.J. 195:183-190 (1981); Peretz et al., Biochemistry. 28:6549-6555 (1989)) convert acetone to isopropanol. Methyl ethyl ketone reductase catalyzes the reduction of MEK to 2-butanol. Exemplary MEK reductase enzymes can be found in *Rhodococcus ruber* (Kosjek et al., Biotechnol Bioeng. 86:55-62 (2004)) and *Pyrococcus furiosus* (van der Oost et al., Eur.J.Biochem. 268:3062-3068 (2001)).

| Gene | GenBank Accession No. | GI No. | Organism |
|---|---|---|---|
| *mdh* | AAC76268.1 | 1789632 | *Escherichia coli* |
| *ldhA* | NP_415898.1 | 16129341 | *Escherichia coli* |
| *ldh* | YP_725182.1 | 113866693 | *Ralstonia eutropha* |
| *bdh* | AAA58352.1 | 177198 | *Homo sapiens* |
| *adh* | AAA23199.2 | 60592974 | *Clostridium beijerinckii* NRRL B593 |
| *adh* | P14941.1 | 113443 | *Thermoanaerobacter brockii* HTD4 |
| *sadh* | CAD36475 | 21615553 | *Rhodococcus ruber* |
| *adhA* | AAC25556 | 3288810 | *Pyrococcus furiosus* |

A number of organisms encode genes that catalyze the reduction of 3-oxobutanol to 1,3-butanediol, including those belonging to the genus *Bacillus, Brevibacterium, Candida,* and *Klebsiella* among others, as described by Matsuyama et al. J Mol Cat B Enz, 11:513-521 (2001). One of these enzymes, SADH from *Candida parapsilosis,* was cloned and characterized in *E. coli.* A mutated *Rhodococcus* phenylacetaldehyde reductase (Sar268) and a *Leifonia* alcohol dehydrogenase have also been shown to catalyze this transformation at high yields (Itoh et al., Appl.Microbiol Biotechnol. 75:1249-1256 (2007)).

| Gene | GenBank Accession No. | GI No. | Organism |
|---|---|---|---|
| *sadh* | BAA24528.1 | 2815409 | *Candida parapsilosis* |

Alcohol dehydrogenase enzymes that reduce 3-oxoacyl-CoA substrates to their corresponding 3-hydroxyacyl-CoA product are also relevant to the pathways depicted in Figure 2 (step B) and Figure 3 (step B). Exemplary enzymes include 3-oxoacyl-CoA reductase and acetoacetyl-CoA reductase. 3-Oxoacyl-CoA reductase enzymes (EC 1.1.1.35) convert 3-oxoacyl-CoA molecules into 3-hydroxyacyl-CoA molecules and are often involved in fatty acid beta-oxidation or phenylacetate catabolism. For example, subunits of two fatty acid oxidation complexes in *E. coli,* encoded by *fadB* and *fadJ,* function as 3-hydroxyacyl-CoA dehydrogenases (Binstock et al., Methods Enzymol. 71 Pt C:403-411 (1981)). Given the proximity in *E. coli* of *paaH* to other genes in the phenylacetate degradation operon (Nogalcs ct al., 153:357-365 (2007)) and the fact that *paaH* mutants cannot grow on phenylacetate (Ismail et al., Eur. J Biochem. 270:3047-3054 (2003)), it is expected that the *E. coli paaH* gene also encodes a 3-hydroxyacyl-CoA dehydrogenase. Additional 3-oxoacyl-CoA enzymes include the gene products of *phaC* in *Pseudomonas putida* (Olivera et al., Proc.Natl.Acad.Sci U.S.A 95:6419-6424 (1998)) and *paaC* in *Pseudomonas fluorescens* (Di et al., 188:117-125 (2007)). These enzymes catalyze the reversible oxidation of 3-hydroxyadipyl-CoA to 3-oxoadipyl-CoA during the catabolism of phenylacetate or styrene.

Acetoacetyl-CoA reductase (EC 1.1.1.36) catalyzes the reduction of acetoacetyl-CoA to 3-hydroxybutyryl-CoA. This enzyme participates in the acetyl-CoA fermentation pathway to butyrate in several species of *Clostridia* and has been studied in detail (Jones et al., Microbiol Rev. 50:484-524 (1986)). Acetoacetyl-CoA reducatse also participates in polyhydroxybutyrate biosynthesis in many organisms, and has also been used in metabolic engineering applications for overproducing PHB and 3-hydroxyisobutyrate (Liu et al., Appl. Microbiol. Biotechnol. 76:811-818 (2007); Qui et al., Appl. Microbiol. Biotechnol. 69:537-542 (2006)). The enzyme from *Clostridium acetobutylicum,* encoded by *hbd,* has been cloned and functionally expressed in *E. coli* (Youngleson et al., J Bacteriol. 171:6800-6807 (1989)). Additional gene candidates include *phbB* from *Zoogloea ramigera* (Ploux et al., Eur.J Biochem. 174:177-182 (1988)) and *phaB* from *Rhodobacter sphaeroides* (Alber et al., Mol.Microbiol 61:297-309 (2006)). The *Z*. *ramigera* gene is NADPH-dependent and the gene has been expressed in *E. coli* (Peoples et al., Mol.Microbiol 3:349-357 (1989)). Substrate specificity studies on the gene led to the conclusion that it could accept 3-oxopropionyl-CoA as a substrate besides acetoacetyl-CoA (Ploux et al., Eur.J Biochem. 174:177-182 (1988)). Additional genes include *phaB* in *Paracoccus denitrificans, Hbd1* (C-terminal domain) and *Hbd2* (N-terminal domain) in *Clostridium kluyveri* (Hillmer and Gottschalk, Biochim. Biophys. Acta 3334:12-23 (1974)) and *HSD17B10* in *Bos taurus* (Wakil et al., J Biol.Chem. 207:631-638 (1954)). The enzyme from *Paracoccus denitrificans* has been functionally expressed and characterized in *E. coli* (Yabutani et al., FEMS Microbiol Lett. 133:85-90 (1995)). A number of similar enzymes have been found in other species of *Clostridia* and in *Metallosphaera sedula* (Berg et al., Science. 318:1782-1786 (2007)). The enzyme from *Candida tropicalis* is a component of the peroxisomal fatty acid beta-oxidation multifunctional enzyme type 2 (MFE-2). The dehydrogenase B domain of this protein is catalytically active on acetoacetyl-CoA. The domain has been functionally expressed in *E. coli,* a crystal structure is available, and the catalytic mechanism is well-understood (Ylianttila et al., Biochem Biophys Res Commun 324:25-30 (2004); Ylianttila et al., J Mol Biol 358:1286-1295 (2006)).

| Protein | GENBANK ID | GI NUMBER | ORGANISM |
|---|---|---|---|
| *fadB* | P21177.2 | 119811 | *Escherichia coli* |
| *fadJ* | P77399.1 | 3334437 | *Escherichia coli* |
| *paaH* | NP_415913.1 | 16129356 | *Escherichia coli* |
| *Hhd2* | EDK34807.1 | 146348271 | *Clostridium kluyveri* |
| *Hbd1* | EDK32512.1 | 146345976 | *Clostridium kluyveri* |
| *phaC* | NP_745425.1 | 26990000 | *Pseudomonas putida* |
| *paaC* | ABF82235.1 | 106636095 | *Pseudomonas fluorescens* |
| *HSD17B10* | 002691.3 | 3183024 | *Bos taurus* |
| *phbB* | P23238.1 | 130017 | *Zoogloea ramigera* |
| *phaB* | YP_353825.1 | 77464321 | *Rhodobacter sphaeroides* |
| *phaB* | BAA08358 | 675524 | *Paracoccus denitrificans* |
| *Hbd* | NP_349314.1 | 15895965 | *Clostridium acetobutylicum* |
| *Hbd* | AAM14586.1 | 20162442 | *Clostridium beijerinckii* |
| *Msed_1423* | YP_001191505 | 146304189 | *Metallosphaera sedula* |
| *Msed_0399* | YP_001190500 | 146303184 | *Metallosphaera sedula* |
| *Msed_0389* | YP_001190490 | 146303174 | *Metallosphaera sedula* |
| *Msed_1993* | YP_001192057 | 146304741 | *Metallosphaera sedula* |
| *Fox2* | Q02207 | 399508 | *Candida tropicalis* |

### 1.1.1.c Oxidoreductase (acyl-CoA to alcohol)

Bifunctional oxidoreductases convert an acyl-CoA to its corresponding alcohol. Enzymes with this activity are required to convert 3-hydroxybutyryl-CoA to 1,3-butanediol (Step L of Figure 5).

Exemplary bifunctional oxidoreductases that convert an acyl-CoA to alcohol include those that transform substrates such as acetyl-CoA to ethanol (e.g., *adhE* from *E. coli* (Kessler et al., FEBS.Lett. 281:59-63 (1991))) and butyryl-CoA to butanol (e.g. *adhE2* from *C. acetobutylicum* (Fontaine et al., J.Bacteriol. 184:821-830 (2002))). The C. *acetobutylicum* enzymes encoded by *bdh* I and *bdh* II (Walter, et al., J. Bacteriol. 174:7149-7158 (1992)), reduce acetyl-CoA and butyryl-CoA to ethanol and butanol, respectively. In addition to reducing acetyl-CoA to ethanol, the enzyme encoded by *adhE* in *Leuconostoc mesenteroides* has been shown to oxide the branched chain compound isobutyraldehyde to isobutyryl-CoA (Kazahaya et al., J. Gen.Appl.Microbiol. 18:43-55 (1972); Koo et al., Biotechnol Lett, 27:505-510 (2005)). Another exemplary enzyme can convert malonyl-CoA to 3-HP. An NADPH-dependent enzyme with this activity has characterized in *Chloroflexus aurantiacus* where it participates in the 3-hydroxypropionate cycle (Hugler et al., J Bacteriol, 184:2404-2410 (2002); Strauss et al., Eur J Biochem, 215:633-643 (1993)). This enzyme, with a mass of 300 kDa, is highly substrate-specific and shows little sequence similarity to other known oxidoreductases (Hugler et al., *supra*). No enzymes in other organisms have been shown to catalyze this specific reaction; however there is bioinformatic evidence that other organisms may have similar pathways (Klatt et al., Env Microbiol, 9:2067-2078 (2007)). Enzyme candidates in other organisms including *Roseiflexus castenholzii, Erythrobacter sp. NAP1* and marine gamma proteobacterium HTCC2080 can be inferred by sequence similarity.

| **Protein** | **GenBank ID** | **GI Number** | **Organism** |
|---|---|---|---|
| *adhE* | NP_415757.1 | 16129202 | *Escherichia coli* |
| *adhE2* | AAK09379.1 | 12958626 | *Clostridium acetobutylicum* |
| *bdh* I | NP_349892.1 | 15896543 | *Clostridium acetobutylicum* |
| *bdh* II | NP_349891.1 | 15896542 | *Clostridium acetobutylicum* |
| *adhE* | AAV66076.1 | 55818563 | *Leuconostoc mesenteroides* |
| *mcr* | AAS20429.1 | 42561982 | *Chloroflexus aurantiacus* |
| *Rcas_2929* | YP_001433009.1 | 156742880 | *Roseiflexus castenholzii* |
| *NAP1_02720* | ZP_01039179.1 | 85708113 | *Erythrobacter sp. NAP1* |
| *MGP2080_00535* | ZP_01626393.1 | 119504313 | *marine gamma proteobacterium HTCC2080* |

Longer chain acyl-CoA molecules can be reduced to their corresponding alcohols by enzymes such as the jojoba (*Simmondsia chinensis*) *FAR* which encodes an alcohol-forming fatty acyl-CoA reductase. Its overexpression in *E. coli* resulted in FAR activity and the accumulation of fatty alcohol (Metz et al., Plant Physiol, 122:635-644 (2000)).

| **Protein** | **GenBank ID** | **GI Number** | **Organism** |
|---|---|---|---|
| FAR | AAD38039.1 | 5020215 | *Simmondsia chinensis* |

Another candidate for catalyzing these steps is 3-hydroxy-3-methylglutaryl-CoA reductase (or HMG-CoA reductase). This enzyme naturally reduces the CoA group in 3-hydroxy-3-methylglutaryl-CoA to an alcohol forming mevalonate. The *hmgA* gene of Sulfolobus *solfataricus,* encoding 3-hydroxy-3-methylglutaryl-CoA reductase, has been cloned, sequenced, and expressed in *E. coli* (Bochar et al., J Bacteriol. 179:3632-3638 (1997)). *S*. *cerevisiae* also has two HMG-CoA reductases in it (Basson et al., Proc.Natl.Acad.Sci. U.S.A 83:5563-5567 (1986)). The gene has also been isolated from *Arabidopsis thaliana* and has been shown to complement the HMG-COA reductase activity in *S*. *cerevisiae* (Learned et al., Proc.Natl.Acad.Sci. U.S.A 86:2779-2783 (1989)).

| ***Protein*** | ***GenBank ID*** | ***GI Number*** | ***Organism*** |
|---|---|---|---|
| *HMG1* | CAA86503.1 | 587536 | *Saccharomyces cerevisiae* |
| *HMG2* | NP_013555 | 6323483 | *Saccharomyces cerevisiae* |
| *HMG1* | CAA70691.1 | 1694976 | *Arabidopsis thaliana* |
| *hmgA* | AAC45370.1 | 2130564 | *Sulfolobus solfataricus* |

### 1.2.1.b Oxidoreductase (acyl-CoA to aldehyde)

Acyl-CoA reductases in the 1.2.1 family reduce an acyl-CoA to its corresponding aldehyde. Such a conversion is required to catalyze the reduction of glutaryl-CoA to glutarate semialdehyde (step S of Figure 1) and 3-hydroxybutyryl-CoA to 3-hydroxybutyraldehyde (step I of Figure 5). Several acyl-CoA reductase enzymes have been described in the open literature and represent suitable candidates for this step. These are described below.

Acyl-CoA reductases or acylating aldehyde dehydrogenases reduce an acyl-CoA to its corresponding aldehyde. Exemplary enzymes include fatty acyl-CoA reductase, succinyl-CoA reductase (EC 1.2.1.76), acetyl-CoA reductase, butyryl-CoA reductase and propionyl-CoA reductase (EC 1.2.1.3). Exemplary fatty acyl-CoA reductases enzymes are encoded by *acr1* of *Acinetobacter calcoaceticus* (Reiser, Journal of Bacteriology 179:2969-2975 (1997)) and *Acinetobacter sp. M-1* (Ishige et al., Appl. Environ. Microbiol. 68:1192-1195 (2002)). Enzymes with succinyl-CoA reductase activity are encoded by *sucD* of *Clostridium kluyveri* (Sohling, J. Bacteriol. 178:871-880 (1996)) *and sucD* of *P. gingivalis* (Takahashi, J. Bacteriol 182:4704-4710 (2000)). Additional succinyl-CoA reductase enzymes participate in the 3-hydroxypropionate/4-hydroxybutyrate cycle of thermophilic archaea including *Metallosphaera sedula* (Berg et al., Science 318:1782-1786 (2007)) and *Thermoproteus neutrophilia* (Ramos-Vera et al., J Bacteriol., 191:4286-4297 (2009)). The *M*. *sedula* enzyme, encoded by *Msed_0709,* is strictly NADPH-dependent and also has malonyl-CoA reductase activity. The *T. neutrophilus* enzyme is active with both NADPH and NADH. The enzyme acylating acetaldehyde dehydrogenase in *Pseudomonas sp,* encoded by *bphG,* is yet another as it has been demonstrated to oxidize and acylate acetaldehyde, propionaldehyde, butyraldehyde, isobutyraldehyde and formaldehyde (Powlowski, J. Bacteriol. 175:377-385 (1993)). In addition to reducing acctyl-CoA to cthanol, the enzyme encoded by *adhE* in *Leuconostoc mesenteroides* has been shown to oxidize the branched chain compound isobutyraldehyde to isobutyryl-CoA (Kazahaya, J. Gen. Appl. Microbiol. 18:43-55 (1972); and Koo et al., Biotechnol Lett. 27:505-510 (2005)). Butyraldehyde dehydrogenase catalyzes a similar reaction, conversion of butyryl-CoA to butyraldehyde, in solventogenic organisms such as *Clostridium saccharoperbutylacetonicum* (Kosaka et al., Biosci Biotechnol Biochem., 71:58-68 (2007)). Exemplary propionyl-CoA reductase enzymes include *pduP* of *Salmonella typhimurium LT2* (Leal, Arch. Microbiol. 180:353-361 (2003)) and *eutE* from *E. coli* (Skraly, WO Patent No. 2004/024876). The propionyl-CoA reductase of *Salmonella typhimurium LT2,* which naturally converts propionyl-CoA to propionaldehyde, also catalyzes the reduction of 5-hydroxyvaleryl-CoA to 5-hydroxypentanal (WO 2010/068953A2).

| Protein | GenBank ID | GI Number | Organism |
|---|---|---|---|
| *acr1* | YP_047869.1 | 50086359 | *Acinetobacter calcoaceticus* |
| *acr1* | AAC45217 | 1684886 | *Acinetobacter baylyi* |
| *acr1* | BAB85476.1 | 18857901 | *Acinetobacter sp. Strain M-1* |
| *MSED_0709* | YP_001190808.1 | 146303492 | *Metallosphaera sedula* |
| *Tneu_0421* | ACB39369.1 | 170934108 | *Thermoproteus neutrophilia* |
| *sucD* | P38947.1 | 172046062 | *Clostridium kluyveri* |
| *sucD* | NP_904963.1 | 34540484 | *Porphyromonas gingivalis* |
| *bphG* | BAA03892.1 | 425213 | *Pseudomonas sp* |
| *adhE* | AAV66076.1 | 55818563 | *Leuconostoc mesenteroides* |
| *bld* | AAP42563.1 | 31075383 | *Clostridium saccharoperbutylacetonicum* |
| *pduP* | NP_460996 | 16765381 | *Salmonella typhimurium LT2* |
| *eutE* | NP_416950 | 16130380 | *Escherichia coli* |

An additional enzyme that converts an acyl-CoA to its corresponding aldehyde is malonyl-CoA reductase which transforms malonyl-CoA to malonic semialdehyde. Malonyl-CoA reductase is a key enzyme in autotrophic carbon fixation via the 3-hydroxypropionate cycle in thermoacidophilic archaeal bacteria (Berg, Science 318:1782-1786 (2007); and Thauer, Science 318:1732-1733 (2007)). The enzyme utilizes NADPH as a cofactor and has been characterized in *Metallosphaera* and *Sulfolobus sp.* (Alber et al., J. Bacteriol. 188:8551-8559 (2006); and Hugler, J. Bacteriol. 184:2404-2410 (2002)). The enzyme is encoded by *Msed_0709* in *Metallosphaera sedula* (Alber et al., J. Bacteriol. 188:8551-8559 (2006); and Berg, Science 318:1782-1786 (2007)). A gene encoding a malonyl-CoA reductase from *Sulfolobus tokodaii* was cloned and heterologously expressed in *E. coli* (Alber et al., J. Bacteriol 188:8551-8559 (2006). This enzyme has also been shown to catalyze the conversion of methylmalonyl-CoA to its corresponding aldehyde (WO2007141208 (2007)). Although the aldehyde dehydrogenase functionality of these enzymes is similar to the bifunctional dehydrogenase from *Chloroflexus aurantiacus,* there is little sequence similarity. Both malonyl-CoA reductase enzyme candidates have high sequence similarity to aspartate-semialdehyde dehydrogenase, an enzyme catalyzing the reduction and concurrent dephosphorylation of aspartyl-4-phosphate to aspartate semialdehyde. Additional gene candidates can be found by sequence homology to proteins in other organisms including *Sulfolobus solfataricus* and *Sulfolobus acidocaldarius* and have been listed below. Yet another candidate for CoA-acylating aldehyde dehydrogenase is the *ald* gene from *Clostridium beijerinckii* (Toth, Appl. Environ. Microbiol. 65:4973-4980 (1999). This enzyme has been reported to reduce acetyl-CoA and butyryl-CoA to their corresponding aldehydes. This gene is very similar to *eutE* that encodes acetaldehyde dehydrogenase of *Salmonella typhimurium and E. coli* (Toth, Appl. Environ. Microbiol. 65:4973-4980 (1999).

| Protein | GenBank ID | GI Number | Organism |
|---|---|---|---|
| *Msed_0709* | YP_001190808.1 | 146303492 | *Metallosphaera sedula* |
| *Mcr* | NP_378167.1 | 15922498 | *Sulfolobus tokodaii* |
| *asd-2* | NP_343563.1 | 15898958 | *Sulfolobus solfataricus* |
| *Saci_2370* | YP_256941.1 | 70608071 | *Sulfolobus acidocaldarius* |
| *Ald* | AAT66436 | 49473535 | *Clostridium beijerinckii* |
| *eutE* | AAA80209 | 687645 | *Salmonella typhimurium* |
| *eutE* | P77445 | 2498347 | *Escherichia coli* |

### 1.2.1.c Oxidoreductase 2-oxoacid to acyl-CoA, decarboxylation

The reductive decarboxylation and acylation of 2-oxoadipate to glutarate semialdehyde (step D of Figure 1) is catalyzed by an oxidoreductase in EC class 1.2. A similar enzyme is required to convert 4-hydroxy-2-oxovalerate to 3-hydroxybutyryl-CoA (step H of Figure 5). Exemplary enzymes are found in the 2-ketoacid dehydrogenase and 2-ketoglutarate ferredoxin oxidoreductase (OFOR) families.

Alpha-ketoglutarate dehydrogenase (AKGD) converts alpha-ketoglutarate to succinyl-CoA and is the primary site of control of metabolic flux through the TCA cycle (Hansford, Curr. Top.Bioenerg. 10:217-278 (1980)). Encoded by genes *sucA, sucB* and *lpd* in *E. coli,* AKGD gene expression is downregulated under anaerobic conditions and during growth on glucose (Park et al., 15:473-482 (1995)). Although the substrate range of AKGD is narrow, structural studies of the catalytic core of the E2 component pinpoint specific residues responsible for substrate specificity (Knapp et al., J.Mol.Biol. 280:655-668 (1998)). The *Bacillus subtilis* AKGD, encoded by *odhAB* (E1 and E2) and *pdhD* (E3, shared domain), is regulated at the transcriptional level and is dependent on the carbon source and growth phase of the organism (Rcsnckov ct al., Mol. Gen. Genet. 234:285-296 (1992)). In yeast, the *LPD1* gene encoding the E3 component is regulated at the transcriptional level by glucose (Roy et al., J.Gen.Microbiol. 133:925-933 (1987)). The E1 component, encoded by *KGD1,* is also regulated by glucose and activated by the products of HAP2 and HAP3 (Repetto et al., Mol. Cell Biol. 9:2695-2705 (1989)). The AKGD enzyme complex, inhibited by products NADH and succinyl-CoA, is well-studied in mammalian systems, as impaired function of has been linked to several neurological diseases.

| *Gene* | GI # | Accession No. | *Organism* |
|---|---|---|---|
| *sucA* | 16128701 | NP_415254.1 | *Escherichia coli* |
| *sucB* | 16128702 | NP_415255.1 | *Escherichia coli* |
| *lpd* | 16128109 | NP_414658.1 | *Escherichia coli* |
| *odhA* | 51704265 | P23129.2 | *Bacillus subtilis* |
| *odhB* | 129041 | P16263.1 | *Bacillus subtilis* |
| *pdhD* | 118672 | P21880.1 | *Bacillus subtilis* |
| *KGD1* | 6322066 | NP_012141.1 | *Saccharomyces cerevisiae* |
| *KGD2* | 6320352 | NP_010432.1 | *Saccharomyces cerevisiae* |
| *LPD1* | 14318501 | NP_116635.1 | *Saccharomyces cerevisiae* |

Branched-chain 2-keto-acid dehydrogenase complex (BCKAD), also known as 2-oxoisovalerate dehydrogenase, participates in branched-chain amino acid degradation pathways, converting 2-keto acids derivatives of valine, leucine and isoleucine to their acyl-CoA derivatives and CO₂. The complex has been studied in many organisms including *Bacillus subtilis* (Wang et al., Eur.J.Biochem. 213:1091-1099 (1993)), *Rattus norvegicus* (Namba et al., J.Biol.Chem. 244:4437-4447 (1969)) and *Pseudomonas putida* (Sokatch et al., 148:647-652 (1981)). In *Bacillus subtilis* the enzyme is encoded by genes *pdhD* (E3 component), *bfmBB* (E2 component), *bfmBAA* and *bfmBAB* (E1 component) (Wang et al., EurJ.Biochem. 213:1091-1099 (1993)). In mammals, the complex is regulated by phosphorylation by specific phosphatases and protein kinases. The complex has been studied in rat hepatocites (Chicco et al., J.Biol.Chem. 269:19427-19434 (1994)) and is encoded by genes *Bckdha* (E1 alpha), *Bckdhb (*E1 beta), *Dbt (*E2*), and Dld (*E3). The E1 and E3 components of the *Pseudomonas putida* BCKAD complex have been crystallized (Aevarsson et al., Nat.Struct.Biol. 6:785-792 (1999); Mattevi et al., Science. 255:1544-1550 (1992)) and the enzyme complex has been studied (Sokatch et al., 148:647-652 (1981)). Transcription of the *P. putida* BCKAD genes is activated by the gene product of *bkdR* (Hester et al., 233:828-836 (1995)). In some organisms including *Rattus norvegicus* (Paxton et al., Biochem.J. 234:295-303 (1986)) and *Saccharomyces cerevisiae* (Sinclair et al., Biochem.Mol.Biol.Int. 31:911-922 (1993)), this complex has been shown to have a broad substrate range that includes linear oxo-acids such as 2-oxobutanoate and alpha-ketoglutarate, in addition to the branched-chain amino acid precursors. The active site of the bovine BCKAD was engineered to favor alternate substrate acetyl-CoA (Meng et al., Biochemistry. 33:12879-12885 (1994)).

| *Gene* | Accession No. | GI # | *Organism* |
|---|---|---|---|
| *bfmBB* | NP_390283.1 | 16079459 | *Bacillus subtilis* |
| *bfmBAA* | NP_390285.1 | 16079461 | *Bacillus subtilis* |
| *bfmBAB* | NP_390284.1 | 16079460 | *Bacillus subtilis* |
| *pdhD* | P21880.1 | 118672 | *Bacillus subtilis* |
| *lpdV* | P09063.1 | 118677 | *Pseudomonas putida* |
| *bkdB* | P09062.1 | 129044 | *Pseudomonas putida* |
| *bkdA1* | NP_746515.1 | 26991090 | *Pseudomonas putida* |
| *bkdA2* | NP_746516.1 | 26991091 | *Pseudomonas putida* |
| *Bckdha* | NP_036914.1 | 77736548 | *Rattus norvegicus* |
| *Bckdhb* | NP_062140.1 | 158749538 | *Rattus norvegicus* |
| *Dbt* | NP_445764.1 | 158749632 | *Rattus norvegicus* |
| *Dld* | NP_955417.1 | 40786469 | *Rattus norvegicus* |

The pyruvate dehydrogenase complex, catalyzing the conversion of pyruvate to acetyl-CoA, has also been extensively studied. In the *E. coli* enzyme, specific residues in the E1 component are responsible for substrate specificity (Bisswanger, 256:815-822 (1981); Bremer, 8:535-540 (1969); Gong et al., 275:13645-13653 (2000)). As mentioned previously, enzyme engineering efforts have improved the *E. coli* PDH enzyme activity under anaerobic conditions (Kim et al., J.Bacteriol. 190:3851-3858 (2008); Kim et al., Appl.Environ.Microbiol. 73:1766-1771 (2007); Zhou et al., Biotechnol.Lett. 30:335-342 (2008)). In contrast to the *E. coli* PDH, the *B. subtilis* complex is active and required for growth under anaerobic conditions (Nakano et al., 179:6749-6755 (1997)). The *Klebsiella pneumoniae* PDH, characterized during growth on glycerol, is also active under anaerobic conditions (Menzel et al., 56:135-142 (1997)). Crystal structures of the enzyme complex from bovine kidney (Zhou et al., 98:14802-14807 (2001)) and the E2 catalytic domain from *Azotobacter vinelandii* are available (Mattevi et al., Science. 255:1544-1550 (1992)). Some mammalian PDH enzymes complexes can react on alternate substrates such as 2-oxobutanoate. Comparative kinetics of *Rattus norvegicus* PDH and BCKAD indicate that BCKAD has higher activity on 2-oxobutanoate as a substrate (Paxton et al., Biochem.J. 234:295-303 (1986)). The *S*. *cerevisiae* complex consists of an E2 (LAT1) core that binds E1 (PDA1, PDB1), E3 (LPD1), and Protein X (PDX1) components (Pronk et al., Yeast 12:1607-1633 (1996)).

| *Gene* | Accession No. | GI# | *Organism* |
|---|---|---|---|
| *aceE* | NP_414656.1 | 16128107 | *Escherichia coli* |
| *aceF* | NP_414657.1 | 16128108 | *Escherichia coli* |
| *lpd* | NP_414658.1 | 16128109 | *Escherichia coli* |
| *pdhA* | P21881.1 | 3123238 | *Bacillus subtilis* |
| *pdhB* | P21882.1 | 129068 | *Bacillus subtilis* |
| *pdhC* | P21883.2 | 129054 | *Bacillus subtilis* |
| *pdhD* | P21880.1 | 118672 | *Bacillus subtilis* |
| *aceE* | YP_001333808.1 | 152968699 | *Klebsiella pneumonia* |
| *aceF* | YP_001333809.1 | 152968700 | *Klebsiella pneumonia* |

| *Gene* | Accession No. | GI # | *Organism* |
|---|---|---|---|
| *lpdA* | YP_001333810.1 | 152968701 | *Klebsiella pneumonia* |
| *Pdha1* | NP_001004072.2 | 124430510 | *Rattus norvegicus* |
| *Pdha2* | NP_446446.1 | 16758900 | *Rattus norvegicus* |
| *Dlat* | NP_112287.1 | 78365255 | *Rattus norvegicus* |
| *Dld* | NP_955417.1 | 40786469 | *Rattus norvegicus* |
| *LAT1* | NP_014328 | 6324258 | *Saccharomyces cerevisiae* |
| *PDA1* | NP_011105 | 37362644 | *Saccharomyces cerevisiae* |
| *PDB1* | NP_009780 | 6319698 | *Saccharomyces cerevisiae* |
| *LPD1* | NP_116635 | 14318501 | *Saccharomyces cerevisiae* |
| *PDX1* | NP_011709 | 6321632 | *Saccharomyces cerevisiae* |

As an alternative to the large multienzyme 2-keto-acid dehydrogenase complexes described above, some anaerobic organisms utilize enzymes in the 2-ketoacid oxidoreductase family (OFOR) to catalyze acylating oxidative decarboxylation of 2-ketoacids. Unlike the dehydrogenase complexes, these enzymes contain iron-sulfur clusters, utilize different cofactors, and use ferredoxin, flavodixin or FAD as electron donors in lieu of NAD(P)H. While most enzymes in this family are specific to pyruvate as a substrate (POR) some 2-keto-acid:ferredoxin oxidoreductases have been shown to accept a broad range of 2-ketoacids as substrates including alpha-ketoglutarate and 2-oxobutanoate (Zhang et al., J.Biochem. 120:587-599 (1996); Fukuda et al., Biochim.Biophys.Acta 1597:74-80 (2002)). One such enzyme is the OFOR from the thermoacidophilic archaeon *Sulfolobus tokodaii* 7, which contains an alpha and beta subunit encoded by gene *ST2300* (Zhang et al., J.Biochem. 120:587-599 (1996); Fukuda and Wakagi, Biochim.Biophys.Acta 1597:74-80 (2002)). A plasmid-based expression system has been developed for efficiently expressing this protein in *E. coli* (Fukuda et al., Eur.J.Biochem. 268:5639-5646 (2001)) and residues involved in substrate specificity were determined (Fukuda and Wakagi, Biochim.Biophys.Acta 1597:74-80 (2002)). The 2-oxoacid:ferredoxin oxidoreductase from *Sulfolobus solfataricus* P1 is also active on a broad range of 2-oxoacids (Park et al., J.Biochem.Mol.Biol. 39:46-54 (2006)). The OFOR enzyme encoded by Ape1472/Ape1473 from *Aeropyrum pernix* str. K1 was recently cloned into *E. coli,* characterized, and found to react with 2-oxoglutarate and a broad range of 2-oxoacids (Nishizawa et al., FEBS Lett. 579:2319-2322 (2005)). There is bioinformatic evidence that similar enzymes are present in all archaea, some anaerobic bacteria and amitochondrial eukarya (Fukuda and Wakagi, *supra*)*.* OFOR enzymes are also found in organisms that fix carbon by the RTCA cycle including Hydrogenobacter *thermophilus, Desulfobacter hydrogenophilus* and *Chlorobium* species (Shiba et al., Archives of Microbiology 141:198-203 (1985); Evans et al., Proc.Natl.Acad.Sci.U.S.A 55:928-934 (1966)). The two-subunit enzyme from *H. thermophilus,* encoded by *korAB,* was cloned and expressed in *E. coli* (Yun et al., Biochem.Biophys.Res.Commun. 282:589-594 (2001)).

| *Gene* | GI # | Accession No. | *Organism* |
|---|---|---|---|
| *ST2300* | NP_378302.1 | 15922633 | *Sulfolobus tokodaii 7* |
| *Ape1472* | BAA80470.1 | 5105156 | *Aeropyrum pernix* |
| *Ape1473* | BAA80471.2 | 116062794 | *Aeropyrum pernix* |
| *korA* | BAB21494 | 12583691 | *Hydrogenobacter thermophilus* |
| *korB* | BAB21495 | 12583692 | *Hydrogenobacter thermophilus* |

### 1.2.1.d Oxidoreductase (dephosphorylating)

The reduction of a phosphonic acid to its corresponding aldehyde is catalyzed by an oxidoreductase or phosphate reductase in the EC class 1.2.1. Step E of Figure 6 requires such an enzyme for the reduction of 2,4-pentadienoyl-phosphate to its corresponding aldehyde. This transformation has not been characterized in the literature to date. Exemplary phosphonate reductase enzymes include glyceraldehyde-3-phosphate dehydrogenase (EC 1.2.1.12), aspartate-semialdehyde dehydrogenase (EC 1.2.1.11) acetylglutamylphosphate reductase (EC 1.2.1.38) and glutamate-5-semialdehyde dehydrogenase (EC 1.2.1.-). Aspartate semialdehyde dehydrogenase (ASD, EC 1.2.1.11) catalyzes the NADPH-dependent reduction of 4-aspartyl phosphate to aspartate-4-semialdehyde. ASD participates in amino acid biosynthesis and recently has been studied as an antimicrobial target (Hadfield et al., Biochemistry 40:14475-14483 (2001)). The *E. coli* ASD structure has been solved (Hadfield et al., J Mol.Biol. 289:991-1002 (1999)) and the enzyme has been shown to accept the alternate substrate beta-3-methylaspartyl phosphate (Shames et al., J Biol.Chem. 259:15331-15339 (1984)). The *Haemophilus influenzae* enzyme has been the subject of enzyme engineering studies to alter substrate binding affinities at the active site (Blanco et al., Acta Crystallogr.D.Biol.Crystallogr. 60:1388-1395 (2004); Blanco et al., Acta Crystallogr.D.Biol.Crystallogr. 60:1808-1815 (2004)). Other ASD candidates are found in *Mycobacterium tuberculosis* (Shafiani et al., J Appl Microbiol 98:832-838 (2005)), *Methanococcus jannaschii* (Faehnle et al., J Mol.Biol. 353:1055-1068 (2005)), and the infectious microorganisms *Vibrio cholera* and *Heliobacter pylori* (Moore et al., Protein Expr.Purif 25:189-194 (2002)). A related enzyme candidate is acetylglutamylphosphate reductase (EC 1.2.1.38), an enzyme that naturally reduces acetylglutamylphosphate to acetylglutamate-5-semialdehyde, found in *S*. *cerevisiae* (Pauwels et al., Eur.J Biochem. 270:1014-1024 (2003)), *B. subtilis* (O'Reilly et al., Microbiology 140 (Pt 5):1023-1025 (1994)), *E. coli* (Parsot et al., Gene. 68:275-283 (1988)), and other organisms. Additional phosphate reductase enzymes of *E. coli* include glyceraldehyde 3-phosphate dehydrogenase (*gapA* (Branlant et al., Eur.J.Biochem. 150:61-66 (1985))) and glutamate-5-semialdehyde dehydrogenase (*proA* (Smith et al., J. Bacteriol. 157:545-551 (1984))). Genes encoding glutamate-5-semialdehyde dehydrogenase enzymes from *Salmonella typhimurium* (Mahan et al., J Bacteriol. 156:1249-1262 (1983)) and *Campylobacter jejuni* (Louie et al., Mol.Gen.Genet. 240:29-35 (1993)) were cloned and expressed in *E. coli.*

| **Protein** | **GenBank ID** | **GI Number** | **Organism** |
|---|---|---|---|
| *asd* | NP_417891.1 | 16131307 | *Escherichia coli* |
| *asd* | YP_248335.1 | 68249223 | *Haemophilus influenzae* |
| *asd* | AAB49996 | 1899206 | *Mycobacterium tuberculosis* |
| *VC2036* | NP_231670 | 15642038 | *Vibrio cholera* |
| *asd* | YP_002301787.1 | 210135348 | *Heliobacter pylori* |
| *ARG5,6* | NP_010992.1 | 6320913 | *Saccharomyces cerevisiae* |
| *argC* | NP_389001.1 | 16078184 | *Bacillus subtilis* |
| *argC* | NP_418393.1 | 16131796 | *Escherichia coli* |
| *gapA* | P0A9B2.2 | 71159358 | *Escherichia coli* |
| *proA* | NP_414778.1 | 16128229 | *Escherichia coli* |
| *proA* | NP_459319.1 | 16763704 | *Salmonella typhimurium* |
| *proA* | P53000.2 | 9087222 | *Campylobacter jejuni* |

### 1.2.1.e Oxidoreductase (acid to aldehyde)

The conversion of an acid to an aldehyde is thermodynamically unfavorable and typically requires energy-rich cofactors and multiple enzymatic steps. Direct conversion of the acid to aldehyde by a single enzyme is catalyzed by an acid reductase enzyme in the 1.2.1 family. An enzyme in this EC class is required to convert 3-hydroxybutyrate to 3-hydroxybutanal (Step 5K of Figure 5) and 2,4-pentadienoate to penta-2,4-dienal (Step A of Figure 6).

Exemplary acid reductase enzymes include carboxylic acid reductase, alpha-aminoadipate reductase and retinoic acid reductase. Carboxylic acid reductase (CAR), found in *Nocardia iowensis,* catalyzes the magnesium, ATP and NADPH-dependent reduction of carboxylic acids to their corresponding aldehydes (Venkitasubramanian et al., J Biol. Chem. 282:478-485 (2007)). The natural substrate of this enzyme is benzoate and the enzyme exhibits broad acceptance of aromatic substrates including p-toluate (Venkitasubramanian et al., Biocatalysis in Pharmaceutical and Biotechnology Industries. CRC press (2006)). The enzyme from *Nocardia iowensis*, encoded by *car,* was cloned and functionally expressed in *E. coli* (Venkitasubramanian et al., J Biol.Chem. 282:478-485 (2007)). CAR requires post-translational activation by a phosphopantetheine transferase (PPTase) that converts the inactive apo-enzyme to the active holo-enzyme (Hansen et al., Appl.Environ.Microbiol 75:2765-2774 (2009)). Expression of the *npt* gene, encoding a specific PPTase, product improved activity of the enzyme. An additional enzyme candidate found in *Streptomyces griseus* is encoded by the *griC* and *griD* genes. This enzyme is believed to convert 3-amino-4-hydroxybenzoic acid to 3-amino-4-hydroxybenzaldehyde as deletion of either *griC* or *griD* led to accumulation of extracellular 3-acetylamino-4-hydroxybenzoic acid, a shunt product of 3-amino-4-hydroxybenzoic acid metabolism (Suzuki, et al., J. Antibiot. 60(6):380-387 (2007)). Co-expression of *griC* and *griD* with SGR_665, an enzyme similar in sequence to the *Nocardia iowensis npt,* can be beneficial.

| Gene | GenBank Accession No. | GI No. | Organism |
|---|---|---|---|
| *car* | AAR91681.1 | 40796035 | *Nocardia iowensis* |
| *npt* | ABI83656.1 | 114848891 | *Nocardia iowensis* |
| *griC* | YP_001825755.1 | 182438036 | *Streptomyces griseus* |
| *griD* | YP_001825756.1 | 182438037 | *Streptomyces griseus* |

Additional *car* and *npt* genes can be identified based on sequence homology.

| Gene name | GI No. | GenBank Accession No. | Organism |
|---|---|---|---|
| *fadD9* | 121638475 | YP_978699.1 | *Mycobacterium bovis* BCG |
| *BCG_2812c* | 121638674 | YP_978898.1 | *Mycobacterium bovis* BCG |
| *nfa20150* | 54023983 | YP_118225.1 | *Nocardia farcinica* IFM 10152 |
| *nfa40540* | 54026024 | YP_120266.1 | *Nocardia farcinica* IFM 10152 |
| *SGR_6790* | 182440583 | YP_001828302.1 | *Streptomyces griseus* subsp. *griseus* NBRC 13350 |
| *SGR_665* | 182434458 | YP_001822177.1 | *Streptomyces griseus* subsp. *griseus* NBRC 13350 |
| *MSMEG*_*2956* | YP_887275.1 | YP_887275.1 | *Mycobacterium smegmatis MC2 155* |
| *MSMEG*_*5739* | YP_889972.1 | 118469671 | *Mycobacterium smegmatis MC2 155* |
| *MSMEG_2648* | YP_886985.1 | 118471293 | *Mycobacterium smegmatis MC2 155* |
| *MAP1040c* | NP_959974.1 | 41407138 | *Mycobacterium avium subsp. paratuberculosis K-10* |
| *MAP2899c* | NP_961833.1 | 41408997 | *Mycobacterium avium subsp. paratuberculosis K-10* |
| *MMAR*_*2117* | YP_001850422.1 | 183982131 | *Mycobacterium marinum* M |
| *MMAR*_*2936* | YP_001851230. | 183982939 | *Mycobacterium marinum* |
| *MMAR*_*1916* | YP_001850220.1 | 183981929 | *Mycobacterium marinum M* |
| *TpauDRAFT_33060* | ZP_04027864.1 | 227980601 | *Tsukamurella paurometabola DSM 20162* |
| *TpauDRAFT_20920* | ZP_04026660.1 | ZP_04026660.1 | *Tsukamurella paurometabola DSM 20162* |
| *CPCC7001-132_1320* | ZP_05045132.1 | 254431429 | *Cyanobium PCC7001* |
| *DDBDRAFT_0187729* | XP_636931.1 | 66806417 | *Dictyostelium discoideum AX4* |

An enzyme with similar characteristics, alpha-aminoadipate reductase (AAR, EC 1.2.1.31), participates in lysine biosynthesis pathways in some fungal species. This enzyme naturally reduces alpha-aminoadipate to alpha-aminoadipate semialdehyde. The carboxyl group is first activated through the ATP-dependent formation of an adenylate that is then reduced by NAD(P)H to yield the aldehyde and AMP. Like CAR, this enzyme utilizes magnesium and requires activation by a PPTase. Enzyme candidates for AAR and its corresponding PPTase are found in *Saccharomyces cerevisiae* (Morris et al., Gene 98:141-145 (1991)), *Candida albicans* (Guo et al., Mot. Genet. Genomics 269:271-279 (2003)), and *Schizosaccharomyces pombe* (Ford et al., Curr.Genet. 28:131-137 (1995)). The AAR from *S. pombe* exhibited significant activity when expressed in *E. coli* (Guo et al., Yeast 21:1279-1288 (2004)). The AAR from *Penicillium chrysogenum* accepts S-carboxymethyl-L-cysteine as an alternate substrate, but did not react with adipate, L-glutamate or diaminopimelate (Hijarrubia et al., J Biol.Chem. 278:8250-8256 (2003)). The gene encoding the *P. chrysogenum* PPTase has not been identified to date and no high-confidence hits were identified by sequence comparison homology searching.

| Gene | GenBank Accession No. | GI No. | Organism |
|---|---|---|---|
| *LYS2* | AAA34747.1 | 171867 | *Saccharomyces cerevisiae* |
| *LYS5* | P50113.1 | 1708896 | *Saccharomyces cerevisiae* |
| *LYS2* | AAC02241.1 | 2853226 | *Candida albicans* |
| *LYS5* | AA026020.1 | 28136195 | *Candida albicans* |
| *Lys1p* | P40976.3 | 13124791 | *Schizosaccharomyces pombe* |
| *Lys7p* | Q10474.1 | 1723561 | *Schizosaccharomyces pombe* |
| *Lys2* | CAA74300.1 | 3282044 | *Penicillium chrysogenum* |

### 1.3.1.a Oxidoreducatse (alkane to alkene)

Several transformations in Figure 1 involve the oxidation of an alkane to an alkene, including steps B, F and M. Steps B and F are catalyzed by a dehydrogenase or enoate reductase operating in the reverse direction. Step M is catalyzed by 5-hydroxyvaleryl-CoA dehydrogenase, an acyl-CoA dehydrogenase or enoate reductase. Steps E and G of Figure 3 entail oxidation of pent-3-enoyl-CoA or pent-2-enoyl-CoA, respectively, to 2,4-pentadienoyl-CoA. Exemplary enzyme candidates are described below.

The oxidation of pent-3-enoyl-CoA or pent-2-enoyl-CoA to 2,4-pentadienoyl-CoA is catalyzed by 2,4-pentadienoyl-CoA forming dehydrogenase enzymes. 2,4-Dienoyl-CoA reductase enzymes (EC 1.3.1.34) are suitable candidates for these transformations. Generally, bacterial 2,4-dienoyl-CoA reductases yield 2-enoyl-CoA products, whereas eukaryotic 2,4-dienoyl-CoA reductases yield 3-enoyl-CoA products (Dommes and Kunau, J Biol Chem, 259:1781-1788 (1984)). The *fadH* gene product of *E*. *coli* is an NADPH-dependent 2,4-dienoyl-CoA reductase, which participates in the beta-oxidation of unsaturated fatty acids (Tu et al, Biochem, 47:1167-1175 (2008). A series of mutant DCR enzymes were constructed and shown to yield both 2-enoyl-CoA and 3-enoyl-CoA products (Tu et al, *supra*)*.* Eukaryotic DCR enzymes have been characterized in humans and the mouse (Koivuranta et al, Biochem J, 304:787-792 (1994); Geisbrecht et al, J Biol Chem 274:25814-20 (1999); Miinalainen et al, PLoS genet. 5: E1000543 (2009)). The 2,4-pentadienoyl-CoA reductase of *Clostridium aminovalericum* was shown to catalyze the oxidation of 3-pent-3-enoyl-CoA to 2,4-pentadienoyl-CoA. This enzyme has been purified, characterized and crystallized (Eikmanns, Acta Cryst, D50: 913-914 (1994) and Eikmanns and Buckel, Eur J Biochem 198:263-266 (1991)). The electron carrier of this enzyme is not known; however, it is not NAD(P)H. The sequence of the enzyme has not been published to date.

| Protein | GenBank ID | GI Number | Organism |
|---|---|---|---|
| *fadH* | *NP_417552.1* | 16130976 | *Escherichia coli* |
| *Decr1* | Q16698.1 | 3913456 | *Homo sapiens* |
| *Pdcr* | Q9WV68.1 | 90109767 | *Mus musculus* |
| *Decr* | NP_080448.1 | 13385680 | *Mus musculus* |

2-Enoate reductase enzymes in the EC classes 1.3.* are known to catalyze the reversible reduction of a wide variety of α, β-unsaturated carboxylic acids and aldehydes (Rohdich et al., J Biol Chem 276:5779-5787 (2001)). In the recently published genome sequence of *C*. *kluyveri,* 9 coding sequences for enoate reductases were reported, out of which one has been characterized (Seedorf et al., PNAS 105:2128-2133 (2008)). The *enr* genes from both *C*. *tyrobutyricum* and *Moorella thermoaceticum* have been cloned and sequenced and show 59% identity to each other. The former gene is also found to have approximately 75% similarity to the characterized gene in *C*. *kluyveri* (Gicscl et al., 135:51-57 (1983)). It has been reported based on these sequence results that the *C*. *tyrobutyricum enr* is very similar to the FadH dienoyl CoA reductase of *E. coli* (Rohdich et al., *supra*)*.* The *M*. *thermoaceticum enr* gene was expressed in a catalytically active form in *E. coli* (Rohdich et al., *supra*)*.* This enzyme exhibits activity on a broad range of alpha, beta-unsaturated carbonyl compounds.

| Protein | GenBank ID | GI Number | Organism |
|---|---|---|---|
| *enr* | ACA54153.1 | 169405742 | *Clostridium botulinum A3 str* |
| *enr* | CAA71086.1 | 2765041 | *Clostridium tyrobutyricum* |
| *enr* | CAA76083.1 | 3402834 | *Clostridium kluyveri* |
| *enr* | YP_430895.1 | 83590886 | *Moorella thermoacetica* |

Another candidate 2-enoate reductase is maleylacetate reductase (MAR, EC 1.3.1.32), an enzyme catalyzing the reduction of 2-maleylacetate (4-oxohex-2-enedioate) to 3-oxoadipate. MAR enzymes naturally participate in aromatic degradation pathways (Kaschabek et al., J Bacteriol. 175:6075-6081 (1993); Kaschabek et al., J Bacteriol. 177:320-325 (1995); Camara et al., J Bacteriol. (2009); Huang et al., Appl Environ.Microbiol 72:7238-7245 (2006)). The enzyme activity was identified and characterized in *Pseudomonas* sp. strain B13 (Kaschabek et al., 175:6075-6081 (1993); Kaschabek et al., 177:320-325 (1995)), and the coding gene was cloned and sequenced (Kasberg et al., J Bacteriol. 179:3801-3803 (1997)). Additional MAR gene candidates include *clcE* gene from *Pseudomonas* sp. strain B13 (Kasberg et al., J Bacteriol. 179:3801-3803 (1997)), *macA* gene from *Rhodococcus opacus* (Seibert et al., 180:3503-3508 (1998)), the *macA* gene from *Ralstonia eutropha* (also known as *Cupriavidus necator*) (Seibert et al., Microbiology 150:463-472 (2004)), *tfdFII* from *Ralstonia eutropha* (Seibert et al., J Bacteriol. 175:6745-6754 (1993)) and *NCgl1112* in *Corynebacterium glutamicum* (Huang et al., Appl Environ.Microbiol 72:7238-7245 (2006)). A MAR in *Pseudomonas reinekei MT1*, encoded by *ccaD,* was recently identified (Camara et al., J Bacteriol. (2009)).

| *Gene* | GI # | Accession No. | *Organism* |
|---|---|---|---|
| *clcE* | 3913241 | O30847.1 | *Pseudomonas* sp. strain B13 |
| *macA* | 7387876 | O84992.1 | *Rhodococcus opacus* |
| *macA* | 5916089 | AAD55886 | *Cupriavidus necator* |
| *tfdFII* | 1747424 | AC44727.1 | *Ralstonia eutropha JMP134* |
| *NCgl1112* | 19552383 | NP_600385 | *Corynebacterium glutamicum* |
| *ccaD* | ABO61029.1 | 134133940 | *Pseudomonas reinekei MT1* |

An exemplary enoate reductase that favors the alkene-forming oxidative direction is succinate dehydrogenase (EC classes 1.3.99 or 1.3.5), also known as succinate-ubiquinone oxidoreductase and complex II. SDH is a membrane-bound enzyme complex that converts succinate to fumarate and transfers electrons to ubiquinone. The enzyme is composed of two catalytic subunits, encoded by *sdhAB,* and two membrane subunits encoded by *sdhCD.* Although the *E. coli* SDH is reversible, the enzyme is 50-fold more proficient in oxidizing succinate than reducing fumarate (Maklashina et al J Biol.Chem. 281:11357-11365 (2006)).

| Protein | GenBank ID | GI Number | Organism |
|---|---|---|---|
| *sdhA* | AAC73817.1 | 1786942 | *Escherichia coli* |
| *sdhB* | AAC73818.1 | 1786943 | *Escherichia coli* |
| *sdhC* | AAC73815.1 | 1786940 | *Escherichia coli* |
| *sdhD* | AAC73816.1 | 1786941 | *Escherichia coli* |

An exemplary acyl-CoA dehydrogenase or enoyl-CoA reductase is the gene product of *bcd* from *Clostridium acetobutylicum* (Atsumi et al., 10:305-311 (2008); Boynton et al., J Bacteriol. 178:3015-3024 (1996)), which naturally catalyzes the reduction of crotonyl-CoA to butyryl-CoA (EC 1.3.99.2). This enzyme participates in the acetyl-CoA fermentation pathway to butyrate in *Clostridial* species (Jones et al., Microbiol Rev. 50:484-524 (1986)). Activity of butyryl-CoA reductase can be enhanced by expressing *bcd* in conjunction with expression of the *C*. *acetobutylicum etfAB* genes, which encode an electron transfer flavoprotein. An additional candidate for the enoyl-CoA reductase step is the mitochondrial enoyl-CoA reductase (EC 1.3.1.44) from *E. gracilis* (Hoffmeister et al., J Biol.Chem 280:4329-4338 (2005)). A construct derived from this sequence following the removal of its mitochondrial targeting leader sequence was cloned in *E. coli* resulting in an active enzyme (Hoffmeister et al, *supra).* A close homolog of the protein from the prokaryote *Treponema denticola,* encoded by TDE0597, has also been cloned and expressed in *E. coli* (Tucci et al., FEBS Lett, 581:1561-1566 (2007)). Six genes in *Syntrophus aciditrophicus* were identified by sequence homology to the *C*. *acetobutylicum bcd* gene product. The *S. aciditrophicus* genes *syn_02637* and *syn_02636* bear high sequence homology to the *etfAB* genes of *C*. *acetobutylicum,* and are predicted to encode the alpha and beta subunits of an electron transfer flavoprotein.

| Protein | GenBank ID | GI Number | Organism |
|---|---|---|---|
| *bcd* | NP_349317.1 | 15895968 | *Clostridium acetobutylicum* |
| *etfA* | NP_349315.1 | 15895966 | *Clostridium acetobutylicum* |
| *etfB* | NP_349316.1 | 15895967 | *Clostridium acetobutylicum* |
| *TER* | Q5EU90.1 | 62287512 | *Euglena gracilis* |
| *TDE0597* | NP_971211.1 | 42526113 | *Treponema denticola* |
| *syn_02587* | ABC76101 | 85721158 | *Syntrophus aciditrophicus* |
| *syn_02586* | ABC76100 | 85721157 | *Syntrophus aciditrophicus* |
| *syn_01146* | ABC76260 | 85721317 | *Syntrophus aciditrophicus* |
| *syn_00480* | ABC77899 | 85722956 | *Syntrophus aciditrophicus* |
| *syn_02128* | ABC76949 | 85722006 | *Syntrophus aciditrophicus* |
| *syn_01699* | ABC78863 | 85723920 | *Syntrophus aciditrophicus* |
| *syn_02637* | ABC78522.1 | 85723579 | *Syntrophus aciditrophicus* |
| *syn_02636* | ABC78523.1 | 85723580 | *Syntrophus aciditrophicus* |

Additional enoyl-CoA reductase enzyme candidates are found in organisms that degrade aromatic compounds. *Rhodopseudomonas palustris,* a model organism for benzoate degradation, has the enzymatic capability to degrade pimelate via beta-oxidation of pimeloyl-CoA. Adjacent genes in the *pim* operon, *pimC* and *pimD*, bear sequence homology to *C*. *acetobutylicum bcd* and are predicted to encode a flavin-containing pimeloyl-CoA dehydrogenase (Harrison et al., 151:727-736 (2005)). The genome of nitrogen-fixing soybean symbiont *Bradyrhizobium japonicum* also contains a *pim* operon composed of genes with high sequence similarity to *pimC* and *pimD* of *R. palustris* (Harrison and Harwood, Microbiology 151:727-736 (2005)).

| Protein | GenBank ID | GI Number | Organism |
|---|---|---|---|
| *pimC* | CAE29155 | 39650632 | *Rhodopseudomonas palustris* |
| *pimD* | CAE29154 | 39650631 | *Rhodopseudomonas palustris* |
| *pimC* | BAC53083 | 27356102 | *Bradyrhizobium japonicum* |
| *pimD* | BAC53082 | 27356101 | *Bradyrhizobium japonicum* |

An additional candidate is 2-methyl-branched chain enoyl-CoA reductase (EC 1.3.1.52 and EC 1.3.99.12), an enzyme catalyzing the reduction of sterically hindered trans-enoyl-CoA substrates. This enzyme participates in branched-chain fatty acid synthesis in the nematode *Ascarius suum* and is capable of reducing a variety of linear and branched chain substrates including 2-methylvaleryl-CoA, 2-methylbutanoyl-CoA, 2-methylpentanoyl-CoA, octanoyl-CoA and pentanoyl-CoA (Duran et al., 268:22391-22396 (1993)). Two isoforms of the enzyme, encoded by genes *acadl* and *acad,* have been characterized.

| Protein | GenBank ID | GI Number | Organism |
|---|---|---|---|
| *acad1* | AAC48316.1 | 2407655 | *Ascarius suum* |
| *acad* | AAA16096.1 | 347404 | *Ascarius suum* |

### 1.4.1.a Oxidoreductase (amine to oxo)

Enzymes in the EC class 1.4.1 catalyze the oxidative deamination of amines to aldehydes or ketones. Enzymes in this EC class typically employ NAD+, NADP+ or FAD as an electron acceptor, and the reactions are typically reversible. Steps C, H and I of Figure 1 can be catalyzed by a deaminating oxidoreductase. Enzyme candidates are described below.

Glutamate dehydrogenase (EC 1.4.1.2), leucine dehydrogenase (EC 1.4.1.9), and aspartate dehydrogenase (EC 1.4.1.21) convert amino acids to their corresponding 2-keto acids. The *gdhA* gene product from *Escherichia coli* (Korber et al., J Mol.Biol. 234:1270-1273 (1993); McPherson et al., Nucleic Acids Res. 11:5257-5266 (1983)), *gdh* from *Thermotoga maritime* (Kort et al., Extremophiles. 1:52-60 (1997); Lebbink et al., J Mol.Biol. 280:287-296 (1998); Lebbink et al., J Mol.Biol. 289:357-369 (1999)), and *gdhA1* from *Halobacterium salinarum* (Ingoldsby et al., Gene 349:237-244 (2005)) catalyze the reversible conversion of glutamate to 2-oxoglutarate and ammonia, while favoring NADP(H), NAD(H), or both, respectively. Additional glutamate dehydrogenase gene candidates are found in *Bacillus subtilis* (Khan et al., Biosci.Biotechnol Biochem. 69:1861-1870 (2005)), *Nicotiana tabacum* (Purnell et al., Planta 222:167-180 (2005)), *Oryza sativa* (Abiko et al., Plant Cell Physiol 46:1724-1734 (2005)), *Haloferax mediterranei* (Diaz et al., Extremophiles. 10:105-115 (2006)) and *Halobactreium salinarum* (Hayden et al., FEMS Microbiol Lett. 211:37-41 (2002)). The *Nicotiana tabacum* enzyme is composed of alpha and beta subunits encoded by *gdh1* and *gdh2* (Purnell et al., Planta 222:167-180 (2005)). Overexpression of the NADH-dependent glutamate dehydrogenase was found to improve ethanol production in engineered strains of *S. cerevisiae* (Roca et al., Appl Environ.Microbiol 69:4732-4736 (2003)). The *ldh* gene of *Bacillus cereus* encodes the LeuDH protein that accepts a wide of range of substrates including leucine, isoleucine, valine, and 2-aminobutanoate (Ansorge et al., Biotechnol Bioeng 68:557-562 (2000); Stoyan et al., J Biotechnol 54:77-80 (1997)). The *nadX gene* from *Thermotoga maritima* encodes aspartate dehydrogenase, involved in the biosynthesis of NAD (Yang et al., J Biol. Chem. 278:8804-8808 (2003)).

| Protein | GenBank ID | GI Number | Organism |
|---|---|---|---|
| *gdhA* | P00370 | 118547 | *Escherichia coli* |
| *gdh* | P96110.4 | 6226595 | *Thermotoga maritima* |
| *gdhA1* | NP_279651.1 | 15789827 | *Halobacterium salinarum* |
| *rocG* | NP_391659.1 | 16080831 | *Bacillus subtilis* |
| *gdh1* | AAR11534.1 | 38146335 | *Nicotiana tabacum* |
| *gdh2* | AAR11535.1 | 38146337 | *Nicotiana tabacum* |
| *GDH* | Q852M0 | 75243660 | *Oryza sativa* |
| *GDH* | Q977U6 | 74499858 | *Haloferax mediterranei* |
| *GDH* | P29051 | 118549 | *Halobactreium salinarum* |
| *GDH2* | NP_010066.1 | 6319986 | *Saccharomyces cerevisiae* |
| *ldh* | P0A393 | 61222614 | *Bacillus cereus* |
| *nadX* | NP_229443.1 | 15644391 | *Thermotoga maritima* |

An exemplary enzyme for catalyzing the conversion of primary amines to their corresponding aldehydes is lysine 6-dehydrogenase (EC 1.4.1.18), encoded by the *lysDH* genes. This enzyme catalyzes the oxidative deamination of the 6-amino group of L-lysine to form 2-aminoadipate-6-semialdehyde (Misono et al., J Bacteriol. 150:398-401 (1982)). Exemplary lysine 6-dehydrogenase enzymes are found in *Geobacillus stearothermophilus* (Heydari et al., AEM 70:937-942 (2004)), *Agrobacterium tumefaciens* (Hashimoto et al., J Biochem. 106:76-80 (1989); Misono and Nagasaki, J Bacteriol. 150:398-401 (1982)), and *Achromobacter denitrificans* (Ruldeekulthamrong et al., BMB.Rep. 41:790-795 (2008)).

| Protein | GenBank ID | GI Number | Organism |
|---|---|---|---|
| *lysDH* | BAB39707 | 13429872 | *Geobacillus stearothermophilus* |
| *lysDH* | NP_353966 | 15888285 | *Agrobacterium tumefaciens* |
| *lysDH* | AAZ94428 | 74026644 | *Achromobacter denitrificans* |

### 1.4.3.a Amine oxidase

Amine oxidase enzymes in EC class 1.4.3 catalyze the oxidative deamination of amino groups to their corresponding aldehydes or ketones. This class of enzymes utilizes oxygen as the electron acceptor, converting an amine, O2 and water to an aldehyde or ketone, ammonia and hydrogen peroxide. L-Amino-acid oxidase catalyzes the oxidative deamination of a number of L-amino acids to their respective 2-oxoacids. The *Streptococcus oligofermentans* enzyme was overexpressed in *E. coli* (Tong et al, J Bacteriol 190:4716-21 (2008)). Other amine oxidase enzymes such as lysine-6-oxidase (EC 1.4.3.20) and putrescine oxidase (EC 1.4.3.10), are specific to terminal amines. Lysine-6-oxidase enzymes are encoded by *lodA* of *Marinomonas mediterranea* (Lucas-Elio et al, J Bacteriol 188:2493-501 (2006)) and *alpP* of *Pseudoalteromonas tunicata* (Mai-Prochnow et al, J Bacteriol 190:5493-501 (2008)). Putrescine oxidase enzymes are encoded by *puo* of *Kocuria rosea* (Ishizuka et al, J Gen Microbiol 139:425-32 (1993)) and *ATAO1* of *Arabidopsis thaliana* (Moller and McPherson, Plant J 13:781-91 (1998)).

| **Protein** | **GenBank ID** | **GI Number** | **Organism** |
|---|---|---|---|
| EU495328.1:1..1176 | ACA52024.1 | 169260271 | *Streptococcus oligofermentans* |
| *lodA* | AAY33849.1 | 83940756 | *Marinomonas mediterranea* |
| alpP | AAP73876.1 | 32396307 | *Pseudoalteromonas tunicata* |
| *puo* | BAA02074.1 | 303641 | *Kocuria rosea* |
| *ATAO1* | AAB87690.1 | 2654118 | *Arabidopsis thaliana* |

### 2.3.1.a Acyltransferase (transferring phosphate group to CoA; phosphotransacylase)

An enzyme with 2,4-pentadienoyl-CoA phosphotransferase activity is required to convert 2,4-pentadienoyl-CA to 2,4-pentadienoyl-phosphate (Figure 6, Step D). Exemplary phosphate-transferring acyltransferases include phosphotransacetylase (EC 2.3.1.8) and phosphotransbutyrylase (EC 2.3.1.19). The *pta* gene from *E. coli* encodes a phosphotransacetylase that reversibly converts acetyl-CoA into acetyl-phosphate (Suzuki, Biochim.Biophys.Acta 191:559-569 (1969)). This enzyme can also utilize propionyl-CoA as a substrate, forming propionate in the process (Hesslinger et al., Mol.Microbiol 27:477-492 (1998)). Other phosphate acetyltransferases that exhibit activity on propionyl-CoA are found in *Bacillus subtilis* (Rado et al., Biochim.Biophys.Acta 321:114-125 (1973)), *Clostridium* kluyveri (Stadtman, Methods Enzymol 1:596-599 (1955)), and *Thermotoga maritima* (Bock et al., J Bacteriol. 181:1861-1867 (1999)). Similarly, the *ptb* gene from C. *acetobutylicum* encodes phosphotransbutyrylase, an enzyme that reversibly converts butyryl-CoA into butyryl-phosphate (Wiesenborn et al., Appl Environ.Microbiol 55:317-322 (1989); Walter et al., Gene 134:107-111 (1993)). Additional *ptb* genes are found in butyrate-producing bacterium L2-50 (Louis et al., J.Bacteriol. 186:2099-2106 (2004)) and *Bacillus megaterium* (Vazquez et al., Curr.Microbiol 42:345-349 (2001)).

| **Protein** | **GenBank ID** | **GI Number** | **Organism** |
|---|---|---|---|
| *pta* | NP_416800.1 | 71152910 | *Escherichia coli* |
| *pta* | P39646 | 730415 | *Bacillus subtilis* |
| *pta* | A5N801 | 146346896 | *Clostridium kluyveri* |
| *pta* | Q9X0L4 | 6685776 | *Thermotoga maritima* |
| *ptb* | NP_349676 | 34540484 | *Clostridium acetobutylicum* |
| *ptb* | AAR19757.1 | 38425288 | butyrate-producing bacterium L2-50 |
| *ptb* | CAC07932.1 | 10046659 | *Bacillus megaterium* |

### 2.3.1.b Beta-ketothiolase

Beta-ketothiolase enzymes in the EC class 2.3.1 catalyze the condensation of two acyl-CoA substrates. Several transforms in Figures 2-4 require a beta-ketothiolase, including step A of Figure 2, step
A of Figure 3 and step A of Figure 4.

Exemplary beta-ketothiolases with acetoacetyl-CoA thiolase activity include the gene products of *atoB* from *E*. *coli* (Martin et al., Nat.Biotechnol 21:796-802 (2003)), *thlA and thlB* from *C*. acetobutylicum (Hanai et al., Appl Environ Microbiol 73:7814-7818 (2007); Winzer et al., J.Mol.Microbiol Biotechnol 2:531-541 (2000)), and *ERG10* from *S. cerevisiae* (Hiser et al., J.Biol.Chem. 269:31383-31389 (1994)).

| Protein | GenBank ID | GI Number | Organism |
|---|---|---|---|
| *atoB* | NP_416728 | 16130161 | *Escherichia coli* |
| *thlA* | NP_349476.1 | 15896127 | *Clostridium acetobutylicum* |
| *thlB* | NP_149242.1 | 15004782 | *Clostridium acetobutylicum* |
| *ERG10* | NP_015297 | 6325229 | *Saccharomyces cerevisiae* |

Beta-ketothiolase enzymes catalyzing the formation of beta-ketovalerate from acetyl-CoA and propionyl-CoA are also suitable candidates. *Zoogloea ramigera* possesses two ketothiolases that can form 3-ketovaleryl-CoA from propionyl-CoA and acetyl-CoA and *R. eutropha* has a beta-oxidation ketothiolase that is also capable of catalyzing this transformation (Gruys et al., US Patent 5,958,745 (1999)). The sequences of these genes or their translated proteins have not been reported, but several candidates in *R. eutropha, Z. ramigera,* or other organisms can be identified based on sequence homology to *bktB* from *R. eutropha.* These include:

| Protein | GenBank ID | GI Number | Organism |
|---|---|---|---|
| *phaA* | YP_725941.1 | 113867452 | *Ralstonia eutropha* |
| *h16_A1713* | YP_726205.1 | 113867716 | *Ralstonia eutropha* |
| *pcaF* | YP_728366.1 | 116694155 | *Ralstonia eutropha* |
| *h16_B1369* | YP_840888.1 | 116695312 | *Ralstonia eutropha* |
| *h16_A0170* | YP_724690.1 | 113866201 | *Ralstonia eutropha* |
| *h16_A0462* | YP_724980.1 | 113866491 | *Ralstonia eutropha* |
| *h16_A1528* | YP_726028.1 | 113867539 | *Ralstonia eutropha* |
| *h16_B0381* | YP_728545.1 | 116694334 | *Ralstonia eutropha* |
| *h16_B0662* | YP_728824.1 | 116694613 | *Ralstonia eutropha* |
| *h16_B0759* | YP_728921.1 | 116694710 | *Ralstonia eutropha* |
| *h16_B0668* | YP_728830.1 | 116694619 | *Ralstonia eutropha* |
| *h16_A1720* | YP_726212.1 | 113867723 | *Ralstonia eutropha* |
| *h16_A1887* | YP_726356.1 | 113867867 | *Ralstonia eutropha* |
| *phbA* | P07097.4 | 135759 | *Zoogloea ramigera* |
| *bktB* | YP_002005382.1 | 194289475 | *Cupriavidus taiwanensis* |
| *Rmet_1362* | YP_583514.1 | 94310304 | *Ralstonia metallidurans* |
| *Bphy_0975* | YP_001857210.1 | 186475740 | *Burkholderia phymatum* |

Another suitable candidate is 3-oxoadipyl-CoA thiolase (EC 2.3.1.174), which converts beta-ketoadipyl-CoA to succinyl-CoA and acetyl-CoA, and is a key enzyme of the beta-ketoadipate pathway for aromatic compound degradation. The enzyme is widespread in soil bacteria and fungi including *Pseudomonas putida* (Harwood et al., J Bacteriol. 176:6479-6488 (1994)) and *Acinetobacter calcoaceticus* (Doten et al., J Bacteriol. 169:3168-3174 (1987)). The gene products encoded by *pcaF* in *Pseudomonas* strain B13 (Kaschabck et al., J Bacteriol. 184:207-215 (2002)), *phaD* in *Pseudomonas putida U* (Olivera et al., Proc.Natl.Acad.Sci U.S.A 95:6419-6424 (1998)), *paaE* in *Pseudomonas fluorescens ST* (Di et al., Arch.Microbiol 188:117-125 (2007)), and *paaJ* from *E. coli* (Nogalcs et al., Microbiology 153:357-365 (2007)) also catalyze this transformation. Several beta-ketothiolases exhibit significant and selective activities in the oxoadipyl-CoA forming direction including *bkt* from *Pseudomonas putida, pcaF* and *bkt* from *Pseudomonas aeruginosa PAO1, bkt* from *Burkholderia ambifaria* AMMD, *paaJ* from *E. coli,* and *phaD* from *P. putida.*

| Gene name | GI# | GenBank Accession # | Organism |
|---|---|---|---|
| *paaJ* | 16129358 | NP_415915.1 | *Escherichia coli* |
| *pcaF* | 17736947 | AAL02407 | *Pseudomonas knackmussii* (B13) |
| *phaD* | 3253200 | AAC24332.1 | *Pseudomonas putida* |
| *pcaF* | 506695 | AAA85138.1 | *Pseudomonas putida* |
| *pcaF* | 141777 | AAC37148.1 | *Acinetobacter calcoaceticus* |
| *paaE* | 106636097 | ABF82237.1 | *Pseudomonas fluorescens* |
| *bkt* | 115360515 | YP_777652.1 | *Burkholderia ambifaria AMMD* |
| *bkt* | 9949744 | AAG06977.1 | *Pseudomonas aeruginosa PAO1* |
| *pcaF* | 9946065 | AAG03617.1 | *Pseudomonas aeruginosa PAO1* |

### 2.3.1.d Formate C-acyltransferase

Formate C-acyltransferase enzymes in the EC class 2.3.1 catalyze the acylation of ketoacids and concurrent release of formate. Such an enzyme is suitable for the conversion of 2-oxoadipate to glutaryl-CoA in Figure 1 (step R) and the conversion of 4-hydroxy-2-oxovalerate to 3-hydroxybutyryl-CoA in step H of Figure 5.

Enzymes in this class include pyruvate formate-lyase and ketoacid formate-lyase. Pyruvate formate-lyase (PFL, EC 2.3.1.54), encoded by *pflB* in *E. coli,* converts pyruvate into acetyl-CoA and formate. The active site of PFL contains a catalytically essential glycyl radical that is posttranslationally activated under anaerobic conditions by PFL-activating enzyme (PFL-AE, EC 1.97.1.4) encoded by *pflA* (Knappe et al., Proc.Natl.Acad.Sci U.S.A 81:1332-1335 (1984); Wong et al., Biochemistry 32:14102-14110 (1993)). Keto-acid formate-lyase (EC 2.3.1.-), also known as 2-ketobutyrate formate-lyase (KFL) and pyruvate formate-lyase 4, is the gene product of *tdcE* in *E*. coli. This enzyme catalyzes the conversion of 2-ketobutyrate to propionyl-CoA and formate during anaerobic threonine degradation, and can also substitute for pyruvate formate-lyase in anaerobic catabolism (Simanshu et al., J Biosci. 32:1195-1206 (2007)). The enzyme is oxygen-sensitive and, like PflB, requires post-translational modification by PFL-AE to activate a glycyl radical in the active site (Hesslinger et al., Mol.Microbiol 27:477-492 (1998)). A pyruvate formate-lyase from *Archaeglubus fulgidus* encoded by *pflD* has been cloned, expressed in *E. coli* and characterized (Lehtio et al., Protein Eng Des Sel 17:545-552 (2004)). The crystal structures of the *A. fulgidus* and *E. coli* enzymes have been resolved (Lehtio et al., J Mol.Biol. 357:221-235 (2006); Leppanen et al., Structure. 7:733-744 (1999)). Additional PFL and PFL-AE candidates are found in *Lactococcus lactis* (Melchiorsen et al., Appl Microbiol Biotechnol 58:338-344 (2002)), and *Streptococcus mutans* (Takahashi-Abbe et al., Oral.Microbiol Immunol. 18:293-297 (2003)), *Chlamydomonas reinhardtii* (Hemschemeier et al., Eukaryot.Cell 7:518-526 (2008b); Atteia et al., J.Biol.Chem. 281:9909-9918 (2006)) and *Clostridium pasteurianum* (Weidner et al., J Bacteriol. 178:2440-2444 (1996)).

| Protein | GenBank ID | GI Number | Organism |
|---|---|---|---|
| *pflB* | NP_415423 | 16128870 | *Escherichia coli* |
| *pflA* | NP_415422.1 | 16128869 | *Escherichia coli* |
| *tdcE* | AAT48170.1 | 48994926 | *Escherichia coli* |
| *pflD* | NP_070278.1 | 11499044 | *Archaeglubus fulgidus* |
| *pfl* | CAA03993 | 2407931 | *Lactococcus lactis* |
| *pfl* | BAA09085 | 1129082 | *Streptococcus mutans* |
| *PFL1* | XP_001689719.1 | 159462978 | *Chlamydomonas reinhardtii* |
| *pflA1* | XP_001700657.1 | 159485246 | *Chlamydomonas reinhardtii* |
| *pfl* | Q46266.1 | 2500058 | *Clostridium pasteurianum* |
| *act* | CAA63749.1 | 1072362 | *Clostridium pasteurianum* |

### 2.3.1.h 3-Oxoacyl-CoA Synthase

3-Oxoacyl-CoA products such as acetoacetyl-CoA, 3-oxopentanoyl-CoA, 3-oxo-5-hydroxypentanoyl-CoA can be synthesized from acyl-CoA and malonyl-CoA substrates by 3-oxoacyl-CoA synthases (Steps 2A, 3A, 4A, 7AS). As enzymes in this class catalyze an essentially irreversible reaction, they are particularly useful for metabolic engineering applications for overproducing metabolites, fuels or chemicals derived from 3-oxoacyl-CoA intermediates such as acetoacetyl-CoA. Acetoacetyl-CoA synthase, for example, has been heterologously expressed in organisms that biosynthesize butanol (Lan et al, PNAS USA (2012)) and poly-(3-hydroxybutyrate) (Matsumoto et al, Biosci Biotech Biochem, 75:364-366 (2011). An acetoacetyl-CoA synthase (EC 2.3.1.194) enzyme (FhsA) has been characterized in the soil bacterium *Streptomyces* sp. CL190 where it participates in mevalonate biosynthesis (Okamura et al, PNAS USA 107:11265-70 (2010)). Other acetoacetyl-CoA synthase genes can be identified by sequence homology to *fhsA.*

| Protein | GenBank ID | GI Number | Organism |
|---|---|---|---|
| *fhsA* | BAJ83474.1 | 325302227 | *Streptomyces sp CL190* |
| *AB183750.1:1* | BAD86806.1 | 57753876 | *Streptomyces* sp. KO-3988 |
| *1991..12971* | | | |
| *epzT* | ADQ43379.1 | 312190954 | *Streptomyces cinnamonensis* |
| *ppzT* | CAX48662.1 | 238623523 | *Streptomyces anulatus* |
| *O3I_22085* | ZP_09840373.1 | 378817444 | *Nocardia brasiliensis* |

### 2.6.1.a Aminotransferase

Aminotransferases reversibly convert an amino group to an aldehyde or ketone. Exemplary enzymes for converting aldehydes to primary amines include lysine-6-aminotransferase (EC 2.6.1.36), 5-aminovalerate aminotransferase (EC 2.6.1.48), gamma-aminobutyrate aminotransferase and diamine aminotransferases such as putrescine aminotransferase (EC 2.6.1.82 and 2.6.1.29). These enzymes are suitable for catalyzing steps C and I of Figure 1. Aspartate aminotransferase and similar enzymes convert amino acids to their corresponding 2-ketoacids. Amino acid aminotransferases are suitable candidates for interconverting 2-oxoadipate and 2-aminoadipate (Step H of Figure 1).

Lysine-6-aminotransferase (EC 2.6.1.36) converts lysine to alpha-aminoadipate semialdehyde, and has been charactierized in yeast and bacteria. Candidates from *Candida utilis* (Hammer et al., J Basic Microbiol 32:21-27 (1992)), *Flavobacterium lutescens* (Fujii et al., J Biochem. 128:391-397 (2000)) and *Streptomyces clavuligenus* (Romero et al., J Ind.Microbiol Biotechnol 18:241-246 (1997)) have been characterized. A recombinant lysine-6-aminotransferase from *S. clavuligenus* was functionally expressed in *E. coli* (Tobin et al., J Bacteriol. 173:6223-6229 (1991)). The *F. lutescens* enzyme is specific to alpha-ketoglutarate as the amino acceptor (Soda et al., Biochemistry 7:4110-4119 (1968)). A related enzyme, diaminobutyrate aminotransferase (EC 2.6.1.46 and EC 2.6.1.76), is encoded by the *dat* gene products in *Acinetobacter baumanii* and *Haemophilus influenza* (Ikai et al., J Bacteriol. 179:5118-5125 (1997); Tkai et al., Biol Pharm.Bull. 21:170-173 (1998)). In addition to its natural substrate, 2,4-diaminobutyrate, the *A. baumanii* DAT transaminates the terminal amines of lysine, 4-aminobutyrate and ornithine. Additional diaminobutyrate aminotransferase gene candidates include the *ectB* gene products of *Marinococcus halophilus* and *Halobacillus dabanensis (*Zhao et al., Curr Microbiol 53:183-188 (2006*);* Louis et al., Microbiology 143 (Pt 4):1141-1149 (1997*))* and the *pvdH* gene product of *Pseudomonas aeruginosa* (Vandenende et al., J Bacteriol. 186:5596-5602 (2004)). The beta-alanine aminotransferase of *Pseudomonas fluorescens* also accepts 2,4-diaminobutyrate as a substrate (Hayaishi et al., J Biol Chem 236:781-790 (1961)); however, this activity has not been associated with a gene to date.

| Gene | GenBank ID | GI Number | Organism |
|---|---|---|---|
| *lat* | BAB13756.1 | 10336502 | *Flavobacterium lutescens* |
| *lat* | AAA26777.1 | 153343 | *Streptomyces clavuligenus* |
| *dat* | P56744.1 | 6685373 | *Acinetobacter baumanii* |
| *dat* | P44951.1 | 1175339 | *Haemophilus influenzae* |
| *ectB* | AAB57634.1 | 2098609 | *Marinococcus halophilus* |
| *ectB* | AAZ57191.1 | 71979940 | *Halobacillus dabanensis* |
| *pvdH* | AAG05801.1 | 9948457 | *Pseudomonas aeruginosa* |

The conversion of an aldehyde to a terminal amine can also be catalyzed by gamma-aminobutyrate (GABA) transaminase (EC 2.6.1.19) or 5-aminovalerate transaminase. GABA aminotransferase interconverts succinic semialdehyde and glutamate to 4-aminobutyrate and alpha-ketoglutarate and is known to have a broad substrate range (Schulz et al., 56:1-6 (1990); Liu et al., 43:10896-10905 (2004)). The two GABA transaminases in *E. coli* are encoded by *gabT* (Bartsch et al., J Bacteriol. 172:7035-7042 (1990)) and *puuE* (Kurihara et al., J.Biol.Chem. 280:4602-4608 (2005)). GABA transaminases in *Mus musculus* and *Sus scrofa* also catalyze the transamination of alternate substrates including 6-aminocaproic acid (Cooper, Methods Enzymol. 113:80-82 (1985)). 5-Aminovalerate aminotransferase (EC 2.6.1.48) converts 5-aminovalerate to 5-oxovalerate during lysine degradation. The enzyme is encoded by *davT* of *Pseudomonas putida* (Espinosa-Urgel et al, Appl Env Microbiol, 67:5219-24 (2001)) and *PA0266* of *Pseudomonas aeruginosa* (Yamanishi et al, FEBS J. 274:2262-73 (2007)). A 5-aminovalerate aminotransferase from *Clostridium aminovalericum* was purified and characterized but the sequence has not been published to date (Barker et al, J Biol Chem, 262:8994-9003 (1987)).

| Gene | GenBank ID | GI Number | Organism |
|---|---|---|---|
| *gabT* | NP_417148.1 | 16130576 | *Escherichia coli* |
| *puuE* | NP_415818.1 | 16129263 | *Escherichia coli* |
| *Abat* | NP_766549.2 | 37202121 | *Mus musculus* |
| *gabT* | YP_257332.1 | 70733692 | *Pseudomonas fluorescens* |
| *Abat* | NP_999428.1 | 47523600 | *Sus scrofa* |
| *davT* | AAK97868.1 | 15428718 | *Pseudomonas putida* |
| PA0266 | NP_248957.1 | 15595463 | *Pseudomonas aeruginosa* |

Putrescine aminotransferase (EC 2.6.1.82) and other diamine aminotransferases (EC 2.6.1.29) also catalyze the intcrconvcrsion of aldehydes and primary amines. The *E. coli* putrescine aminotransferase is encoded by the *ygjG* gene and the purified enzyme transaminates the alternative substrates cadaverine, spermidine and 1,7-diaminoheptane (Samsonova et al., BMC.Microbiol 3:2 (2003)). Activity of this enzyme with amino acceptors other than 2-oxoglutarate (e.g., pyruvate, 2-oxobutanoate) has been reported (Samsonova et al., BMC.Microbiol 3:2 (2003); KIM, J Biol.Chem. 239:783-786 (1964)). Another putrescine aminotransferase enzyme is encoded by *spuC* gene of *Pseudomonas aeruginosa* (Lu et al., J Bacteriol. 184:3765-3773 (2002)).

| Gene | GenBank ID | GI Number | Organism |
|---|---|---|---|
| *ygjG* | NP_417544 | 145698310 | *Escherichia coli* |
| *spuC* | AAG03688 | 9946143 | *Pseudomonas aeruginosa* |

Several aminotransferases transaminate the amino groups of amino acid groups to form 2-oxoacids. Aspartate aminotransferase is an enzyme that naturally transfers an oxo group from oxaloacetate to glutamate, forming alpha-ketoglutarate and aspartate. Aspartate aminotransferase activity is catalyzed by, for example, the gene products of *aspC* from *Escherichia coli* (Yagi et al., 100:81-84 (1979); Yagi et al., 113:83-89 (1985)), *AAT2* from *Saccharomyces cerevisiae* (Yagi et al., 92:35-43 (1982)) and *ASP5* from *Arabidopsis thaliana* (Kwok et al., 55:595-604 (2004); de la et al., 46:414-425 (2006); Wilkie et al., Protein Expr.Purif. 12:381-389 (1998)). The enzyme from *Rattus norvegicus* has been shown to transaminate alternate substrates such as 2-aminohexanedioic acid and 2,4-diaminobutyric acid (Rccascns ct al., Biochemistry 19:4583-4589 (1980)). Aminotransferases that work on other amino-acid substrates may also be able to catalyze these transformations. Valine aminotransferase catalyzes the conversion of valine and pyruvate to 2-ketoisovalerate and alanine. The *E. coli* gene, *avtA,* encodes one such enzyme (Whalen et al., J.Bacteriol. 150:739-746 (1982)). This gene product also catalyzes the transamination of α-ketobutyrate to generate α-aminobutyrate, although the amine donor in this reaction has not been identified (Whalen et al., J.Bacteriol. 158:571-574 (1984)). The gene product of the *E. coli serC* catalyzes two reactions, phosphoserine aminotransferase and phosphohydroxythreonine aminotransferase (Lam et al., J.Bacteriol. 172:6518-6528 (1990)), and activity on non-phosphorylated substrates could not be detected (Drewke et al., FEBS.Lett. 390:179-182 (1996)). Another enzyme candidate is alpha-aminoadipate aminotransferase (EC 2.6.1.39), an enzyme that participates in lysine biosynthesis and degradation in some organisms. This enzyme interconverts 2-aminoadipate and 2-oxoadipate, using alpha-ketoglutarate as the amino acceptor. Gene candidates are found in *Homo sapiens* (Okuno et al., Enzyme Protein 47:136-148 (1993)) and *Thermus thermophilus* (Miyazaki et al., Microbiology 150:2327-2334 (2004)). The *Thermus thermophilus* enzyme, encoded by *lysN,* is active with several alternate substrates including oxaloacetate, 2-oxoisocaproate, 2-oxoisovalerate, and 2-oxo-3-methylvalerate.

| Protein | GenBank ID | GI Number | Organism |
|---|---|---|---|
| *aspC* | NP_415448.1 | 16128895 | *Escherichia coli* |
| *AAT2* | P23542.3 | 1703040 | *Saccharomyces cerevisiae* |
| *ASP5* | P46248.2 | 20532373 | *Arabidopsis thaliana* |
| *got2* | P00507 | 112987 | *Rattus norvegicus* |
| *avtA* | YP_026231.1 | 49176374 | *Escherichia coli* |
| *lysN* | BAC76939.1 | 31096548 | *Thermus thermophilus* |
| *AadAT-II* | Q8N5Z0.2 | 46395904 | *Homo sapiens* |

### 2.7.2.a Phosphotransferase (kinase)

Kinase or phosphotransferase enzymes in the EC class 2.7.2 transform carboxylic acids to phosphonic acids with concurrent hydrolysis of one ATP. An enzyme with 2,4-pentadienoate kinase activity is required in step H of Figure 6. Exemplary enzyme candidates include butyrate kinase (EC 2.7.2.7), isobutyrate kinase (EC 2.7.2.14), aspartokinase (EC 2.7.2.4), acetate kinase (EC 2.7.2.1) and glycerate kinase. Butyrate kinase catalyzes the reversible conversion of butyryl-phosphate to butyrate during acidogenesis in *Clostridial* species (Cary et al., Appl Environ Microbiol 56:1576-1583 (1990)). The *Clostridium acetobutylicum* enzyme is encoded by either of the two *buk* gene products (Huang et al., J Mol.Microbiol Biotechnol 2:33-38 (2000)). Other butyrate kinase enzymes are found in *C*. *butyricum* and *C.* tetanomorphum (Twarog et al., J Bacteriol. 86:112-117 (1963)). A related enzyme, isobutyrate kinase from *Thermotoga maritima,* was expressed in *E. coli* and crystallized (Diao et al., J Bacteriol. 191:2521-2529 (2009); Diao et al., Acta Crystallogr.D.Biol.Crystallogr. 59:1100-1102 (2003)). Aspartokinase catalyzes the ATP-dependent phosphorylation of aspartate and participates in the synthesis of several amino acids. The aspartokinase III enzyme in *E. coli,* encoded by *lysC,* has a broad substrate range and the catalytic residues involved in substrate specificity have been elucidated (Keng et al., Arch Biochem Biophys 335:73-81 (1996)). Two additional kinases in *E. coli* are also acetate kinase and gamma-glutamyl kinase. The *E. coli* acetate kinase, encoded by *ackA* (Skarstedt et al., J.Biol. Chem. 251:6775-6783 (1976)), phosphorylates propionate in addition to acetate (Hesslinger et al., Mol.Microbiol 27:477-492 (1998)). The *E. coli* gamma-glutamyl kinase, encoded by *proB* (Smith et al., J.Bacteriol. 157:545-551 (1984)), phosphorylates the gamma carbonic acid group of glutamate.

| **Protein** | **GenBank ID** | **GI Number** | **Organism** |
|---|---|---|---|
| *buk1* | NP_349675 | 15896326 | *Clostridium acetobutylicum* |
| *buk2* | Q97II1 | 20137415 | *Clostridium acetobutylicum* |
| *buk2* | Q9X278.1 | 6685256 | *Thermotoga maritima* |
| *lysC* | NP_418448.1 | 16131850 | *Escherichia coli* |
| *ackA* | NP_416799.1 | 16130231 | *Escherichia coli* |
| *proB* | NP_414777.1 | 16128228 | *Escherichia coli* |

Glycerate kinase (EC 2.7.1.31) activates glycerate to glycerate-2-phosphate or glycerate-3-phosphate. Three classes of glycerate kinase have been identified. Enzymes in class I and II produce glycerate-2-phosphate, whereas the class III enzymes found in plants and yeast produce glycerate-3-phosphate (Bartsch et al., FEBS Lett. 582:3025-3028 (2008)). In a recent study, class III glycerate kinase enzymes from *Saccharomyces cerevisiae, Oryza sativa* and *Arabidopsis thaliana* were heterologously expressed in *E. coli* and characterized (Bartsch et al., FEBS Lett. 582:3025-3028 (2008)). This study also assayed the *glxK* gene product of *E. coli* for ability to form glycerate-3-phosphate and found that the enzyme can only catalyze the formation of glycerate-2-phosphate, in contrast to previous work (Doughty et al., J Biol.Chem. 241:568-572 (1966)).

| **Protein** | **GenBank ID** | **GI Number** | **Organism** |
|---|---|---|---|
| *glxK* | AAC73616.1 | 1786724 | *Escherichia coli* |
| YGR205W | AAS56599.1 | 45270436 | *Saccharomyces cerevisiae* |
| *Os01g0682500* | BAF05800.1 | 113533417 | *Oryza sativa* |
| *At1g80380* | BAH57057.1 | 227204411 | *Arabidopsis thaliana* |

### 2.8.3.a CoA transferase

Enzymes in the 2.8.3 family catalyze the reversible transfer of a CoA moiety from one molecule to another. Such a transformation is required by steps L, P and O of Figure 1, step F of Figure 3, step B of Figure 4, step J of Figure 5 and step F of Figure 6. Several CoA transferase enzymes have been described in the open literature and represent suitable candidates for these steps. These are described below.

Many transferases have broad specificity and thus can utilize CoA acceptors as diverse as acetate, succinate, propionate, butyrate, 2-methylacetoacetate, 3-ketohexanoate, 3-ketopentanoate, valerate, crotonate, 3-mercaptopropionate, propionate, vinylacetate, butyrate, among others. For example, an enzyme from *Roseburia* sp. A2-183 was shown to have butyryl-CoA:acetate:CoA transferase and propionyl-CoA:acetate:CoA transferase activity (Charrier et al., Microbiology 152, 179-185 (2006)). Close homologs can be found in, for example, *Roseburia intestinalis* L1-82, *Roseburia inulinivorans* DSM 16841, *Eubacterium rectale* ATCC 33656. Another enzyme with propionyl-CoA transferase activity can be found in *Clostridium propionicum* (Selmer et al., Eur J Biochem 269, 372-380 (2002)). This enzyme can use acetate, (R)-lactate, (S)-lactate, acrylate, and butyrate as the CoA acceptor (Selmer et al., Eur J Biochem 269, 372-380 (2002); Schweiger and Buckel, FEBS Letters, 171(1) 79-84 (1984)). Close homologs can be found in, for example, *Clostridium novyi* NT, *Clostridium beijerinckii* NCIMB 8052, and *Clostridium botulinum* C str. Eklund. *YgfH* encodes a propionyl CoA:succinate CoA transferase in *E. coli* (Haller et al., Biochemistry, 39(16) 4622-4629). Close homologs can be found in, for example, *Citrobacter youngae* ATCC 29220, *Salmonella enterica* subsp. *arizonae serovar,* and *Yersinia intermedia* ATCC 29909. These proteins are identified below .

| **Protein** | **GenBank ID** | **GI Number** | **Organism** |
|---|---|---|---|
| *Ach1* | AAX19660.1 | 60396828 | *Roseburia* sp. A2-183 |
| *ROSINTL182_*07121 | ZP_04743841.2 | 257413684 | *Roseburia* intestinalis L1-82 |
| *ROSEINA2194_03642* | ZP_03755203.1 | 225377982 | *Roseburia inulinivorans* |
| *EUBREC_3075* | YP_002938937.1 | 238925420 | *Eubacterium rectale ATCC 33656* |
| *Pct* | CAB77207.1 | 7242549 | *Clostridium propionicum* |
| *NT01CX_2372* | YP_878445.1 | 118444712 | *Clostridium novyi NT* |
| *Cbei_4543* | YP_001311608.1 | 150019354 | *Clostridium beijerinckii* |
| *CBC_A0889* | ZP_02621218.1 | 168186583 | *Clostridium botulinum C str. Eklund* |
| *ygfH* | NP_417395.1 | 16130821 | *Escherichia coli* |
| *CIT292_04485* | ZP_03838384.1 | 227334728 | *Citrobacter youngae ATCC 29220* |
| *SARI_04582* | YP_001573497.1 | 161506385 | *Salmonella enterica subsp. arizonae serovar* |
| *yinte0001_14430* | ZP_04635364.1 | 238791727 | *Yersinia intermedia ATCC 29909* |

An additional candidate enzyme is the two-unit enzyme encoded by *pcaI* and *pcaJ* in *Pseudomonas,* which has been shown to have 3-oxoadipyl-CoA/succinate transferase activity (Kaschabek et al., *supra*)*.* Similar enzymes based on homology exist in *Acinetobacter* sp. ADP1 (Kowalchuk et al., Gene 146:23-30 (1994)) and *Streptomyces coelicolor.* Additional exemplary succinyl-CoA:3:oxoacid-CoA transferases are present in *Helicobacter pylori* (Corthesy-Theulaz et al., J.Biol.Chem. 272:25659-25667 (1997)) and *Bacillus subtilis* (Stols et al., Protein.Expr.Purif. 53:396-403 (2007)). These proteins are identified below.

| **Protein** | **GenBank ID** | **GI Number** | **Organism** |
|---|---|---|---|
| *pcaI* | AAN69545.1 | 24985644 | *Pseudomonas putida* |
| *pcaJ* | NP_746082.1 | 26990657 | *Pseudomonas putida* |
| *pcaI* | YP_046368.1 | 50084858 | *Acinetobacter sp. ADP1* |
| *pcaJ* | AAC37147.1 | 141776 | *Acinetobacter sp. ADP1* |
| *pcaI* | NP_630776.1 | 21224997 | *Streptomyces coelicolor* |
| *pcaJ* | NP_630775.1 | 21224996 | *Streptomyces coelicolor* |
| *HPAG1_0676* | YP_627417 | 108563101 | *Helicobacter pylori* |
| *HPAG1_0677* | YP_627418 | 108563102 | *Helicobacter pylori* |
| *ScoA* | NP_391778 | 16080950 | *Bacillus subtilis* |
| *ScoB* | NP_391777 | 16080949 | *Bacillus subtilis* |

A CoA transferase that can utilize acetate as the CoA acceptor is acetoacetyl-CoA transferase, encoded by the *E. coli atoA* (alpha subunit) and *atoD* (beta subunit) genes (Vanderwinkel et al., Biochem.Biophys.Res Commun. 33:902-908 (1968); Korolev et al., Acta Crystallogr.D Biol Crystallogr, 58:2116-2121 (2002)). This enzyme has also been shown to transfer the CoA moiety to acetate from a variety of branched and linear acyl-CoA substrates, including isobutyrate (Matthies et al., Appl Environ Microbiol 58:1435-1439 (1992)), valerate (Vanderwinkel et al., *supra)* and butanoate (Vanderwinkel et al., *supra*)*.* Similar enzymes exist in *Corynebacterium glutamicum* ATCC 13032 (Duncan et al., Appl Environ Microbiol 68:5186-5190 (2002)), *Clostridium acetobutylicum* (Cary et al., Appl Environ Microbiol 56:1576-1583 (1990)), and *Clostridium saccharoperbutylacetonicum* (Kosaka et al., Biosci.Biotechnol Biochem. 71:58-68 (2007)). These proteins are identified below.

| **Protein** | **GenBank ID** | **GI Number** | **Organism** |
|---|---|---|---|
| *atoA* | P76459.1 | 2492994 | *Escherichia coli K12* |
| *atoD* | P76458.1 | 2492990 | *Escherichia coli K12* |
| *actA* | YP_226809.1 | 62391407 | *Corynebacterium glutamicum ATCC 13032* |
| *cg0592* | YP_224801.1 | 62389399 | *Corynebacterium glutamicum ATCC 13032* |
| *ctfA* | NP_149326.1 | 15004866 | *Clostridium acetobutylicum* |
| *ctfB* | NP_149327.1 | 15004867 | *Clostridium acetobutylicum* |
| *ctfA* | AAP42564.1 | 31075384 | *Clostridium saccharoperbutylacetonicum* |
| *ctfB* | AAP42565.1 | 31075385 | *Clostridium saccharoperbutylacetonicum* |

Additional exemplary transferase candidates are catalyzed by the gene products of *cat1*, *cat2,* and *cat3* of *Clostridium kluyveri* which have been shown to exhibit succinyl-CoA, 4-hydroxybutyryl-CoA, and butyryl-CoA transferase activity, respectively (Seedorf et al., *supra;* Sohling et al., Eur.J Biochem. 212:121-127 (1993); Sohling et al., J Bacteriol. 178:871-880 (1996)). Similar CoA transferase activities are also present in *Trichomonas vaginalis* (van Grinsven et al., J.Biol.Chem. 283:1411-1418 (2008)) and *Trypanosoma brucei* (Riviere et al., J.Biol.Chem. 279:45337-45346 (2004)). These proteins are identified below.

| **Protein** | **GenBank ID** | **GI Number** | **Organism** |
|---|---|---|---|
| *cat1* | P38946.1 | 729048 | *Clostridium kluyveri* |
| *cat2* | P38942.2 | 172046066 | *Clostridium kluyveri* |
| *cat3* | EDK35586.1 | 146349050 | *Clostridium kluyveri* |
| *TVAG_395550* | XP_001330176 | 123975034 | *Trichomonas vaginalis G3* |
| *Tb11.02.0290* | XP_828352 | 71754875 | *Trypanosoma brucei* |

The glutaconate-CoA-transferase (EC 2.8.3.12) enzyme from anaerobic bacterium *Acidaminococcus fermentans* reacts with diacid glutaconyl-CoA and 3-butenoyl-CoA (Mack et al., FEBS Lett. 405:209-212 (1997)). The genes encoding this enzyme are *gctA* and *gctB.* This enzyme has reduced but detectable activity with other CoA derivatives including glutaryl-CoA, 2-hydroxyglutaryl-CoA, adipyl-CoA and acrylyl-CoA (Buckel et al., Eur.J.Biochem. 118:315-321 (1981)). The enzyme has been cloned and expressed in *E. coli* (Mack et al., Eur. J.Biochem. 226:41-51 (1994)). These proteins are identified below.

| **Protein** | **GenBank ID** | **GI Number** | **Organism** |
|---|---|---|---|
| *gctA* | CAA57199.1 | 559392 | *Acidaminococcus fermentans* |
| *gctB* | CAA57200.1 | 559393 | *Acidaminococcus fermentans* |

### 3.1.2.a CoA hydrolase

Enzymes in the 3.1.2 family hydrolyze acyl-CoA molecules to their corresponding acids. Such a transformation is required by step O of Figure 1, step F of Figure 3, step B of Figure 4, step J of Figure 5 and step F of Figure 6. Several such enzymes have been described in the literature and represent suitable candidates for these steps.

For example, the enzyme encoded by *acot12* from *Rattus norvegicus* brain (Robinson et al., Biochem.Biophys.Res.Commun. 71:959-965 (1976)) can react with butyryl-CoA, hexanoyl-CoA and malonyl-CoA. The human dicarboxylic acid thioesterase, encoded by *acot8,* exhibits activity on glutaryl-CoA, adipyl-CoA, suberyl-CoA, sebacyl-CoA, and dodecanedioyl-CoA (Westin et al., J.Biol.Chem. 280:38125-38132 (2005)). The closest *E*. *coli* homolog to this enzyme, *tesB,* can also hydrolyze a range of CoA thiolesters (Naggert et al., J Biol Chem 266:11044-11050 (1991)). A similar enzyme has also been characterized in the rat liver (Deana R., Biochem Int 26:767-773 (1992)). Additional enzymes with hydrolase activity in *E. coli* include *ybgC, paaI,* and *ybdB* (Kuznetsova, et al., FEMS Microbiol Rev, 2005, 29(2):263-279; Song et al., J Biol Chem, 2006, 281(16):11028-38). Though its sequence has not been reported, the enzyme from the mitochondrion of the pea leaf has a broad substrate specificity, with demonstrated activity on acetyl-CoA, propionyl-CoA, butyryl-CoA, palmitoyl-CoA, oleoyl-CoA, succinyl-CoA, and crotonyl-CoA (Zeiher et al., Plant.Physiol. 94:20-27 (1990)) The acetyl-CoA hydrolase, *ACH1,* from *S. cerevisiae* represents another candidate hydrolase (Buu et al., J.Biol.Chem. 278:17203-17209 (2003)).

| Protein | GenBank Accession # | GI# | Organism |
|---|---|---|---|
| *acot12* | NP_570103.1 | 18543355 | *Rattus norvegicus* |
| *tesB* | NP_414986 | 16128437 | *Escherichia coli* |
| *acot8* | CAA15502 | 3191970 | *Homo sapiens* |
| *acot8* | NP_570112 | 51036669 | *Rattus norvegicus* |
| *tesA* | NP_415027 | 16128478 | *Escherichia coli* |
| *ybgC* | NP_415264 | 16128711 | *Escherichia coli* |
| *paaI* | NP_415914 | 16129357 | *Escherichia coli* |
| *ybdB* | NP_415129 | 16128580 | *Escherichia coli* |
| *ACH1* | NP_009538 | 6319456 | *Saccharomyces cerevisiae* |

Additional hydrolase enzymes include 3-hydroxyisobutyryl-CoA hydrolase which has been described to efficiently catalyze the conversion of 3-hydroxyisobutyryl-CoA to 3-hydroxyisobutyrate during valine degradation (Shimomura et al., J Biol Chem. 269:14248-14253 (1994)). Genes encoding this enzyme include *hibch* of *Rattus norvegicus* (Shimomura et al., Methods Enzymol. 324:229-240 (2000)) and *Homo sapiens* (Shimomura et al., *supra).* Similar gene candidates can also be identified by sequence homology, including *hibch* of *Saccharomyces cerevisiae* and *BC_2292* of *Bacillus cereus.*

| Protein | GenBank Accession # | GI# | Organism |
|---|---|---|---|
| *hibch* | Q5XIE6.2 | 146324906 | *Rattus norvegicus* |
| *hibch* | Q6NVY1.2 | 146324905 | *Homo sapiens* |
| *hibch* | P28817.2 | 2506374 | *Saccharomyces cerevisiae* |
| *BC_2292* | AP09256 | 29895975 | *Bacillus cereus* |

Yet another candidate hydrolase is the glutaconate CoA-transferase from *Acidaminococcus fermentans.* This enzyme was transformed by site-directed mutagenesis into an acyl-CoA hydrolase with activity on glutaryl-CoA, acetyl-CoA and 3-butenoyl-CoA (Mack et al., FEBS.Lett. 405:209-212 (1997)).This suggests that the enzymes encoding succinyl-CoA:3-ketoacid-CoA transferases and acetoacetyl-CoA:acetyl-CoA transferases may also serve as candidates for this reaction step but would require certain mutations to change their function. GeneBank accession numbers for the *gctA* and *gctB* genes are listed above.

### 4.1.1.a Decarboxylase

Decarboxylase enzymes in the EC class 4.1.1 are required to catalyze steps A, D, T and U of Figure 1, step D of Figure 2, step C of Figure 4, steps C and F of Figure 5 and step G of Figure 6.

The decarboxylation reactions of 2,4-pentadienoate to butadiene (step T of Figure 1 and step G of Figure 6) and 5-hydroxypent-2-enoate to 3-buten-1-ol (step U of Figure 1) are catalyzed by enoic acid decarboxylase enzymes. Exemplary enzymes are sorbic acid decarboxylase, aconitate decarboxylase, 4-oxalocrotonate decarboxylase and cinnamate decarboxylase. Sorbic acid decarboxylase converts sorbic acid to 1,3-pentadiene. Sorbic acid decarboxylation by *Aspergillus niger* requires three genes: *padA1, ohbA1,* and *sdrA* (Plumridge et al. Fung. Genet. Bio, 47:683-692 (2010). *PadA1* is annotated as a phenylacrylic acid decarboxylase, *ohbA1* is a putative 4-hydroxybenzoic acid decarboxylase, and *sdrA* is a sorbic acid decarboxylase regulator. Additional species have also been shown to decarboxylate sorbic acid including several fungal and yeast species (Kinderlerler and Hatton, Food Addit Contam., 7(5):657-69 (1990); Casas et al., IntJ Food Micro., 94(1):93-96 (2004); Pinches and Apps, Int. J. Food Microbiol. 116: 182-185 (2007)). For example, *Aspergillus oryzae* and *Neosartorya fischeri* have been shown to decarboxylate sorbic acid and have close homologs to *padA1, ohbA1,* and *sdrA.*

| Gene name | GenBankID | GI Number | Organism |
|---|---|---|---|
| *padA1* | XP_001390532.1 | 145235767 | *Aspergillus niger* |
| *ohbA1* | XP_001390534.1 | 145235771 | *Aspergillus niger* |
| *sdrA* | XP_001390533.1 | 145235769 | *Aspergillus niger* |
| *padA1* | XP_001818651.1 | 169768362 | *Aspergillus oryzae* |
| *ohbA1* | XP_001818650.1 | 169768360 | *Aspergillus oryzae* |
| *sdrA* | XP_001818649.1 | 169768358 | *Aspergillus oryzae* |
| *padA1* | XP_001261423.1 | 119482790 | *Neosartorya fischeri* |
| *ohbA1* | XP_001261424.1 | 119482792 | *Neosartorya fischeri* |
| *sdrA* | XP_001261422.1 | 119482788 | *Neosartorya fischeri* |

Aconitate decarboxylase (EC 4.1.1.6) catalyzes the final step in itaconate biosynthesis in a strain of *Candida* and also in the filamentous fungus *Aspergillus terreus* (Bonnarme et al. J Bacteriol. 177:3573-3578 (1995); Willke and Vorlop, Appl Microbiol. Biotechnol 56:289-295 (2001)). A cis-aconitate decarboxylase (CAD) (EC 4.1.16) has been purified and characterized from *Aspergillus terreus* (Dwiarti et al., J. Biosci. Bioeng. 94(1): 29-33 (2002)). Recently, the gene has been cloned and functionally characterized (Kanamasa et al., Appl.Microbiol Biotechnol 80:223-229 (2008)) and (WO/2009/014437). Several close homologs of CAD are listed below (EP 2017344A1; WO 2009/014437 A1). The gene and protein sequence of CAD were reported previously (EP 2017344 A1; WO 2009/014437 A1), along with several close homologs listed in the table below.

| Gene name | GenBankID | GI Number | Organism |
|---|---|---|---|
| *CAD* | XP_001209273 | 115385453 | *Aspergillus terreus* |
| | XP_001217495 | 115402837 | *Aspergillus terreus* |
| | XP_001209946 | 115386810 | *Aspergillus terreus* |
| | BAE66063 | 83775944 | *Aspergillus oryzae* |
| | XP_001393934 | 145242722 | *Aspergillus niger* |
| | XP_391316 | 46139251 | *Gibberella zeae* |
| | XP_001389415 | 145230213 | *Aspergillus niger* |
| | XP_001383451 | 126133853 | *Pichia stipitis* |
| | YP_891060 | 118473159 | *Mycobacterium smegmatis* |
| | NP_961187 | 41408351 | *Mycobacterium avium subsp. pratuberculosis* |
| | YP_880968 | 118466464 | *Mycobacterium avium* |
| | ZP_01648681 | 119882410 | *Salinispora arenicola* |

An additional class of decarboxylases has been characterized that catalyze the conversion of cinnamate (phenylacrylate) and substituted cinnamate derivatives to the corresponding styrene derivatives. These enzymes are common in a variety of organisms and specific genes encoding these enzymes that have been cloned and expressed in *E. coli* are: *pad 1* from *Saccharomyces cerevisae* (Clausen et al., Gene 142:107-112 (1994)), *pdc* from *Lactobacillus plantarum* (Barthelmebs et al., 67:1063-1069 (2001); Qi et al., Metab Eng 9:268-276 (2007); Rodriguez et al., J.Agric.Food Chem. 56:3068-3072 (2008)), *pofK* (*pad*) from *Klebsiella oxytoca* (Uchiyama ct al., Biosci.Biotechnol.Biochem. 72:116-123 (2008); Hashidoko et al., Biosci.Biotech.Biochem. 58:217-218 (1994)), *Pedicoccus pentosaceus* (Barthelmebs et al., 67:1063-1069 (2001)), and *padC* from *Bacillus subtilis* and *Bacillus pumilus* (Shinglcr ct al., 174:711-724 (1992)). A fcrulic acid decarboxylase from *Pseudomonas fluorescens* also has been purified and characterized (Huang et al., J.Bacteriol. 176:5912-5918 (1994)). Importantly, this class of enzymes have been shown to be stable and do not require either exogenous or internally bound co-factors, thus making these enzymes ideally suitable for biotransformations (Sariaslani, Annu.Rev.Microbiol. 61:51-69 (2007)).

| Protein | GenBank ID | GI Number | Organism |
|---|---|---|---|
| *pad1* | AAB64980.1 | 1165293 | *Saccharomyces cerevisae* |
| *pdc* | AAC45282.1 | 1762616 | *Lactobacillus plantarum* |
| *pad* | BAF65031.1 | 149941608 | *Klebsiella oxytoca* |
| *padC* | NP_391320.1 | 16080493 | *Bacillus subtilis* |
| *pad* | YP_804027.1 | 116492292 | *Pedicoccus pentosaceus* |
| *pad* | CAC18719.1 | 11691810 | *Bacillus pumilus* |

4-Oxalocronate decarboxylase catalyzes the decarboxylation of 4-oxalocrotonate to 2-oxopentanoate. This enzyme has been isolated from numerous organisms and characterized. The decarboxylase typically functions in a complex with vinylpyruvate hydratase. Genes encoding this enzyme include *dmpH* and *dmpE* in *Pseudomonas sp. (strain 600)* (Shingler et al., 174:711-724 (1992)), *xyIII* and *xylIII* from *Pseudomonas putida* (Kato et al., Arch.Microbiol 168:457-463 (1997); Stanley et al., Biochemistry 39:3514 (2000); Lian et al., J.Am.Chem.Soc. 116:10403-10411 (1994)) and *Reut_B5691* and *Reut_B5692* from *Ralstonia eutropha JMP134* (Hughes et al., J Bacteriol, 158:79-83 (1984)). The genes encoding the enzyme from *Pseudomonas sp. (strain 600)* have been cloned and expressed in *E. coli* (Shingler et al., J. Bacteriol. 174:711-724 (1992)). The 4-oxalocrotonate decarboxylase encoded by *xylI* in *Pseudomonas putida* functions in a complex with vinylpyruvate hydratase. A recombinant form of this enzyme devoid of the hydratase activity and retaining wild type decarboxylase activity has been characterized (Stanley et al., Biochem. 39:718-26 (2000)). A similar enzyme is found in *Ralstonia pickettii* (formerly *Pseudomonas pickettii*) (Kukor et al., J Bacteriol. 173:4587-94 (1991)).

| Gene | GenBank | GI Number | Organism |
|---|---|---|---|
| *dmpH* | CAA43228.1 | 45685 | *Pseudomonas sp. CF600* |
| *dmpE* | CAA43225.1 | 45682 | *Pseudomonas sp. CF600* |
| *xylII* | YP_709328.1 | 111116444 | *Pseudomonas putida* |
| *xylIII* | YP_709353.1 | 111116469 | *Pseudomonas putida* |
| *Reut_B5691* | YP_299880.1 | 73539513 | *Ralstonia eutropha JMP134* |
| *Reut_B5692* | YP_299881.1 | 73539514 | *Ralstonia eutropha JMP134* |
| *xylI* | P49155.1 | 1351446 | *Pseudomonas putida* |
| *tbuI* | YP_002983475.1 | 241665116 | *Ralstonia pickettii* |
| *nbaG* | BAC65309.1 | 28971626 | *Pseudomonas fluorescens KU-7* |

The decarboxylation of 2-keto-acids such as 2-oxoadipate (step D of Figure 1) is catalyzed by a variety of enzymes with varied substrate specificities, including pyruvate decarboxylase (EC 4.1.1.1), benzoylformate decarboxylase (EC 4.1.1.7), alpha-ketoglutarate decarboxylase and branched-chain alpha-ketoacid decarboxylase. Pyruvate decarboxylase (PDC), also termed keto-acid decarboxylase, is a key enzyme in alcoholic fermentation, catalyzing the decarboxylation of pyruvate to acetaldehyde. The enzyme from *Saccharomyces cerevisiae* has a broad substrate range for aliphatic 2-keto acids including 2-ketobutyrate, 2-ketovalerate, 3-hydroxypyruvate and 2-phenylpyruvate (22). This enzyme has been extensively studied, engineered for altered activity, and functionally expressed in *E. coli* (Killenberg-Jabs et al., Eur.J.Biochem. 268:1698-1704 (2001); Li et al., Biochemistry. 38:10004-10012 (1999); ter Schure et al., Appl.Environ.Microbiol. 64:1303-1307 (1998)). The PDC from *Zymomonas mobilus,* encoded by *pdc,* also has a broad substrate range and has been a subject of directed engineering studies to alter the affinity for different substrates (Siegert et al., Protein Eng Des Sel 18:345-357 (2005)). The crystal structure of this enzyme is available (Killenberg-Jabs et al., Eur.J.Biochem. 268:1698-1704 (2001)). Other well-characterized PDC candidates include the enzymes from *Acetobacter pasteurians* (Chandra et al., 176:443-451 (2001)) and *Kluyveromyces lactis* (Krieger et al., 269:3256-3263 (2002)).

| Protein | GenBank ID | GI Number | Organism |
|---|---|---|---|
| *pdc* | P06672.1 | 118391 | *Zymomonas mobilis* |
| *pdc1* | P06169 | 30923172 | *Saccharomyces cerevisiae* |
| *pdc* | Q8L388 | 20385191 | *Acetobacter pasteurians* |
| *pdc1* | Q12629 | 52788279 | *Kluyveromyces lactis* |

Like PDC, benzoylformate decarboxylase (EC 4.1.1.7) has a broad substrate range and has been the target of enzyme engineering studies. The enzyme from *Pseudomonas putida* has been extensively studied and crystal structures of this enzyme are available (Polovnikova et al., 42:1820-1830 (2003); Hasson et al., 37:9918-9930 (1998)). Site-directed mutagenesis of two residues in the active site of the *Pseudomonas putida* enzyme altered the affinity (Km) of naturally and non-naturally occurring substrates (Siegert et al., Protein Eng Des Sel 18:345-357 (2005)). The properties of this enzyme have been further modified by directed engineering (Lingen et al., Chembiochem. 4:721-726 (2003); Lingen et al., Protein Eng 15:585-593 (2002)). The enzyme from *Pseudomonas aeruginosa,* encoded by *mdlC,* has also been characterized experimentally (Barrowman et al., 34:57-60 (1986)). Additional gene candidates from *Pseudomonas stutzeri, Pseudomonas fluorescens* and other organisms can be inferred by sequence homology or identified using a growth selection system developed in *Pseudomonas putida* (Henning et al., Appl.Environ.Microbiol. 72:7510-7517 (2006)).

| Protein | GenBank ID | GI Number | Organism |
|---|---|---|---|
| *mdlC* | P20906.2 | 3915757 | *Pseudomonas putida* |
| *mdlC* | Q9HUR2.1 | 81539678 | *Pseudomonas aeruginosa* |
| *dpgB* | ABN80423.1 | 126202187 | *Pseudomonas stutzeri* |
| *ilvB-1* | YP_260581.1 | 70730840 | *Pseudomonas fluorescens* |

A third enzyme capable of decarboxylating 2-oxoacids is alpha-ketoglutarate decarboxylase (KGD). The substrate range of this class of enzymes has not been studied to date. The KDC from *Mycobacterium tuberculosis* (Tian et al., 102:10670-10675 (2005)) has been cloned and functionally expressed. KDC enzyme activity has been detected in several species of rhizobia including *Bradyrhizobium japonicum* and *Mesorhizobium loti* (Green et al., 182:2838-2844 (2000)). Although the KDC-encoding gene(s) have not been isolated in these organisms, the genome sequences are available and several genes in each genome are annotated as putative KDCs. A KDC from *Euglena gracilis* has also been characterized but the gene associated with this activity has not been identified to date (Shigeoka et al., Arch.Biochem.Biophys. 288:22-28 (1991)). The first twenty amino acids starting from the N-terminus were sequenced MTYKAPVKDVKFLLDKVFKV (SEQ ID NO.) (Shigeoka and Nakano, Arch.Biochem.Biophys. 288:22-28 (1991)). The gene could be identified by testing candidate genes containing this N-terminal sequence for KDC activity.

| Protein | GenBank ID | GI Number | Organism |
|---|---|---|---|
| *kgd* | O50463.4 | 160395583 | *Mycobacterium tuberculosis* |
| *kgd* | NP_767092.1 | 27375563 | *Bradyrhizobium japonicum USDA110* |
| *kgd* | NP_105204.1 | 13473636 | *Mesorhizobium loti* |

A fourth candidate enzyme for catalyzing this reaction is branched chain alpha-ketoacid decarboxylase (BCKA). This class of enzyme has been shown to act on a variety of compounds varying in chain length from 3 to 6 carbons (Oku et al., J Biol Chem. 263:18386-18396 (1988); Smit et al., Appl Environ Microbiol 71:303-311 (2005)). The enzyme in *Lactococcus lactis* has been characterized on a variety of branched and linear substrates including 2-oxobutanoate, 2-oxohexanoate, 2-oxopentanoate, 3-methyl-2-oxobutanoate, 4-methyl-2-oxobutanoate and isocaproate (Smit et al., Appl Environ Microbiol 71:303-311 (2005)). The enzyme has been structurally characterized (Berg et al., Science. 318:1782-1786 (2007)). Sequence alignments between the *Lactococcus lactis* enzyme and the pyruvate decarboxylase of *Zymomonas mobilus* indicate that the catalytic and substrate recognition residues are nearly identical (Siegert et al., Protein Eng Des Sel 18:345-357 (2005)), so this enzyme would be a promising candidate for directed engineering. Decarboxylation of alpha-ketoglutarate by a BCKA was detected in *Bacillus subtilis;* however, this activity was low (5%) relative to activity on other branched-chain substrates (Oku and Kaneda, J Biol Chem. 263:18386-18396 (1988)) and the gene encoding this enzyme has not been identified to date. Additional BCKA gene candidates can be identified by homology to the *Lactococcus lactis* protein sequence. Many of the high-scoring BLASTp hits to this enzyme are annotated as indolepyruvate decarboxylases (EC 4.1.1.74). Indolepyruvate decarboxylase (IPDA) is an enzyme that catalyzes the decarboxylation of indolepyruvate to indoleacetaldehyde in plants and plant bacteria. Recombinant branched chain alpha-keto acid decarboxylase enzymes derived from the E1 subunits of the mitochondrial branched-chain keto acid dehydrogenase complex from *Homo sapiens* and *Bos taurus* have been cloned and functionally expressed in *E. coli* (Davie et al., J.Biol.Chem. 267:16601-16606 (1992); Wynn et al., J.Biol.Chem. 267:12400-12403 (1992); Wynn et al., J.Biol.Chem. 267:1881-1887 (1992)). In these studies, the authors found that co-expression of chaperonins GroEL and GroES enhanced the specific activity of the decarboxylase by 500-fold (Wynn et al., J.Biol.Chem. 267:12400-12403 (1992)). These enzymes are composed of two alpha and two beta subunits.

| Protein | GenBank ID | GI Number | Organism |
|---|---|---|---|
| *kdcA* | AAS49166.1 | 44921617 | *Lactococcus lactis* |
| *BCKDHB* | NP_898871.1 | 34101272 | *Homo sapiens* |
| *BCKDHA* | NP_000700.1 | 11386135 | *Homo sapiens* |
| *BCKDHB* | P21839 | 115502434 | *Bos taurus* |
| *BCKDHA* | P11178 | 129030 | *Bos taurus* |

A decarboxylase enzyme suitable for decarboxylating 3-ketoacids is acetoacetate decarboxylase (EC 4.1.1.4). The enzyme from *Clostridium acetobutylicum,* encoded by *adc,* has a broad substrate specificity and has been shown to decarboxylate numerous alternate substrates including 2-ketocyclohexane carboxylate, 3-oxopentanoate, 2-oxo-3-phenylpropionic acid, 2-methyl-3-oxobutyrate and benzoyl-acetate (Rozzel et al., J.Am.Chem.Soc. 106:4937-4941 (1984); Benner and Rozzell, J.Am.Chem.Soc. 103:993-994 (1981*);* Autor et al., J Biol. Chem. 245:5214-5222 (1970*)).* An acetoacetate decarboxylase has also been characterized in *Clostridium beijerinckii* (Ravagnani et al., Mol.Microbiol 37:1172-1185 (2000)). The acetoacetate decarboxylase from *Bacillus polymyxa,* characterized in cell-free extracts, also has a broad substrate specificity for 3-keto acids and can decarboxylate 3-oxopentanoate (Matiasek et al., Curr.Microbiol 42:276-281 (2001)). The gene encoding this enzyme has not been identified to date and the genome sequence of *B. polymyxa* is not yet available. Another *adc* is found in *Clostridium saccharoperbutylacetonicum* (Kosaka, et al., Biosci.Biotechnol Biochem. 71:58-68 (2007)). Additional gene candidates in other organisms, including *Clostridium botulinum* and *Bacillus amyloliquefaciens FZB42,* can be identified by sequence homology.

| Protein | GenBank ID | GI Number | Organism |
|---|---|---|---|
| *adc* | NP_149328.1 | 15004868 | *Clostridium acetobutylicum* |
| *adc* | AAP42566.1 | 31075386 | *Clostridium saccharoperbutylacetonicum* |
| *adc* | YP_001310906.1 | 150018652 | *Clostridium beijerinckii* |
| *CLL_*A2135 | YP_001886324.1 | 187933144 | *Clostridium botulinum* |
| *RBAM_*030030 | YP_001422565.1 | 154687404 | *Bacillus amyloliquefaciens* |

Numerous characterized enzymes dccarboxylatc amino acids and similar compounds, including aspartate decarboxylase, lysine decarboxylase and ornithine decarboxylase. Aspartate decarboxylase (EC 4.1.1.11) decarboxylates aspartate to form beta-alanine. This enzyme participates in pantothenate biosynthesis and is encoded by gene *panD* in *Escherichia coli* (Dusch et al., Appl.Environ.Microbiol 65:1530-1539 (1999); Ramjee et al., Biochem.J 323 (Pt 3):661-669 (1997); Merkel et al., FEMS Microbiol Lett. 143:247-252 (1996); Schmitzberger et al., EMBO J 22:6193-6204 (2003)). The enzymes from *Mycobacterium tuberculosis* (Chopra et al., Protein Expr.Purif. 25:533-540 (2002)) and *Corynebacterium glutanicum* (Dusch et al., Appl.Environ.Microbiol 65:1530-1539 (1999)) have been expressed and characterized in *E. coli.*

| Protein | GenBank ID | GI Number | Organism |
|---|---|---|---|
| *panD* | P0A790 | 67470411 | *Escherichia coli K12* |
| *panD* | Q9X4N0 | 18203593 | *Corynebacterium glutanicum* |
| *panD* | P65660.1 | 54041701 | *Mycobacterium tuberculosis* |

Lysine decarboxylase (EC 4.1.1.18) catalyzes the decarboxylation of lysine to cadaverine. Two isozymes of this enzyme are encoded in the *E. coli* genome by genes *cadA* and *ldcC.* CadA is involved in acid resistance and is subject to positive regulation by the *cadC* gene product (Lemonnier et al., Microbiology 144 (Pt 3):751-760 (1998)). CadC accepts hydroxylysine and S-aminoethylcysteine as alternate substrates, and 2-aminopimelate and 6-aminocaproate act as competitive inhibitors to this enzyme (Sabo et al., Biochemistry 13:662-670 (1974)). The constitutively expressed *ldc* gene product is less active than CadA (Lemonnier and Lane, Microbiology 144 (Pt 3):751-760 (1998)). A lysine decarboxylase analogous to CadA was recently identified in *Vibrio parahaemolyticus* (Tanaka et al., J Appl Microbiol 104:1283-1293 (2008)). The lysine decarboxylase from *Selenomonas ruminantium,* encoded by *ldc,* bears sequence similarity to eukaryotic ornithine decarboxylases, and accepts both L-lysine and L-ornithine as substrates (Takatsuka et al., Biosci.Biotechnol Biochem. 63:1843-1846 (1999)). Active site residues were identified and engineered to alter the substrate specificity of the enzyme (Takatsuka et al., J Bacteriol. 182:6732-6741 (2000)). Several ornithine decarboxylase enzymes (EC 4.1.1.17) also exhibit activity on lysine and other similar compounds. Such enzymes are found in *Nicotiana glutinosa* (Lee et al., Biochem.J 360:657-665 (2001)), *Lactobacillus sp. 30a* (Guirard et al., J Biol.Chem. 255:5960-5964 (1980)) and *Vibrio vulnificus* (Lee et al., J Biol.Chem. 282:27115-27125 (2007)). The enzymes from *Lactobacillus sp. 30a* (Momany et al., J Mol.Biol. 252:643-655 (1995)) and *V. vulnificus* have been crystallized. The *V. vulnificus* enzyme efficiently catalyzes lysine decarboxylation and the residues involved in substrate specificity have been elucidated (Lee et al., J Biol. Chem. 282:27115-27125 (2007)). A similar enzyme has been characterized in *Trichomonas vaginalis* but the gene encoding this enzyme is not known (Yarlett et al., Biochem.J 293 (Pt 2):487-493 (1993)).

| Protein | GenBank ID | GI Number | Organism |
|---|---|---|---|
| *cadA* | AAA23536.1 | 145458 | *Escherichia coli* |
| *ldcC* | AAC73297.1 | 1786384 | *Escherichia coli* |
| *ldc* | 050657.1 | 13124043 | *Selenomonas ruminantium* |
| *cadA* | AB124819.1 | 44886078 | *Vibrio parahaemolyticus* |
| *AF323910.1:1..1299* | AAG45222.1 | 12007488 | *Nicotiana glutinosa* |
| *odc1* | P43099.2 | 1169251 | *Lactobacillus sp. 30a* |
| *VV2_1235* | NP_763142.1 | 27367615 | *Vibrio vulnificus* |

Glutaryl-CoA dehydrogenase (GCD, EC 1.3.99.7 and EC 4.1.1.70) is a bifunctional enzyme that catalyzes the oxidative decarboxylation of glutaryl-CoA to crotonyl-CoA (Figure 3, step 3). Bifunctional GCD enzymes are homotetramers that utilize electron transfer flavoprotein as an electron acceptor (Hartcl ct al., Arch.Microbiol 159:174-181 (1993)). Such enzymes were first characterized in cell extracts of *Pseudomonas* strains KB740 and K172 during growth on aromatic compounds (Hartel et al., Arch.Microbiol 159:174-181 (1993)), but the associated genes in these organisms is unknown. Genes encoding glutaryl-CoA dehydrogenase (*gcdH*) and its cognate transcriptional regulator (*gcdR*) were identified in *Azoarcus sp. CIB* (Blazquez et al., Environ.Microbiol 10:474-482 (2008)). An *Azoarcus* strain deficient in gcdH activity was used to identify a heterologous *gcdH* gene from *Pseudomonas putida* (Blazquez et al., Environ.Microbiol 10:474-482 (2008)). The cognate transcriptional regulator in *Pseudomonas putida* has not been identified but the locus *PP_0157* has a high sequence homology (> 69% identity) to the *Azoarcus* enzyme. Additional GCD enzymes are found in *Pseudomonas fluorescens* and *Paracoccus denitrificans* (Husain et al., J Bacteriol. 163:709-715 (1985)). The human GCD has been extensively studied, overexpressed in *E. coli* (Dwyer et al., Biochemistry 39:11488-11499 (2000)), crystallized, and the catalytic mechanism involving a conserved glutamate residue in the active site has been described (Fu et al., Biochemistry 43:9674-9684 (2004)). A GCD in *Syntrophus aciditrophicus* operates in the CO₂-assimilating direction during growth on crotonate (Mouttaki et al., 73:930-938 (2007))). Two GCD genes in *S*. *aciditrophicus* were identified by protein sequence homology to the *Azoarcus* GcdH: *syn*_*00480* (31%) and *syn*_*01146* (31%). No significant homology was found to the *Azoarcus* GcdR regulatory protein.

| Protein | GenBank ID | GI Number | Organism |
|---|---|---|---|
| *gcdH* | ABM69268.1 | 123187384 | *Azoarcus sp. CIB* |
| *gcdR* | ABM69269.1 | 123187385 | *Azoarcus sp. CIB* |
| *gcdH* | AAN65791.1 | 24981507 | *Pseudomonas putida KT2440* |
| *PP_0157* (*gcdR*) | AAN65790.1 | 24981506 | *Pseudomonas putida KT2440* |
| *gcdH* | YP_257269.1 | 70733629 | *Pseudomonas fluorescens Pf-5,* |
| *gcvA* (*gcdR*) | YP_257268.1 | 70733628 | *Pseudomonas fluorescens Pf-5* |
| *gcd* | YP_918172.1 | 119387117 | *Paracoccus denitrificans* |
| *gcdR* | YP_918173.1 | 119387118 | *Paracoccus denitrificans* |
| *gcd* | AAH02579.1 | 12803505 | *Homo sapiens* |
| *syn_00480* | ABC77899 | 85722956 | *Syntrophus aciditrophicus* |
| *syn_01146* | ABC76260 | 85721317 | *Syntrophus aciditrophicus* |

Alternatively, the carboxylation of crotonyl-CoA to glutaconyl-CoA and subsequent reduction to glutaryl-CoA can be catalyzed by separate enzymes: glutaconyl-CoA decarboxylase and glutaconyl-CoA reductase. Glutaconyl-CoA decarboxylase enzymes, characterized in glutamate-fermenting anaerobic bacteria, are sodium-ion translocating decarboxylases that utilize biotin as a cofactor and are composed of four subunits (alpha, beta, gamma, and delta) (Boiangiu et al., J Mol.Microbiol Biotechnol 10:105-119 (2005); Buckel, Biochim.Biophys.Acta 1505:15-27 (2001)). Such enzymes have been characterized in *Fusobacterium nucleatum* (Beatrix et al., Arch.Microbiol 154:362-369 (1990)) and *Acidaminococcus fermentans* (Braune et al., Mol.Microbiol 31:473-487 (1999)). Analogs to the *F. nucleatum* glutaconyl-CoA decarboxylase alpha, beta and delta subunits are found in *S. aciditrophicus.* A gene annotated as an enoyl-CoA dehydrogenase, *syn_00480,* another GCD, is located in a predicted operon between a biotin-carboxyl carrier (*syn_00479*) and a glutaconyl-CoA decarboxylase alpha subunit (*syn_00481*)*.* The protein sequences for exemplary gene products can be found using the following GenBank accession numbers shown below. Enoyl-CoA reductase enzymes are described above (see EC 1.3.1).

| Protein | GenBank ID | GI Number | Organism |
|---|---|---|---|
| *gcdA* | CAA49210 | 49182 | *Acidaminococcus fermentans* |
| *gcdC* | AAC69172 | 3777506 | *Acidaminococcus fermentans* |
| *gcdD* | AAC69171 | 3777505 | *Acidaminococcus fermentans* |
| *gcdB* | AAC69173 | 3777507 | *Acidaminococcus fermentans* |
| FN0200 | AAL94406 | 19713641 | *Fusobacterium nucleatum* |
| FN0201 | AAL94407 | 19713642 | *Fusobacterium nucleatum* |
| FN0204 | AAL94410 | 19713645 | *Fusobacterium nucleatum* |
| *syn_00479* | YP_462066 | 85859864 | *Syntrophus aciditrophicus* |
| *syn_00481* | YP_462068 | 85859866 | *Syntrophus aciditrophicus* |
| *syn_01431* | YP_460282 | 85858080 | *Syntrophus aciditrophicus* |
| *syn_00480* | ABC77899 | 85722956 | *Syntrophus aciditrophicus* |

### 4.1.1.b Decarboxylase, alkene forming

An olefin-forming decarboxylase enzyme catalyzes the conversion of 5-hydroxyvalerate to 3-buten-1-ol (step W of Figure 1). A terminal olefin-forming fatty acid decarboxylase is encoded by the *ole T* gene product of *Jeotgalicoccus* sp. ATCC8456 (Rude et al, AEM 77(5):1718-27 (2011)). This recently discovered enzyme is a member of the cytochrome P450 family of enzymes and is similar to P450s that catalyze fatty acid hydroxylation. OleT and homologs are listed in the table below. Additional fatty acid decarboxylase enzymes are found in US 2011/0196180.

| Protein | GenBank ID | GI Number | Organism |
|---|---|---|---|
| *oleT* | ADW41779.1 | 320526718 | *Jeotgalicoccus* sp. ATCC8456 |
| *MCCL_0804* | BAH17511.1 | 222120176 | *Macrococcus caseolyticus* |
| *SPSE_1582* | ADX76840.1 | 323464687 | *Staphylococcus pseudintermedius* |
| *faaH* | ADC49546.1 | 288545663 | *Bacillus pseudofirmus* |
| *cypC2* | EGQ19322.1 | 339614630 | *Sporosarcina newyorkensis* |
| *cypC* | BAK15372.1 | 32743900 | *Solibacillus silvestris* |
| *Bcoam_010100017440* | ZP_03227611.1 | 205374818 | *Bacillus coahuilensis* |

### 4.1.99.a Decarbonylase

The conversion of penta-2,4-dienal to butadiene is catalyzed by a decarbonylase (Step B of Figure 6). Decarbonylase enzymes catalyze the final step of alkane biosynthesis in plants, mammals, and bacteria (Dennis et al., Arch.Biochem.Biophys. 287:268-275 (1991)). Non-oxidative decarbonylases transfom aldehydes into alkanes with the concurrent release of CO. Exemplary decarbonylase enzymes include octadecanal decarbonylase (EC 4.1.99.5), sterol desaturase and fatty aldehyde decarbonylase. A cobalt-porphyrin containing decarbonylase was purified and characterized in the algae *Botryococcus braunii;* however, no gene is associated with this activity to date (Dennis et al., Proc.Natl.Acad.Sci. U.S.A 89:5306-5310 (1992)). A copper-containing decarbonylase from *Pisum sativum* was also purified and characterized (Schneider-Belhaddad et al., Arch.Biochem.Biophys. 377:341-349 (2000)). The CER1 gene of Arabidopsis thaliana encodes a fatty acid decarbonylase involved in epicuticular wax formation (US 6,437,218). Additional fatty acid decarbonylases are found in *Medicago truncatula, Vitis vinifera* and *Oryza sativa* (US Patent Application 2009/0061493).

| Protein | GenBank ID | GI Number | Organism |
|---|---|---|---|
| *CER1* | NP_850932 | 145361948 | *Arabidopsis thaliana* |
| *MtrDRAFT_AC153128g2v2* | ABN07985 | 124359969 | *Medicago truncatula* |
| *VITISV_029045* | CAN60676 | 147781102 | *Vitis vinifera* |
| OSJNBa0004N05.14 | CAE03390.2 | 38345317 | *Oryza sativa* |

Alternately, an oxidative decarbonylase can convert an aldehyde into an alkane. Oxidative decarbonylases arc cytochrome P450 enzymes that utilize NADPH and O₂ as cofactors and release CO₂, water and NADP⁺. This activity was demonstrated in the *CYP4G2v1* and *CXP4G1* gene products of *Musca domestica* and *Drosophila melanogaster* (US Patent Application 2010/0136595). Additional enzyme candidates with oxidative decarbonylase activity can be identified in other organisms, for example *Mamestra brassicae*, *Helicoverpa zea* and *Acyrthosiphon pisum,* by sequence homology.

| Protein | GenBank ID | GI Number | Organism |
|---|---|---|---|
| *CYP4G2v1* | ABV48808.1 | 157382740 | *Musca domestica* |
| *CYP4G1* | NP_525031.1 | 17933498 | *Drosophila melanogaster* |
| *CYP4G25* | BAD81026.1 | 56710314 | *Antheraea yamamai* |
| *CYP4M6* | AAM54722.1 | 21552585 | *Helicoverpa zea* |
| *LOC100164072* | XP_001944205.1 | 193650239 | *Acyrthosiphon pisum* |

### 4.1.3.a Lyase

The condensation of pyruvate and acetaldehyde to 4-hydroxy-2-oxovalerate (Step A of Figure 5) is catalyzed by 4-hydroxy-2-oxovalerate aldolase (EC 4.1.3.39). This enzyme participates in pathways for the degradation of phenols, cresols and catechols. The *E. coli* enzyme, encoded by *mhpE*, is highly specific for acetaldehyde as an acceptor but accepts the alternate substrates 2-ketobutyrate or phenylpyruvate as donors (Pollard et al., Appl Environ Microbiol 64:4093-4094 (1998)). Similar enzymes are encoded by the *cmtG* and *todH* genes of *Pseudomonas putida* (Lau et al., Gene 146:7-13 (1994); Eaton, J Bacteriol. 178:1351-1362 (1996)). In *Pseudomonas* CF600, this enzyme is part of a bifunctional aldolase-dehydrogenase heterodimer encoded by *dmpFG (*Manjasetty et al., Acta Crystallogr.D.Biol Crystallogr. 57:582-585 (2001*)).* The dehydrogenase functionality interconverts acetaldehyde and acetyl-CoA, providing the advantage of reduced cellular concentrations of acetaldehyde, toxic to some cells. A similar aldolase-dehydrogenase complex is encoded by BphIJ of *Burkholderia xenovorans* (Baker et al, Biochem 48:6551-8 (2009)).

| Gene | GenBank ID | GI Number | Organism |
|---|---|---|---|
| *mhpE* | AAC73455.1 | 1786548 | *Escherichia coli* |
| *cmtG* | AAB62295.1 | 1263190 | *Pseudomonas putida* |
| *todH* | AAA61944.1 | 485740 | *Pseudomonas putida* |
| *dmpG* | CAA43227.1 | 45684 | *Pseudomonas sp. CF600* |
| *dmpF* | CAA43226.1 | 45683 | *Pseudomonas sp. CF600* |
| *bphl* | ABE37049.1 | 91693852 | *Burkholderia xenovorans* |
| *bphJ* | ABE37050.1 | 91693853 | *Burkholderia xenovorans* |

### 4.2.1.a Hydro-lyase

The removal of water to form a double bond is catalyzed by dehydratase enzymes in the 4.2.1 family of enzymes. Hydratase enzymes are sometimes reversible and also catalyze dehydration. Dehydratase enzymes are sometimes reversible and also catalyze hydration. The removal of water from a given substrate is required by steps G, N and V of Figure 1, step C of Figure 2, step C of Figure 3 and steps B and E of Figure 5. Several hydratase and dehydratase enzymes have been described in the literature and represent suitable candidates for these steps.

For example, many dehydratase enzymes catalyze the alpha, beta-elimination of water which involves activation of the alpha-hydrogen by an electron-withdrawing carbonyl, carboxylate, or CoA-thiol ester group and removal of the hydroxyl group from the beta-position (Buckel et al, J Bacteriol, 117:1248-60 (1974); Martins et al, PNAS 101:15645-9 (2004)). Exemplary enzymes include 2-(hydroxymethyl)glutarate dehydratase (EC 4.2.1.-), fumarase (EC 4.2.1.2), 3-dehydroquinate dehydratase (EC 4.2.1.10), cyclohexanone hydratase (EC 4.2.1.-) and 2-keto-4-pentenoate dehydratase (EC 4.2.1.80), citramalate hydrolyase and dimethylmaleate hydratase.

2-(Hydroxymethyl)glutarate dehydratase is a [4Fe-4S]-containing enzyme that dehydrates 2-(hydroxymethyl)glutarate to 2-methylene-glutarate, studied for its role in nicontinate catabolism in *Eubacterium barkeri* (formerly *Clostridium barkeri*) (Alhapel et al., Proc Natl Acad Sci 103:12341-6 (2006)). Similar enzymes with high sequence homology are found in *Bacteroides capillosus*, *Anaerotruncus colihominis*, and *Natranaerobius thermophilius.* These enzymes are homologous to the alpha and beta subunits of [4Fe-4S]-containing bacterial serine dehydratases (e.g., *E. coli* enzymes encoded by *tdcG, sdhB,* and *sdaA*). An enzyme with similar functionality in *E. barkeri* is dimethylmaleate hydratase, a reversible Fe²⁺-dependent and oxygen-sensitive enzyme in the aconitase family that hydrates dimcthylmacatc to form (2R,3S)-2,3-dimcthylmalatc. This enzyme is encoded by *dmdAB* (Alhapel et al., Proc Natl Acad Sci U S A 103:12341-6 (2006); Kollmann-Koch et al., Hoppe Seylers.Z.Physiol Chem. 365:847-857 (1984)).

| Protein | GenBank ID | GI Number | Organism |
|---|---|---|---|
| *hmd* | ABC88407.1 | 86278275 | *Eubacterium barkeri* |
| *BACCAP 02294* | ZP_02036683.1 | 154498305 | *Bacteroides capillosus* |
| *ANACOL*_*02527* | ZP_02443222.1 | 167771169 | *Anaerotruncus colihominis* |
| *NtherDRAFT_2368* | ZP_02852366.1 | 169192667 | *Natranaerobius thermophilus* |
| *dmdA* | ABC88408 | 86278276 | *Eubacterium barkeri* |
| *dmdB* | ABC88409 | 86278277 | *Eubacterium barkeri* |

Fumarate hydratase (EC 4.2.1.2) enzymes naturally catalyze the reversible hydration of fumarate to malate. Although the ability of fumarate hydratase to react with 3-oxobutanol as a substrate has not been described in the literature, a wealth of structural information is available for this enzyme and other researchers have successfully engineered the enzyme to alter activity, inhibition and localization (Weaver, 61:1395-1401 (2005)). *E.* coli has three fumarases: FumA, FumB, and FumC that are regulated by growth conditions. FumB is oxygen sensitive and only active under anaerobic conditions. FumA is active under microanaerobic conditions, and FumC is the only active enzyme in aerobic growth (Tseng et al., J Bacteriol, 183:461-467 (2001); Woods et al., 954:14-26 (1988); Guest et al., J Gen Microbiol 131:2971-2984 (1985)). Additional enzyme candidates are found in *Campylobacter jejuni* (Smith et al., Int.J Biochem.Cell Biol 31:961-975 (1999)), *Thermus thermophilus* (Mizobata et al., Arch.Biochem.Biophys. 355:49-55 (1998)) and *Rattus norvegicus* (Kobayashi et al., J. Biochem, 89:1923-1931 (1981)). Similar enzymes with high sequence homology include *fum1* from *Arabidopsis thaliana* and *fumC* from *Corynebacterium glutamicum.* The *MmcBC* fumarase from *Pelotomaculum thermopropionicum* is another class of fumarase with two subunits (Shimoyama et al., FEMS Microbiol Lett, 270:207-213 (2007)).

| Protein | GenBank ID | GI Number | Organism |
|---|---|---|---|
| *fumA* | NP_416129.1 | 16129570 | *Escherichia coli* |
| *fumB* | NP_418546.1 | 16131948 | *Escherichia coli* |
| *fumC* | NP_416128.1 | 16129569 | *Escherichia coli* |
| *fumC* | 069294 | 9789756 | *Campylobacter jejuni* |
| *fumC* | P84127 | 75427690 | *Thermus thermophilus* |
| *fumH* | P14408 | 120605 | *Rattus norvegicus* |
| *fumI* | P93033 | 39931311 | *Arabidopsis thaliana* |
| *fumC* | Q8NRN8 | 39931596 | *Corynebacterium glutamicum* |
| *MmcB* | YP_001211906 | 147677691 | *Pelotomaculum thermopropionicum* |
| *MmcC* | YP_001211907 | 147677692 | *Pelotomaculum thermopropionicum* |

Dehydration of 4-hydroxy-2-oxovalerate to 2-oxopentenoate is catalyzed by 4-hydroxy-2-oxovalerate hydratase (EC 4.2.1.80). This enzyme participates in aromatic degradation pathways and is typically co-transcribed with a gene encoding an enzyme with 4-hydroxy-2-oxovalerate aldolase activity. Exemplary gene products are encoded by *mhpD of E. coli* (Ferrandez et al., J Bacteriol. 179:2573-2581 (1997); Pollard et al., Eur J Biochem. 251:98-106 (1998)), *todG* and *cmtF* of *Pseudomonas putida* (Lau et al., Gene 146:7-13 (1994); Eaton, J Bacteriol. 178:1351-1362 (1996)), *cnbE* of *Comamonas* sp. CNB-1 (Ma et al., Appl Environ Microbiol 73:4477-4483 (2007)) and mhpD of *Burkholderia xenovorans* (Wang et al., FEBS J 272:966-974 (2005)). A closely related enzyme, 2-oxohepta-4-ene-1,7-dioate hydratase, participates in 4-hydroxyphenylacetic acid degradation, where it converts 2-oxo-hept-4-ene-1,7-dioate (OHED) to 2-oxo-4-hydroxy-hepta-1,7-dioate using magnesium as a cofactor (Burks et al., J.Am.Chem.Soc. 120: (1998)). OHED hydratase enzyme candidates have been identified and characterized in *E. coli C* (Roper et al., Gene 156:47-51 (1995); Izumi et al., J Mol.Biol. 370:899-911 (2007)) and *E*. *coli W* (Prieto et al., J Bacteriol. 178:111-120 (1996)). Sequence comparison reveals homologs in a wide range of bacteria, plants and animals. Enzymes with highly similar sequences are contained in *Klebsiella pneumonia* (91% identity, eval = 2e-138) and *Salmonella enterica* (91% identity, eval = 4e-138), among others.

| Protein | GenBank Accession No. | GI No. | Organism |
|---|---|---|---|
| *mhpD* | AAC73453.2 | 87081722 | *Escherichia coli* |
| *cmtF* | AAB62293.1 | 1263188 | *Pseudomonas putida* |
| *todG* | AAA61942.1 | 485738 | *Pseudomonas putida* |
| *cnbE* | YP_001967714.1 | 190572008 | *Comamonas* sp. CNB-1 |
| *mhpD* | Q13VU0 | 123358582 | *Burkholderia xenovorans* |
| *hpcG* | CAA57202.1 | 556840 | *Escherichia coli C* |
| *hpaH* | CAA86044.1 | 757830 | *Escherichia coli W* |
| *hpaH* | ABR80130.1 | 150958100 | *Klebsiella pneumoniae* |
| *Sari_01896* | ABX21779.1 | 160865156 | *Salmonella enterica* |

Another enzyme candidate is citramalate hydrolyase (EC 4.2.1.34), an enzyme that naturally dehydrates 2-methylmalate to mesaconate. This enzyme has been studied in *Methanocaldococcus jannaschii* in the context of the pyruvate pathway to 2-oxobutanoate, where it has been shown to have a broad substrate range (Drevland et al., J Bacteriol. 189:4391-4400 (2007)). This enzyme activity was also detected in *Clostridium tetanomorphum*, *Morganella morganii*, *Citrobacter amalonaticus* where it is thought to participate in glutamate degradation (Kato et al., Arch.Microbiol 168:457-463 (1997)). The *M. jannaschii* protein sequence does not bear significant homology to genes in these organisms.

| Protein | GenBank ID | GI Number | Organism |
|---|---|---|---|
| *leuD* | Q58673.1 | 3122345 | *Methanocaldococcus jannaschii* |

Dimethylmaleate hydratase (EC 4.2.1.85) is a reversible Fe²⁺-dependent and oxygen-sensitive enzyme in the aconitase family that hydrates dimethylmaeate to form (2R,3S)-2,3-dimethylmalate. This enzyme is encoded by *dmdAB* in *Eubacterium barkeri* (Alhapel et al., *supra*; Kollmann-Koch et al., Hoppe Seylers.Z.Physiol Chem. 365:847-857 (1984)).

| Protein | GenBank ID | GI Number | Organism |
|---|---|---|---|
| *dmdA* | ABC88408 | 86278276 | *Eubacterium barkeri* |
| *dmdB* | ABC88409.1 | 86278277 | *Eubacterium barkeri* |

Oleate hydratases represent additional suitable candidates as suggested in WO2011076691. Examples include the following proteins.

| Protein | GenBank ID | GI Number | Organism |
|---|---|---|---|
| OhyA | ACT54545.1 | 254031735 | *Elizabethkingia meningoseptica* |
| HMPREF0841_1446 | ZP_07461147.1 | 306827879 | *Streptococcus pyogenes ATCC 10782* |
| P700755_13397 | ZP_01252267.1 | 91215295 | *Psychroflexus torquis ATCC 700755* |
| RPB 2430 | YP_ 486046.1 | 86749550 | *Rhodopseudomonas palustris* |

Enoyl-CoA hydratases (EC 4.2.1.17) catalyze the dehydration of a range of 3-hydroxyacyl-CoA substrates (Roberts et al., Arch.Microbiol 117:99-108 (1978); Agnihotri et al., Bioorg.Med.Chem. 11:9-20 (2003); Conrad et al., J Bacteriol. 118:103-111 (1974)). The enoyl-CoA hydratase of *Pseudomonas putida,* encoded by *ech,* catalyzes the conversion of 3-hydroxybutyryl-CoA to crotonyl-CoA (Roberts et al., Arch.Microbiol 117:99-108 (1978)). This transformation is also catalyzed by the *crt* gene product of *Clostridium acetobutylicum,* the *crt1* gene product of *C*. *kluyveri*, and other clostridial organisms Atsumi et al., Metab Eng 10:305-311 (2008); Boynton et al., J Bacteriol. 178:3015-3024 (1996); Hillmer et al., FEBS Lett. 21:351-354 (1972)). Additional enoyl-CoA hydratase candidates are *phaA* and *phaB*, of *P. putida*, *and paaA and paaB* from *P*. *fluorescens* (Olivera et al., Proc.Natl.Acad.Sci U.S.A 95:6419-6424 (1998)). The gene product of *pimF* in *Rhodopseudomonas palustris* is predicted to encode an enoyl-CoA hydratase that participates in pimeloyl-CoA degradation (Harrison et al., Microbiology 151:727-736 (2005)). Lastly, a number of *Escherichia coli* genes have been shown to demonstrate enoyl-CoA hydratase functionality including *maoC* (Park et al., J Bacteriol. 185:5391-5397 (2003)), *paaF* (Ismail et al., Eur. J Biochem. 270:3047-3054 (2003); Park et al., Appl.Biochent.Biotechnol 113-116:335-346 (2004); Park et al., Biotechnol Bioeng 86:681-686 (2004)) and *paaG* (Ismail et al., Eur.J Biochem. 270:3047-3054 (2003); Park and Lee, Appl.Biochem.Biotechnol 113-116:335-346 (2004); Park and Yup, Biotechnol Bioeng 86:681-686 (2004)).

| Protein | GenBank Accession No. | GI No. | Organism |
|---|---|---|---|
| *ech* | NP_745498.1 | 26990073 | *Pseudomonas putida* |
| *crt* | NP_349318.1 | 15895969 | *Clostridium acetobutylicum* |
| *crt1* | YP_001393856 | 153953091 | *Clostridium kluyveri* |
| *phaA* | ABF82233.1 | 26990002 | *Pseudomonas putida* |
| *phaB* | ABF82234.1 | 26990001 | *Pseudomonas putida* |
| *paaA* | NP_745427.1 | 106636093 | *Pseudomonas fluorescens* |
| *paaB* | NP_745426.1 | 106636094 | *Pseudomonas fluorescens* |
| *maoC* | NP_415905.1 | 16129348 | *Escherichia coli* |
| *paaF* | NP_415911.1 | 16129354 | *Escherichia coli* |
| *paaG* | NP_415912.1 | 16129355 | *Escherichia coli* |

Alternatively, the *E. coli* gene products of *fadA and fadB* encode a multienzyme complex involved in fatty acid oxidation that exhibits enoyl-CoA hydratase activity (Yang et al., Biochemistry 30:6788-6795 (1991); Yang, J Bacteriol. 173:7405-7406 (1991); Nakahigashi et al., Nucleic Acids Res. 18:4937 (1990)). Knocking out a negative regulator encoded by *fadR* can be utilized to activate the *fadB* gene product (Sato et al., J Biosci.Bioeng 103:38-44 (2007)). The *fadI* and *fadJ* genes encode similar functions and are naturally expressed under anaerobic conditions (Campbell et al., Mol.Microbiol 47:793-805 (2003)).

| Protein | GenBank ID | GI Number | Organism |
|---|---|---|---|
| *fadA* | YP_026272.1 | 49176430 | *Escherichia coli* |
| *fadB* | NP_418288.1 | 16131692 | *Escherichia coli* |
| *fadI* | NP_416844.1 | 16130275 | *Escherichia coli* |
| *fadJ* | NP_416843.1 | 16130274 | *Escherichia coli* |
| *fadR* | NP_415705.1 | 16129150 | *Escherichia coli* |

### 4.3.1.a Ammonia-lyase

In step J of Figure 1, an ammonia lyase in EC class 4.3.1 is required to catalyze the deamination of 5-aminopen-2-enoate to 2,4-pentadienoate. Exemplary enzymes are aspartase and 3-methylaspartase. Aspartase (EC 4.3.1.1), catalyzing the deamination of aspartate to fumarate, has been characterized extensively (Viola, Adv Enzym Relat Areas Mol Biol, 74:295-341 (2000)). The *E. coli* enzyme is active on a variety of alternate substrates including aspartatephenylmethylester, asparagine, benzyl-aspartate and malate (Ma et al., Ann NY Acad Sci, 672:60-65 (1992)). In addition, directed evolution was employed on this enzyme to alter substrate specificity (Asano et al., Biomol Eng 22:95-101 (2005)). The crystal structure of the *E. coli* aspartase, encoded by *aspA,* has been solved (Shi et al., Biochem, 36:9136-9144 (1997)). Enzymes with aspartase functionality have also been characterized in *Haemophilus influenzae* (Sjostrom et al., Biochim.Biophys.Acta 1324:182-190 (1997)), *Pseudomonas fluorescens* (Takagi et al., J.Biochem. 96:545-552 (1984)), *Bacillus subtilis* (Sjostrom et al, Biochim Biophys Acta 1324:182-190 (1997)) and *Serratia marcescens* (Takagi et al., J Bacteriol, 161:1-6 (1985)). 3-Methylaspartase catalyzes the deamination of threo-3-methylasparatate to mesaconate. The 3-methylaspartase from *Clostridium tetanomorphum* has been cloned, functionally expressed in *E. coli,* and crystallized (Asuncion et al., Acta Cryst D Biol Crystallog, 57:731-733 (2001); Asuncion et al., J Biol Chem. 277:8306-8311 (2002); Botting et al., Biochem 27:2953-2955 (1988); Goda et al., Biochem 31:10747-10756 (1992)). In *Citrobacter amalonaticus,* this enzyme is encoded by *BAA28709* (Kato et al., Arch.Microbiol 168:457-463 (1997)). 3-methylaspartase has also been crystallized from *E*. *coli YG1002* (Asano et al., FEMS Microbiol Lett. 118:255-258 (1994)) although the protein sequence is not listed in public databases such as GenBank. Sequence homology can be used to identify additional candidate genes, including *CTC_02563* in *C*. *tetani* and *ECs0761* in *Escherichia coli 0157:H7.*

| Protein | GenBank ID | GI Number | Organism |
|---|---|---|---|
| *aspA* | NP_418562 | 90111690 | *Escherichia coli K12 subsp. MG1655* |
| *aspA* | P44324.1 | 1168534 | *Haemophilus influenzae* |
| *aspA* | P07346.1 | 114273 | *Pseudomonas fluorescens* |
| *ansB* | P26899.1 | 251757243 | *Bacillus subtilis* |
| *aspA* | P33109.1 | 416661 | *Serratia marcescens* |
| *MAL* | AAB24070.1 | 259429 | *Clostridium tetanomorphum* |
| *BAA28709* | BAA28709.1 | 3184397 | *Citrobacter amalonaticus* |
| *CTC*_*02563* | NP_783085.1 | 28212141 | *Clostridium tetani* |
| *ECs0761* | BAB34184.1 | 13360220 | *Escherichia coli O157:H7 str. Sakai* |

### 5.3.3.a Delta-isomerase

Several characterized enzymes shift the double bond of enoyl-CoA substrates from the 2-to the 3- position. Such a transformation is required in step D of Figure 3. Exemplary enzymes include 4-hydroxybutyryl-CoA dehydratase/vinylacetyl-CoA delta-isomerase (EC 5.3.3.3), delta-3, delta-2-enoyl-CoA isomerase (EC 5.3.3.8) and fatty acid oxidation complexes. 4-Hydroxybutyrul-CoA dehydratase enzymes catalyze the reversible conversion of 4-hydroxybutyryl-CoA to crotonyl-CoA. These enzymes are bifunctional, catalyzing both the dehydration of 4-hydroxybutyryl-CoA to vinylacetyl-CoA, and also the isomerization of vinylacetyl-CoA and crotonyl-CoA. 4-Hydroxybutyrul-CoA dehydratase enzymes from *C*. *aminobutyrium* and *C. kluyveri* were purified, characterized, and sequenced at the N-terminus (Scherf et al., Arch.Microbiol 161:239-245 (1994); Scherf and Buckel, Eur.J Biochem. 215:421-429 (1993)). The *C. kluyveri* enzyme, encoded by *abfD,* was cloned, sequenced and expressed in *E. coli* (Gerhardt et al., Arch.Microbiol 174:189-199 (2000)). The *abfD* gene product from *Porphyromonas gingivalis ATCC* 33277 is closely related by sequence homology to the Clostridial gene products. 4-Hydroxybutyryl-CoA dehydratase/isomerase activity was also detected in *Metallosphaera sedula*, and is likely associated with the *Msed_1220* gene (Berg et al, Science 318(5857):1782-6 (2007). Delta isomerization reactions are also catalyzed by the fatty acid oxidation complex. In *E. coli,* the *fadJ* and *fadB* gene products convert cis-3-enoyl-CoA molecules to trans-2-enoyl-CoA molecules under aerobic and anaerobic conditions, respectively (Campbell et al, Mol Micro 47(3):793-805 (2003)). A monofunctional delta-isomerase isolated from *Cucumis sativus* peroxisomes catalyzes the reversible conversion of both cis- and trans-3-enoyl-CoA into trans-2-enoyl-CoA (Engeland et al, Eur J Biochem, 196 (3):699-705 (1991). The gene associated with this enzyme has not been identified to date. A number of multifunctional proteins (MFP) from *Cucumis sativus* also catalyze this activity, including the gene product of MFP-a (Preisig-Muller et al, J Biol Chem 269:20475-81 (1994)).

| Gene | GenBank | GI Number | Organism |
|---|---|---|---|
| *abfD* | P55792 | 84028213 | *Clostridium aminobutyricum* |
| *abfD* | YP_001396399.1 | 153955634 | *Clostridium kluyveri* |
| *abfD* | YP_001928843 | 188994591 | *Porphyromonas gingivalis* |
| *Msed_1220* | ABP95381.1 | 145702239 | *Metallosphaera sedula* |
| *fadJ* | AAC75401.1 | 1788682 | *Escherichia coli* |
| *fadB* | AAC76849.1 | 1790281 | *Escherichia coli* |
| *MFP-a* | Q39659.1 | 34922495 | *Cucumis sativus* |

### 6.2.1.a Acid-thiol ligase

The conversion of acyl-CoA substrates to their acid products can be catalyzed by a CoA acid-thiol ligase or CoA synthetase in the 6.2.1 family of enzymes, several of which arc reversible. Several reactions shown in Figures 1-6 are catalyzed by acid-thiol ligase enzymes. These reactions include Steps L, P and O of Figure 1, step F of Figure 3, step B of Figure 4, step J of Figure 5 and step F of Figure 6. Several enzymes catalyzing CoA acid-thiol ligase or CoA synthetase activities have been described in the literature and represent suitable candidates for these steps.

For example, ADP-forming acetyl-CoA synthetase (ACD, EC 6.2.1.13) is an enzyme that couples the conversion of acyl-CoA esters to their corresponding acids with the concomitant synthesis of ATP. ACD I from *Archaeoglobus fulgidus*, encoded by AF1211, was shown to operate on a variety of linear and branched-chain substrates including isobutyrate, isopentanoate, and fumarate (Musfeldt et al., J Bacteriol. 184:636-644 (2002)). A second reversible ACD in *Archaeoglobus fulgidus,* encoded by AF1983, was also shown to have a broad substrate range with high activity on cyclic compounds phenylacetate and indoleacetate (Musfeldt and Schonheit, J Bacteriol. 184:636-644 (2002)). The enzyme from *Haloarcula marismortui* (annotated as a succinyl-CoA synthetase) accepts propionate, butyrate, and branched-chain acids (isovalerate and isobutyrate) as substrates, and was shown to operate in the forward and reverse directions (Brasen et al., Arch Microbiol 182:277-287 (2004)). The ACD encoded by *PAE3250* from hyperthermophilic crenarchaeon *Pyrobaculum aerophilum* showed the broadest substrate range of all characterized ACDs, reacting with acetyl-CoA, isobutyryl-CoA (preferred substrate) and phenylacetyl-CoA (Brasen et al, *supra*). Directed evolution or engineering can be used to modify this enzyme to operate at the physiological temperature of the host organism. The enzymes from *A. fulgidus*, *H. marismortui* and *P. aerophilum* have all been cloned, functionally expressed, and characterized in *E. coli* (Brasen and Schonheit, *supra*; Musfeldt and Schonheit, J Bacteriol 184:636-644 (2002)). An additional candidate is succinyl-CoA synthetase, encoded by *sucCD* of *E. coli* and *LSC1* and *LSC2* genes of *Saccharomyces cerevisiae.* These enzymes catalyze the formation of succinyl-CoA from succinate with the concomitant consumption of one ATP in a reaction which is reversible *in vivo* (Buck et al., Biochemistry 24:6245-6252 (1985)). The acyl CoA ligase from *Pseudomonas putida* has been demonstrated to work on several aliphatic substrates including acetic, propionic, butyric, valeric, hexanoic, heptanoic, and octanoic acids and on aromatic compounds such as phenylacetic and phenoxyacetic acids (Fernandez-Valverde et al., Appl.Environ.Microbiol. 59:1149-1154 (1993)). A related enzyme, malonyl CoA synthetase (6.3.4.9) from *Rhizobium leguminosarum* could convert several diacids, namely, ethyl-, propyl-, allyl-, isopropyl-, dimethyl-, cyclopropyl-, cyclopropylmethylene-, cyclobutyl-, and benzyl-malonate into their corresponding monothioesters (Pohl et al., J.Am.Chem.Soc. 123:5822-5823 (2001)).

| Protein | GenBank ID | GI Number | Organism |
|---|---|---|---|
| *AF1211* | NP_070039.1 | 11498810 | *Archaeoglobus fulgidus* |
| *AF1983* | NP_070807.1 | 11499565 | *Archaeoglobus fulgidus* |
| *Scs* | YP_135572.1 | 55377722 | *Haloarcula marismortui* |
| *PAE3250* | NP_560604.1 | 18313937 | *Pyrobaculum aerophilum* str. IM2 |
| *sucC* | NP_415256.1 | 16128703 | *Escherichia coli* |
| *sucD* | AAC73823.1 | 1786949 | *Escherichia coli* |
| *LSC1* | NP_014785 | 6324716 | *Saccharomyces cerevisiae* |
| *LSC2* | NP_011760 | 6321683 | *Saccharomyces cerevisiae* |
| *paaF* | AAC24333.2 | 22711873 | *Pseudomonas putida* |
| *matB* | AAC83455.1 | 3982573 | *Rhizobium leguminosarum* |

Another candidate enzyme for these steps is 6-carboxyhexanoate-CoA ligase, also known as pimeloyl-CoA ligase (EC 6.2.1.14), which naturally activates pimelate to pimeloyl-CoA during biotin biosynthesis in gram-positive bacteria. The enzyme from *Pseudomonas mendocina*, cloned into *E. coli,* was shown to accept the alternate substrates hexanedioate and nonanedioate (Binieda et al., Biochem.J 340 (Pt 3):793-801 (1999)). Other candidates are found in *Bacillus subtilis* (Bower et al., J Bacteriol. 178:4122-4130 (1996)) and *Lysinibacillus sphaericus* (formerly *Bacillus sphaericus*) (Ploux et al., Biochem.J 287 (Pt 3):685-690 (1992)).

| Protein | GenBank ID | GI Number | Organism |
|---|---|---|---|
| *bioW* | NP_390902.2 | 50812281 | *Bacillus subtilis* |
| *bioW* | CAA10043.1 | 3850837 | *Pseudomonas mendocina* |
| *bioW* | P22822.1 | 115012 | *Bacillus sphaericus* |

Additional CoA-ligases include the rat dicarboxylate-CoA ligase for which the sequence is yet uncharacterized (Vamecq et al., Biochem.J 230:683-693 (1985)), either of the two characterized phenylacetate-CoA ligases from *P*. *chrysogenum* (Lamas-Maceiras et al., Biochem.J 395:147-155 (2006); Wang et al., 360:453-458 (2007)), the phenylacetate-CoA ligase from *Pseudomonas putida* (Martinez-Blanco et al., J Biol Chem 265:7084-7090 (1990)) and the 6-carboxyhexanoate-CoA ligase from *Bacillus subtilis* (Bower et al. J Bacteriol 178(14):4122-4130 (1996)). Acetoacetyl-CoA synthetases *from Mus musculus* (Hasegawa et al., Biochim Biophys Acta 1779:414-419 (2008)) and *Homo sapiens* (Ohgami et al., Biochem. Pharmacol. 65:989-994 (2003)) naturally catalyze the ATP-dependent conversion of acetoacetate into acetoacetyl-CoA.

| Protein | Accession No. | GI No. | Organism |
|---|---|---|---|
| *phl* | CAJ15517.1 | 77019264 | *Penicillium chrysogenum* |
| *phlB* | ABS19624.1 | 152002983 | *Penicillium chrysogenum* |
| *paaF* | AAC24333.2 | 22711873 | *Pseudomonas putida* |
| *bioW* | NP_390902.2 | 50812281 | *Bacillus subtilis* |
| *AACS* | NP_084486.1 | 21313520 | *Mus musculus* |
| *AACS* | NP_076417.2 | 31982927 | *Homo sapiens* |

Like enzymes in other classes, certain enzymes in the EC class 6.2.1 have been determined to have broad substrate specificity. The acyl CoA ligase from *Pseudomonas putida* has been demonstrated to work on several aliphatic substrates including acetic, propionic, butyric, valeric, hexanoic, heptanoic, and octanoic acids and on aromatic compounds such as phenylacetic and phenoxyacetic acids (Fernandez-Valverde et al., Applied and Environmental Microbiology 59:1149-1154 (1993)). A related enzyme, malonyl CoA synthetase (6.3.4.9) from *Rhizobium trifolii* could convert several diacids, namely, ethyl-, propyl-, allyl-, isopropyl-, dimethyl-, cyclopropyl-, cyclopropylmethylene-, cyclobutyl-, and benzyl-malonate into their corresponding monothioesters (Pohl et al., J.Am.Chem.Soc. 123:5822-5823 (2001)).

### N/A (No EC number)

In step Q of Figure 1, the conversion of 5-hydroxyvaleryl-CoA to 2,4-pentadienoyl-CoA is catalyzed by 5-hydroxyvaleryl-CoA dehydratase/dehydratase, a bifunctional enzyme. Participating in 5-aminovalerate fermentation by *Clostridium aminovalericum,* this enzyme was purified characterized and crystallized (Eikmanns et al, Proteins: Struct Fun Gen 19:269-271 (1994), Eikmanns and Buckel, Eur J Biochem, 197:661-668 (1991)). The protein sequence is known but has not been assigned a GcnBank identifier to date. Homologs with similar protein sequences are listed in the table below.

| Gene | GenBank ID | GI Number | Organism |
|---|---|---|---|
| *CLOSS21*_*02963* | ZP_02440459.1 | 167768406 | *Clostridium sp. SS2*/*1* |
| *CK3*_*30740* | CBL42530.1 | 291563714 | *butyrate-producing bacterium SS3*/*4* |
| *ANACAC_01346* | ZP_02418762.1 | 167746635 | *Anaerostipes caccae DSM 14662* |
| *mmgC2* | ZP_07921990.1 | 315925783 | *Pseudoramibacter alactolyticus* |
| *ANACAC_01346* | ZP_07822451.1 | 167746635 | *Peptoniphilus harei* |
| *FgonA2_010100002879* | ZP_05630680.1 | 257466369 | *Fusobacterium gonidiaformans* |
| *FNP_2146* | ZP_04969457.1 | 254302099 | *Fusobacterium nucleatum* |
| *acdA2* | ZP_07921487.1 | 315925275 | *Pseudoramibacter alactolyticus* |
| *CHY_1732* | YP_360552.1 | 78043883 | *Carboxydothermus hydrogenoformans* |
| *acdA* | ZP_07454495.1 | 306820875 | *Eubacterium yurii* |

### Example VIII

### Chemical dehydration of 1,3-butanediol and 3-buten-1-ol to butadiene

Alcohols can be converted to olefins by reaction with a suitable dehydration catalyst under appropriate conditions. Typical dehydration catalysts that convert alcohols such as butanols and pentanols into olefins include various acid treated and untreated alumina (e.g., γ-alumina) and silica catalysts and clays including zeolites (e.g., β-type zeolites, ZSM-5 or Y-type zeolites, fluoride-treated β-zeolite catalysts, fluoride-treated clay catalysts, etc.), sulfonic acid resins (e.g., sulfonated styrenic resins such as Amberlyst® 15), strong acids such as phosphoric acid and sulfuric acid, Lewis acids such boron trifluoride and aluminum trichloride, and many different types of metal salts including metal oxides (e.g., zirconium oxide or titanium dioxide) and metal chlorides (e.g., Latshaw B E, Dehydration of Isobutanol to Isobutylene in a Slurry Reactor, Department of Energy Topical Report, February 1994).

Dehydration reactions can be carried out in both gas and liquid phases with both heterogeneous and homogeneous catalyst systems in many different reactor configurations. Typically, the catalysts used are stable to the water that is generated by the reaction. The water is usually removed from the reaction zone with the product. The resulting alkene(s) either exit the reactor in the gas or liquid phase (e.g., depending upon the reactor conditions) and are captured by a downstream purification process or are further converted in the reactor to other compounds (such as butadiene or isoprene) as described herein. The water generated by the dehydration reaction exits the reactor with unreacted alcohol and alkene product(s) and is separated by distillation or phase separation. Because water is generated in large quantities in the dehydration step, the dehydration catalysts used are generally tolerant to water and a process for removing the water from substrate and product may be part of any process that contains a dehydration step. For this reason, it is possible to use wet (i.e., up to about 95% or 98% water by weight) alcohol as a substrate for a dehydration reaction and remove this water with the water generated by the dehydration reaction (e.g., using a zeolite catalyst as described U.S. Pat. Nos. 4,698,452 and 4,873,392). Additionally, neutral alumina and zeolites will dehydrate alcohols to alkenes but generally at higher temperatures and pressures than the acidic versions of these catalysts.

Dehydration of 1,3-butaediol to 3-buten-1-ol and butadiene is known in the art. For example, 3-buten-1-ol is synthesized from 1,3-butanediol by heating the diol in the presence of a trivalent metal sulfate to a temperature in the range of 70-100 degrees Celcius (US Patent 4400562). The dehydration of 1,3-butanediol to butadiene entails, for example, heating 1,3-butanediol in the presence of superheated steam and a phosphate-phosphoric acid catalyst (Sato, et al, Catalysis Communications, 5 (8), 2004, p. 397-400). Dehydration of 3-buten-1-ol to butadiene is also well known in the art (Gustav. Egloff and George. Hulla, Chem. Rev., 1945, 36 (1), pp 63-141).

### EXAMPLE IX

### Exemplary Hydrogenase and CO Dehydrogenase Enzymes for Extracting Reducing Equivalents from Syngas and Exemplary Reductive TCA Cycle Enzymes

Enzymes of the reductive TCA cycle useful in the non-naturally occurring microbial organisms of the present invention include one or more of ATP-citrate lyase and three CO₂-fixing enzymes: isocitrate dehydrogenase, alpha-ketoglutarate:ferredoxin oxidoreductase, pyruvate:ferredoxin oxidoreductase. The presence of ATP-citrate lyase or citrate lyase and alpha-ketoglutarate:ferredoxin oxidoreductase indicates the presence of an active reductive TCA cycle in an organism. Enzymes for each step of the reductive TCA cycle are shown below.

ATP-citrate lyase (ACL, EC 2.3.3.8), also called ATP citrate synthase, catalyzes the ATP-dependent cleavage of citrate to oxaloacetate and acetyl-CoA. ACL is an enzyme of the RTCA cycle that has been studied in green sulfur bacteria *Chlorobium limicola* and *Chlorobium tepidum.* The alpha(4)beta(4) heteromeric enzyme from *Chlorobium limicola* was cloned and characterized in *E. coli* (Kanao et al., Eur. J. Biochem. 269:3409-3416 (2002). The *C*. *limicola* enzyme, encoded by *aclAB,* is irreversible and activity of the enzyme is regulated by the ratio of ADP/ATP. A recombinant ACL from *Chlorobium tepidum* was also expressed in *E. coli* and the holoenzyme was reconstituted *in vitro,* in a study elucidating the role of the alpha and beta subunits in the catalytic mechanism (Kim and Tabita, J. Bacteriol. 188:6544-6552 (2006). ACL enzymes have also been identified in *Balnearium lithotrophicum, Sulfurihydrogenibium subterraneum* and other members of the bacterial phylum *Aquificae* (Hugler et al., Environ. Microbiol. 9:81-92 (2007)). This acitivy has been reported in some fungi as well. Exemplary organisms include *Sordaria macrospora (*Nowrousian et al., Curr. Genet. 37:189-93 (2000*), Aspergillus nidulans , Yarrowia lipolytica (*Hynes and Murray, Eukaryotic Cell, July: 1039-1048, (2010*)* and *Aspergillus niger (*Meijer et al. J. Ind. Microbiol. Biotechnol. 36:1275-1280 (2009*).* Other candidates can be found based on sequence homology. Information related to these enzymes is tabulated below:

| Protein | GenBank ID | GI Number | Organism |
|---|---|---|---|
| *aclA* | BAB21376.1 | 12407237 | *Chlorobium limicola* |
| *aclB* | BAB21375.1 | 12407235 | *Chlorobium limicola* |
| *aclA* | AAM72321.1 | 21647054 | *Chlorobium tepidum* |
| *aclB* | AAM72322.1 | 21647055 | *Chlorobium tepidum* |
| *aclA* | ABI50076.1 | 114054981 | *Balnearium lithotrophicum* |
| *aclB* | *ABI*50075.1 | 114054980 | *Balnearium lithotrophicum* |
| *aclA* | *ABI50085.1* | *114055040* | *Sulfurihydrogenibium subterraneum* |
| *aclB* | ABI50084.1 | 114055039 | *Sulfurihydrogenibium subterraneum* |
| *aclA* | AAX76834.1 | 62199504 | *Sulfurimonas denitrificans* |
| *aclB* | AAX76835.1 | 62199506 | *Sulfurimonas denitrificans* |
| *acl1* | XP_504787.1 | 50554757 | *Yarrowia lipolytica* |
| *acl2* | XP_503231.1 | 50551515 | *Yarrowia lipolytica* |
| *SPBC1703.07* | NP_596202.1 | 19112994 | *Schizosaccharomyces pombe* |
| *SPAC22A12.16* | NP_593246.1 | 19114158 | *Schizosaccharomyces pombe* |
| *acl1* | CAB76165.1 | 7160185 | *Sordaria macrospora* |
| *acl2* | CAB76164.1 | 7160184 | *Sordaria macrospora* |
| *aclA* | CBF86850.1 | 259487849 | *Aspergillus nidulans* |
| *aclB* | CBF86848 | 259487848 | *Aspergillus nidulans* |

In some organisms the conversion of citrate to oxaloacctatc and acctyl-CoA proceeds through a citryl-CoA intermediate and is catalyzed by two separate enzymes, citryl-CoA synthetase (EC 6.2.1.18) and citryl-CoA lyase (EC 4.1.3.34) (Aoshima, M., Appl. Microbiol. Biotechnol. 75:249-255 (2007). Citryl-CoA synthetase catalyzes the activation of citrate to citryl-CoA. The *Hydrogenobacter thermophilus* enzyme is composed of large and small subunits encoded by *ccsA* and *ccsB*, respectively (Aoshima et al., Mol. Micrbiol. 52:751-761 (2004)). The citryl-CoA synthetase of *Aquifex aeolicus* is composed of alpha and beta subunits encoded by *sucC1* and *sucD1* (Hugler et al., Environ. Microbiol. 9:81-92 (2007)). Citryl-CoA lyase splits citryl-CoA into oxaloacetate and acetyl-CoA. This enzyme is a homotrimer encoded by *ccl* in *Hydrogenobacter thermophilus* (Aoshima et al., Mol. Microbiol. 52:763-770 (2004)) and *aq_150* in *Aquifex aeolicus* (Hugler et al., *supra* (2007)). The genes for this mechanism of converting citrate to oxaloacetate and citryl-CoA have also been reported recently in *Chlorobium tepidum* (Eisen et al., PNAS 99(14): 9509-14 (2002).

| Protein | GenBank ID | GI Number | Organism |
|---|---|---|---|
| *ccsA* | BAD17844.1 | 46849514 | *Hydrogenobacter thermophilus* |
| *ccsB* | BAD17846.1 | 46849517 | *Hydrogenobacter thermophilus* |
| *sucC1* | AAC07285 | 2983723 | *Aquifex aeolicus* |
| *sucD1* | AAC07686 | 2984152 | *Aquifex aeolicus* |
| *ccl* | BAD17841.1 | 46849510 | *Hydrogenobacter thermophilus* |
| *aq_150* | AAC06486 | 2982866 | *Aquifex aeolicus* |
| *CT0380* | NP_661284 | 21673219 | *Chlorobium tepidum* |
| *CT0269* | NP_661173.1 | 21673108 | *Chlorobium tepidum* |
| *CT1834* | AAM73055.1 | 21647851 | *Chlorobium tepidum* |

Oxaloacetate is converted into malate by malate dehydrogenase (EC 1.1.1.37), an enzyme which functions in both the forward and reverse direction. *S. cerevisiae* possesses three copies of malate dehydrogenase, *MDH1* (McAlister-Henn and Thompson, J. Bacteriol. 169:5157-5166 (1987), *MDH2* (Minard and McAlister-Henn, Mol. Cell. Biol. 11:370-380 (1991); Gibson and McAlister-Henn, J. Biol. Chem. 278:25628-25636 (2003)), and *MDH3* (Steffan and McAlister-Henn, J. Biol. Chem. 267:24708-24715 (1992)), which localize to the mitochondrion, cytosol, and peroxisome, respectively. *E. coli* is known to have an active malate dehydrogenase encoded by *mdh.*

| Protein | GenBank ID | GI Number | Organism |
|---|---|---|---|
| *MDH1* | NP_012838 | 6322765 | *Saccharomyces cerevisiae* |
| *MDH2* | NP_014515 | 116006499 | *Saccharomyces cerevisiae* |
| *MDH3* | NP_010205 | 6320125 | *Saccharomyces cerevisiae* |
| *Mdh* | NP_417703.1 | 16131126 | *Escherichia coli* |

Fumarate hydratase (EC 4.2.1.2) catalyzes the reversible hydration of fumarate to malate. The three fumarases *of E. coli,* encoded by *fumA, fumB* and *fumC,* are regulated under different conditions of oxygen availability. FumB is oxygen sensitive and is active under anaerobic conditions. FumA is active under microanaerobic conditions, and FumC is active under aerobic growth conditions (Tseng et al., J. Bacteriol. 183:461-467 (2001);Woods et al., Biochim. Biophys. Acta 954:14-26 (1988); Guest et al., J. Gen. Microbiol. 131:2971-2984 (1985)). *S. cerevisiae* contains one copy of a fumarase-encoding gene, *FUM1,* whose product localizes to both the cytosol and mitochondrion (Sass et al., J. Biol. Chem. 278:45109-45116 (2003)). Additional fumarase enzymes are found in *Campylobacter jejuni* (Smith et al., Int. J. Biochem. Cell. Biol. 31:961-975 (1999)), *Thermus thermophilus* (Mizobata et al., Arch. Biochem. Biophys. 355:49-55 (1998)) and *Rattus norvegicus* (Kobayashi et al., J. Biochem. 89:1923-1931 (1981)). Similar enzymes with high sequence homology include *fum1* from *Arabidopsis thaliana* and *fumC* from *Corynebacterium glutamicum.* The *MmcBC* fumarase from *Pelotomaculum thermopropionicum* is another class of fumarase with two subunits (Shimoyama et al., FEMS Microbiol. Lett. 270:207-213 (2007)).

| Protein | GenBank ID | GI Number | Organism |
|---|---|---|---|
| *fumA* | NP_416129.1 | 16129570 | *Escherichia coli* |
| *fumB* | NP_418546.1 | 16131948 | *Escherichia coli* |
| *fumC* | NP_416128.1 | 16129569 | *Escherichia coli* |
| *FUM1* | NP_015061 | 6324993 | *Saccharomyces cerevisiae* |
| *fumC* | Q8NRN8.1 | 39931596 | *Corynebacterium glutamicum* |
| *fumC* | 069294.1 | 9789756 | *Campylobacter jejuni* |
| *fumC* | P84127 | 75427690 | *Thermus thermophilus* |
| *fumH* | P14408.1 | 120605 | *Rattus norvegicus* |
| *MmcB* | YP_001211906 | 147677691 | *Pelotomaculum thermopropionicum* |
| *MmcC* | YP_001211907 | 147677692 | *Pelotomaculum thermopropionicum* |

Fumarate reductase catalyzes the reduction of fumarate to succinate. The fumarate reductase of *E. coli,* composed of four subunits encoded by *frdABCD,* is membrane-bound and active under anaerobic conditions. The electron donor for this reaction is menaquinone and the two protons produced in this reaction do not contribute to the proton gradient (Iverson et al., Science 284:1961-1966 (1999)). The yeast genome encodes two soluble fumarate reductase isozymes encoded by FRDS1 (Enomoto et al., DNA Res. 3:263-267 (1996)) and FRDS2 (Muratsubaki et al., Arch. Biochem. Biophys. 352:175-181 (1998)), which localize to the cytosol and promitochondrion, respectively, and are used during anaerobic growth on glucose (Arikawa et al., FEMS Microbiol. Lett. 165:111-116 (1998)).

| Protein | GenBank ID | GI Number | Organism |
|---|---|---|---|
| *FRDS1* | P32614 | 418423 | *Saccharomyces cerevisiae* |
| *FRDS2* | NP_012585 | 6322511 | *Saccharomyces cerevisiae* |
| *frdA* | NP_418578.1 | 16131979 | *Escherichia coli* |
| *frdB* | NP_418577.1 | 16131978 | *Escherichia coli* |
| *fraC* | NP_418576.1 | 16131977 | *Escherichia coli* |
| *frdD* | NP_418475.1 | 16131877 | *Escherichia coli* |

The ATP-dependent acylation of succinate to succinyl-CoA is catalyzed by succinyl-CoA synthetase (EC 6.2.1.5). The product of the *LSC1* and *LSC2* genes of *S. cerevisiae* and the *sucC* and *sucD* genes *of E. coli* naturally form a succinyl-CoA synthetase complex that catalyzes the formation of succinyl-CoA from succinate with the concomitant consumption of one ATP, a reaction which is reversible *in vivo* (Buck et al., Biochemistry 24:6245-6252 (1985)). These proteins are identified below:

| Protein | GenBank ID | GI Number | Organism |
|---|---|---|---|
| *LSC1* | NP_014785 | 6324716 | *Saccharomyces cerevisiae* |
| *LSC2* | NP_011760 | 6321683 | *Saccharomyces cerevisiae* |
| *sucC* | NP_415256.1 | 16128703 | *Escherichia coli* |
| *sucD* | AAC73823.1 | 1786949 | *Escherichia coli* |

Alpha-ketoglutarate:ferredoxin oxidoreductase (EC 1.2.7.3), also known as 2-oxoglutarate synthase or 2-oxoglutarate:ferredoxin oxidoreductase (OFOR), forms alpha-ketoglutarate from CO2 and succinyl-CoA with concurrent consumption of two reduced ferredoxin equivalents. OFOR and pyruvate:ferredoxin oxidoreductase (PFOR) are members of a diverse family of 2-oxoacid:ferredoxin (flavodoxin) oxidoreductases which utilize thiamine pyrophosphate, CoA and iron-sulfur clusters as cofactors and ferredoxin, flavodoxin and FAD as electron carriers *(*Adams et al., Archaea. Adv. Protein Chem. 48:101-180 (1996*)).* Enzymes in this class are reversible and function in the carboxylation direction in organisms that fix carbon by the RTCA cycle such as *Hydrogenobacter thermophilus*, *Desulfobacter hydrogenophilus* and *Chlorobium species* (Shiba et al. 1985; Evans et al., Proc. Natl. Acad. ScI. U.S.A. 55:92934 (1966); Buchanan, 1971). The two-subunit enzyme from *H. thermophilus*, encoded by *korAB,* has been cloned and expressed in *E. coli* (Yun et al., Biochem. Biophys. Res. Commun. 282:589-594 (2001)). A five subunit OFOR from the same organism with strict substrate specificity for succinyl-CoA, encoded by *forDABGE,* was recently identified and expressed in *E. coli* (Yun et al. 2002). The kinetics of CO2 fixation of both *H. thermophilus* OFOR enzymes have been characterized (Yamamoto et al., Extremophiles 14:79-85 (2010)). A CO2-fixing OFOR from *Chlorobium thiosulfatophilum* has been purified and characterized but the genes encoding this enzyme have not been identified to date. Enzyme candidates in *Chlorobium* species can be inferred by sequence similarity to the *H. thermophilus* genes. For example, the *Chlorobium limicola* genome encodes two similar proteins. Acetogenic bacteria such as *Moorella thermoacetica* are predicted to encode two OFOR enzymes. The enzyme encoded by *Moth_0034* is predicted to function in the CO2-assimilating direction. The genes associated with this enzyme, *Moth_0034* have not been experimentally validated to date but can be inferred by sequence similarity to known OFOR enzymes.

OFOR enzymes that function in the decarboxylation direction under physiological conditions can also catalyze the reverse reaction. The OFOR from the thermoacidophilic archaeon *Sulfolobus* sp. strain 7, encoded by ST2300, has been extensively studied (Zhang ct al. 1996. A plasmid-bascd expression system has been developed for efficiently expressing this protein in *E. coli* (Fukuda et al., Eur. J. Biochem. 268:5639-5646 (2001)) and residues involved in substrate specificity were determined (Fukuda and Wakagi, Biochim. Biophys. Acta 1597:74-80 (2002)). The OFOR encoded by *Ape1472*/*Ape1473* from *Aeropyrum pernix* str. K1 was recently cloned into *E. coli,* characterized, and found to react with 2-oxoglutarate and a broad range of 2-oxoacids (Nishizawa et al., FEBS Lett. 579:2319-2322 (2005)). Another exemplary OFOR is encoded by *oorDABC* in *Helicobacter pylori* (Hughes et al. 1998). An enzyme specific to alpha-ketoglutarate has been reported in *Thauera aromatica* (Dorner and Boll, J, Bacteriol. 184 (14), 3975-83 (2002). A similar enzyme can be found in *Rhodospillum rubrum* by sequence homology. A two subunit enzyme has also been identified in *Chlorobium tepidum* (Eisen et al., PNAS 99(14): 9509-14 (2002)).

| Protein | GenBank ID | GI Number | Organism |
|---|---|---|---|
| *korA* | BAB21494 | 12583691 | *Hydrogenobacter thermophilus* |
| *korB* | BAB21495 | 12583692 | *Hydrogenobacter thermophilus* |
| *forD* | BAB62132.1 | 14970994 | *Hydrogenobacter thermophilus* |
| *forA* | BAB62133.1 | 14970995 | *Hydrogenobacter thermophilus* |
| *forB* | BAB62134.1 | 14970996 | *Hydrogenobacter thermophilus* |
| *forG* | BAB62135.1 | 14970997 | *Hydrogenobacter thermophilus* |
| *forE* | BAB62136.1 | 14970998 | *Hydrogenobacter thermophilus* |
| *Clim_0204* | ACD89303.1 | 189339900 | *Chlorobium limicola* |
| *Clim_0205* | ACD89302.1 | 189339899 | *Chlorobium limicola* |
| *Clim_1123* | ACD90192.1 | 189340789 | *Chlorobium limicola* |
| *Clim_1124* | ACD90193.1 | 189340790 | *Chlorobium limicola* |
| *Moth_1984* | YP_430825.1 | 83590816 | *Moorella thermoacetica* |
| *Moth_1985* | YP_430826.1 | 83590817 | *Moorella thermoacetica* |
| *Moth*_ *0034* | YP_428917.1 | 83588908 | *Moorella thermoacetica* |
| *ST2300* | NP_378302.1 | 15922633 | *Sulfolobus* sp. strain 7 |
| *Ape1472* | BAA80470.1 | 5105156 | *Aeropyrum pernix* |
| *Ape1473* | BAA80471.2 | 116062794 | *Aeropyrum pernix* |
| *oorD* | NP_207383.1 | 15645213 | *Helicobacter pylori* |
| *oorA* | NP_207384.1 | 15645214 | *Helicobacter pylori* |
| *oorB* | NP_207383.1 | 15645215 | *Helicobacter pylori* |
| *oorC* | NP_207386.1 | 15645216 | *Helicobacter pylori* |
| *CT0163* | NP_661069.1 | 21673004 | *Chlorobium tepidum* |
| *CT0162* | NP_661068.1 | 21673003 | *Chlorobium tepidum* |
| *korA* | CAA12243.2 | 19571179 | *Thauera aromatica* |
| *korB* | CAD27440.1 | 19571178 | *Thauera aromatica* |
| *Rru_A2721* | YP_427805.1 | 83594053 | *Rhodospirillum rubrum* |
| *Rru_A2722* | YP_427806.1 | 83594054 | *Rhodospirillum rubrum* |

Isocitrate dehydrogenase catalyzes the reversible decarboxylation of isocitrate to 2-oxoglutarate coupled to the reduction of NAD(P)⁺. IDH enzymes in *Saccharomyces cerevisiae* and *Escherichia coli* are encoded by *IDP1* and *icd,* respectively (Haselbeck and McAlister-Henn, J. Biol. Chem. 266:2339-2345 (1991); Nimmo, H.G., Biochem. J. 234:317-2332 (1986)). The reverse reaction in the reductive TCA cycle, the reductive carboxylation of 2-oxoglutarate to isocitrate, is favored by the NADPH-dependent CO₂-fixing IDH from *Chlorobium limicola* and was functionally expressed in *E. coli* (Kanao et al., Eur. J. Biochem. 269:1926-1931 (2002)). A similar enzyme with 95% sequence identity is found in the C. *tepidum* genome in addititon to some other candidates listed below.

| Protein | GenBank ID | GI Number | Organism |
|---|---|---|---|
| *Icd* | ACI84720.1 | 209772816 | *Escherichia coli* |
| *IDP1* | AAA34703.1 | 171749 | *Saccharomyces cerevisiae* |
| *Idh* | BAC00856.1 | 21396513 | *Chlorobium limicola* |
| *Icd* | AAM71597.1 | 21646271 | *Chlorobium tepidum* |
| *icd* | NP_952516.1 | 39996565 | *Geobacter sulfurreducens* |
| *icd* | YP_393560. | 78777245 | *Sulfurimonas denitrificans* |

In *H. thermophilus* the reductive carboxylation of 2-oxoglutarate to isocitrate is catalyzed by two enzymes: 2-oxoglutarate carboxylase and oxalosuccinate reductase. 2-Oxoglutarate carboxylase (EC 6.4.1.7) catalyzes the ATP-dependent carboxylation of alpha-ketoglutarate to oxalosuccinate (Aoshima and Igarashi, Mol. Microbiol. 62:748-759 (2006)). This enzyme is a large complex composed of two subunits. Biotinylation of the large (A) subunit is required for enzyme function (Aoshima et al., Mol. Microbiol. 51:791-798 (2004)). Oxalosuccinate reductase (EC 1.1.1.-) catalyzes the NAD-dependent conversion of oxalosuccinate to D-*threo*-isocitrate. The enzyme is a homodimer encoded by *icd* in *H. thermophilus.* The kinetic parameters of this enzyme indicate that the enzyme only operates in the reductive carboxylation direction *in vivo,* in contrast to isocitrate dehydrogenase enzymes in other organisms (Aoshima and Igarashi, J. Bacteriol. 190:2050-2055 (2008)). Based on sequence homology, gene candidates have also been found in *Thiobacillus denitrificans* and *Thermocrinis albus.*

| Protein | GenBank ID | GI Number | Organism |
|---|---|---|---|
| *cfiA* | BAF34932.1 | 116234991 | *Hydrogenobacter thermophilus* |
| *cifB* | BAF34931.1 | 116234990 | *Hydrogenobacter thermophilus* |
| *Icd* | BAD02487.1 | 38602676 | *Hydrogenobacter thermophilus* |
| *Tbd_1556* | YP_315314 | 74317574 | *Thiobacillus denitrificans* |
| *Tbd_1555* | YP_315313 | 74317573 | *Thiobacillus denitrificans* |
| *Tbd_0854* | YP_314612 | 74316872 | *Thiobacillus denitrificans* |
| *Thal_0268* | YP_003473030 | 289548042 | *Thermocrinis albus* |
| *Thal_0267* | YP_003473029 | 289548041 | *Thermocrinis albus* |
| *Thal_0646* | YP_003473406 | 289548418 | *Thermocrinis albus* |

Aconitase (EC 4.2.1.3) is an iron-sulfur-containing protein catalyzing the reversible isomerization of citrate and iso-citrate via the intermediate *cis*-aconitate. Two aconitase enzymes are encoded in the *E. coli* genome by *acnA* and *acnB.* AcnB is the main catabolic enzyme, while AcnA is more stable and appears to be active under conditions of oxidative or acid stress (Cunningham et al., Microbiology 143 (Pt 12):3795-3805 (1997)). Two isozymes of aconitase in *Salmonella typhimurium* are encoded by *acnA* and *acnB* (Horswill and Escalante-Semerena, Biochemistry 40:4703-4713 (2001)). The *S. cerevisiae* aconitase, encoded by *ACO1,* is localized to the mitochondria where it participates in the TCA cycle (Gangloff et al., Mol. Cell. Biol. 10:3551-3561 (1990)) and the cytosol where it participates in the glyoxylate shunt (Regev-Rudzki et al., Mol. Biol. Cell. 16:4163-4171 (2005)).

| Protein | GenBank ID | GI Number | Organism |
|---|---|---|---|
| *acnA* | AAC7438.1 | 1787531 | *Escherichia coli* |
| *acnB* | AAC73229.1 | 2367097 | *Escherichia coli* |
| *acnA* | NP_460671.1 | 16765056 | *Salmonella typhimurium* |
| *HP0779* | NP_207572.1 | 15645398 | *Helicobacter pylori 26695* |
| *H16_B0568* | CAJ95365.1 | 113529018 | *Ralstonia eutropha* |
| *DesfrDRAFT_3783* | ZP_07335307.1 | 303249064 | *Desulfovibrio fructosovorans JJ* |
| *Suden_1040 (acnB)* | ABB44318.1 | 78497778 | *Sulfurimonas denitrificans* |
| *Hydth_0755* | ADO45152.1 | 308751669 | *Hydrogenobacter thermophilus* |
| *CT0543 (acn)* | AAM71785.1 | 21646475 | *Chlorobium tepidum* |
| *Clim_2436* | YP_001944436.1 | 189347907 | *Chlorobium limicola* |
| *Clim_0515* | ACD89607.1 | 189340204 | *Chlorobium limicola* |
| *acnB* | NP_459163.1 | 16763548 | *Salmonella typhimurium* |
| *ACO1* | AAA34389.1 | 170982 | *Saccharomyces cerevisiae* |

Pyruvate:ferredoxin oxidoreductase (PFOR) catalyzes the reversible oxidation of pyruvate to form acetyl-CoA. The PFOR from *Desulfovibrio africanus* has been cloned and expressed in *E. coli* resulting in an active recombinant enzyme that was stable for several days in the presence of oxygen (Pieulle et al., J. Bacteriol. 179:5684-5692 (1997)). Oxygen stability is relatively uncommon in PFORs and is believed to be conferred by a 60 residue extension in the polypeptide chain of the *D. africanus* enzyme. Two cysteine residues in this enzyme form a disulfide bond that prtotects it against inactivation in the form of oxygen. This disulfide bond and the stability in the presence of oxygen has been found in other *Desulfovibrio* species also (Vita et al., Biochemistry, 47: 957-64 (2008)). The *M. thermoacetica* PFOR is also well characterized (Menon and Ragsdale, Biochemistry 36:8484-8494 (1997)) and was shown to have high activity in the direction of pyruvate synthesis during autotrophic growth (Furdui and Ragsdale, J. Biol. Chem. 275:28494-28499 (2000)). Further, *E. coli* possesses an uncharacterized open reading frame, *ydbK,* encoding a protein that is 51% identical to the *M. thermoacetica* PFOR. Evidence for pyruvate oxidoreductase activity in *E. coli* has been described (Blaschkowski et al., Eur. J. Biochem. 123:563-569 (1982)). PFORs have also been described in other organisms, including *Rhodobacter capsulatas* (Yakunin and Hallenbeck, Biochimica et Biophysica Acta 1409 (1998) 39-49 (1998)) and *Choloboum tepidum* (Eisen et al., PNAS 99(14): 9509-14 (2002)). The five subunit PFOR from *H. thermophilus*, encoded by *porEDABG,* was cloned into *E. coli* and shown to function in both the decarboxylating and CO₂-assimilating directions (Ikeda et al. 2006; Yamamoto et al., Extremophiles 14:79-85 (2010)). Homologs also exist in C. *carboxidivorans P7.* Several additional PFOR enzymes are described in the following review (Ragsdale, S.W., Chem. Rev. 103:2333-2346 (2003)). Finally, flavodoxin reductases (e*.*g*.,fqrB* from *Helicobacter pylori* or *Campylobacter jejuni*) (St Maurice et al., J. Bacteriol. 189:4764-4773 (2007)) or Rnf-type proteins (Seedorf et al., Proc. Natl. Acad. Sci. U.S.A. 105:2128-2133 (2008); and Herrmann, J. Bacteriol 190:784-791 (2008)) provide a means to generate NADH or NADPH from the reduced ferredoxin generated by PFOR. These proteins are identified below.

| Protein | GenBank ID | GI Number | Organism |
|---|---|---|---|
| *DesfrDRAFT_0121* | ZP_07331646.1 | 303245362 | *Desulfovibrio fructosovorans JJ* |
| Por | CAA70873.1 | 1770208 | *Desulfovibrio africanus* |
| por | YP_012236.1 | 46581428 | *Desulfovibrio vulgaris str. Hildenborough* |
| Dde_3237 | ABB40031.1 | 78220682 | *Desulfo Vibrio desulfuricans G20* |
| Ddes_0298 | YP_002478891.1 | 220903579 | *Desulfovibrio desulfuricans subsp. desulfuricans str. ATCC 27774* |
| Por | YP_428946.1 | 83588937 | *Moorella thermoacetica* |
| YdbK | NP_415896.1 | 16129339 | *Escherichia coli* |
| nifJ (CT1628) | NP_662511.1 | 21674446 | *Chlorobium tepidum* |
| CJE1649 | YP_179630.1 | 57238499 | *Campylobacter jejuni* |
| nifJ | ADE85473.1 | 294476085 | *Rhodobacter capsulatus* |
| *porE* | BAA95603.1 | 7768912 | *Hydrogenobacter thermophilus* |
| *porD* | BAA95604.1 | 7768913 | *Hydrogenobacter thermophilus* |
| *porA* | BAA95605.1 | 7768914 | *Hydrogenobacter thermophilus* |
| *porB* | BAA95606.1 | 776891 | *Hydrogenobacter thermophilus* |
| *porG* | BAA95607.1 | 7768916 | *Hydrogenobacter thermophilus* |
| FqrB | YP_001482096.1 | 157414840 | *Campylobacter jejuni* |
| HP1164 | NP_207955.1 | 15645778 | *Helicobacter pylori* |
| RnfC | EDK33306.1 | 146346770 | *Clostridium kluyveri* |
| RnfD | EDK33307.1 | 146346771 | *Clostridium kluyveri* |
| RnfG | EDK33308.1 | 146346772 | *Clostridium kluyveri* |
| RnfE | EDK33309.1 | 146346773 | *Clostridium kluyveri* |
| RnfA | EDK33310.1 | 146346774 | *Clostridium kluyveri* |
| RnfB | EDK33311.1 | 146346775 | *Clostridium kluyveri* |

The conversion of pyruvate into acetyl-CoA can be catalyzed by several other enzymes or their combinations thereof. For example, pyruvate dehydrogenase can transform pyruvate into acetyl-CoA with the concomitant reduction of a molecule of NAD into NADH. It is a multi-enzyme complex that catalyzes a series of partial reactions which results in acylating oxidative decarboxylation of pyruvate. The enzyme comprises of three subunits: the pyruvate decarboxylase (E1), dihydrolipoamide acyltransferase (E2) and dihydrolipoamide dehydrogenase (E3). This enzyme is naturally present in several organisms, including *E. coli* and *S. cerevisiae.* In the *E. coli* enzyme, specific residues in the E1 component are responsible for substrate specificity (Bisswanger, H., J. Biol. Chem. 256:815-82 (1981); Bremer, J., Eur. J. Biochem. 8:535-540 (1969); Gong et al., J. Biol. Chem. 275:13645-13653 (2000)). Enzyme engineering efforts have improved the *E. coli* PDH enzyme activity under anaerobic conditions (Kim et al., J. Bacteriol. 190:3851-3858 (2008); Kim et al., Appl. Environ. Microbiol. 73:1766-1771 (2007); Zhou et al., Biotechnol. Lett. 30:335-342 (2008)). In contrast to the *E. coli* PDH, the *B. subtilis* complex is active and required for growth under anaerobic conditions (Nakano et al., J. Bacteriol. 179:6749-6755 (1997)). The *Klebsiella pneumoniae* PDH, characterized during growth on glycerol, is also active under anaerobic conditions (5). Crystal structures of the enzyme complex from bovine kidney (18) and the E2 catalytic domain from *Azotobacter vinelandii* are available (4). Yet another enzyme that can catalyze this conversion is pyruvate formate lyase. This enzyme catalyzes the conversion of pyruvate and CoA into acetyl-CoA and formate. Pyruvate formate lyase is a common enzyme in prokaryotic organisms that is used to help modulate anaerobic redox balance. Exemplary enzymes can be found in *Escherichia coli* encoded by *pflB* (Knappe and Sawers, FEMS.Microbiol Rev. 6:383-398 (1990)), *Lactococcus lactis* (Melchiorsen et al., Appl Microbiol Biotechnol 58:338-344 (2002)), and *Streptococcus mutans* (Takahashi-Abbe et al., Oral.Microbiol Immunol. 18:293-297 (2003)). *E. coli* possesses an additional pyruvate formate lyase, encoded by *tdcE,* that catalyzes the conversion of pyruvate or 2-oxobutanoate to acetyl-CoA or propionyl-CoA, respectively (Hesslinger et al., Mol. Microbiol 27:477-492 (1998)). Both *pflB* and *tdcE* from *E. coli* require the presence of pyruvate formate lyase activating enzyme, encoded by *pflA.* Further, a short protein encoded by *yfiD* in *E. coli* can associate with and restore activity to oxygen-cleaved pyruvate formate lyase (Vey et al., Proc.Natl. Acad. Sci. U.S.A. 105:16137-16141 (2008). Note that *pflA* and *pflB* from *E. coli* were expressed in *S. cerevisiae* as a means to increase cytosolic acetyl-CoA for butanol production as described in WO/2008/080124]. Additional pyruvate formate lyase and activating enzyme candidates, encoded by *pfl* and *act,* respectively, are found in *Clostridium pasteurianum* (Weidner et al., J Bacteriol. 178:2440-2444 (1996)).

Further, different enzymes can be used in combination to convert pyruvate into acetyl-CoA. For example, in *S. cerevisiae,* acetyl-CoA is obtained in the cytosol by first decarboxylating pyruvate to form acetaldehyde; the latter is oxidized to acetate by acetaldehyde dehydrogenase and subsequently activated to form acetyl-CoA by acetyl-CoA synthetase. Acetyl-CoA synthetase is a native enzyme in several other organisms including *E. coli* (Kumari et al., J. Bacteriol. 177:2878-2886 (1995)), *Salmonella enterica* (Starai et al., Microbiology 151:3793-3801 (2005); Starai et al., J. Biol. Chem. 280:26200-26205 (2005)), and *Moorella thermoacetica* (described already). Alternatively, acetate can be activated to form acetyl-CoA by acetate kinase and phosphotransacetylase. Acetate kinase first converts acetate into acetyl-phosphate with the accompanying use of an ATP molecule. Acetyl-phosphate and CoA are next converted into acetyl-CoA with the release of one phosphate by phosphotransacetylase. Both acetate kinase and phosphotransacetlyase are well-studied enzymes in several *Clostridia* and *Methanosarcina thermophila.*

Yet another way of converting pyruvate to acetyl-CoA is via pyruvate oxidase. Pyruvate oxidase converts pyruvate into acetate, using ubiquione as the electron acceptor. In *E. coli,* this activity is encoded by *poxB. PoxB* has similarity to pyruvate decarboxylase *of S. cerevisiae* and *Zymomonas mobilis.* The enzyme has a thiamin pyrophosphate cofactor (Koland and Gennis, Biochemistry 21:4438-4442 (1982)); O'Brien et al., Biochemistry 16:3105-3109 (1977); O'Brien and Gennis, J. Biol. Chem. 255:3302-3307 (1980)) and a flavin adenine dinucleotide (FAD) cofactor. Acetate can then be converted into acctyl-CoA by either acetyl-CoA synthetase or by acetate kinase and phosphotransacetylase, as described earlier. Some of these enzymes can also catalyze the reverse reaction from acetyl-CoA to pyruvate.

For enzymes that use reducing equivalents in the form of NADH or NADPH, these reduced carriers can be generated by transferring electrons from reduced ferredoxin. Two enzymes catalyze the reversible transfer of electrons from reduced ferredoxins to NAD(P)⁻, ferredoxin:NAD⁺ oxidoreductase (EC 1.18.1.3) and ferredoxin:NADP⁺ oxidoreductase (FNR, EC 1.18.1.2). Ferredoxin:NADP⁺ oxidoreductase (FNR, EC 1.18.1.2) has a noncovalently bound FAD cofactor that facilitates the reversible transfer of electrons from NADPH to low-potential acceptors such as ferredoxins or flavodoxins (Blaschkowski et al., Eur. J. Biochem. 123:563-569 (1982); Fujii et al., 1977). The *Helicobacter pylori* FNR, encoded by *HP1164 (fqrB),* is coupled to the activity of pyruvate:ferredoxin oxidoreductase (PFOR) resulting in the pyruvate-dependent production of NADPH (St et al. 2007). An analogous enzyme is found in *Campylobacter jejuni* (St et al. 2007). A ferredoxin:NADP⁺ oxidoreductase enzyme is encoded in the *E. coli* genome by *fpr* (Bianchi et al. 1993). Ferredoxin:NAD⁺ oxidoreductase utilizes reduced ferredoxin to generate NADH from NAD⁺. In several organisms, including *E. coli,* this enzyme is a component of multifunctional dioxygenase enzyme complexes. The ferredoxin:NAD⁺ oxidoreductase of *E. coli,* encoded by *hcaD,* is a component of the 3-phenylproppionate dioxygenase system involved in involved in aromatic acid utilization (Diaz et al. 1998). NADH:ferredoxin reductase activity was detected in cell extracts of *Hydrogenobacter thermophilus* strain TK-6, although a gene with this activity has not yet been indicated (Yoon et al. 2006). Finally, the energy-conserving membrane-associated Rnf-type proteins (Seedorf et al., Proc. Natl. Acad. Sci. U.S.A. 105:2128-2133 (2008); Herrmann et al., J. Bacteriol. 190:784-791 (2008)) provide a means to generate NADH or NADPH from reduced ferredoxin. Additional ferredoxin:NAD(P)+ oxidoreductases have been annotated in *Clostridium carboxydivorans* P7.

| Protein | GenBank ID | GI Number | Organism |
|---|---|---|---|
| *HP1164* | NP_207955.1 | 15645778 | *Helicobacter pylori* |
| *RPA3954* | CAE29395.1 | 39650872 | *Rhodopseudomonas palustris* |
| *fpr* | BAH29712.1 | 225320633 | *Hydrogenobacter thermophilus* |
| *yumC* | NP_391091.2 | 255767736 | *Bacillus subtilis* |
| CJE0663 | AAW35824.1 | 57167045 | *Campylobacter jejuni* |
| *fpr* | P28861.4 | 399486 | *Escherichia coli* |
| *hcaD* | AAC75595.1 | 1788892 | *Escherichia coli* |
| *LOC100282643* | NP_001149023.1 | 226497434 | *Zea mays* |
| *RnfC* | EDK33306.1 | 146346770 | *Clostridium kluyveri* |
| *RnfD* | EDK33307.1 | 146346771 | *Clostridium kluyveri* |
| *RnfG* | EDK33308.1 | 146346772 | *Clostridium kluyveri* |
| *RnfE* | EDK33309.1 | 146346773 | *Clostridium kluyveri* |
| *RnfA* | EDK33310.1 | 146346774 | *Clostridium kluyveri* |
| *RnfB* | EDK33311.1 | 146346775 | *Clostridium kluyveri* |
| *CcarbDRAFT_2639* | ZP_05392639.1 | 255525707 | *Clostridium carboxidivorans P7* |
| *CcarbDRAFT_2638* | ZP_05392638.1 | 255525706 | *Clostridium carboxidivorans P7* |
| *CcarbDRAFT_2636* | ZP_05392636.1 | 255525704 | *Clostridium carboxidivorans P7* |
| *CcarbDRAFT_5060* | ZP_05395060.1 | 255528241 | *Clostridium carboxidivorans P7* |
| *CcarbDRAFT_2450* | ZP_05392450.1 | 255525514 | *Clostridium carboxidivorans P7* |
| *CcarbDRAFT_1084* | ZP_05391084.1 | 255524124 | *Clostridium carboxidivorans P7* |

Ferredoxins are small acidic proteins containing one or more iron-sulfur clusters that function as intracellular electron carriers with a low reduction potential. Reduced ferredoxins donate electrons to Fe-dependent enzymes such as ferredoxin-NADP⁺ oxidoreductase, pyruvate:ferredoxin oxidoreductase (PFOR) and 2-oxoglutarate:ferredoxin oxidoreductase (OFOR). The *H. thermophilus* gene *fdx1* encodes a [4Fe-4S]-type ferredoxin that is required for the reversible carboxylation of 2-oxoglutarate and pyruvate by OFOR and PFOR, respectively (Yamamoto et al., Extremophiles 14:79-85 (2010)). The ferredoxin associated with the *Sulfolobus solfataricus* 2-oxoacid:ferredoxin reductase is a monomeric dicluster [3Fe-4S][4Fe-4S] type ferredoxin (Park et al. 2006). While the gene associated with this protein has not been fully sequenced, the N-terminal domain shares 93% homology with the *zfx* ferredoxin from *S. acidocaldarius.* The *E. coli* genome encodes a soluble ferredoxin of unknown physiological function, *fdx*. Some evidence indicates that this protein can function in iron-sulfur cluster assembly (Takahashi and Nakamura, 1999). Additional ferredoxin proteins have been characterized in *Helicobacter pylori* (Mukhopadhyay et al. 2003) and *Campylobacter jejuni* (van Vliet et al. 2001). A 2Fe-2S ferredoxin from *Clostridium pasteurianum* has been cloned and expressed in *E. coli* (Fujinaga and Meyer, Biochemical and Biophysical Research Communications, 192(3): (1993)). Acetogenic bacteria such as *Moorella thermoacetica, Clostridium carboxidivorans P7* and *Rhodospirillum rubrum* are predicted to encode several ferredoxins, listed in the table below.

| **Protein** | **GenBank ID** | **GI Number** | **Organism** |
|---|---|---|---|
| *fdx1* | BAE02673.1 | 68163284 | *Hydrogenobacter thermophilus* |
| M11214.1 | AAA83524.1 | 144806 | *Clostridium pasteurianum* |
| *Zfx* | AAY79867.1 | 68566938 | *Sulfolobus acidocalarius* |
| *Fdx* | AAC75578.1 | 1788874 | *Escherichia coli* |
| *hp_ 0277* | AAD07340.1 | 2313367 | *Helicobacter pylori* |
| *fdxA* | CAL34484.1 | 112359698 | *Campylobacter jejuni* |
| *Moth_0061* | ABC18400.1 | 83571848 | *Moorella thermoacetica* |
| *Moth_1200* | ABC19514.1 | 83572962 | *Moorella thermoacetica* |
| *Moth_1888* | ABC20188.1 | 83573636 | *Moorella thermoacetica* |
| *Moth_2112* | ABC20404.1 | 83573852 | *Moorella thermoacetica* |
| Moth_1037 | ABC19351.1 | 83572799 | *Moorella thermoacetica* |
| CcarbDRAFT_4383 | ZP_05394383.1 | 255527515 | *Clostridium carboxidivorans P7* |
| CcarbDRAFT_2958 | ZP_05392958.1 | 255526034 | *Clostridium carboxidivorans P7* |
| CcarbDRAFT_2281 | ZP_05392281.1 | 255525342 | *Clostridium carboxidivorans P7* |
| CcarbDRAFT_5296 | ZP_05395295.1 | 255528511 | *Clostridium carboxidivorans P7* |
| CcarbDRAFT_1615 | ZP_05391615.1 | 255524662 | *Clostridium carboxidivorans P7* |
| CcarbDRAFT_1304 | ZP_05391304.1 | 255524347 | *Clostridium carboxidivorans P7* |
| cooF | AAG29808.1 | 11095245 | *Carboxydothermus hydrogenoformans* |
| fdxN | CAA35699.1 | 46143 | *Rhodobacter capsulatus* |
| Rru_A2264 | ABC23064.1 | 83576513 | *Rhodospirillum rubrum* |
| Rru_A1916 | ABC22716.1 | 83576165 | *Rhodospirillum rubrum* |
| Rru_A2026 | ABC22826.1 | 83576275 | *Rhodospirillum rubrum* |
| cooF | AAC45122.1 | 1498747 | *Rhodospirillum rubrum* |
| fdxN | AAA26460.1 | 152605 | *Rhodospirillum rubrum* |
| Alvin_2884 | ADC63789.1 | 288897953 | *Allochromatium vinosum DSM 180* |
| fdx | YP_002801146.1 | 226946073 | *Azotobacter vinelandii DJ* |
| CKL_3790 | YP_001397146.1 | 153956381 | *Clostridium kluyveri DSM 555* |
| fer1 | NP_949965.1 | 39937689 | *Rhodopseudomonas palustris CGA009* |
| fdx | CAA12251.1 | 3724172 | *Thauera aromatica* |
| CHY_2405 | YP_361202.1 | 78044690 | *Carboxydothermus hydrogenoformans* |
| fer | YP_359966.1 | 78045103 | *Carboxydothermus hydrogenoformans* |
| fer | AAC83945.1 | 1146198 | *Bacillus subtilis* |
| fdx1 | NP_249053.1 | 15595559 | *Pseudomonas aeruginosa PA01* |
| yfhL | AP_003148.1 | 89109368 | *Escherichia coli K-12* |

Succinyl-CoA transferase catalyzes the conversion of succinyl-CoA to succinate while transferring the CoA moiety to a CoA acceptor molecule. Many transferases have broad specificity and can utilize CoA acceptors as diverse as acetate, succinate, propionate, butyrate, 2-methylacetoacetate, 3-ketohexanoate, 3-ketopentanoate, valerate, crotonate, 3-mercaptopropionate, propionate, vinylacetate, and butyrate, among others.

The conversion of succinate to succinyl-CoA can be carried by a transferase which does not require the direct consumption of an ATP or GTP. This type of reaction is common in a number of organisms. The conversion of succinate to succinyl-CoA can also be catalyzed by succinyl-CoA:Acetyl-CoA transferase. The gene product of *cat1* of *Clostridium kluyveri* has been shown to exhibit succinyl-CoA: acetyl-CoA transferase activity (Sohling and Gottschalk, J. Bacteriol. 178:871-880 (1996)). In addition, the activity is present in *Trichomonas vaginalis* (van Grinsven et al. 2008) and *Trypanosoma brucei* (Riviere et al. 2004). The succinyl-CoA:acetate CoA-transferase from *Acetobacter aceti,* encoded by *aarC,* replaces succinyl-CoA synthetase in a variant TCA cycle (Mullins et al. 2008). Similar succinyl-CoA transferase activities are also present in *Trichomonas vaginalis* (van Grinsven et al. 2008), *Trypanosoma brucei* (Riviere et al. 2004) and *Clostridium kluyveri* (Sohling and Gottschalk, 1996c). The beta-ketoadipate:succinyl-CoA transferase encoded by *pcaI* and *pcaJ* in *Pseudomonas putida* is yet another candidate (Kaschabek et al. 2002). The aforementioned proteins are identified below.

| Protein | GenBank ID | GI Number | Organism |
|---|---|---|---|
| *cat1* | P38946.1 | 729048 | *Clostridium kluyveri* |
| *TVAG_395550* | XP_001330176 | 123975034 | *Trichomonas vaginalis G3* |
| *Tb11.02.0290* | XP_828352 | 71754875 | *Trypanosoma brucei* |
| *pcaI* | AAN69545.1 | 24985644 | *Pseudomonas putida* |
| *pcaJ* | NP_746082.1 | 26990657 | *Pseudomonas putida* |
| *aarC* | ACD85596.1 | 189233555 | *Acetobacter aceti* |

An additional exemplary transferase that converts succinate to succinyl-CoA while converting a 3-ketoacyl-CoA to a 3-ketoacid is succinyl-CoA:3:ketoacid-CoA transferase (EC 2.8.3.5). Exemplary succinyl-CoA:3:ketoacid-CoA transferases are present in *Helicobacter pylori* (Corthesy-Theulaz et al. 1997), *Bacillus subtilis,* and *Homo sapiens* (Fukao et al. 2000; Tanaka et al. 2002). The aforementioned proteins are identified below.

| Protein | GenBank ID | GI Number | Organism |
|---|---|---|---|
| *HPAG1_0676* | YP_627417 | 108563101 | *Helicobacter pylori* |
| *HPAG1_0677* | YP_627418 | 108563102 | *Helicobacter pylori* |
| *ScoA* | NP_391778 | 16080950 | *Bacillus subtilis* |
| *ScoB* | NP_391777 | 16080949 | *Bacillus subtilis* |
| *OXCT1* | NP_000427 | 4557817 | *Homo sapiens* |
| *OXCT2* | NP_071403 | 11545841 | *Homo sapiens* |

Converting succinate to succinyl-CoA by succinyl-CoA:3:ketoacid-CoA transferase requires the simultaneous conversion of a 3-ketoacyl-CoA such as acetoacetyl-CoA to a 3-ketoacid such as acetoacetate. Conversion of a 3-ketoacid back to a 3-ketoacyl-CoA can be catalyzed by an acetoacetyl-CoA:acetate:CoA transferase. Acetoacetyl-CoA:acetate:CoA transferase converts acetoacetyl-CoA and acetate to acetoacetate and acetyl-CoA, or vice versa. Exemplary enzymes include the gene products of *atoAD* from *E. coli* (Hanai et al., Appl Environ Microbiol 73:7814-7818 (2007), *ctfAB* from *C. acetobutylicum* (Jojima et al., Appl Microbiol Biotechnol 77:1219-1224 (2008), and *ctfAB* from *Clostridium saccharoperbutylacetonicum* (Kosaka et al., Biosci.Biotechnol Biochem. 71:58-68 (2007)) are shown below.

| Protein | GenBank ID | GI Number | Organism |
|---|---|---|---|
| *AtoA* | NP_416726.1 | 2492994 | *Escherichia coli* |
| *AtoD* | NP_416725.1 | 2492990 | *Escherichia coli* |
| *CtfA* | NP_149326.1 | 15004866 | *Clostridium acetobutylicum* |
| *CtfB* | NP_149327.1 | 15004867 | *Clostridium acetobutylicum* |
| *CtfA* | AAP42564.1 | 31075384 | *Clostridium saccharoperbutylacetonicum* |
| *CtfB* | AAP42565.1 | 31075385 | *Clostridium saccharoperbutylacetonicum* |

Yet another possible CoA acceptor is benzylsuccinate. Succinyl-CoA:(R)-Benzylsuccinate CoA-Transferase functions as part of an anaerobic degradation pathway for toluene in organisms such as *Thauera aromatica* (Leutwein and Heider, J. Bact. 183(14) 4288-4295 (2001)). Homologs can be found in *Azoarcus* sp. T, *Aromatoleum aromaticum* EbN1, and *Geobacter metallireducens* GS-15. The aforementioned proteins are identified below.

| Protein | GenBank ID | GI Number | Organism |
|---|---|---|---|
| *bbsE* | AAF89840 | 9622535 | *Thauera aromatic* |
| *Bbsf* | AAF89841 | 9622536 | *Thauera aromatic* |
| *bbsE* | AAU45405.1 | 52421824 | *Azoarcus sp. T* |
| *bbsF* | AAU45406.1 | 52421825 | *Azoarcus sp. T* |
| *bbsE* | YP_158075.1 | 56476486 | *Aromatoleum aromaticum EbN1* |
| *bbsF* | YP_158074.1 | 56476485 | *Aromatoleum aromaticum EbN1* |
| *Gmet_1521* | YP_384480.1 | 78222733 | *Geobacter metallireducens GS-15* |
| *Gmet_1522* | YP_84481.1 | 78222734 | *Geobacter metallireducens GS-15* |

Additionally, *ygfH* encodes a propionyl CoA:succinate CoA transferase in *E. coli* (Haller et al., Biochemistry, 39(16) 4622-4629). Close homologs can be found in, for example, *Citrobacter youngae* ATCC 29220, *Salmonella enterica* subsp. *arizonae serovar*, and *Yersinia intermedia* ATCC 29909. The aforementioned proteins are identified below.

| Protein | GenBank ID | GI Number | Organism |
|---|---|---|---|
| *ygfH* | NP_417395.1 | 16130821 | *Escherichia coli str. K-12 substr. MG1655* |
| *CIT292_04485* | ZP_03838384.1 | 227334728 | *Citrobacter youngae ATCC 29220* |
| *SARI_04582* | YP_001573497.1 | 161506385 | *Salmonella enterica subsp. arizonae serovar* |
| *yinte0001_14430* | ZP_04635364.1 | 238791727 | *Yersinia intermedia ATCC 29909* |

Citrate lyase (EC 4.1.3.6) catalyzes a series of reactions resulting in the cleavage of citrate to acetate and oxaloacetate. The enzyme is active under anaerobic conditions and is composed of three subunits: an acyl-carrier protein (ACP, gamma), an ACP transferase (alpha), and a acyl lyase (beta). Enzyme activation uses covalent binding and acetylation of an unusual prosthetic group, 2'-(5"-phosphoribosyl)-3-'-dephospho-CoA, which is similar in structure to acctyl-CoA. Acylation is catalyzed by CitC, a citrate lyase synthetase. Two additional proteins, CitG and CitX, are used to convert the apo enzyme into the active holo enzyme (Schneider et al., Biochemistry 39:9438-9450 (2000)). Wild type *E. coli* does not have citrate lyase activity; however, mutants deficient in molybdenum cofactor synthesis have an active citrate lyase (Clark, FEMS Microbiol. Lett. 55:245-249 (1990)). The *E. coli* enzyme is encoded by *citEFD* and the citrate lyase synthetase is encoded by *citC* (Nilekani and SivaRaman, Biochemistry 22:4657-4663 (1983)). The *Leuconostoc mesenteroides* citrate lyase has been cloned, characterized and expressed in *E. coli* (Bekal et al., J. Bacteriol. 180:647-654 (1998)). Citrate lyase enzymes have also been identified in enterobacteria that utilize citrate as a carbon and energy source, including *Salmonella typhimurium* and *Klebsiella pneumoniae* (Bott, Arch. Microbiol. 167: 78-88 (1997); Bott and Dimroth, Mol. Microbiol. 14:347-356 (1994)). The aforementioned proteins are tabulated below.

| **Protein** | **GenBank ID** | **GI Number** | **Organism** |
|---|---|---|---|
| *citF* | AAC73716.1 | 1786832 | *Escherichia coli* |
| *Cite* | AAC73717.2 | 87081764 | *Escherichia coli* |
| *citD* | AAC73718.1 | 1786834 | *Escherichia coli* |
| *citC* | AAC73719.2 | 87081765 | *Escherichia coli* |
| *citG* | AAC73714.1 | 1786830 | *Escherichia coli* |
| *citX* | AAC73715.1 | 1786831 | *Escherichia coli* |
| *citF* | CAA71633.1 | 2842397 | *Leuconostoc mesenteroides* |
| *Cite* | CAA71632.1 | 2842396 | *Leuconostoc mesenteroides* |
| *citD* | CAA71635.1 | 2842395 | *Leuconostoc mesenteroides* |
| *citC* | CAA71636.1 | 3413797 | *Leuconostoc mesenteroides* |
| *citG* | CAA71634.1 | 2842398 | *Leuconostoc mesenteroides* |
| *citX* | CAA71634.1 | 2842398 | *Leuconostoc mesenteroides* |
| *citF* | NP_459613.1 | 16763998 | *Salmonella typhimurium* |
| *cite* | AAL19573.1 | 16419133 | *Salmonella typhimurium* |
| *citD* | NP_459064.1 | 16763449 | *Salmonella typhimurium* |
| *citC* | NP_459616.1 | 16764001 | *Salmonella typhimurium* |
| *citG* | NP_459611.1 | 16763996 | *Salmonella typhimurium* |
| *citX* | NP_459612.1 | 16763997 | *Salmonella typhimurium* |
| *citF* | CAA56217.1 | 565619 | *Klebsiella pneumoniae* |
| *cite* | CAA56216.1 | 565618 | *Klebsiella pneumoniae* |
| *citD* | CAA56215.1 | 565617 | *Klebsiella pneumoniae* |
| *citC* | BAH66541.1 | 238774045 | *Klebsiella pneumoniae* |
| *citG* | CAA56218.1 | 565620 | *Klebsiella pneumoniae* |
| *citX* | AAL60463.1 | 18140907 | *Klebsiella pneumoniae* |

Acetate kinase (EC 2.7.2.1) catalyzes the reversible ATP-dependent phosphorylation of acetate to acetylphosphate. Exemplary acetate kinase enzymes have been characterized in many organisms including *E. coli, Clostridium acetobutylicum* and Methanosarcina *thermophila* (Ingram-Smith et al., J. Bacteriol. 187:2386-2394 (2005); Fox and Roseman, J. Biol. Chem. 261:13487-13497 (1986); Winzer et al., Microbioloy 143 (Pt 10):3279-3286 (1997)). Acetate kinase activity has also been demonstrated in the gene product of *E. coli purT* (Marolewski et al., Biochemistry 33:2531-2537 (1994). Some butyrate kinase enzymes (EC 2.7.2.7), for example *buk1* and *buk2* from *Clostridium acetobutylicum*, also accept acetate as a substrate (Hartmanis, M.G., J. Biol. Chem. 262:617-621 (1987)).

| Protein | GenBank ID | GI Number | Organism |
|---|---|---|---|
| *ackA* | NP_416799.1 | 16130231 | *Escherichia coli* |
| *Ack* | AAB18301.1 | 1491790 | *Clostridium acetobutylicum* |
| *Ack* | AAA72042.1 | 349834 | *Methanosarcina thermophila* |
| *purT* | AAC74919.1 | 1788155 | *Escherichia coli* |
| *buk1* | NP_349675 | 15896326 | *Clostridium acetobutylicum* |
| *buk2* | Q97II1 | 20137415 | *Clostridium acetobutylicum* |

The formation of acetyl-CoA from acetylphosphate is catalyzed by phosphotransacetylase (EC 2.3.1.8). The *pta* gene from *E. coli* encodes an enzyme that reversibly converts acetyl-CoA into acetyl-phosphate (Suzuki, T., Biochim. Biophys. Acta 191:559-569 (969)). Additional acetyltransferase enzymes have been characterized in *Bacillus subtilis* (Rado and Hoch, Biochim. Biophys. Acta 321:114-125 (1973), *Clostridium kluyveri* (Stadtman, E., Methods Enzymol. 1:5896-599 (1955), and *Thermotoga maritima* (Bock et al., J. Bacteriol. 181:1861-1867 (1999)). This reaction is also catalyzed by some phosphotranbutyrylase enzymes (EC 2.3.1.19) including the *ptb* gene products from *Clostridium acetobutylicum* (Wiesenborn et al., App. Environ. Microbiol. 55:317-322 (1989); Walter et al., Gene 134:107-111 (1993)). Additional *ptb* genes are found in butyrate-producing bacterium L2-50 (Louis et al., J. Bacteriol. 186:2099-2106 (2004) and *Bacillus megaterium* (Vazquez et al., Curr. Microbiol. 42:345-349 (2001).

| **Protein** | **GenBank ID** | **GI Number** | **Organism** |
|---|---|---|---|
| *Pta* | NP_416800.1 | 71152910 | *Escherichia coli* |
| *Pta* | P39646 | 730415 | *Bacillus subtilis* |
| *Pta* | A5N801 | 146346896 | *Clostridium kluyveri* |
| *Pta* | Q9X0L4 | 6685776 | *Thermotoga maritima* |
| *Ptb* | NP_349676 | 34540484 | *Clostridium acetobutylicum* |
| *Ptb* | AAR19757.1 | 38425288 | butyrate-producing bacterium L2-50 |
| *Ptb* | CAC07932.1 | 10046659 | *Bacillus megaterium* |

The acylation of acetate to acetyl-CoA is catalyzed by enzymes with acetyl-CoA synthetase activity. Two enzymes that catalyze this reaction are AMP-forming acetyl-CoA synthetase (EC 6.2.1.1) and ADP-forming acetyl-CoA synthetase (EC 6.2.1.13). AMP-forming acetyl-CoA synthetase (ACS) is the predominant enzyme for activation of acetate to acetyl-CoA. Exemplary ACS enzymes are found in *E. coli* (Brown et al., J. Gen. Microbiol. 102:327-336 (1977)), *Ralstonia eutropha* (Priefert and Steinbuchel, J. Bacteriol. 174:6590-6599 (1992)), *Methanothermobacter thermautotrophicus* (Ingram-Smith and Smith, Archaea 2:95-107 (2007)), *Salmonella enterica* (Gulick et al., Biochemistry 42:2866-2873 (2003)) and *Saccharomyces cerevisiae* (Jogl and Tong, Biochemistry 43:1425-1431 (2004)). ADP-forming acetyl-CoA synthetases are reversible enzymes with a generally broad substrate range (Musfeldt and Schonheit, J. Bacteriol. 184:636-644 (2002)). Two isozymes of ADP-forming acetyl-CoA synthetases are encoded in the *Archaeoglobus fulgidus* genome by are encoded by AF1211 and AF1983 (Musfeldt and Schonheit, *supra* (2002)). The enzyme from *Haloarcula marismortui* (annotated as a succinyl-CoA synthetase) also accepts acetate as a substrate and reversibility of the enzyme was demonstrated (Brasen and Schonheit, Arch. Microbiol. 182:277-287 (2004)). The ACD encoded by *PAE3250* from hyperthermophilic crenarchaeon *Pyrobaculum aerophilum* showed the broadest substrate range of all characterized ACDs, reacting with acetate, isobutyryl-CoA (preferred substrate) and phenylacetyl-CoA (Brasen and Schonheit, *supra* (2004)). Directed evolution or engineering can be used to modify this enzyme to operate at the physiological temperature of the host organism. The enzymes from *A. fulgidus, H. marismortui* and *P. aerophilum* have all been cloned, functionally expressed, and characterized in *E. coli* (Brasen and Schonheit, *supra* (2004); Musfeldt and Schonheit, *supra* (2002)). Additional candidates include the succinyl-CoA synthetase encoded by *sucCD* in *E. coli* (Buck et al., Biochemistry 24:6245-6252 (1985)) and the acyl-CoA ligase from *Pseudomonas putida* (Fernandez-Valverde et al., Appl. Environ. Microbiol. 59:1149-1154 (1993)). The aforementioned proteins are tabulated below.

| **Protein** | **GenBank ID** | **GI Number** | **Organism** |
|---|---|---|---|
| *acs* | AAC77039.1 | 1790505 | *Escherichia coli* |
| *acoE* | AAA21945.1 | 141890 | *Ralstonia eutropha* |
| *acs1* | ABC87079.1 | 86169671 | *Methanothermobacter thermautotrophicus* |
| *acs1* | AAL23099.1 | 16422835 | *Salmonella enterica* |
| *ACS1* | Q01574.2 | 257050994 | *Saccharomyces cerevisiae* |
| *AF1211* | NP_070039.1 | 11498810 | *Archaeoglobus fulgidus* |
| *AF1983* | NP_070807.1 | 11499565 | *Archaeoglobus fulgidus* |
| *scs* | YP_135572.1 | 55377722 | *Haloarcula marismortui* |
| *PAE3250* | NP_560604.1 | 18313937 | *Pyrobaculum aerophilum* str. IM2 |
| *sucC* | NP_415256.1 | 16128703 | *Escherichia coli* |
| *sucD* | AAC73823.1 | 1786949 | *Escherichia coli* |
| *paaF* | AAC24333.2 | 22711873 | *Pseudomonas putida* |

The product yields per C-mol of substrate of microbial cells synthesizing reduced fermentation products such as 2,4-pentadienoate, butadiene, 1,3-butanediol or 3-buten-1-ol, are limited by insufficient reducing equivalents in the carbohydrate feedstock. Reducing equivalents, or electrons, can be extracted from synthesis gas components such as CO and H₂ using carbon monoxide dehydrogenase (CODH) and hydrogenase enzymes, respectively. The reducing equivalents are then passed to acceptors such as oxidized ferredoxins, oxidized quinones, oxidized cytochromes, NAD(P)+, water, or hydrogen peroxide to form reduced ferredoxin, reduced quinones, reduced cytochromes, NAD(P)H, H₂, or water, respectively. Reduced ferredoxin and NAD(P)H are particularly useful as they can serve as redox carriers for various Wood-Ljungdahl pathway and reductive TCA cycle enzymes.

Here, we show specific examples of how additional redox availability from CO and/or H₂ can improve the yields of reduced products such as 2,4-pentadienoate, butadiene, 1,3-butanediol or 3-buten-1-ol.

In some embodiments of the invention, a combined feedstock strategy where syngas is combined with a sugar-based feedstock or other carbon substrate can greatly improve the theoretical yields. In this co-feeding appoach, syngas components H₂ and CO can be utilized by the hydrogenase and CO dehydrogenase to generate reducing equivalents, that can be used to power chemical production pathways in which the carbons from sugar or other carbon substrates will be maximally conserved and the theoretical yields improved. Such improvements provide environmental and economic benefits and greatly enhance sustainable chemical production.

Herein below the enzymes and the corresponding genes used for extracting redox from synags components are described. CODH is a reversible enzyme that interconverts CO and CO₂ at the expense or gain of electrons. The natural physiological role of the CODH in ACS/CODH complexes is to convert CO₂ to CO for incorporation into acetyl-CoA by acetyl-CoA synthase. Nevertheless, such CODH enzymes are suitable for the extraction of reducing equivalents from CO due to the reversible nature of such enzymes. Expressing such CODH enzymes in the absence of ACS allows them to operate in the direction opposite to their natural physiological role (i.e., CO oxidation).

In *M. thermoacetica, C. hydrogenoformans, C. carboxidivorans P7,* and several other organisms, additional CODH encoding genes are located outside of the ACS/CODH operons. These enzymes provide a means for extracting electrons (or reducing equivalents) from the conversion of carbon monoxide to carbon dioxide. The *M. thermoacetica* gene (Genbank Accession Number: YP_430813) is expressed by itself in an operon and is believed to transfer electrons from CO to an external mediator like ferredoxin in a "Ping-pong" reaction. The reduced mediator then couples to other reduced nicolinamide adenine dinucleotide phosphate (NAD(P)H) carriers or ferredoxin-dependent cellular processes (Ragsdale, Annals of the New York Academy of Sciences 1125: 129-136 (2008)). The genes encoding the *C. hydrogenoformans* CODH-II and CooF, a neighboring protein, were cloned and sequenced (Gonzalez and Robb, FEMS Microbiol Lett. 191:243-247 (2000)). The resulting complex was membrane-bound, although cytoplasmic fractions of CODH-II were shown to catalyze the formation of NADPH suggesting an anabolic role (Svctlitchnyi ct al., J Bacteriol. 183:5134-5144 (2001)). The crystal structure of the CODH-II is also available (Dobbek et al., Science 293:1281-1285 (2001)). Similar ACS-free CODH enzymes can be found in a diverse array of organisms including *Geobacter metallireducens* GS-15, *Chlorobium phaeobacteroides* DSM 266, *Clostridium cellulolyticum* H10, *Desulfovibrio desulfuricans* subsp. *desulfuricans* str. ATCC 27774, *Pelobacter carbinolicus* DSM 2380, and *Campylobacter curvus* 525.92.

| Protein | GenBank ID | GI Number | Organism |
|---|---|---|---|
| CODH (putative) | YP_430813 | 83590804 | *Moorella thermoacetica* |
| CODH-II (CooS-II) | YP_358957 | 78044574 | *Carboxydothermus hydrogenoformans* |
| CooF | YP_358958 | 78045112 | *Carboxydothermus hydrogenoformans* |
| CODH (putative) | ZP_05390164.1 | 255523193 | *Clostridium carboxidivorans P7* |
| CcarbDRAFT_0341 | ZP_05390341.1 | 255523371 | *Clostridium carboxidivorans P7* |
| CcarbDRAFT_1756 | ZP_05391756.1 | 255524806 | *Clostridium carboxidivorans P7* |
| CcarbDRAFT_2944 | ZP_05392944.1 | 255526020 | *Clostridium carboxidivorans P7* |
| CODH | YP_384856.1 | 78223109 | *Geobacter metallireducens GS-15* |
| *Cpha266_0148 (cytochrome c)* | YP_910642.1 | 119355998 | *Chlorobium phaeobacteroides DSM 266* |
| *Cpha266_0149 (CODH)* | YP_910643.1 | 119355999 | *Chlorobium phaeobacteroides DSM 266* |
| *Ccel_0438* | YP_002504800.1 | 220927891 | *Clostridium cellulolyticum H10* |
| *Ddes_0382 (CODH)* | YP_002478973.1 | 220903661 | *Desulfovibrio desulfuricans subsp. desulfuricans str. ATCC 27774* |
| *Ddes_ 0381 (CooC)* | YP_002478972.1 | 220903660 | *Desulfovibrio desulfuricans subsp. desulfuricans str. ATCC 27774* |
| *Pcar_0057 (CODH)* | YP_355490.1 | 7791767 | *Pelobacter carbinolicus DSM 2380* |
| *Pcar_0058 (CooC)* | YP_355491.1 | 7791766 | *Pelobacter carbinolicus DSM 2380* |
| *Pcar_0058 (HypA)* | YP_355492.1 | 7791765 | *Pelobacter carbinolicus DSM 2380* |
| *CooS (CODH)* | YP_001407343.1 | 154175407 | *Campylobacter curvus 525.92* |

In some cases, hydrogenase encoding genes are located adjacent to a CODH. In *Rhodospirillum rubrum,* the encoded CODH/hydrogenase proteins form a membrane-bound enzyme complex that has been indicated to be a site where energy, in the form of a proton gradient, is generated from the conversion of CO and H₂O to CO₂ and H₂ (Fox et al., J Bacteriol. 178:6200-6208 (1996)). The CODH-I of *C. hydrogenoformans* and its adjacent genes have been proposed to catalyze a similar functional role based on their similarity to the *R. rubrum* CODH/hydrogenase gene cluster (Wu et al., PLoS Genet. 1:e65 (2005)). The *C. hydrogenoformans* CODH-I was also shown to exhibit intense CO oxidation and CO₂ reduction activities when linked to an electrode (Parkin et al., J Am.Chem.Soc. 129:10328-10329 (2007)). The protein sequences of exemplary CODH and hydrogenase genes can be identified by the following GenBank accession numbers.

| Protein | GenBank ID | GI Number | Organism |
|---|---|---|---|
| CODH-I (CooS-I) | YP_360644 | 78043418 | *Carboxydothermus hydrogenoformans* |
| CooF | YP_360645 | 78044791 | *Carboxydothermus hydrogenoformans* |
| HypA | YP_360646 | 78044340 | *Carboxydothermus hydrogenoformans* |
| CooH | YP_360647 | 78043871 | *Carboxydothermus hydrogenoformans* |
| CooU | YP_360648 | 78044023 | *Carboxydothermus hydrogenoformans* |
| CooX | YP_360649 | 78043124 | *Carboxydothermus hydrogenoformans* |
| CooL | YP_360650 | 78043938 | *Carboxydothermus hydrogenoformans* |
| CooK | YP_ 360651 | 78044700 | *Carboxydothermus hydrogenoformans* |
| CooM | YP_360652 | 78043942 | *Carboxydothermus hydrogenoformans* |
| CooC | *YP_360654.1* | 78043296 | *Carboxydothermus hydrogenoformans* |
| CooA-1 | YP_360655.1 | 78044021 | *Carboxydothermus hydrogenoformans* |
| CooL | AAC45118 | 1515468 | *Rhodospirillum rubrum* |
| CooX | AAC45119 | 1515469 | *Rhodospirillum rubrum* |
| CooU | AAC45120 | 1515470 | *Rhodospirillum rubrum* |
| CooH | AAC45121 | 1498746 | *Rhodospirillum rubrum* |
| CooF | AAC45122 | 1498747 | *Rhodospirillum rubrum* |
| CODH (CooS) | AAC45123 | 1498748 | *Rhodospirillum rubrum* |
| CooC | AAC45124 | 1498749 | *Rhodospirillum rubrum* |
| CooT | AAC45125 | 1498750 | *Rhodospirillum rubrum* |
| CooJ | AAC45126 | 1498751 | *Rhodospirillum rubrum* |

Native to *E. coli* and other enteric bacteria are multiple genes encoding up to four hydrogenases (Sawers, G., Antonie Van Leeuwenhoek 66:57-88 (1994); Sawers et al., J Bacteriol. 164:1324-1331 (1985); Sawers and Boxer, Eur.J Biochem. 156:265-275 (1986); Sawers et al., J Bacteriol. 168:398-404 (1986)). Given the multiplicity of enzyme activities, *E. coli* or another host organism can provide sufficient hydrogenase activity to split incoming molecular hydrogen and reduce the corresponding acceptor. *E. coli* possesses two uptake hydrogenases, Hyd-1 and Hyd-2, encoded by the *hyaABCDEF* and *hybOABCDEFG* gene clusters, respectively (Lukey et al., How E. coli is equipped to oxidize hydrogen under different redox conditions, J Biol Chem published online Nov 16, 2009). Hyd-1 is oxygen-tolerant, irreversible, and is coupled to quinone reduction via the *hyaC* cytochrome. Hyd-2 is sensitive to O₂, reversible, and transfers electrons to the periplasmic ferredoxin *hybA* which, in turn, reduces a quinone via the *hybB* integral membrane protein. Reduced quinones can serve as the source of electrons for fumarate reductase in the reductive branch of the TCA cycle. Reduced ferredoxins can be used by enzymes such as NAD(P)H:ferredoxin oxidoreductases to generate NADPH or NADH. They can alternatively be used as the electron donor for reactions such as pyruvate ferredoxin oxidoreductase, AKG ferredoxin oxidoreductase, and 5,10-methylene-H4folate reductase.

| Protein | GenBank ID | GI Number | Organism |
|---|---|---|---|
| HyaA | AAC74057.1 | 1787206 | *Escherichia coli* |
| HyaB | AAC74058.1 | 1787207 | *Escherichia coli* |
| HyaC | AAC74059.1 | 1787208 | *Escherichia coli* |
| HyaD | AAC74060.1 | 1787209 | *Escherichia coli* |
| HyaE | AAC74061.1 | 1787210 | *Escherichia coli* |
| HyaF | AAC74062.1 | 1787211 | *Escherichia coli* |

| Protein | GenBank ID | GI Number | Organism |
|---|---|---|---|
| HybO | AAC76033.1 | 1789371 | *Escherichia coli* |
| HybA | AAC76032.1 | 1789370 | *Escherichia coli* |
| HybB | AAC76031.1 | 2367183 | *Escherichia coli* |
| HybC | AAC76030.1 | 1789368 | *Escherichia coli* |
| HybD | AAC76029.1 | 1789367 | *Escherichia coli* |
| HybE | AAC76028.1 | 1789366 | *Escherichia coli* |
| HybF | AAC76027.1 | 1789365 | *Escherichia coli* |
| HybG | AAC76026.1 | 1789364 | *Escherichia coli* |

The hydrogen-lyase systems of *E. coli* include hydrogenase 3, a membrane-bound enzyme complex using ferredoxin as an acceptor, and hydrogenase 4 that also uses a ferredoxin acceptor. Hydrogenase 3 and 4 are encoded by the *hyc* and *hyf* gene clusters, respectively. Hydrogenase 3 has been shown to be a reversible enzyme (Maeda et al., Appl Microbiol Biotechnol 76(5):1035-42 (2007)). Hydrogenase activity in *E. coli* is also dependent upon the expression of the *hyp* genes whose corresponding proteins are involved in the assembly of the hydrogenase complexes (Jacobi et al., Arch.Microbiol 158:444-451 (1992); Rangarajan ct al., J. Bacteriol. 190:1447-1458 (2008)).

| Protein | GenBank ID | GI Number | Organism |
|---|---|---|---|
| HycA | NP_417205 | 16130632 | *Escherichia coli* |
| HycB | NP_417204 | 16130631 | *Escherichia coli* |
| HycC | NP_417203 | 16130630 | *Escherichia coli* |
| HycD | NP_417202 | 16130629 | *Escherichia coli* |
| HycE | NP_417201 | 16130628 | *Escherichia coli* |
| HycF | NP_417200 | 16130627 | *Escherichia coli* |
| HycG | NP_417199 | 16130626 | *Escherichia coli* |
| HycH | NP_417198 | 16130625 | *Escherichia coli* |
| HycI | NP_417197 | 16130624 | *Escherichia coli* |

| Protein | GenBank ID | GI Number | Organism |
|---|---|---|---|
| HyfA | NP_416976 | 90111444 | *Escherichia coli* |
| HyfB | NP_416977 | 16130407 | *Escherichia coli* |
| HyfC | NP_416978 | 90111445 | *Escherichia coli* |
| HyfD | NP_416979 | 16130409 | *Escherichia coli* |
| HyfE | NP_416980 | 16130410 | *Escherichia coli* |
| HyfF | NP_416981 | 16130411 | *Escherichia coli* |
| HyfG | NP_416982 | 16130412 | *Escherichia coli* |
| HyfH | NP_416983 | 16130413 | *Escherichia coli* |
| HyfI | NP_416984 | 16130414 | *Escherichia coli* |
| HyfJ | NP_416985 | 90111446 | *Escherichia coli* |
| HyfR | NP_416986 | 90111447 | *Escherichia coli* |

| Protein | GenBank ID | GI Number | Organism |
|---|---|---|---|
| HypA | NP_417206 | 16130633 | *Escherichia coli* |
| HypB | NP_417207 | 16130634 | *Escherichia coli* |
| HypC | NP_417208 | 16130635 | *Escherichia coli* |
| HypD | NP_417209 | 16130636 | *Escherichia coli* |
| HypE | NP_417210 | 226524740 | *Escherichia coli* |
| HypF | NP_417192 | 16130619 | *Escherichia coli* |

The *M. thermoacetica* hydrogenases are suitable for a host that lacks sufficient endogenous hydrogenase activity. *M. thermoacetica* can grow with CO₂ as the exclusive carbon source indicating that reducing equivalents are extracted from H₂ to enable acetyl-CoA synthesis via the Wood-Ljungdahl pathway (Drake, H. L., J. Bacteriol. 150:702-709 (1982); Drake and Daniel, Res. Microbiol. 155:869-883 (2004); Kellum and Drake, J. Bacteriol. 160:466-469 (1984)) (see Figure 7). *M. thermoacetica* has homologs to several *hyp, hyc,* and *hyf* genes from *E. coli.* The protein sequences encoded for by these genes are identified by the following GenBank accession numbers.

Proteins in *M. thermoacetica* whose genes are homologous to the *E. coli hyp* genes are shown below.

| Protein | GenBank ID | GI Number | Organism |
|---|---|---|---|
| Moth_2175 | YP_431007 | 83590998 | *Moorella thermoacetica* |
| Moth_2176 | YP_431008 | 83590999 | *Moorella thermoacetica* |
| Moth_2177 | YP_431009 | 83591000 | *Moorella thermoacetica* |
| Moth 2178 | YP_431010 | 83591001 | *Moorella thermoacetica* |
| Moth_2179 | YP_431011 | 83591002 | *Moorella thermoacetica* |
| Moth_2180 | YP_431012 | 83591003 | *Moorella thermoacetica* |
| Moth_2181 | YP_431013 | 83591004 | *Moorella thermoacetica* |

Proteins in *M.* thermoacetica that are homologous to the *E. coli* Hydrogenase 3 and/or 4 proteins are listed in the following table.

| Protein | GenBank ID | GI Number | Organism |
|---|---|---|---|
| Moth_2182 | YP_431014 | 83591005 | *Moorella thermoacetica* |
| Moth_2183 | YP_431015 | 83591006 | *Moorella thermoacetica* |
| Moth_2184 | YP_431016 | 83591007 | *Moorella thermoacetica* |
| Moth_2185 | YP_431017 | 83591008 | *Moorella thermoacetica* |
| Moth_2186 | YP_431018 | 83591009 | *Moorella thermoacetica* |
| Moth_2187 | YP_431019 | 83591010 | *Moorella thermoacetica* |
| Moth_2188 | YP_431020 | 83591011 | *Moorella thermoacetica* |
| Moth_2189 | YP_431021 | 83591012 | *Moorella thermoacetica* |
| Moth_2190 | YP_431022 | 83591013 | *Moorella thermoacetica* |
| Moth_2191 | YP_431023 | 83591014 | *Moorella thermoacetica* |
| Moth_2192 | YP_431024 | 83591015 | *Moorella thermoacetica* |

In addition, several gene clusters encoding hydrogenase functionality are present in *M.* thermoacetica and their corresponding protein sequences are provided below.

| Protein | GenBank ID | GI Number | Organism |
|---|---|---|---|
| Moth_0439 | YP_429313 | 83589304 | *Moorella thermoacetica* |
| Moth_0440 | YP_429314 | 83589305 | *Moorella thermoacetica* |
| Moth_0441 | YP_429315 | 83589306 | *Moorella thermoacetica* |
| Moth_0442 | YP_429316 | 83589307 | *Moorella thermoacetica* |
| Moth_0809 | YP_429670 | 83589661 | *Moorella thermoacetica* |
| Moth_0810 | YP_429671 | 83589662 | *Moorella thermoacetica* |
| Moth_0811 | YP_429672 | 83589663 | *Moorella thermoacetica* |
| Moth_0812 | YP_429673 | 83589664 | *Moorella thermoacetica* |
| Moth_0814 | YP_429674 | 83589665 | *Moorella thermoacetica* |
| Moth_0815 | YP_429675 | 83589666 | *Moorella thermoacetica* |
| Moth_0816 | YP_429676 | 83589667 | *Moorella thermoacetica* |
| Moth_1193 | YP_430050 | 83590041 | *Moorella thermoacetica* |
| Moth_1194 | YP_430051 | 83590042 | *Moorella thermoacetica* |
| Moth_1195 | YP_430052 | 83590043 | *Moorella thermoacetica* |
| Moth_1196 | YP_430053 | 83590044 | *Moorella thermoacetica* |
| Moth_1717 | YP_430562 | 83590553 | *Moorella thermoacetica* |
| Moth_1718 | YP_430563 | 83590554 | *Moorella thermoacetica* |
| Moth_1719 | YP_430564 | 83590555 | *Moorella thermoacetica* |
| Moth_1883 | YP_430726 | 83590717 | *Moorella thermoacetica* |
| Moth_1884 | YP_430727 | 83590718 | *Moorella thermoacetica* |
| Moth_1885 | YP_430728 | 83590719 | *Moorella thermoacetica* |
| Moth_1886 | YP_430729 | 83590720 | *Moorella thermoacetica* |
| Moth_1887 | YP_430730 | 83590721 | *Moorella thermoacetica* |
| Moth_1888 | YP_430731 | 83590722 | *Moorella thermoacetica* |
| Moth_1452 | YP_430305 | 83590296 | *Moorella thermoacetica* |
| Moth_1453 | YP_430306 | 83590297 | *Moorella thermoacetica* |
| Moth_1454 | YP_ 430307 | 83590298 | *Moorella thermoacetica* |

*Ralstonia eutropha* H16 uses hydrogen as an energy source with oxygen as a terminal electron acceptor. Its membrane-bound uptake [NiFe]-hydrogenase is an "O2-tolerant" hydrogenase (Cracknell, et al. Proc Nat Acad Sci, 106(49) 20681-20686 (2009)) that is periplasmically-oriented and connected to the respiratory chain via a b-type cytochrome (Schink and Schlegel, Biochim. Biophys. Acta, 567, 315-324 (1979); Bernhard et al., Eur. J. Biochem. 248, 179-186 (1997)). *R. eutropha* also contains an O₂-tolerant soluble hydrogenase encoded by the *Hox* operon which is cytoplasmic and directly reduces NAD+ at the expense of hydrogen (Schneider and Schlegel, Biochim. Biophys. Acta 452, 66-80 (1976); Burgdorf, J. Bact. 187(9) 3122-3132(2005)). Soluble hydrogenase enzymes are additionally present in several other organisms including *Geobacter sulfurreducens* (Coppi, Microbiology 151, 1239-1254 (2005)), *Synechocystis* str. PCC 6803 (Germer, J. Biol. Chem., 284(52), 36462-36472 (2009)), and *Thiocapsa roseopersicina* (Rakhely, Appl. Environ. Microbiol. 70(2) 722-728 (2004)). The *Synechocystis* enzyme is capable of generating NADPH from hydrogen. Overexpression of both the *Hox* operon from *Synechocystis* str. PCC 6803 and the accessory genes encoded by the *Hyp* operon from *Nostoc* sp. PCC 7120 led to increased hydrogenase activity compared to expression of the *Hox* genes alone (Germer, J. Biol. Chem. 284(52), 36462-36472 (2009)).

| Protein | GenBank ID | GI Number | Organism |
|---|---|---|---|
| HoxF | NP_942727.1 | 38637753 | *Ralstonia eutropha H16* |
| HoxU | NP_942728.1 | 38637754 | *Ralstonia eutropha H16* |
| HoxY | NP_942729.1 | 38637755 | *Ralstonia eutropha H16* |
| HoxH | NP_942730.1 | 38637756 | *Ralstonia eutropha H16* |
| HoxW | NP_942731.1 | 38637757 | *Ralstonia eutropha H16* |
| HoxI | NP_942732.1 | 38637758 | *Ralstonia eutropha H16* |
| HoxE | NP_953767.1 | *39997816* | *Geobacter sulfurreducens* |
| HoxF | NP_953766.1 | *39997815* | *Geobacter sulfurreducens* |
| HoxU | NP_953765.1 | *39997814* | *Geobacter sulfurreducens* |
| HoxY | NP_953764.1 | *39997813* | *Geobacter sulfurreducens* |
| HoxH | NP_953763.1 | *39997812* | *Geobacter sulfurreducens* |
| GSU2717 | NP_953762.1 | *39997811* | *Geobacter sulfurreducens* |
| HoxE | NP_441418.1 | 16330690 | *Synechocystis* str. PCC 6803 |
| HoxF | NP_441417.1 | 16330689 | *Synechocystis* str. PCC 6803 |
| Unknown function | NP_441416.1 | 16330688 | *Synechocystis* str. PCC 6803 |
| HoxU | NP_441415.1 | 16330687 | *Synechocystis* str. PCC 6803 |
| HoxY | NP_441414.1 | 16330686 | *Synechocystis* str. PCC 6803 |
| Unknown function | NP_ 441413.1 | 16330685 | *Synechocystis* str. PCC 6803 |
| Unknown function | NP_441412.1 | 16330684 | *Synechocystis* str. PCC 6803 |
| HoxH | NP_ 441411.1 | 16330683 | *Synechocystis* str. PCC 6803 |
| HypF | NP_484737.1 | 17228189 | *Nostoc* sp. PCC 7120 |
| HypC | NP_484738.1 | 17228190 | *Nostoc* sp. PCC 7120 |
| HypD | NP_484739.1 | 17228191 | *Nostoc* sp. PCC 7120 |
| Unknown function | NP_484740.1 | 17228192 | *Nostoc* sp. PCC 7120 |
| HypE | NP_484741.1 | 17228193 | *Nostoc* sp. PCC 7120 |
| HypA | NP_484742.1 | 17228194 | *Nostoc* sp. PCC 7120 |
| HypB | NP_484743.1 | 17228195 | *Nostoc* sp. PCC 7120 |
| Hox1E | AAP50519.1 | 37787351 | *Thiocapsa roseopersicina* |
| Hox1F | AAP50520.1 | 37787352 | *Thiocapsa roseopersicina* |
| Hox1U | AAP50521.1 | 37787353 | *Thiocapsa roseopersicina* |
| Hox1Y | AAP50522.1 | 37787354 | *Thiocapsa roseopersicina* |
| Hox1H | AAP50523.1 | 37787355 | *Thiocapsa roseopersicina* |

Several enzymes and the corresponding genes used for fixing carbon dioxide to either pyruvate or phosphoenolpyruvate to form the TCA cycle intermediates, oxaloacetate or malate are described below.

Carboxylation of phosphoenolpyruvate to oxaloacetate is catalyzed by phosphoenolpyruvate carboxylase. Exemplary PEP carboxylase enzymes are encoded by *ppc in E. coli* (Kai et al., Arch. Biochem. Biophys. 414:170-179 (2003), *ppcA* in *Methylobacterium extorquens AM1* (Arps et al., J. Bacteriol. 175:3776-3783 (1993), and *ppc* in *Corynebacterium glutamicum* (Eikmanns et al., Mol. Gen. Genet. 218:330-339 (1989).

| Protein | GenBank ID | GI Number | Organism |
|---|---|---|---|
| *Ppc* | NP_418391 | 16131794 | *Escherichia coli* |
| *ppcA* | AAB58883 | 28572162 | *Methylobacterium extorquens* |
| *Ppc* | ABB53270 | 80973080 | *Corynebacterium glutamicum* |

An alternative enzyme for converting phosphoenolpyruvate to oxaloacetate is PEP carboxykinase, which simultaneously forms an ATP while carboxylating PEP. In most organisms PEP carboxykinase serves a gluconeogenic function and converts oxaloacetate to PEP at the expense of one ATP. *S. cerevisiae* is one such organism whose native PEP carboxykinase, *PCK1,* serves a gluconeogenic role (Valdes-Hevia et al., FEBS Lett. 258:313-316 (1989). *E. coli* is another such organism, as the role of PEP carboxykinase in producing oxaloacetate is believed to be minor when compared to PEP carboxylase, which does not form ATP, possibly due to the higher Kₘ for bicarbonate of PEP carboxykinase (Kim et al., Appl. Environ. Microbiol. 70:1238-1241 (2004)). Nevertheless, activity of the native *E. coli* PEP carboxykinase from PEP towards oxaloacetate has been recently demonstrated in *ppc* mutants of *E. coli* K-12 (Kwon et al., J. Microbiol. Biotechnol. 16:1448-1452 (2006)). These strains exhibited no growth defects and had increased succinate production at high NaHCO₃ concentrations. Mutant strains of *E. coli* can adopt Pck as the dominant CO2-fixing enzyme following adaptive evolution (Zhang et al. 2009). In some organisms, particularly rumen bacteria, PEP carboxykinase is quite efficient in producing oxaloacetate from PEP and generating ATP. Examples of PEP carboxykinase genes that have been cloned into *E. coli* include those from *Mannheimia succiniciproducens* (Lee et al., Biotechnol. Bioprocess Eng. 7:95-99 (2002)), *Anaerobiospirillum succiniciproducens* (Laivenieks et al., Appl. Environ. Microbiol. 63:2273-2280 (1997), and *Actinobacillus succinogenes* (Kim et al. *supra*). The PEP carboxykinase enzyme encoded by *Haemophilus influenza* is effective at forming oxaloacetate from PEP.

| Protein | GenBank ID | GI Number | Organism |
|---|---|---|---|
| *PCK1* | NP_013023 | 6322950 | *Saccharomyces cerevisiae* |
| *pck* | NP_417862.1 | 16131280 | *Escherichia coli* |
| *pckA* | YP_089485.1 | 52426348 | *Mannheimia succiniciproducens* |
| *pckA* | O09460.1 | 3122621 | *Anaerobiospirillum succiniciproducens* |
| *pckA* | Q6W6X5 | 75440571 | *Actinobacillus succinogenes* |
| *pckA* | P43923.1 | 1172573 | *Haemophilus influenza* |

Pyruvate carboxylase (EC 6.4.1.1) directly converts pyruvate to oxaloacetate at the cost of one ATP. Pyruvate carboxylase enzymes are encoded by *PYC1* (Walker et al., Biochem. Biophys. Res. Commun. 176:1210-1217 (1991) and *PYC2* (Walker et al., *supra*) in *Saccharomyces cerevisiae*, and pyc in *Mycobacterium smegmatis* (Mukhopadhyay and Purwantini, Biochim. Biophys. Acta 1475:191-206 (2000)).

| Protein | GenBank ID | GI Number | Organism |
|---|---|---|---|
| *PYC1* | NP_011453 | 6321376 | *Saccharomyces cerevisiae* |
| *PYC2* | NP_009777 | 6319695 | *Saccharomyces cerevisiae* |
| *Pyc* | YP_890857.1 | 118470447 | *Mycobacterium smegmatis* |

Malic enzyme can be applied to convert CO₂ and pyruvate to malate at the expense of one reducing equivalent. Malic enzymes for this purpose can include, without limitation, malic enzyme (NAD-dependent) and malic enzyme (NADP-dependent). For example, one of the *E. coli* malic enzymes (Takeo, J. Biochem. 66:379-387 (1969)) or a similar enzyme with higher activity can be expressed to enable the conversion of pyruvate and CO₂ to malate. By fixing carbon to pyruvate as opposed to PEP, malic enzyme allows the high-energy phosphate bond from PEP to be conserved by pyruvate kinase whereby ATP is generated in the formation of pyruvate or by the phosphotransferase system for glucose transport. Although malic enzyme is typically assumed to operate in the direction of pyruvate formation from malate, overexpression of the NAD-dependent enzyme, encoded by maeA, has been demonstrated to increase succinate production in *E. coli* while restoring the lethal pfl- ldhA phenotype under anaerobic conditions by operating in the carbon-fixing direction (Stols and Donnelly, Appl. Environ. Microbiol. 63(7) 2695-2701 (1997)). A similar observation was made upon overexpressing the malic enzyme from *Ascaris suum* in *E. coli* (Stols ct al., Appl. Biochem. Biotechnol. 63-65(1), 153-158 (1997)). The second *E. coli* malic enzyme, encoded by *maeB*, is NADP-dependent and also decarboxylates oxaloacetate and other alpha-keto acids (Iwakura et al., J. Biochem. 85(5):1355-65 (1979)).

| Protein | GenBank ID | GI Number | Organism |
|---|---|---|---|
| *maeA* | NP_415996 | 90111281 | *Escherichia coli* |
| *maeB* | NP_416958 | 16130388 | *Escherichia coli* |
| *NAD-ME* | P27443 | 126732 | *Ascaris suum* |

The enzymes used for converting oxaloacetate (formed from, for example, PEP carboxylase, PEP carboxykinase, or pyruvate carboxylase) or malate (formed from, for example, malic enzyme or malate dehydrogenase) to succinyl-CoA via the reductive branch of the TCA cycle are malate dehydrogenase, fumarate dehydratase (fumarase), fumarate reductase, and succinyl-CoA transferase. The genes for each of the enzymes are described herein above.

Enzymes, genes and methods for engineering pathways from succinyl-CoA to various products into a microorganism are now known in the art. The additional reducing equivalents obtained from CO and/or H₂, as disclosed herein, improve the yields of 2,4-pentadienoate, butadiene, 1,3-butanediol or 3-buten-1-ol when utilizing carbohydrate-based feedstock.

Enzymes, genes and methods for engineering pathways from glycolysis intermediates to various products into a microorganism are known in the art. The additional reducing equivalents obtained from CO and H₂, as described herein, improve the yields of all these products on carbohydrates.

### EXAMPLE X

### Methods for Handling CO and Anaerobic Cultures

This example describes methods used in handling CO and anaerobic cultures.
**A. Handling of CO in small quantities for assays and small cultures.** CO is an odorless, colorless and tasteless gas that is a poison. Therefore, cultures and assays that utilized CO required special handling. Several assays, including CO oxidation, acetyl-CoA synthesis, CO concentration using myoglobin, and CO tolerance/utilization in small batch cultures, called for small quantities of the CO gas that were dispensed and handled within a fume hood. Biochemical assays called for saturating very small quantities (<2 mL) of the biochemical assay medium or buffer with CO and then performing the assay. All of the CO handling steps were performed in a fume hood with the sash set at the proper height and blower turned on; CO was dispensed from a compressed gas cylinder and the regulator connected to a Schlenk line. The latter ensures that equal concentrations of CO were dispensed to each of several possible cuvettes or vials. The Schlenk line was set up containing an oxygen scrubber on the input side and an oil pressure release bubbler and vent on the other side. Assay cuvettes were both anaerobic and CO-containing. Threfore, the assay cuvettes were tightly sealed with a rubber stopper and reagents were added or removed using gas-tight needles and syringes. Secondly, small (∼50 mL) cultures were grown with saturating CO in tightly stoppered serum bottles. As with the biochemical assays, the CO-saturated microbial cultures were equilibrated in the fume hood using the Schlenk line setup. Both the biochemical assays and microbial cultures were in portable, sealed containers and in small volumes making for safe handling outside of the fume hood. The compressed CO tank was adjacent to the fume hood. Typically, a Schlenk line was used to dispense CO to cuvettes, each vented. Rubber stoppers on the cuvettes were pierced with 19 or 20 gage disposable syringe needles and were vented with the same. An oil bubbler was used with a CO tank and oxygen scrubber. The glass or quartz spectrophotometer cuvettes have a circular hole on top into which a Kontes stopper sleeve, Sz7 774250-0007 was fitted. The CO detector unit was positioned proximal to the fume hood.
**B. Handling of CO in larger quantities fed to large-scale cultures.** Fermentation cultures are fed either CO or a mixture of CO and H₂ to simulate syngas as a feedstock in fermentative production. Therefore, quantities of cells ranging from 1 liter to several liters can include the addition of CO gas to increase the dissolved concentration of CO in the medium. In these circumstances, fairly large and continuously administered quantities of CO gas are added to the cultures. At different points, the cultures are harvested or samples removed. Alternatively, cells are harvested with an integrated continuous flow centrifuge that is part of the fermenter.
   The fermentative processes are carried out under anaerobic conditions. In some cases, it is uneconomical to pump oxygen or air into fermenters to ensure adequate oxygen saturation to provide a respiratory environment. In addition, the reducing power generated during anaerobic fermentation may be needed in product formation rather than respiration. Furthermore, many of the enzymes for various pathways are oxygen-sensitive to varying degrees. Classic acetogens such as *M. thermoacetica* are obligate anaerobes and the enzymes in the Wood-Ljungdahl pathway are highly sensitive to irreversible inactivation by molecular oxygen. While there are oxygen-tolerant acetogens, the repertoire of enzymes in the Wood-Ljungdahl pathway might be incompatible in the presence of oxygen because most are metallo-enzymes, key components are ferredoxins, and regulation can divert metabolism away from the Wood-Ljungdahl pathway to maximize energy acquisition. At the same time, cells in culture act as oxygen scavengers that moderate the need for extreme measures in the presence of large cell growth.
**C. Anaerobic chamber and conditions.** Exemplary anaerobic chambers are available commercially (see, for example, Vacuum Atmospheres Company, Hawthorne CA; MBraun, Newburyport MA). Conditions included an O₂ concentration of 1 ppm or less and 1 atm pure N₂. In one example, 3 oxygen scrubbers/catalyst regenerators were used, and the chamber included an O₂ electrode (such as Teledyne; City of Industry CA). Nearly all items and reagents were cycled four times in the airlock of the chamber prior to opening the inner chamber door. Reagents with a volume >5mL were sparged with pure N2 prior to introduction into the chamber. Gloves are changed twice/yr and the catalyst containers were regenerated periodically when the chamber displays increasingly sluggish response to changes in oxygen levels. The chamber's pressure was controlled through one-way valves activated by solenoids. This feature allowed setting the chamber pressure at a level higher than the surroundings to allow transfer of very small tubes through the purge valve.
   The anaerobic chambers achieved levels of O2 that were consistently very low and were needed for highly oxygen sensitive anaerobic conditions. However, growth and handling of cells does not usually require such precautions. In an alternative anaerobic chamber configuration, platinum or palladium can be used as a catalyst that requires some hydrogen gas in the mix. Instead of using solenoid valves, pressure release can be controlled by a bubbler. Instead of using instrument-based O2 monitoring, test strips can be used instead.
D. Anaerobic microbiology. Small cultures were handled as described above for CO handling. In particular, serum or media bottles are fitted with thick rubber stoppers and aluminum crimps are employed to seal the bottle. Medium, such as Terrific Broth, is made in a conventional manner and dispensed to an appropriately sized serum bottle. The bottles are sparged with nitrogen for ∼30 min of moderate bubbling. This removes most of the oxygen from the medium and, after this step, each bottle is capped with a rubber stopper (such as Bellco 20 mm septum stoppers; Bellco, Vineland, NJ) and crimp-sealed (Bellco 20 mm). Then the bottles of medium are autoclaved using a slow (liquid) exhaust cycle. At least sometimes a needle can be poked through the stopper to provide exhaust during autoclaving; the needle needs to be removed immediately upon removal from the autoclave. The sterile medium has the remaining medium components, for example buffer or antibiotics, added via syringe and needle. Prior to addition of reducing agents, the bottles are equilibrated for 30 - 60 minutes with nitrogen (or CO depending upon use). A reducing agent such as a 100 x 150 mM sodium sulfide, 200 mM cysteine-HCl is added. This is made by weighing the sodium sulfide into a dry beaker and the cysteine into a serum bottle, bringing both into the anaerobic chamber, dissolving the sodium sulfide into anaerobic water, then adding this to the cysteine in the serum bottle. The bottle is stoppered immediately as the sodium sulfide solution generates hydrogen sulfide gas upon contact with the cysteine. When injecting into the culture, a syringe filter is used to sterilize the solution. Other components are added through syringe needles, such as B12 (10 µM cyanocobalamin), nickel chloride (NiCl₂, 20 microM final concentration from a 40 mM stock made in anaerobic water in the chamber and sterilized by autoclaving or by using a syringe filter upon injection into the culture), and ferrous ammonium sulfate (final concentration needed is 100 µM-made as 100-1000x stock solution in anaerobic water in the chamber and sterilized by autoclaving or by using a syringe filter upon injection into the culture). To facilitate faster growth under anaerobic conditions, the 1 liter bottles were inoculated with 50 mL of a preculture grown anaerobically. Induction of the pA1-lacO1 promoter in the vectors was performed by addition of isopropyl β-D-1-thiogalactopyranoside (IPTG) to a final concentration of 0.2 mM and was carried out for about 3 hrs.

Large cultures can be grown in larger bottles using continuous gas addition while bubbling. A rubber stopper with a metal bubbler is placed in the bottle after medium addition and sparged with nitrogen for 30 minutes or more prior to setting up the rest of the bottle. Each bottle is put together such that a sterile filter will sterilize the gas bubbled in and the hoses on the bottles are compressible with small C clamps. Medium and cells are stirred with magnetic stir bars. Once all medium components and cells are added, the bottles are incubated in an incubator in room air but with continuous nitrogen sparging into the bottles.

### EXAMPLE XI

### CO oxidation (CODH) Assay

### This example describes assay methods for measuring CO oxidation (CO dehydrogenase; CODH).

The 7 gene CODH/ACS operon of Moorella thermoacetica was cloned into E. coli expression vectors. The intact ∼10 kbp DNA fragment was cloned, and it is likely that some of the genes in this region arc expressed from their own endogenous promoters and all contain endogenous ribosomal binding sites. These clones were assayed for CO oxidation, using an assay that quantitatively measures CODH activity. Antisera to the M. thermoacetica gene products was used for Western blots to estimate specific activity. M. thermoacetica is Gram positive, and ribosome binding site elements are expected to work well in E. coli. This activity, described below in more detail, was estimated to be ∼1/50th of the M. thermoacetica specific activity. It is possible that CODH activity of recombinant E. coli cells could be limited by the fact that M. thermoacetica enzymes have temperature optima around 55oC. Therefore, a mesophilic CODH/ACS pathway could be advantageous such as the close relative of Moorella that is mesophilic and does have an apparently intact CODH/ACS operon and a Wood-Ljungdahl pathway, Desulfitobacterium hafniense. Acetogens as potential host organisms include, but are not limited to, Rhodospirillum rubrum, Moorella thermoacetica and Desulfitobacterium hafniense.

CO oxidation is both the most sensitive and most robust of the CODH/ACS assays. It is likely that an E. coli-based syngas using system will ultimately need to be about as anaerobic as Clostridial (i.e., Moorella) systems, especially for maximal activity. Improvement in CODH should be possible but will ultimately be limited by the solubility of CO gas in water.

Initially, each of the genes was cloned individually into expression vectors. Combined expression units for multiple subunits/1 complex were generated. Expression in E. coli at the protein level was determined. Both combined M. thermoacetica CODH/ACS operons and individual expression clones were made.

CO oxidation assay. This assay is one of the simpler, reliable, and more versatile assays of enzymatic activities within the Wood-Ljungdahl pathway and tests CODH (Seravalli et al., Biochemistry 43:3944-3955 (2004)). A typical activity of M. thermoacetica CODH specific activity is 500 U at 55oC or ∼60U at 25oC. This assay employs reduction of methyl viologen in the presence of CO. This is measured at 578 nm in stoppered, anaerobic, glass cuvettes.

In more detail, glass rubber stoppered cuvettes were prepared after first washing the cuvette four times in deionized water and one time with acetone. A small amount of vacuum grease was smeared on the top of the rubber gasket. The cuvette was gassed with CO, dried 10 min with a 22 Ga. needle plus an exhaust needle. A volume of 0.98 m1L of reaction buffer (50 mM Hepes, pH 8.5, 2mM dithiothreitol (DTT) was added using a 22 Ga. needle, with exhaust needled, and 100%CO. Methyl viologen (CH₃ viologen) stock was 1 M in water. Each assay used 20 microliters for 20 mM final concentration. When methyl viologen was added, an 18 Ga needle (partial) was used as a jacket to facilitate use of a Hamilton syringe to withdraw the CH₃ viologen. 4 -5 aliquots were drawn up and discarded to wash and gas equilibrate the syringe. A small amount of sodium dithionite (0.1 M stock) was added when making up the CH₃ viologen stock to slightly reduce the CH₃ viologen. The temperature was equilibrated to 55°C in a heated Olis spectrophotometer (Bogart GA). A blank reaction (CH₃ viologen + buffer) was run first to measure the base rate of CH₃ viologen reduction. Crude *E. coli* cell extracts of ACS90 and ACS91 (CODH-ACS operon of *M. thermoacetica* with and without, respectively, the first *cooC*)*.* 10 microliters of extract were added at a time, mixed and assayed. Reduced CH₃ viologen turns purple. The results of an assay are shown in Table I.

**Table I. Crude extract CO Oxidation Activities.**

| | | | | |
|---|---|---|---|---|
| ACS90 | 7.7 mg/ml | ACS91 | 11.8 mg/ml | |
| Mta98 | 9.8 mg/ml | Mta99 | 11.2 mg/ml | |
| | | | | |

| **Extract** | **Vol** | **OD/** | **U/ml** | **U/mg** |
|---|---|---|---|---|
| ACS90 | 10 microliters | 0.073 | 0.376 | 0.049 |
| ACS91 | 10 microliters | 0.096 | 0.494 | 0.042 |
| Mta99 | 10 microliters | 0.0031 | 0.016 | 0.0014 |
| ACS90 | 10 microliters | 0.099 | 0.51 | 0.066 |
| Mta99 | 25 microliters | 0.012 | 0.025 | 0.0022 |
| ACS91 | 25 microliters | 0.215 | 0.443 | 0.037 |
| Mta98 | 25 microliters | 0.019 | 0.039 | 0.004 |
| ACS91 | 10 microliters | 0.129 | 0.66 | 0.056 |
| | | | | |

| Averages | | | | |
|---|---|---|---|---|
| ACS90 | 0.057 U/mg | | | |
| ACS91 | 0.045 U/mg | | | |
| Mta99 | 0.0018 U/mg | | | |

Mta98/Mta99 are *E. coli* MG1655 strains that express methanol methyltransferase genes from *M. thermoacetia* and, therefore, are negative controls for the ACS90 ACS91 E. coli strains that contain M. thermoacetica CODH operons.

If ∼ 1% of the cellular protein is CODH, then these figures would be approximately 100X less than the 500 U/mg activity of pure M. thermoacetica CODH. Actual estimates based on Western blots are 0.5% of the cellular protein, so the activity is about 50X less than for M. thermoacetica CODH. Nevertheless, this experiment demonstrates CO oxidation activity in recombinant E. coli with a much smaller amount in the negative controls. The small amount of CO oxidation (CH3 viologen reduction) seen in the negative controls indicates that E. coli may have a limited ability to reduce CH3 viologen.

To estimate the final concentrations of CODH and Mtr proteins, SDS-PAGE followed by Western blot analyses were performed on the same cell extracts used in the CO oxidation, ACS, methyltransferase, and corrinoid Fe-S assays. The antisera used were polyclonal to purified M. thermoacetica CODH-ACS and Mtr proteins and were visualized using an alkaline phosphatase-linked goat-anti-rabbit secondary antibody. The Westerns were performed and results are shown in Figure 9. The amounts of CODH in ACS90 and ACS91 were estimated at 50 ng by comparison to the control lanes. Expression of CODH-ACS operon genes including 2 CODH subunits and the methyltransferase were confirmed via Western blot analysis. Therefore, the recombinant E. coli cells express multiple components of a 7 gene operon. In addition, both the methyltransferase and corrinoid iron sulfur protein were active in the same recombinant E. coli cells. These proteins are part of the same operon cloned into the same cells.

The CO oxidation assays were repeated using extracts of Moorella thermoacetica cells for the positive controls. Though CODH activity in E. coli ACS90 and ACS91 was measurable, it was at about 130 - 150 X lower than the M. thermoacetica control. The results of the assay are shown in Figure 10. Briefly, cells (M. thermoacetica or E. coli with the CODH/ACS operon; ACS90 or ACS91 or empty vector: pZA33S) were grown and extracts prepared as described above. Assays were performed as described above at 55°C at various times on the day the extracts were prepared. Reduction of methylviologen was followed at 578 nm over a 120 sec time course.

These results describe the CO oxidation (CODH) assay and results. Recombinant *E. coli* cells expressed CO oxidation activity as measured by the methyl viologen reduction assay.

### EXAMPLE XII

### E. coli CO Tolerance Experiment and CO Concentration Assay (myoglobin assay)

This example describes the tolerance of E. coli for high concentrations of CO.

To test whether or not E. coli can grow anaerobically in the presence of saturating amounts of CO, cultures were set up in 120 ml serum bottles with 50 ml of Terrific Broth medium (plus reducing solution, NiCl2, Fc(II)NH4SO4, cyanocobalamin, IPTG, and chloramphenicol) as described above for anaerobic microbiology in small volumes. One half of these bottles were equilibrated with nitrogen gas for 30 min. and one half was equilibrated with CO gas for 30 min. An empty vector (pZA33) was used as a control, and cultures containing the pZA33 empty vector as well as both ACS90 and ACS91 were tested with both N₂ and CO. All were inoculated and grown for 36 hrs with shaking (250 rpm) at 37°C. At the end of the 36 hour period, examination of the flasks showed high amounts of growth in all. The bulk of the observed growth occurred overnight with a long lag.

Given that all cultures appeared to grow well in the presence of CO, the final CO concentrations were confirmed. This was performed using an assay of the spectral shift of myoglobin upon exposure to CO. Myoglobin reduced with sodium dithionite has an absorbance peak at 435 nm; this peak is shifted to 423 nm with CO. Due to the low wavelength and need to record a whole spectrum from 300 nm on upwards, quartz cuvettes must be used. CO concentration is measured against a standard curve and depends upon the Henry's Law constant for CO of maximum water solubility = 970 micromolar at 20oC and 1 atm.

For the myoglobin test of CO concentration, cuvettes were washed 10X with water, 1X with acetone, and then stoppered as with the CODH assay. N2 was blown into the cuvettes for ∼10 min. A volume of 1 ml of anaerobic buffer (HEPES, pH 8.0, 2mM DTT) was added to the blank (not equilibrated with CO) with a Hamilton syringe. A volume of 10 microliter myoglobin (∼1 mM-can be varied, just need a fairly large amount) and 1 microliter dithionite (20 mM stock) were added. A CO standard curve was made using CO saturated buffer added at 1 microliter increments. Peak height and shift was recorded for each increment. The cultures tested were pZA33/CO, ACS90/CO, and ACS91/CO. Each of these was added in 1 microliter increments to the same cuvette. Midway through the experiment a second cuvette was set up and used. The results arc shown in Table II.

**Table II. Carbon Monoxide Concentrations, 36 hrs.**

| **Strain and Growth Conditions** | **Final CO concentration (micromolar)** |
|---|---|
| **pZA33-CO** | 930 |
| | |
| **ACS90-CO** | 638 |
| | 494 |
| | 734 |
| | 883 |
| ave | **687** |
| SD | **164** |
| | |
| **ACS91-CO** | 728 |
| | 812 |
| | 760 |
| | 611 |
| ave. | **728** |
| SD | **85** |

The results shown in Table II indicate that the cultures grew whether or not a strain was cultured in the presence of CO or not. These results indicate that E. coli can tolerate exposure to CO under anaerobic conditions and that E. coli cells expressing the CODH-ACS operon can metabolize some of the CO.

These results demonstrate that E. coli cells, whether expressing CODH/ACS or not, were able to grow in the presence of saturating amounts of CO. Furthermore, these grew equally well as the controls in nitrogen in place of CO. This experiment demonstrated that laboratory strains of *E. coli* are insensitive to CO at the levels achievable in a syngas project performed at normal atmospheric pressure. In addition, preliminary experiments indicated that the recombinant *E. coli* cells expressing CODH/ACS actually consumed some CO, probably by oxidation to carbon dioxide.

### EXAMPLE XIII

### Pathways to 1,3-butanediol, propylene and crotyl alcohol

Pathways to 1,3-butanediol, propylene and crotyl alcohol are shown in Figure 7. These pathways can begin with the initiation of fatty acid biosynthesis, in which malonyl-ACP is condensed with acetyl-CoA or acetyl-ACP to form acetoacetyl-ACP (step A). The second step involves reduction of acetoacetyl-ACP to 3-hydroxybutyryl-ACP. Following dehydration to crotonyl-ACP and another reduction, butyryl-ACP is formed. The chain elongation typically continues with further addition of malonyl-ACP until a long-chain acyl chain is formed, which is then hydrolyzed by a thioesterase into a free C16 fatty acid. Bacterial fatty acid synthesis systems (FAS II) utilize discreet proteins for each step, whereas fungal and mammalian fatty acid synthesis systems (FAS I) utilize complex multifunctional proteins. The pathways utilize one or more enzymes of fatty acid biosynthesis to produce the C3 and C4 products, propylene, 1,3-butanediol and crotyl alcohol.

Several pathways are shown in Figure 7 for converting acetoacetyl-ACP to 1,3-butanediol. In some pathways, acetoacetyl-ACP is first converted to acetoacetyl-CoA (step D). Alternatively, acetoacetyl-CoA can also be synthesized from acetyl-CoA and malonyl-CoA by acetoacetyl-CoA synthase (EC 2.3.1.194). Acetoacetyl-CoA can then be hydrolyzed to acetoacetate by a CoA transferase, hydrolase or synthetase (step E). Acetoacetate is then reduced to 3-oxobutyraldehyde by a carboxylic acid reductase (step F). Alternately, acetoacetyl-CoA is converted directly to 3-oxobutyraldehyde by a CoA-dependent aldehyde dehydrogenase (step I). In yet another embodiement, acetoacetyl-ACP is converted directly to 3-oxobutyraldehyde by an acyl-ACP reductase (step J). 3-Oxobutyraldehyde is further reduced to 1,3-butanediol via a 4-hydroxy-2-butanone or 3-hydroxybutyraldehyde intermediate (steps G and S, or steps R and AA). Another option is the direct conversion of acetoacetyl-CoA to 4-hydroxy-2-butanone by a bifunctional enzyme with aldehyde dehydrogenase/alcohol dehydrogenase activity (step K). Pathways to 1,3-butanediol can also proceed through a 3-hydroxybutyryl-CoA intermediate. This intermediate is formed by the reduction of acetoacetyl-CoA (step P) or the transacylation of 3-hydroxybutyryl-ACP (step X). 3-Hydroxybutyryl-CoA is further converted to 3-hydroxybutyrate (step Y), 3-hydroxybutyraldehyde (step N) or 1,3-butanediol (step O). Alternately, the 3-hydroxybutyrate intermediate is formed from acetoacetate (step Q) or via hydrolysis of 3-hydroxybutyryl-ACP (step L). The 3-hydroxybutyraldehyde intermediate is also the product of 3-hydroxybutyrl-ACP reductase (step M).

Figure 7 also shows pathways from malonyl-ACP to crotyl alcohol. In one embodiment, fatty acid initiation and extension enzymes produce the crotonyl-ACP intermediate (steps A, B, C). Crotonyl-ACP is then transacylated, hydrolyzed or reduced to crotonyl-CoA, crotonate or crotonaldehyde, respectively (steps AE, T, U). Crotonyl-CoA and crotonate arc intcrconvcrtcd by a CoA hydrolase, transferase or synthetase (step AF). Crotonate is reduced to crotonaldehyde by a carboxylic acid reductase (step AG). In the final step of all pathways, crotonaldehyde is reduced to crotyl alcohol by an aldehyde reductase in step AH. Numerous alternate pathways enumerated in the table below arc also encompassed in the invention. Crotonyl-CoA can be reduced to crotonaldehyde or crotyl alcohol (steps V, W). Alternately, the 3-hydroxybutyryl intermediates of the previously described 1,3-butanediol pathways can also be converted to crotyl alcohol precursors. For example, dehydration of 3-hydroxybutyryl-CoA, 3-hydroxybutyrate or 3-hydroxybutyraldehyde yields crotonyl-CoA, crotonate or crotonaldehyde, respectively (step AB, AC, AD).

Pathways to propylene are also shown in Figure 7. In one embodiment, the crotonaldehyde intermediate is decarbonylated to propylene (step AO). In another embodiment, the 3-hydroxybutyrate intermediate is converted to propylene by an alkene-forming decarboxylase (step AR). Decarboxylation of crotonate also forms propylene (step AQ). In yet another embodiment, the enzymes of fatty acid biosynthesis further convert crotonyl-ACP to butyryl-ACP (step AL), which can then be transacylated to butyryl-CoA (step AI) or hydrolyzed to butyrate (step AP). The butyryl-CoA intermediate is also formed from the reduction of crotonyl-CoA (step AM). The butyrate intermediate is also formed from reduction of crotonate or removal of the CoA moiety of butyryl-CoA (step AN or AJ). Propylene is formed from butyrate by an alkene-forming decarboxylase (step AK). Pathways from malonyl-ACP to propylene are listed in the table below.

Exemplary pathways from shown in Figure 7 are listed in the table below:

| Product | Pathways | | |
|---|---|---|---|
| 1,3-BDO | A, D, E, F, G, S | A, D, K, S | AS, E, F, G, S |
| | A, D, E, F, R, AA | A, H, F, G, S | AS, I, G, S |
| | A, D, E, Q, Z, AA | A, H, F, R, AA | AS, K" S |
| | A, D, P, Y, Z, AA | A, H, Q, Z, AA | AS, I, R, AA |
| | A,D,P,O | A, J, G, S | AS, E, F, R, AA |
| | A, D, E, F, G, S | A, J, R, AA | AS, E, Q, Z, AA |
| | A, D, E, F, R, AA | A, B, X, Y, Z, AA | AS, P, N, AA |
| | A, D, P, N, AA | A, B, X, O | AS, P, Y, Z, AA |
| | A, D, I, G, S | A, B, X, N, AA | AS, P, O |
| | A, D, IR, AA | A, B, L, Z, AA | AS, E, F, R, AA |
| | | A, B, M, AA | AS, E, F, G, S |
| Crotyl | A, B, C, AE, AF, AG, AH | A, D, P, AB, AF, AG, AH | A, H, Q, Z, AD, AH |
| alcohol | A, B, C, AE, W | A, D, P, AB, V, AH | A, J, R, AD, AH |
| | A, B, C, AE, V, AH | A, D, P, AB, W | AS, I, R, AD, AH |
| | A, B, C, T, AG, AH | A, D, P, Y, AC, AG, AH | AS, E, F, R, AD, AH |
| | A, B, C, U, AH | A,D, P, Y, Z, AD, AH | AS, E, Q, Z, AD, AH |
| | A, B, X, Y, Z, AD, AH | A, D, P, N, AD, AH | AS, E, Q, AC, AG, AH |
| | A, B, X, Y, AC, AG, AH | A, D, E, F, R, AD, AH | |
| | A, B, X, AB, AF, AG, AH | A, D, E, Q, Z, AD, AH | AS, P, N, AD, AH |
| | A, B, X, AB, V, AH | A, D, E, Q, AC, AG, AH | AS, P, Y, Z, AD, AH |
| | A, B, X, AB, W | A, D, I, R, AD, AH | AS, P, Y, AC, AG, AH |
| | A, B, L, Z, AD, AH | A, H, F, R, AD, AH | AS, P, AB, V, AH |
| | A, B, L, AC, AG, AH | A, H, Q, AC, AG, AH | AS, P, AB, AF, AG, AH |
| | A, B, M, AD, AH | | |
| | | | AS, P, AB, W |
| Propylene | A, B, C, AL, AI, AJ, AK | A, B, L, AC, AG, AO | A, H, Q, AR |
| | A, B, C, AL, AP, AK | A, B, L, AC, AN, AK | A, H, Q, Z, AD, AO |
| | A, B, C, AE, AF, AG, AO | A, B, L, AC, AQ | A, H, Q, AC, AQ |
| | A, B, C, AE, AF, AQ | A, B, M, AD, AO | A, H, Q, AC, AG, AO |
| | A, B, C, AE, AF, AN, AK | A, D, E, F, R, AD, AO | A, H, Q, AC, AN, AK |
| | A, B, C, AE, AM AJ, AK | A, D, E, Q, AR | A, H, Q, V, AG, AO |
| | A, B, C, AE, V, AO | A, D, E, Q, Z, AD, AO | AS, I, R, AD, AO |
| | A, B, C, T, AG, AO | A, D, E, Q, AC, AN, AK | AS, E, F, R, AD, AO |
| | A, B, C, T, AQ | A, D, E, Q, AC, AG, AO | AS, E, Q, AD, AO |
| | A, B, C, T, AN, AK | A, D, E, Q, AC, AQ | AS, P, Y, Z, AD, AO |
| | A, B, C, U, AO | A, D, P, Y, Z, AD, AO | AS, P, N, AD, AO |
| | A, B, X, Y, Z, AD, AO | A, D, P, N, AD, AO | AS, E, Q, AC, AG, AO |
| | A, B, X, Y, AR | A, D, P, Y, AR | |
| | A, B, X, Y, AC, AN, AK | A, D, P, Y, AC, AG, AO | AS, P, Y, AC, AG, AO |
| | A, B, X, Y, AC, AQ | A, D, P, Y, AC, AQ | AS, P, AB, AF, AG, AO |
| | A, B, X, Y, AC, AG, AO | A, D, P, Y, AC, AN, AK | |
| | A, B, X, N, AD, AO | A, D, P, AB, AM, AJ, AK | AS, E, Q, AR |
| | A, B, X, AB, AF, AG, AO | A, D, P, AB, AF, AG, AO | AS, P, Y, AR |
| | A, B, X, AB, AF, AQ | A, D, P, AB, AF, AQ | AS, E, Q, AC, AQ |
| | A, B, X, AB, AF, AN, AK | A, D, P, AB, AF, AN, AK | AS, P, Y, AC, AQ |
| | A, B, X, AB, AM, AJ, AK | A, D, P, AB, V, AO | AS, P, AB, AF, AQ |
| | A, B, X, AB, V, AO | A, D, I, R, AD, AO | AS, E, Q, AC, AN, AK |
| | A, B, L, AR | A, J, R, AD, AO | |
| | A, B, L, Z, AD, AO | A, H, F. R, AD, AO | AS, P, Y, AC, AN, AK |
| | | | AS, P, AB, AF, AN, AK |
| | | | AS, P, AB, AM, AJ, AK |

Enzyme activities required for the reactions shown in Figure 7 are listed in the table below.

| Label | Function | Step |
|---|---|---|
| 1.1.1.a | Oxidoreductase (oxo to alcohol) | 7B, 7G, 7P, 7Q, 7R, 7S, 7AA, 7AH |
| 1.1.1.c | Oxidoreductase (acvl-CoA to alcohol) | 7K, 7O, 7W |
| 1.2.1.b | Oxidoreductase (acyl-CoA to aldehyde) | 71, 7N, 7V |
| 1.2.1.c | Oxidoreductase (acid to aldehyde) | 7F, 7Z, 7AG |
| 1.2.1.f | Oxidoreductase (acyl-ACP to aldehyde) | 7J, 7M, 7U |
| 1.3.1.a | Oxidoreducatse (alkane to alkene) | 7AL, 7AM, 7AN |
| 2.3.1.e | Acyl-ACP C-acyltransferase (decarboxylating) | 7A |
| 2.3.1.f | CoA-ACP acyltransferase | 7D, 7X, 7AE, 7AI |
| 2.3.1.g | Fatty-acid synthase | 7A, 7B, 7C, 7AL |
| 2.8.3.a | CoA transferase | 7E, 7Y, 7AJ, 7AF |
| 3.1.2.a | CoA hydrolase | 7E, 7Y, 7AJ, 7AF |
| 3.1.2.b | Acyl-ACP thioesterase | 7H, 7L, 7T, 7AP |
| 4.1.1.a | Decarboxylase | 7AQ, 7AR |
| 4.1.1.b | Decarboxylase, alkene forming | 7AK |
| 4.1.99.a | Decarbonylase | 7AO |
| 4.2.1.a | Hydro-lyase | 7C, 7AB, 7AC, 7AD |
| 6.2.1.a | CoA synthetase | 7E, 7Y, 7AJ, 7AF |

Enzyme candidates in many of these EC classes have been described earlier and represent suitable candidates for to the transformations depicted in Figure 7. These enzyme classes include EC 1.1.1.a, 1.1.1.c, 1.2.1.b, 1.2.1.e, 2.3.1.b, 2.3.1.h, 2.8.3.a, 3.1.2.a, 4.1.1.a, 4.1.99.a, 4.2.1.a and 6.2.1.a. New enzyme candidates relevant to the Figure 7 pathways are described below.

### 1.1.1.a Oxidoreductase (oxo to alcohol)

Several reactions shown in Figure 7 are catalyzed by alcohol dehydrogenase enzymes. These reactions include Steps B, G, P, Q, R, S, AA and AH. Exemplary alcohol dehydrogenase enzymes for catalyzing steps G, P, Q, R, S, AA and AH were described above in Example VII. Enzyme candidates suitable for catalyzing step B are described below.

The reduction of acetoacetyl-ACP to 3-hydroxyacetyl-ACP is catalyzed by acetoacetyl-ACP reductase or 3-oxoacyl-ACP reductase (EC 1.1.1.100). The *E. coli* 3-oxoacyl-ACP reductase is encoded by *fabG.* Key residues responsible for binding the acyl-ACP substrate to the enzyme have been elucidated (Zhang et al, J Biol Chem 278:52935-43 (2003)). Additional enzymes with this activity have been characterized in *Bacillus anthracis* (Zaccai et al, Prot Struct Funct Gen 70:562-7 (2008)) and *Mycobacterium tuberculosis* (Gurvitz, Mol Genet Genomics 282:407-16 (2009)). The beta-ketoacyl reductase (KR) domain of eukaryotic fatty acid synthase also catalyzes this activity (Smith, FASEB J, 8:1248-59 (1994)).

| Protein | GenBank ID | GI Number | Organism |
|---|---|---|---|
| *fabG* | P0AEK2.1 | 84028081 | *Escherichia coli* |
| *fabG* | AAP27717.1 | 30258498 | *Bacillus anthracis* |
| *FabG1* | NP_215999.1 | 15608621 | *Mycobacterium tuberculosis* |
| *FabG4* | YP_003030167.1 | 253797166 | *Mycobacterium tuberculosis* |

### 1.2.1.f Oxidoreductase (acyl-ACP to aldehyde)

The reduction of an acyl-ACP to its corresponding aldehyde is catalyzed by an acyl-ACP reductase (AAR). Such a transformation is depicted in steps J, M and U of Figure 7. Suitable enzyme candidates include the orf1594 gene product of *Synechococcus elongatus PCC7942* and homologs thereof (Schirmer et al, Science, 329: 559-62 (2010)). The *S. elongates* PCC7942 acyl-ACP reductase is coexpressed with an aldehyde decarbonylase in an operon that appears to be conserved in a majority of cyanobacterial organisms. This enzyme, expressed in *E. coli* together with the aldehyde decarbonylase, conferred the ability to produce alkanes. The *P. marinus* AAR was also cloned into *E. coli* and, together with a decarbonylase, demonstrated to produce alkanes (US Application 2011/0207203).

| Protein | GenBank ID | GI Number | Organism |
|---|---|---|---|
| *orf1594* | YP_400611.1 | 81300403 | *Synechococcus elongatus PCC7942* |
| *PMT9312_0533* | YP_397030.1 | 78778918 | *Prochlorococcus marinus MIT 9312* |
| *syc0051_d* | YP_170761.1 | 56750060 | *Synechococcus elongatus PCC 6301* |
| *Ava_2534* | YP_323044.1 | 75908748 | *Anabaena variabilis ATCC 29413* |
| *alr5284* | NP_489324.1 | 17232776 | *Nostoc sp. PCC 7120* |
| *Aazo_3370* | YP_003722151.1 | 298491974 | *Nostoc azollae* |
| *Cyan7425_0399* | YP_002481152.1 | 220905841 | *Cyanothece sp. PCC 7425* |
| *N9414_21225* | ZP_01628095.1 | 119508943 | *Nodularia spumigena CCY9414* |
| *L8106_07064* | ZP_01619574.1 | 119485189 | *Lyngbya sp. PCC 8106* |

### 1.3.1.a (alkane to alkene)

Several transformations in Figure 7 involve the reduction of an alkene to an alkane. In steps AM and AN, an enoyl-CoA is reduced to its corresponding acyl-CoA. Enzyme candidates for catalyzing these reactions were described previously in Example VII. Step AL depicts the reduction of crotonyl-ACP to butyryl-ACP, catalyzed by a butyryl-ACP reductase. Suitable enzyme candidates for this step are described here.

Enoyl-ACP reductase catalyzes the formation of a saturated acyl-ACP by an NAD(P)H-dependent reduction of the enoyl-ACP double bond. The FabI protein of E. coli is a well-characterized enoyl-ACP reductase that catalyzes the reduction of enoyl substrates of length 4 to 16 carbons (Rafi et al, JBC 281:39285-93 (2006)). FabI is inhibited by acyl-ACP by product inhibition (Heath, J Biol Chem 271:1833-6 (1996)). *Bacillus subtilis* contains two enoyl-ACP reductase isozymes, FabI and FabL (Heath et al, J Biol Chem 275:40128-33 (2000)). The *Streptococcus pneumoniae* FabK protein is a triclosan-resistant flavoprotein catalyzing the same activity (Heath and Rock, Nature 406:145-6 (2000)). An additional candidate is the *Pseudomonas aeruginosa* FabI protein, which was recently crystallized (Lee et al, Acta Cryst Sect F 67:214-216 (2011)).

| Protein | GenBank ID | GI Number | Organism |
|---|---|---|---|
| *fabI* | P0AEK4.2 | 84028072 | *Escherichia coli* |
| *fabI* | P54616.2 | 7531269 | *Bacillus subtilis* |
| *fabL* | P71079.1 | 81817482 | *Bacillus subtilis* |
| *fabK* | AAF98273.1 | 9789231 | *Streptococcus pneumoniae* |
| *fabI* | Q9ZFE4.1 | 7531118 | *Pseudomonas aeruginosa* |

### 2.3.1.e Acyl-ACP C-acyltransferase (decarboxylating)

In step A of Figure 7, acetoacetyl-ACP is formed from malonyl-ACP and either acetyl-CoA or acetyl-ACP. This reaction is catalyzed by an acyl-ACP C-acyltransferase in EC class 2.3.1. The condensation of malonyl-ACP and acetyl-CoA is catalyzed by beta-ketoacyl-ACP synthase (KAS, EC 2.3.1.180). *E. coli* has three KAS enzymes encoded by *fabB*, *fabF* and *fabH.* FabH (KAS III), the key enzyme of initiation of fatty acid biosynthesis in *E. coli,* is selective for the formation of acetoacetyl-ACP. FabB and FabF catalyze the condensation of malonyl-ACP with acyl-ACP substrates and function primarily in fatty acid elongation although they can also react with acetyl-ACP and thereby participate in fatty acid inititation. For example, the *Bacillus subtilis* KAS enzymes are similar to FabH but are less selective, accepting branched acyl-CoA substrates (Choi et al, J Bacteriol 182:365-70 (2000)).

| Protein | GenBank ID | GI Number | Organism |
|---|---|---|---|
| *fabB* | AAC75383.1 | 1788663 | *Escherichia coli* |
| *fabF* | AAC74179.1 | 1787337 | *Escherichia coli* |
| *fabH* | AAC74175.1 | 1787333 | *Escherichia coli* |
| *FabHA* | NP_389015.1 | 16078198 | *Bacillus subtilis* |
| *FabHB* | NP_388898.1 | 16078081 | *Bacillus subtilis* |

Alternately, acetyl-CoA can first be activated to acetyl-ACP and subsequently condensed to acetoacetyl-ACP by two enzymes, acetyl-CoA:ACP transacylase (EC 2.3.1.38) and acetoacetyl-ACP synthase (EC 2.3.1.41). Acetyl-CoA:ACP transacylase converts acetyl-CoA and an acyl carrier protein to acetyl-ACP, releasing CoA. Enzyme candidates for acetyl-CoA:ACP transacylase are described in section EC 2.3.1.f below. Acetoacetyl-ACP synthase enzymes catalyze the condensation of acetyl-ACP and malonyl-ACP. This activity is catalyzed by FabF and FabB *of E. coli*, as well as the multifunctional eukaryotic fatty acid synthase enzyme complexes described in EC 2.3.1.g.

### 2.3.1.f CoA-ACP acyltransferase

The exchange of an ACP moiety for a CoA is catalyzed by enzymes in EC class 2.3.1. This reaction is shown in steps D, X, AE and AI of Figure 7. Activation of acetyl-CoA to acetyl-ACP (step A of Figure 7) is also catalyzed by a CoA:ACP acyltransferase. Enzymes with CoA-ACP acyltransferase activity include acetyl-CoA:ACP transacylase (EC 2.3.1.38) and malonyl-CoA:ACP transacylase (EC 2.3.1.39).

The FabH (KASIII) enzyme of E. coli functions as an acyl-CoA:ACP transacylase, in addition to its primary activity of forming acetoacetyl-ACP. Butyryl-ACP is accepted as an alternate substrate of FabH (Prescott et al, Adv. Enzymol. Relat. Areas Mol, 36:269-311 (1972)). Acetyl-CoA:ACP transacylase enzymes from Plasmodium falciparum and Streptomyces avermitillis have been heterologously expressed in E. coli (Lobo et al, Biochem 40:11955-64 (2001)). A synthetic KASIII (FabH) from P. falciparum expressed in a fabH-deficient Lactococcus lactis host was able to complement the native fadH activity (Du et al, AEM 76:3959-66 (2010)). The acetyl-CoA:ACP transacylase enzyme from Spinacia oleracea accepts other acyl-ACP molecules as substrates, including butyryl-ACP (Shimakata et al, Methods Enzym 122:53-9 (1986)). The sequence of this enzyme has not been determined to date. Malonyl-CoA:ACP transacylase enzymes include FabD *of E. coli* and *Brassica napsus* (Verwoert et al, J Bacteriol, 174:2851-7 (1992); Simon et al, FEBS Lett 435:204-6 (1998)). FabD of *B. napsus* was able to complement *fabD-*deficient *E. coli.* The multifunctional eukaryotic fatty acid synthase enzyme complexes (described in EC 2.3.1.g) also catalyze this activity.

| Protein | GenBank ID | GI Number | Organism |
|---|---|---|---|
| *fabH* | AAC74175.1 | 1787333 | *Escherichia coli* |
| *fadA* | NP 824032.1 | 29829398 | *Streptomyces avermitillis* |
| *fabH* | AAC63960.1 | 3746429 | *Plasmodium falciparum* |
| *Synthetic construct* | ACX34097.1 | 260178848 | *Plasmodium falciparum* |
| *fabH* | CAL98359.1 | 124493385 | *Lactococcus lactis* |
| *fabD* | AAC74176.1 | 1787334 | *Escherichia coli* |
| *fabD* | CAB45522.1 | 5139348 | *Brassica napsus* |

### 2.3.1.g Fatty acid synthase

Steps A, B, C and AL of Figure 7 can together be catalyzed fatty acid synthase or fatty-acyl-CoA synthase, multifunctional enzyme complexes composed of multiple copies of one or more subunits. The fatty acid synthase of *Saccharomyces cerevisiae* is a dodecamer composed of two multifunctional subunits FAS1 and FAS2 that together catalyze all the reactions required for fatty acid synthesis: activation, priming, elongation and termination (Lomakin et al, Cell 129:319-32 (2007)). This enzyme complex catalyzes the formation of long chain fatty acids from acetyl-CoA and malonyl-CoA. The favored product of eukaryotic FAS systems is palmitic acid (C16). Similar fatty acid synthase complexes are found in *Candida parapsilosis* and *Thermomyces lanuginosus* (Nguyen et al, PLoS One 22:e8421 (2009); Jenni et al, Science 316:254-61 (2007)). The multifunctional Fas enzymes of *Mycobacterium tuberculosis* and mammals such as *Homo sapiens* are also suitable candidates (Fernandes and Kolattukudy, Gene 170:95-99 (1996) and Smith et al, Prog Lipid Res 42:289-317 (2003)).

| Protein | GenBank ID | GI Number | Organism |
|---|---|---|---|
| FAS1 | CAA82025.1 | 486321 | *Saccharomyces cerevisiae* |
| FAS2 | CAA97948.1 | 1370478 | *Saccharomyces cerevisiae* |
| *Fas1* | ABO37973.1 | 133751597 | *Thermomyces lanuginosus* |
| *Fas2* | ABO37974.1 | 133751599 | *Thermomyces lanuginosus* |
| *Fas* | AAB03809.1 | 1036835 | *Mycobacterium tuberculosis* |
| *Fas* | NP_004095.4 | 41872631 | *Homo sapiens* |

### 3.1.2.b Acyl-ACP thioesterase

Acyl-ACP thioesterase enzymes convert an acyl-ACP to its corresponding acid. Such a transformation is required in steps H, L, T and AP of Figure 7. Exemplary enzymes include the FatA and FatB isoforms of *Arabidopsis thaliana* (Salas et al, Arch Biochem Biophys 403:25-34 (2002)). The activities of these two proteins vary with carbon chain length, with FatA preferring oleyl-ACP and FatB preferring palmitoyl-ACP. , See 3.1.2.14. A number of thioesterases with different chain length specificities are listed in WO 2008/113041 and are included in the table below [see p 126 Table 2A of patent]. For example, it has been shown previously that expression of medium chain plant thioesterases like FatB from *Umbellularia californica* in *E. coli* results in accumulation of high levels of medium chain fatty acids, primarily laurate (C12:0). Similarly, expression of *Cuphea palustris* FatB1 thioesterase in *E. coli* led to accumulation of C8-10:0 acyl-ACPs (Dehesh et al, Plant Physiol 110:203-10 (1996)). Similarly, *Carthamus tinctorius* thioesterase, when expressed in *E. coli* leads to >50 fold elevation in C 18:1 chain termination and release as free fatty acid (Knutzon et al, Plant Physiol 100:1751-58 (1992)). Methods for altering the substrate specificity of acyl-ACP thioesterases are also known in the art (for example, EP1605048).

| Protein | GenBank ID | GI Number | Organism |
|---|---|---|---|
| *fatA* | AEE76980.1 | 332643459 | *Arabidopsis thaliana* |
| *fatB* | AEE28300.1 | 332190179 | *Arabidopsis thaliana* |
| *fatB2* | AAC49269.1 | 1292906 | *Cuphea hookeriana* |
| *fatB1* | AAC49179.1 | 1215718 | *Cuphea palustris* |
| *M96568.1:94..1251* | AAA33019.1 | 404026 | *Carthamus tinctorius* |
| *fatB1* | Q41635.1 | 8469218 | *Umbellularia californica* |
| *tesA* | AAC73596.1 | 1786702 | *Escherichia coli* |

### 4.1.99.a Decarbonylase

Decarbonylase enzyme candidates described in Example VII are also relevant here. Additional enzyme candidates suitable for catalyzing decarbonylation reactions in Figures 1-7 include the *orf1593* gene product of *Synechococcus elongatus PCC7942* and homologs thereof (US Application 2011/0207203).

| Protein | GenBank ID | GI Number | Organism |
|---|---|---|---|
| *Orf1593* | YP_400610.1 | 81300402 | *Synechococcus elongatus PCC7942* |

### 4.2.1.a Hydro-lyase

Several reactions in Figure 7 depict dehydration reactions, including steps C, AB, AC and AD. Candidate hydro-lyase enzymes described in Example VII are also applicable here. Oleate hydratase enzymes are applicable to catalyze all the dehydration reactions in Figures 1-7, in particular the dehydration of 3-buten-1-ol to butadiene. Oleate hydratase enzymes catalyze the reversible hydration of non-activated alkenes to their corresponding alcohols. These enzymes represent additional suitable candidates as suggested in WO2011076691. Oleate hydratases from *Elizabethkingia meningoseptica* and *Streptococcus pyogenes* have been characterized (WO 2008/119735). Examples include the following proteins.

| Protein | GenBank ID | GI Number | Organism |
|---|---|---|---|
| *OhyA* | ACT54545.1 | 254031735 | *Elizabethkingia meningoseptica* |
| *HMPREF0841_1446* | ZP_07461147.1 | 306827879 | *Streptococcus pyogenes ATCC 10782* |
| *P700755_13397* | ZP_01252267.1 | 91215295 | *Psychroflexus torquis ATCC 700755* |
| *RPB_2430* | YP_486046.1 | 86749550 | *Rhodopseudomonas palustris* |

3-Hydroxyacyl-ACP dehydratase enzymes are suitable candidates for dehydrating 3-hydroxybutyryl-ACP to crotonyl-ACP (step C of Figure 7). Enzymes with this activity include FabA and FabZ of *E. coli,* which posess overlapping broad substrate specificities (Heath, J Biol Chem 271:1833-6 (1996)). Fatty acid synthase complexes, described above, also catalyze this reaction. The FabZ protein from *Plasmodium falciparum* has been crystallized (Kostrew et al, Protein Sci 14:1570-80 (2005)). Additional candidates are the mitochondrial 3-hydroxyacyl-ACP dehydratase encoded by Htd2p in yeast and *TbHTD2* in *Homo sapiens* and *Trypanosoma brucei* (Kastanoitis et al, Mol Micro 53:1407-21 (2004); Kaija et al, FEBS Lett 582:729-33 (2008)).

| Protein | GenBank ID | GI Number | Organism |
|---|---|---|---|
| *fabA* | AAC74040.1 | 1787187 | *Escherichia coli* |
| *fabZ* | AAC73291.1 | 1786377 | *Escherichia coli* |
| *PfFabZ* | AAK83685.1 | 15080870 | *Plasmodium falciparum* |
| *Htd2p* | NP_011934.1 | 6321858 | *Saccharomyces cerevisiae* |
| *HTD2* | P86397.1 | 281312149 | *Homo sapiens* |

### EXAMPLE XIV

### Chemical Production of Butadiene From Crotyl Alcohol

In a typical process for converting crotyl alcohol into butadiene, crotyl alcohol is passed, either neat or in a solvent and either in presence or absence of steam, over a solid inorganic, organic or metal-containing dehydration catalyst heated to temperatures in the range 40-400 °C inside of the reaction vessel or tube, leading to elimination of water and release of butadiene as a gas, which is condensed (butadiene bp = -4.4°C) and collected in a reservoir for further processing, storage, or use. Typical catalysts can include bismuth molybdatc, phosphate-phosphoric acid, cerium oxide, kaolin-iron oxide, kaolin-phosphoric acid, silica-alumina, and alumina. Typical process throughputs are in the range of 0.1-20,000 kg/h. Typical solvents are toluene, heptane, octane, ethylbenzene, and xylene.

### EXAMPLE XV

### Enzymatic pathways for Producing Butadiene from Crotyl Alcohol

This example describes enzymatic pathways for converting crotyl alcohol to butadiene. The two pathways are shown in Figure 12. In one pathway, crotyl alcohol is phosphorylated to 2-butenyl-4-phosphate by a crotyl alcohol kinase (Step A). The 2-butenyl-4-phosphate intermediate is again phosphorylated to 2-butenyl-4-diphosphate (Step B). A butadiene synthase enzyme catalyzes the conversion of 2-butenyl-4-diphosphate to butadiene (Step C). Such a butadiene synthase can be derived from a phosphate lyase enzyme such as isoprene synthase using methods, such as directed evolution, as described herein. In an alternate pathway, crotyl alcohol is directly converted to 2-butenyl-4-diphosphate by a diphosphokinase (step D). Enzyme candidates for steps A-D are provided below.

### Crotyl alcohol kinase (Figure 12, Step A)

Crotyl alcohol kinase enzymes catalyze the transfer of a phosphate group to the hydroxyl group of crotyl alcohol. The enzymes described below naturally possess such activity or can be engineered to exhibit this activity. Kinases that catalyze transfer of a phosphate group to an alcohol group are members of the EC 2.7.1 enzyme class. The table below lists several useful kinase enzymes in the EC 2.7.1 enzyme class.

| Enzyme Commission Number | Enzyme Name | Enzyme Commission Number | Enzyme Name | Enzyme Commission Number | Enzyme Name |
|---|---|---|---|---|---|
| 2.7.1.1 | hexokinase | 2.7.1.48 | uridine kinase | 2.7.1.94 | acylglycerol kinase |
| 2.7.1.2 | glucokinase | 2.7.1.49 | hydroxymethylpyrimidine kinase | 2.7.1.95 | kanamycin kinase |
| 2.7.1.3 | ketohexokinase | 2.7.1.50 | hydroxyethylthiazole kinase | 2.7.1.100 | S-methyl-5-thioribose kinase |
| 2.7.1.4 | fructokinase | 2.7.1.51 | L-fuculokinase | 2.7.1.101 | tagatose kinase |
| 2.7.1.5 | rhamnulokinase | 2.7.1.52 | fucokinase | 2.7.1.102 | hamamelose kinase |
| 2.7.1.6 | galactokinase | 2.7.1.53 | L-xylulokinase | 2.7.1.103 | viomycin kinase |
| 2.7.1.7 | mannokinase | 2.7.1.54 | D-arabinokinase | 2.7.1.105 | 6-phosphofructo-2-kinase |
| 2.7.1.8 | glucosamine kinase | 2.7.1.55 | allose kinase | 2.7.1.106 | glucose-1,6-bisphosphate synthase |
| 2.7.1.10 | phosphoglucokinase | 2.7.1.56 | 1-phosphofructokinase | 2.7.1.107 | diacylglycerol kinase |
| 2.7.1.11 | 6-phosphofructokinase | 2.7.1.58 | 2-dehydro-3-deoxygalactonokinase | 2.7.1.108 | dolichol kinase |
| 2.7.1.12 | gluconokinase | 2.7.1.59 | N-acetylglucosamine kinase | 2.7.1.113 | deoxyguanosine kinase |
| 2.7.1.13 | dehydrogluconokinase | 2.7.1.60 | N-acylmannosamine kinase | 2.7.1.114 | AMP-thymidine kinase |
| 2.7.1.14 | sedoheptulokinase | 2.7.1.61 | acyl-phosphate-hexose phosphotransferase | 2.7.1.118 | ADP-thymidine kinase |
| 2.7.1.15 | ribokinase | 2.7.1.62 | phosphoramidate-hexose phosphotransferase | 2.7.1.119 | hygromycin-B7"-O-kinase |
| 2.7.1.16 | ribulokinase | 2.7.1.63 | polyphosphate-glucose phosphotransferase | 2.7.1.121 | phosphoenolpyruvate-glycerone phosphotransferase |
| 2.7.1.17 | xylulokinase | 2.7.1.64 | inositol 3-kinase | 2.7.1.122 | xylitol kinase |
| 2.7.1.18 | phosphoribokinase | 2.7.1.65 | scyllo-inosamine 4-kinase | 2.7.1.127 | inositol-trisphosphate 3-kinase |
| 2.7.1.19 | phosphoribulokinase | 2.7.1.66 | undecaprenol kinase | 2.7.1.130 | tetraacyldisaccharide 4'-kinase |
| 2.7.1.20 | adenosine kinase | 2.7.1.67 | 1-phosphatidylinositol 4-kinase | 2.7.1.134 | inositol-tetrakisphosphate 1-kinase |
| 2.7.1.21 | thymidine kinase | 2.7.1.68 | 1-phosphatidylinositol-4-phosphate 5-kinase | 2.7.1.136 | macrolide 2'-kinase |
| 2.7.1.22 | ribosylnicotinamide kinase | 2.7.1.69 | protein-Np-phosphohistidine-sugar phosphotransferase | 2.7.1.137 | phosphatidylinositol 3-kinase |
| 2.7.1.23 | NAD+ kinase | 2.7.1.70 | identical to EC 2.7.1.37. | 2.7.1.138 | ceramide kinase |
| 2.7.1.24 | dephospho-CoA kinase | 2.7.1.71 | shikimate kinase | 2.7.1.140 | inositol-tetrakisphosphate 5-kinase |
| 2.7.1.25 | adenylyl-sulfate kinase | 2.7.1.72 | streptomycin 6-kinase | 2.7.1.142 | glycerol-3-phosphate-glucose phosphotransferase |
| 2.7.1.26 | riboflavin kinase | 2.7.1.73 | inosine kinase | 2.7.1.143 | diphosphate-purine nucleoside kinase |
| 2.7.1.27 | erythritol kinase | 2.7.1.74 | deoxycytidine kinase | 2.7.1.144 | tagatose-6-phosphate kinase |
| 2.7.1.28 | triokinase | 2.7.1.76 | deoxyadenosine kinase | 2.7.1.145 | deoxynucleoside kinase |
| 2.7.1.29 | glycerone kinase | 2.7.1.77 | nucleoside phosphotransferase | 2.7.1.146 | ADP-dependent phosphofructokinase |
| 2.7.1.30 | glycerol kinase | 2.7.1.78 | polynucleotide 5'-hydroxyl-kinase | 2.7.1.147 | ADP-dependent glucokinase |
| 2.7.1.31 | glycerate kinase | 2.7.1.79 | diphosphate-glycerol phosphotransferase | 2.7.1.148 | 4-(cytidine 5'-diphospho)-2-C-methyl-D-erythritol kinase |
| 2.7.1.32 | choline kinase | 2.7.1.80 | diphosphate-serine phosphotransferase | 2.7.1.149 | 1-phosphatidylinositol-5-phosphate 4-kinase |
| 2.7.1.33 | pantothenate kinase | 2.7.1.81 | hydroxylysine kinase | 2.7.1.150 | 1-phosphatidylinositol-3-phosphate 5-kinase |
| 2.7.1.34 | pantetheine kinase | 2.7.1.82 | ethanolamine kinase | 2.7.1.151 | inositol- polyphosphate multikinase |
| 2.7.1.35 | pyridoxal kinase | 2.7.1.83 | pseudouridine kinase | 2.7.1.153 | phosphatidylinositol-4,5-bisphosphate 3-kinase |
| 2.7.1.36 | mevalonate kinase | 2.7.1.84 | alkylglycerone kinase | 2.7.1.154 | phosphatidylinositol-4-phosphate 3-kinase |
| 2.7.1.39 | homoserine kinase | 2.7.1.85 | β-glucoside kinase | 2.7.1.156 | adenosylcobinamide kinase |
| 2.7.1.40 | pyruvate kinase | 2.7.1.86 | NADH kinase | 2.7.1.157 | N-acetylgalactosamine kinase |
| 2.7.1.41 | glucose-1 -phosphate phosphodismutase | 2.7.1.87 | streptomycin 3"-kinase | 2.7.1.158 | inositol-pentakisphosphate 2-kinase |
| 2.7.1.42 | riboflavin phosphotransferase | 2.7.1.88 | dihydrostreptomycin-6-phosphate 3'a-kinase | 2.7.1.159 | inositol-1,3,4-trisphosphate 5/6-kinase |
| 2.7.1.43 | glucuronokinase | 2.7.1.89 | thiamine kinase | 2.7.1.160 | 2'-phosphotransferase |
| 2.7.1.44 | galacturonokinase | 2.7.1.90 | diphosphate-fructosc-6-phosphate 1-phosphotransferase | 2.7.1.161 | CTP-dependent riboflavin kinase |
| 2.7.1.45 | 2-dehydro-3-deoxygluconokinase | 2.7.1.91 | sphinganine kinase | 2.7.1.162 | N-acetylhexosamine 1-kinase |
| 2.7.1.46 | L-arabinokinase | 2.7.1.92 | 5-dehydro-2-deoxygluconokinase | 2.7.1.163 | hygromycin B 4-O-kinase |
| 2.7.1.47 | D-ribulokinase | 2.7.1.93 | alkylglycerol kinase | 2.7.1.164 | O-phosphoseryl-tRNASec kinase |

Mevalonate kinase (EC 2.7.1.36) phosphorylates the terminal hydroxyl group of mevalonate. Gene candidates for this step include erg12 from *S. cerevisiae, mvk* from *Methanocaldococcus jannaschi, MVK* from *Homo sapeins,* and *mvk* from *Arabidopsis thaliana col.* Additional mevalonate kinase candidates include the feedback-resistant mevalonate kinase from the archeon *Methanosarcina mazei* (Primak et al, AEM, in press (2011)) and the Mvk protein from *Streptococcus pneumoniae* (Andreassi et al, Protein Sci, 16:983-9 (2007)). Mvk proteins from *S. cerevisiae, S. pneumoniae* and *M. mazei* were heterologously expressed and characterized in *E. coli* (Primak et al, *supra*)*.* The *S*. *pneumoniae* mevalonate kinase was active on several alternate substrates including cylopropylmevalonate, vinylmevalonate and ethynylmevalonate (Kudoh et al, Bioorg Med Chem 18:1124-34 (2010)), and a subsequent study determined that the ligand binding site is selective for compact, electron-rich C(3)-substituents (Lefurgy et al, J Biol Chem 285:20654-63 (2010)).

| Protein | GenBank ID | GI Number | Organism |
|---|---|---|---|
| erg12 | CAA39359.1 | 3684 | Sachharomyces cerevisiae |
| *mvk* | Q58487.1 | 2497517 | *Methanocaldococcus jannaschii* |
| *mvk* | AAH16140.1 | 16359371 | *Homo sapiens* |
| *mvk* | NP_851084.1 | 30690651 | *Arabidopsis thaliana* |
| *mvk* | NP_633786.1 | 21227864 | *Methanosarcina mazei* |
| *mvk* | NP_357932.1 | 15902382 | *Streptococcus pneumoniae* |

Glycerol kinase also phosphorylates the terminal hydroxyl group in glycerol to form glyccrol-3-phosphatc. This reaction occurs in several spccics, including Eschcrichia *coli, Saccharomyces cerevisiae,* and *Thermotoga maritima.* The *E. coli* glycerol kinase has been shown to accept alternate substrates such as dihydroxyacetone and glyceraldehyde (Hayashi et al., J Biol. Chem. 242:1030-1035 (1967)). T, maritime has two glycerol kinases (Nelson et al., Nature 399:323-329 (1999)). Glycerol kinases have been shown to have a wide range of substrate specificity. Crans and Whiteside studied glycerol kinases from four different organisms (*Escherichia coli, S. cerevisiae, Bacillus stearothermophilus,* and *Candida mycoderma*) (Crans et al., J.Am.Chem.Soc. 107:7008-7018 (2010); Nelson et al., *supra,* (1999)). They studied 66 different analogs of glycerol and concluded that the enzyme could accept a range of substituents in place of one terminal hydroxyl group and that the hydrogen atom at C2 could be replaced by a methyl group. Interestingly, the kinetic constants of the enzyme from all four organisms were very similar.

| Protein | GenBank ID | GI Number | Organism |
|---|---|---|---|
| glpK | AP_003883.1 | 89110103 | Escherichia coli K12 |
| *glpK1* | NP_228760.1 | 15642775 | *Thermotoga maritime MSB8* |
| *glpK2* | NP_229230.1 | 15642775 | *Thermotoga maritime MSB8* |
| *Gut1* | NP_011831.1 | 82795252 | *Saccharomyces cerevisiae* |

Homoserine kinase is another possible candidate. This enzyme is also present in a number of organisms including *E. coli,* Streptomyces *sp,* and *S. cerevisiae.* Homoserine kinase from *E. coli* has been shown to have activity on numerous substrates, including, L-2-amino,1,4- butanediol, aspartate semialdehyde, and 2-amino-5-hydroxyvalerate (Huo et al., Biochemistry 35:16180-16185 (1996); Huo et al., Arch.Biochem.Biophys. 330:373-379 (1996)). This enzyme can act on substrates where the carboxyl group at the alpha position has been replaced by an ester or by a hydroxymethyl group. The gene candidates are:

| Protein | GenBank ID | GI Number | Organism |
|---|---|---|---|
| thrB | BAB96580.2 | 85674277 | Escherichia coli K12 |
| SACT1DRAFT_4809 | ZP_06280784.1 | 282871792 | *Streptomyces sp. ACT-1* |
| *Thr1* | AAA35154.1 | 172978 | *Saccharomyces serevisiae* |

### 2-Butenyl-4-phosphate kinase (Figure 12, Step B)

2-Butenyl-4-phosphate kinase enzymes catalyze the transfer of a phosphate group to the phosphate group of 2-butenyl-4-phosphate. The enzymes described below naturally possess such activity or can be engineered to exhibit this activity. Kinases that catalyze transfer of a phosphate group to another phosphate group are members of the EC 2.7.4 enzyme class. The table below lists several useful kinase enzymes in the EC 2.7.4 enzyme class.

| Enzyme Commission Number | Enzyme Name |
|---|---|
| 2.7.4.1 | polyphosphate kinase |
| 2.7.4.2 | phosphomevalonate kinase |
| 2.7.4.3 | adenylate kinase |
| 2.7.4.4 | nucleoside-phosphate kinase |
| 2.7.4.6 | nucleoside-diphosphate kinase |
| 2.7.4.7 | phosphomethylpyrimidine kinase |
| 2.7.4.8 | guanylate kinase |
| 2.7.4.9 | dTMP kinase |
| 2.7.4.10 | nucleoside-triphosphate-adenylate kinase |
| 2.7.4.11 | (deoxy)adenylate kinase |
| 2.7.4.12 | T2-induced deoxynucleotide kinase |
| 2.7.4.13 | (deoxy)nucleoside-phosphate kinase |
| 2.7.4.14 | cytidylate kinase |
| 2.7.4.15 | thiamine-diphosphate kinase |
| 2.7.4.16 | thiamine-phosphate kinase |
| 2.7.4.17 | 3-phosphoglyceroyl-phosphate-polyphosphate phosphotransferase |
| 2.7.4.18 | farnesyl-diphosphate kinase |
| 2.7.4.19 | 5-methyldeoxycytidine-5'-phosphate kinase |
| 2.7.4.20 | dolichyl-diphosphate-polyphosphate phosphotransferase |
| 2.7.4.21 | inositol-hexakisphosphate kinase |
| 2.7.4.22 | UMP kinase |
| 2.7.4.23 | ribose 1,5-bisphosphate phosphokinase |
| 2.7.4.24 | diphosphoinositol-pentakisphosphate kinase |
| 2.7.4.- | Farnesyl monophosphate kinase |
| 2.7.4.- | Geranyl-geranyl monophosphate kinase |
| 2.7.4.- | Phytyl-phosphate kinase |

Phosphomevalonate kinase enzymes are of particular interest. Phosphomevalonate kinase (EC 2.7.4.2) catalyzes the analogous transformation to 2-butenyl-4-phosphate kinase. This enzyme is encoded by *erg8* in *Saccharomyces cerevisiae* (Tsay et al., Mol.Cell Biol. 11:620-631 (1991)) and *mvaK2* in *Streptococcus pneumoniae, Staphylococcus aureus* and *Enterococcus faecalis* (Doun et al., Protein Sci. 14:1134-1139 (2005); Wilding et al., J Bacteriol. 182:4319-4327 (2000)). The *Streptococcus pneumoniae* and *Enterococcus faecalis* enzymes were cloned and characterized in *E. coli* (Pilloff et al., J Biol.Chem. 278:4510-4515 (2003); Doun et al., Protein Sci. 14:1134-1139 (2005)). The *S*. *pneumoniae* phosphomevalonate kinase was active on several alternate substrates including cylopropylmevalonate phosphate, vinylmevalonate phosphate and ethynylmevalonate phosphate (Kudoh et al, Bioorg Med Chem 18:1124-34 (2010)).

| Protein | GenBank ID | GI Number | Organism |
|---|---|---|---|
| *Erg8* | AAA34596.1 | 171479 | *Saccharomyces cerevisiae* |
| *mvaK2* | AAG02426.1 | 9937366 | *Staphylococcus aureus* |
| *mvaK2* | AAG02457.1 | 9937409 | *Streptococcus pneumoniae* |
| *mvaK2* | AAG02442.1 | 9937388 | *Enterococcus faecalis* |

Farnesyl monophosphate kinase enzymes catalyze the CTP dependent phosphorylation of farnesyl monophosphate to farnesyl diphosphate. Similarly, geranylgeranyl phosphate kinase catalyzes CTP dependent phosphorylation. Enzymes with these activities were identified in the microsomal fraction of cultured *Nicotiana tabacum* (Thai et al, PNAS 96:13080-5 (1999)). However, the associated genes have not been identified to date.

### Butadiene synthase (Figure 12, Step C)

Butadiene synthase catalyzes the conversion of 2-butenyl-4-diphosphate to 1,3-butadiene. The enzymes described below naturally possess such activity or can be engineered to exhibit this activity. Carbon-oxygen lyases that operate on phosphates are found in the EC 4.2.3 enzyme class. The table below lists several useful enzymes in EC class 4.2.3.

| Enzyme Commission Number | Enzyme Name |
|---|---|
| 4.2.3.15 | Myrcene synthase |
| 4.2.3.26 | Linalool synthase |
| 4.2.3.27 | Isoprene synthase |
| 4.2.3.36 | Terpentriene sythase |
| 4.2.3.46 | (E, E)-alpha-Farnesene synthase |
| 4.2.3.47 | Beta-Farnesene synthase |
| 4.2.3.49 | Nerolidol synthase |

Particularly useful enzymes include isoprene synthase, myrcene synthase and farnesene synthase. Enzyme candidates are described below.

Isoprene synthase naturally catalyzes the conversion of dimethylallyl diphosphate to isoprene, but can also catalyze the synthesis of 1,3-butadiene from 2-butenyl-4-diphosphate. Isoprene synthases can be found in several organisms including *Populus alba* (Sasaki et al., FEBS Letters, 2005, 579 (11), 2514-2518), *Pueraria montana* (Lindberg et al., Metabolic Eng, 12(1):70-79 (2010); Sharkey et al., Plant Physiol., 137(2):700-712 (2005)), and *Populus tremula* x *Populus alba,* also called *Populus canescens* (Miller et al., Planta, 2001, 213 (3), 483-487). The crystal structure of the *Populus canescens* isoprene synthase was determined (Koksal ct al, J Mol Biol 402:363-373 (2010)). Additional isoprene synthase enzymes are described in (Chotani et al., WO/2010/031079, Systems Using Cell Culture for Production of Isoprene; Cervin et al., US Patent Application 20100003716, Isoprene Synthase Variants for Improved Microbial Production of Isoprene).

| *Protein* | *GenBank ID* | *GI Number* | *Organism* |
|---|---|---|---|
| *ispS* | BAD98243.1 | 63108310 | *Populus alba* |
| *ispS* | AAQ84170.1 | 35187004 | *Pueraria montana* |
| *ispS* | CAC35696.1 | 13539551 | *Populus tremula x Populus alba* |

Myrcene synthase enzymes catalyze the dephosphorylation of geranyl diphosphate to beta-myrcene (EC 4.2.3.15). Exemplary myrcene synthases are encoded by MST2 of *Solanum lycopersicum* (van Schie et al, Plant Mol Biol 64:D473-79 (2007)), TPS-Myr of *Picea abies* (Martin et al, Plant Physiol 135:1908-27 (2004)) g-myr of *Abies grandis* (Bohlmann et al, J Biol Chem 272:21784-92 (1997)) and TPS10 of *Arabidopsis thaliana* (Bohlmann et al, Arch Biochem Biophys 375:261-9 (2000)). These enzymes were heterologously expressed in *E. coli.*

| *Protein* | *GenBank ID* | *GI Number* | *Organism* |
|---|---|---|---|
| *MST2* | ACN58229.1 | 224579303 | *Solanum lycopersicum* |
| *TPS-Myr* | AAS47690.2 | 77546864 | *Picea abies* |
| *G-myr* | O24474.1 | 17367921 | *Abies grandis* |
| *TPS10* | EC07543.1 | 330252449 | *Arabidopsis thaliana* |

Farnesyl diphosphate is converted to alpha-farnesene and beta-farnesene by alpha-farnesene synthase and beta-farnesene synthase, respectively. Exemplary alpha-farnesene synthase enzymes include *TPS03* and *TPS02* of Arabidopsis thaliana (Faldt et al, Planta 216:745-51 (2003); Huang et al, Plant Physiol 153:1293-310 (2010)), *afs* of *Cucumis sativus* (Mercke et al, Plant Physiol 135:2012-14 (2004), *eafar* of *Malus x domestica* (Green et al, Phytochem 68:176-88 (2007)) and TPS-Far of Picea abies (Martin, *supra*)*.* An exemplary beta-farnesene synthase enzyme is encoded by *TPS1* of *Zea mays* (Schnee et al, Plant Physiol 130:2049-60 (2002)).

| *Protein* | *GenBank ID* | *GI Number* | *Organism* |
|---|---|---|---|
| *TPS03* | A4FVP2.1 | 205829248 | *Arabidopsis thaliana* |
| *TPS02* | P0CJ43.1 | 317411866 | *Arabidopsis thaliana* |
| *TPS-Far* | AAS47697.1 | 44804601 | *Picea abies* |
| *afs* | AAU05951.1 | 51537953 | *Cucumis sativus* |
| *eafar* | Q84LB2.2 | 75241161 | *Malus x domestica* |
| *TPS1* | Q84ZW8.1 | 75149279 | *Zea mays* |

### Crotyl alcohol diphosphokinase (Figure 12, Step D)

Crotyl alcohol diphosphokinase enzymes catalyze the transfer of a diphosphate group to the hydroxyl group of crotyl alcohol. The enzymes described below naturally possess such activity or can be engineered to exhibit this activity. Kinases that catalyze transfer of a diphosphate group are members of the EC 2.7.6 enzyme class. The table below lists several useful kinase enzymes in the EC 2.7.6 enzyme class.

| Enzyme Commission Number | Enzyme Name |
|---|---|
| 2.7.6.1 | ribose-phosphate diphosphokinase |
| 2.7.6.2 | thiamine diphosphokinase |
| 2.7.6.3 | 2-amino-4-hydroxy-6-hydroxymethyldihydropteridine diphosphokinase |
| 2.7.6.4 | nucleotide diphosphokinase |
| 2.7.6.5 | GTP diphosphokinase |

Of particular interest are ribose-phosphate diphosphokinase enzymes which have been identified in *Escherichia coli* (Hove-Jenson et al., J Biol Chem, 1986, 261(15);6765-71) and *Mycoplasma pneumoniae* M129 (McElwain et al, International Journal of Systematic Bacteriology, 1988, 38:417-423) as well as thiamine diphosphokinase enzymes. Exemplary thiamine diphosphokinase enzymes are found in *Arabidopsis thaliana* (Ajjawi, Plant Mol Biol, 2007, 65(1-2);151-62).

| Protein | GenBank ID | GI Number | Organism |
|---|---|---|---|
| prs | NP_415725.1 | *16129170* | *Escherichia coli* |
| prsA | NP_109761.1 | *13507812* | *Mycoplasma pneumoniae M129* |
| TPK1 | BAH19964.1 | *222424006* | *Arabidopsis thaliana col* |
| TPK2 | BAH57065.1 | *227204427* | *Arabidopsis thaliana col* |

Throughout this application various publications have been referenced. The disclosures of these publications in their entireties, including GenBank and GI number publications, are hereby incorporated by reference in this application in order to more fully describe the state of the art to which this invention pertains. Although the invention has been described with reference to the examples provided above, it should be understood that various modifications can be made without departing from the spirit of the invention.
1. A non-naturally occurring microbial organism, comprising a microbial organism having a 1,3-butanediol pathway comprising at least one exogenous nucleic acid encoding a 1,3-butanediol pathway enzyme expressed in a sufficient amount to produce 1,3-butanediol, wherein said 1,3-butanediol pathway comprises a pathway selected from:
   (33) 7AS, 7P, 7N and 7AA;
   (1) 4A, 4B, 4C and 4D;
   (2) 5A, 5H, 5J, 5K and 5G;
   (3) 5A, 5H, 51 and 5G;
   (4) 5A, 5H and 5L;
   (5) 5A, 5F and 5G;
   (6) 7A, 7D, 7E, 7F, 7G and 7S;
   (7) 7A, 7D, 71, 7G and 7S;
   (8) 7A, 7D, 7K, and 7S;
   (9) 7A, 7H, 7F, 7G and 7S;
   (10) 7A, 7J, 7G and 7S;
   (11) 7A, 7J, 7R and 7AA;
   (12) 7A, 7H, 7F, 7R and 7AA;
   (13) 7A, 7H, 7Q, 7Z and 7AA;
   (14) 7A, 7D, 71, 7R and 7AA;
   (15) 7A, 7D, 7E, 7F, 7R and 7AA;
   (16) 7A, 7D, 7E, 7Q, 7Z and 7AA;
   (17) 7A, 7D, 7P, 7N and 7AA;
   (18) 7A, 7D, 7P, 7Y, 7Z and 7AA;
   (19) 7A, 7B, 7M and 7AA;
   (20) 7A, 7B, 7L, 7Z and 7AA;
   (21) 7A, 7B, 7X, 7N and 7AA;
   (22) 7A, 7B, 7X, 7Y, 7Z and 7AA;
   (23) 7A, 7D, 7P and 7O;
   (24) 7A, 7B, 7X and 7O;
   (25) 7A, 7D, 7E, 7F, 7R, 7AA;
   (26) 7A, 7D, 7E, 7F, 7G, 7S;
   (27) 7AS, 7E, 7F, 7G and 7S;
   (28) 7AS, 71, 7G and 7S;
   (29) 7AS, 7K, and 7S;
   (30) 7AS, 71, 7R and 7AA;
   (31) 7AS, 7E, 7F, 7R and 7AA;
   (32) 7AS, 7E, 7Q, 7Z and 7AA;
   (34) 7AS, 7P, 7Y, 7Z and 7AA;
   (35) 7AS, 7P and 7O;
   (36) 7AS, 7E, 7F, 7R, and 7AA; and
   (37) 7AS, 7E, 7F, 7G, and 7S,
   wherein 4A is a 3-oxo-5-hydroxypentanoyl-CoA thiolase or a 3-oxo-5-hydroxypentanoyl-CoA synthase, wherein 4B is a 3-oxo-5-hydroxypentanoyl-CoA hydrolase, 3-oxo-5-hydroxypentanoyl-CoA transferase or 3-oxo-5-hydroxypentanoyl-CoA synthetase, wherein 4C is a 3-oxo-5-hydroxypentanoate decarboxylase, wherein 4D is a 3-oxobutanol reductase, wherein in 5A is a 4-hydroxy-2-oxovalerate aldolase, wherein 5F is a 4-hydroxy-2-oxovalerate decarboxylase, wherin 5G is a 3-hydroxybutanal reductase, wherein 5H is a 4-hydroxy-2-oxopentanoate dehydrogenase, a 4-hydroxy-2-oxopentanoate:ferredoxin oxidoreductase or a 4-hydroxy-2-oxopentanoate formate lyase, wherein 51 is a 3-hydroxybutyryl-CoA reductase (aldehyde forming), wherein 5J is a 3-hydroxybutyryl-CoA hydrolase, a 3-hydroxybutyryl-CoA transferase or a 3-hydroxybutyryl-CoA synthetase, wherein 5K is a 3-hydroxybutyrate reductase, wherein 5L is a 3-hydroxybutyryl-CoA reductase (alcohol forming), wherein 7A is a 3-ketoacyl-ACP synthase, wherein 7B is an acetoacetyl-ACP reductase, wherein 7D is an acetoacetyl-CoA:ACP transferase, wherein 7E is an acetoacetyl-CoA hydrolase, acetoacetyl-CoA transferase or acetoacetyl-CoA synthetase, wherein 7F is an acetoacetate reductase (acid reducing), wherein 7G is a 3-oxobutyraldehyde reductase (aldehyde reducing), wherein 7H is an acetoacetyl-ACP thioesterase, wherein 71 is an acetoacetyl-CoA reductase (CoA-dependent, aldehyde forming), wherein 7J is an acetoacetyl-ACP reductase (aldehyde forming), wherein 7K is an acetoacetyl-CoA reductase (alcohol forming), wherein 7L is a 3-hydroxybutyryl-ACP thioesterase, wherein 7M is a 3-hydroxybutyryl-ACP reductase (aldehyde forming), wherein 7N is a 3-hydroxybutyryl-CoA reductase (aldehyde forming), wherein 7O is a 3-hydroxybutyryl-CoA reductase (alcohol forming), wherein 7P is an acetoacetyl-CoA reductase (ketone reducing), wherein 7Q is an acetoacetate reductase (ketone reducing), wherein 7R is a 3-oxobutyraldehyde reductase (ketone reducing), wherein 7S is a 4-hydroxy-2-butanone reductase, wherein 7X is a 3-hydroxybutyryl-CoA:ACP transferase, wherein 7Y is a 3-hydroxybutyryl-CoA hydrolase, a 3-hydroxybutyryl-CoA transferase or a 3-hydroxybutyryl-CoA synthetase, wherein 7Z is a 3-hydroxybutyrate reductase, wherein 7AA is a 3-hydroxybutyraldehyde reductase and wherein 7AS is an acetoacetyl-CoA synthase.
2. The non-naturally occurring microbial organism of item 1, wherein said microbial organism comprises two, three, four or five exogenous nucleic acids each encoding a 1,3-butanediol pathway enzyme.
3 The non-naturally occurring microbial organism of item 2, wherein said microbial organism comprises exogenous nucleic acids encoding each of the enzymes of at least one of the pathways selected from (1)-(37).
4. The non-naturally occurring microbial organism of item 1, wherein said at least one exogenous nucleic acid is a heterologous nucleic acid.
5. The non-naturally occurring microbial organism of item 1, wherein said non-naturally occurring microbial organism is in a substantially anaerobic culture medium.
6. The non-naturally occurring microbial organism of item 1, further comprising:
   (i) a reductive TCA pathway comprising at least one exogenous nucleic acid encoding a reductive TCA pathway enzyme, wherein said at least one exogenous nucleic acid is selected from an ATP-citrate lyase, a citrate lyase, a citryl-CoA synthetase, a citryl-CoA lyase, a fumarate reductase, and an alpha-ketoglutarate:ferredoxin oxidoreductase;
   (ii) a reductive TCA pathway comprising at least one exogenous nucleic acid encoding a reductive TCA pathway enzyme, wherein said at least one exogenous nucleic acid is selected from a pyruvate:ferredoxin oxidorcductasc, a phosphocnolpyruvatc carboxylase, a phosphoenolpyruvate carboxykinase, a CO dehydrogenase, and an H₂ hydrogenase; or
   (iii) at least one exogenous nucleic acid encodes an enzyme selected from a CO dehydrogenase, an H₂ hydrogenase, and combinations thereof.
7. The non-naturally occurring microbial organism of item 6, wherein said microbial organism comprising (i) further comprises an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof.
8. The non-naturally occurring microbial organism of item 6, wherein said microbial organism comprising (ii) further comprises an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof.
9. The non-naturally occurring microbial orgnaism of item 6, wherein said microbial organism comprising (i) comprises three exogenous nucleic acids encoding an ATP-citrate lyase or a citrate lyase, a fumarate reductase, and an alpha-ketoglutarate:ferredoxin oxidoreductase;
   wherein said microbial organism comprising (ii) comprises four exogenous nucleic acids encoding a pyruvate:ferredoxin oxidoreductase; a phosphoenolpyruvate carboxylase or a phosphoenolpyruvate carboxykinase; a CO dehydrogenase; and an H₂ hydrogenase; or
   wherein said microbial organism comprising (iii) comprises two exogenous nucleic acids encoding a CO dehydrogenase and an H₂ hydrogenase.
10. A method for producing 1,3-butanediol, comprising culturing the non-naturally occurring microbial organism of any one of items 1-9 under conditions and for a sufficient period of time to produce 1,3-butanediol.
11. A non-naturally occurring microbial organism, comprising a microbial organism having a 2,4-pentadienoate pathway comprising at least one exogenous nucleic acid encoding a 2,4-pentadienoate pathway enzyme expressed in a sufficient amount to produce 2,4-pentadienoate, wherein said 2,4-pentadienoate pathway comprises a pathway selected from:
   (1) 1D, 1I, 1B, 1C, 1K and 1G;
   (2) 1D, 1E, 1F and 1G;
   (3) 1D, 1E, 1L, 1M, 1P and 1G;
   (4) 1D, 1I, 1B and 1J;
   (5) 1D, 1I, 1B, 1C, 1K, 1P, IN and 1O;
   (6) 1D, 1E, 1F, 1P, IN and 1O;
   (7) 1D, 1E, 1L, 1M, 1N and 1O;
   (8) 1D, 1E, 1L, 1Q and 1O;
   (9) 1S, 1I, 1B, 1C, 1K and 1G;
   (10) 1S, 1E, 1F and 1G;
   (11) 1S, 1I, 1B and 1J;
   (12) 1S, 1I, 1B, 1C, 1K, 1P, IN and 1O;
   (13) 1S, 1E, 1F, 1P, IN and 1O;
   (14) 1S, 1E, 1L, 1M, 1N and 1O;
   (15) 1S, 1E, 1L, 1Q and 1O;
   (16) 1B, 1C, 1K and 1G;
   (17) 1I, 1E, 1F and 1G;
   (18) 1I, 1E, 1L, 1M, 1P and 1G;
   (19) 1B and 1J;
   (20) 1I, 1E, 1F, 1P, IN and 1O;
   (21) 1I, 1E, 1L, 1M, IN and 10;
   (22) 1I, 1E, 1L, 1Q and 1O;
   (23) 3A, 3B, 3C, 3D, 3E and 3F; and
   (24) 3A, 3B, 3C, 3G and 3F,
   wherein 1B is a 5-aminopentanoate reductase, wherein 1C is a 5-aminopent-2-enoate aminotransferase, a 5-aminopent-2-enoate dehydrogenase or an amine oxidase, wherein 1D is a 2-oxoadipate decarboxylase, wherein 1E is a glutarate semialdehyde reductase, wherein 1F is a 5-hydroxyvalerate dehydrogenase, wherein 1G is a 5-hydroxypent-2-enoate dehydratase, wherein 1I is a 5-aminopentanoate aminotransferase, a 5-aminopentanoate dehydrogenase or a 5-aminopentanoate amine oxidase, wherein 1J is a 5-aminopent-2-enoate deaminase, wherin 1K is a 5-hydroxypent-2-enoate reductase, wherein 1L is a 5-hydroxyvaleryl-CoA transferase or a 5-hydroxyvaleryl-CoA synthetase, wherein 1M is a 5-hydroxypentanoyl-CoA dehydrogenase, wherein IN is a 5-hydroxypent-2-enoyl-CoA dehydratase, wherein 1O is a 2,4-pentadienoyl-CoA transferase, a 2,4-pentadienoyl-CoA synthetase or a 2,4-pentadienoyl-CoA hydrolase, wherein 1P is a 5-hydroxypent-2-enoyl-CoA transferase or a 5-hydroxypent-2-enoyl-CoA synthetase, wherein 1Q is a 5-hydroxyvaleryl-CoA dehydratase/dehydrogenase, wherein 1S a glutaryl-CoA reductase, wherein 3A is a 3-oxopentanoyl-CoA thiolase or a 3-oxopentanoyl-CoA synthase, wherein 3B is a 3-oxopentanoyl-CoA reductase, wherein 3C is a 3-hydroxypentanoyl-CoA dehydratase, wherein 3D is a pent-2-enoyl-CoA isomerase, wherein 3E is a pent-3-enoyl-CoA dehydrogenase, wherein 3F is a 2,4-pentadienoyl-CoA hydrolase, a 2,4-pentadienoyl-CoA transferase or a 2,4-pentadienoyl-CoA synthetase, wherein 3G is a pent-2-enoyl-CoA dehydrogenase.
12. The non-naturally occurring microbial organism of item 11, wherein said microbial organism comprises two, three, four, five, six, seven, eight, nine or ten exogenous nucleic acids each encoding a 2,4-pentadienoate pathway enzyme.
13. The non-naturally occurring microbial organism of item 12, wherein said microbial organism comprises exogenous nucleic acids encoding each of the enzymes of at least one of the pathways selected from (1)-(24).
14. The non-naturally occurring microbial organism of item 11, wherein said at least one exogenous nucleic acid is a heterologous nucleic acid.
15. The non-naturally occurring microbial organism of item 11, wherein said non-naturally occurring microbial organism is in a substantially anaerobic culture medium.
16. The non-naturally occurring microbial organism of item 11, wherein said non-naturally occuring microbial organism comprising a 2,4-pentadienoate pathway selected from (9)-(15) further comprises a glutaryl-CoA pathway comprising at least one exogenous nucleic acid encoding a glutaryl-CoA pathway enzyme expressed in a sufficient amount to produce glutaryl-CoA, said glutaryl-CoA pathway comprising a pathway selected from:
   an acetoacetyl-CoA thiolase or an acetoacetyl-CoA synthase; an acetoacetyl-CoA reductase; a 3-hydroxybutyryl-CoA dehydratase; and a glutaryl-CoA dehydrogenase; or
   a 2-aminoadipate aminotransferase, a 2-aminoadipate dehydrogenase or a 2-aminoadipate amine oxidase; and a 2-oxoadipate dehydrogenase, a 2-oxoadipate:ferridoxin oxidoreductase or a 2-oxoadipate formate lyase.
17. The non-naturally occurring microbial organism of item 11, wherein said non-naturally occuring microbial organism comprising a 2,4-pentadienoate pathway selected from (16)-(22) further comprises a 5-aminopcntanoatc pathway comprising at least one exogenous nucleic acid encoding a 5-aminopentanoate pathway enzyme expressed in a sufficient amount to produce 5-aminopentanoate, said 5-aminopentanoate pathway comprising a 2-aminoadipate decarboxylase; or a 2-aminoadipate decarboxylase and a 2-aminoadipate aminotransferase, a 2-aminoadipate dehydrogenase or a 2-aminoadipate amine oxidase.
18. The non-naturally occurring microbial organism of item 11, wherein said non-naturally occuring microbial organism comprising a 2,4-pentadienoate pathway selected from (1)-(8) further comprises a 2-oxoadipate pathway comprising an exogenous nucleic acid encoding a 2-oxoadipate pathway enzyme expressed in a sufficient amount to produce a 2-oxoadipate, said 2-oxoadipate pathway comprising a 2-aminoadipate aminotransferase, a 2-aminoadipate dehydrogenase or a 2-aminoadipate amine oxidase.
19. The non-naturally occurring microbial organism of item 11, further comprising:
   (i) a reductive TCA pathway comprising at least one exogenous nucleic acid encoding a reductive TCA pathway enzyme, wherein said at least one exogenous nucleic acid is selected from an ATP-citrate lyase, a citrate lyase, a citryl-CoA synthetase, a citryl-CoA lyase, a fumarate reductase, and an alpha-ketoglutarate:ferredoxin oxidoreductase;
   (ii) a reductive TCA pathway comprising at least one exogenous nucleic acid encoding a reductive TCA pathway enzyme, wherein said at least one exogenous nucleic acid is selected from a pyruvate:ferredoxin oxidoreductase, a phosphoenolpyruvate carboxylase, a phosphoenolpyruvate carboxykinase, a CO dehydrogenase, and an H₂ hydrogenase; or
   (iii) at least one exogenous nucleic acid encodes an enzyme selected from a CO dehydrogenase, an H₂ hydrogenase, and combinations thereof.
20. The non-naturally occurring microbial organism of item 19, wherein said microbial organism comprising (i) further comprises an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof.
21. The non-naturally occurring microbial organism of item 19, wherein said microbial organism comprising (ii) further comprises an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof.
22. The non-naturally occurring microbial orgnaism of item 19, wherein said microbial organism comprising (i) comprises three exogenous nucleic acids encoding an ATP-citrate lyase or a citrate lyase, a fumarate reductase, and an alpha-ketoglutarate:ferredoxin oxidoreductase;
   wherein said microbial organism comprising (ii) comprises four exogenous nucleic acids encoding a pyruvate:ferredoxin oxidoreductase; a phosphoenolpyruvate carboxylase or a phosphoenolpyruvate carboxykinase; a CO dehydrogenase; and an H₂ hydrogenase; or
   wherein said microbial organism comprising (iii) comprises two exogenous nucleic acids encoding a CO dehydrogenase and an H₂ hydrogenase.
23. A method for producing 2,4-pentadienoate, comprising culturing the non-naturally occurring microbial organism of any one of items 11-22 under conditions and for a sufficient period of time to produce 2,4-pentadienoate.
24. A non-naturally occurring microbial organism, comprising a microbial organism having a butadiene pathway comprising at least one exogenous nucleic acid encoding a butadiene pathway enzyme expressed in a sufficient amount to produce butadiene, wherein said butadiene pathway comprises a pathway selected from:
   (1) 1D, 1I, 1B, 1C, 1K, 1G and 1T;
   (2) 1D, 1E, 1F, 1G and 1T;
   (3) 1D, 1E, 1L, 1M, 1P, 1G and 1T;
   (4) 1D, 1I, 1B, 1J and 1T;
   (5) 1D, 1I, 1B, 1C, 1K, 1P, 1N, 1O and 1T;
   (6) 1D, 1E, 1F, 1P, 1N, 1O and 1T;
   (7) 1D, 1E, 1L, 1M, 1N, 1O and 1T;
   (8) 1D, 1E, 1L, 1Q, 1O and 1T;
   (9) 1D, 1E, 1F, 1U and IV;
   (10) 1D, 1I, 1B, 1C, 1K, 1U and IV;
   (11) 1D, 1E, 1L, 1M, 1P, 1U and 1V;
   (12) 1D, 1E, 1W and 1V;
   (13) 1D, 1I, 1B, 1C, 1K, 1G, 6A and 6B;
   (14) 1D, 1E, 1F, 1G, 6A and 6B;
   (15) 1D, 1E, 1L, 1M, 1P, 1G, 6A and 6B;
   (16) 1D, 1I, 1B, 1J, 6A and 6B;
   (17) 1D, 1I, 1B, 1C, 1K, 1P, 1N, 1O, 6A and 6B;
   (18) 1D, 1E, 1F, 1P, 1N, 1O, 6A and 6B;
   (19) 1D, 1E, 1L, 1M, 1N, 1O, 6A and 6B;
   (20) 1D, 1E, 1L, 1Q, 10, 6A and 6B;
   (21) 1D, 1I, 1B, 1C, 1K, 1G, 6H, 6E and 6B;
   (22) 1D, 1E, 1F, 1G, 6H, 6E and 6B;
   (23) 1D, 1E, 1L, 1M, 1P, 1G, 6H, 6E and 6B;
   (24) 1D, 1I, 1B, 1J, 6H, 6E and 6B;
   (25) 1D, 1I, 1B, 1C, 1K, 1P, 1N, 1O, 6H, 6E and 6B;
   (26) 1D, 1E, 1F, 1P, 1N, 1O, 6H, 6E and 6B;
   (27) 1D, 1E, 1L, 1M, 1N, 1O, 6H, 6E and 6B;
   (28) 1D, 1E, 1L, 1Q, 1O, 6H, 6E and 6B;
   (29) 1D, 1I, 1B, 1C, 1K, 1P, 1N, 6C and 6B;
   (30) 1D, 1E, 1F, 1P, 1N, 6C and 6B;
   (31) 1D, 1E, 1L, 1M, 1N, 6C and 6B;
   (32) 1D, 1E, 1L, 1Q, 6C and 6B;
   (33) 1D, 1I, 1B, 1C, 1K, 1P, 1N, 6D, 6E and 6B;
   (34) 1D, 1E, 1F, 1P, 1N, 6D, 6E and 6B;
   (35) 1D, 1E, 1L, 1M, 1N, 6D, 6E and 6B;
   (36) 1D, 1E, 1L, 1Q, 6D, 6E and 6B;
   (37) 1D, 1I, 1B, 1C, 1K, 1G, 6F, 6C and 6B;
   (38) 1D, 1E, 1F, 1G, 6F, 6C and 6B;
   (39) 1D, 1E, 1L, 1M, 1P, 1G, 6F, 6C and 6B;
   (40) 1D, 1I, 1B, 1C, 1K, 1G, 6F, 6D, 6E and 6B;
   (41) 1D, 1E, 1F, 1G, 6F, 6D, 6E and 6B;
   (42) 1D, 1E, 1L, 1M, 1P, 1G, 6F, 6D, 6E and 6B;
   (43) 1S, 1I, 1B, 1C, 1K, 1G and 1T;
   (44) 1S, 1E, 1F, 1G and 1T;
   (45) 1S, 1I, 1B, 1J and 1T;
   (46) 1S, 1I, 1B, 1C, 1K, 1P, 1N, 1O and 1T;
   (47) 1S, 1E, 1F, 1P, 1N, 10 and 1T;
   (48) 1S, 1E, 1L, 1M, 1N, 1O and 1T;
   (49) 1S, 1E, 1L, 1Q, 1O and 1T;
   (50) 1S, 1E, 1F, 1U and 1V;
   (51) 1S, 1I, 1B, 1C, 1K, 1U and 1V;
   (52) 1S, 1E, 1L, 1M, 1P, 1U and 1V;
   (53) 1S, 1E, 1W and 1V;
   (54) 1S, 1I, 1B, 1C, 1K, 1G, 6A and 6B;
   (55) 1S, 1E, 1F, 1G, 6A and 6B;
   (56) 1S, 1I, 1B, 1J, 6A and 6B;
   (57) 1S, 1I, 1B, 1C, 1K, 1P, 1N, 10, 6A and 6B;
   (58) 1S, 1E, 1F, 1P, IN, 1O, 6A and 6B;
   (59) 1S, 1E, 1L, 1M, IN, 1O, 6A and 6B;
   (60) 1S, 1E, 1L, 1Q, 1O, 6A and 6B;
   (61) 1S, 1I, 1B, 1C, 1K, 1G, 6H, 6E and 6B;
   (62) 1S, 1E, 1F, 1G, 6H, 6E and 6B;
   (63) 1S, 1I, 1B, 1J, 6H, 6E and 6B;
   (64) 1S, 1I, 1B, 1C, 1K, 1P, 1N, 1O, 6H, 6E and 6B;
   (65) 1S, 1E, 1F, 1P, IN, 1O, 6H, 6E and 6B;
   (66) 1S, 1E, 1L, 1M, IN, 1O, 6H, 6E and 6B;
   (67) 1S, 1E, 1L, 1Q, 1O, 6H, 6E and 6B;
   (68) 1S, 1I, 1B, 1C, 1K, 1P, IN, 6C and 6B;
   (69) 1S, 1E, 1F, 1P, IN, 6C and 6B;
   (70) 1S, 1E, 1L, 1M, IN, 6C and 6B;
   (71) 1S, 1E, 1L, 1Q, 6C and 6B;
   (72) 1S, 1I, 1B, 1C, 1K, 1P, IN, 6D, 6E and 6B;
   (73) 1S, 1E, 1F, 1P, IN, 6D, 6E and 6B;
   (74) 1S, 1E, 1L, 1M, IN, 6D, 6E and 6B;
   (75) 1S, 1E, 1L, 1Q, 6D, 6E and 6B;
   (76) 1S, 1I, 1B, 1C, 1K, 1G, 6F, 6C and 6B;
   (77) 1S, 1E, 1F, 1G, 6F, 6C and 6B;
   (78) 1S, 1I, 1B, 1C, 1K, 1G, 6F, 6D, 6E and 6B;
   (79) 1S, 1E, 1F, 1G, 6F, 6D, 6E and 6B;
   (80) 1B, 1C, 1K, 1G and 1T;
   (81) 1I, 1E, 1F, 1G and 1T;
   (82) 1I, 1E, 1L, 1M, IP, 1G and 1T;
   (83) 1B, 1J and 1T;
   (84) 1I, 1E, 1F, 1P, IN, 1O and 1T;
   (85) 1I, 1E, 1L, 1M, IN, 10 and 1T;
   (86) 1I, 1E, 1L, 1Q, 10 and 1T;
   (87) 1B, 1C, 1K, 1U and 1V;
   (88) 1I, 1E, 1F, 1U and 1V;
   (89) 1I, 1E, 1L, 1M, 1P, 1U and 1V;
   (90) 1I, 1E, 1W and 1V;
   (91) 1B, 1C, 1K, 1G, 6A and 6B;
   (92) 1I, 1E, 1F, 1G, 6A and 6B;
   (93) 1I, 1E, 1L, 1M, 1P, 1G, 6A and 6B;
   (94) 1B, 1I, 6A and 6B;
   (95) 1I, 1E, 1F, 1P, IN, 1O, 6A and 6B;
   (96) 1I, 1E, 1L, 1M, IN, 1O, 6A and 6B;
   (97) 1I, 1E, 1L, 1Q, 1O, 6A and 6B;
   (98) 1B, 1C, 1K, 1G, 6H, 6E and 6B;
   (99) 1I, 1E, 1F, 1G, 6H, 6E and 6B;
   (100) 1I, 1E, 1L, 1M, 1P, 1G, 6H, 6E and 6B;
   (101) 1B, 1J, 6H, 6E and 6B;
   (102) 1I, 1E, 1F, 1P, IN, 1O, 6H, 6E and 6B;
   (103) 1I, 1E, 1L, 1M, IN, 1O, 6H, 6E and 6B;
   (104) 1I, 1E, 1L, 1Q, 1O, 6H, 6E and 6B;
   (105) 1I, 1E, 1F, 1P, IN, 6C and 6B;
   (106) 1I, 1E, 1L, 1M, IN, 6C and 6B;
   (107) 1I, 1E, 1L, 1Q, 6C and 6B;
   (108) 1I, 1E, 1F, 1P, IN, 6D, 6E and 6B;
   (109) 1I, 1E, 1L, 1M, IN, 6D, 6E and 6B;
   (110) 1I, 1E, 1L, 1Q, 6D, 6E and 6B;
   (111) 1B, 1C, 1K, 1G, 6F, 6C and 6B;
   (112) 1I, 1E, 1F, 1G, 6F, 6C and 6B;
   (113) 1I, 1E, 1L, 1M, 1P, 1G, 6F, 6C and 6B;
   (114) 1B, 1C, 1K, 1G, 6F, 6D, 6E and 6B;
   (115) 1I, 1E, 1F, 1G, 6F, 6D, 6E and 6B;
   (116) 1I, 1E, 1L, 1M, 1P, 1G, 6F, 6D, 6E and 6B;
   (117) 3A, 3B, 3C, 3D, 3E, 3F and 1T;
   (118) 3A, 3B, 3C, 3D, 3E, 3F, 6A and 6B;
   (119) 3A, 3B, 3C, 3D, 3E, 3F, 6H, 6E and 6B;
   (120) 3A, 3B, 3C, 3D, 3E, 6C and 6B;
   (121) 3A, 3B, 3C, 3D, 3E, 6D, 6E and 6B; and
   (122) 3A, 3B, 3C, 3G, 3F and 1T;
   (123) 3A, 3B, 3C, 3G, 3F, 6A and 6B;
   (124) 3A, 3B, 3C, 3G, 3F, 6H, 6E and 6B;
   (125) 3A, 3B, 3C, 3G, 6C and 6B;
   (126) 3A, 3B, 3C, 3G, 6D, 6E and 6B;
   (127) 5A, 5B, 5C, 5D and 5E;
   (128) 7A, 7J, 7R, 7AD, 7AH, 12A, 12B and 12C;
   (129) 7A, 7H, 7F, 7R, 7AD, 7AH, 12A, 12B and 12C;
   (130) 7A, 7H, 7Q, 7Z, 7AD, 7AH, 12A, 12B and 12C;
   (131) 7A, 7H, 7Q, 7AC, 7AG, 7AH, 12A, 12B and 12C;
   (132) 7A, 7D, 71, 7R, 7AD, 7AH, 12A, 12B and 12C;
   (133) 7A, 7D, 7E, 7F, 7R, 7AD, 7AH, 12A, 12B and 12C;
   (134) 7A, 7D, 7E, 7Q, 7Z, 7AD, 7AH, 12A, 12B and 12C;
   (135) 7A, 7D, 7E, 7Q, 7AC, 7AG, 7AH, 12A, 12B and 12C;
   (136) 7A, 7D, 7P, 7N, 7AD, 7AH, 12A, 12B and 12C;
   (137) 7A, 7D, 7P, 7Y, 7Z, 7AD, 7AH, 12A, 12B and 12C;
   (138) 7A, 7D, 7P, 7Y, 7AC, 7AG, 7AH, 12A, 12B and 12C;
   (139) 7A, 7D, 7P, 7AB, 7V, 7AH, 12A, 12B and 12C;
   (140) 7A, 7D, 7P, 7AB, 7AF, 7AG, 7AH, 12A, 12B and 12C;
   (141) 7A, 7B, 7M, 7AD, 7AH, 12A, 12B and 12C;
   (142) 7A, 7B, 7L, 7Z, 7AD, 7AH, 12A, 12B and 12C;
   (143) 7A, 7B, 7L, 7AC, 7AG, 7AH, 12A, 12B and 12C;
   (144) 7A, 7B, 7X, 7Y, 7Z, 7AD, 7AH, 12A, 12B and 12C;
   (145) 7A, 7B, 7X, 7Y, 7AC, 7AG, 7AH, 12A, 12B and 12C;
   (146) 7A, 7B, 7X, 7AB, 7V, 7AH, 12A, 12B and 12C;
   (147) 7A, 7B, 7X, 7AB, 7AF, 7AG, 7AH, 12A, 12B and 12C;
   (148) 7A, 7B, 7C, 7U, 7AH, 12A, 12B and 12C;
   (149) 7A, 7B, 7C, 7T, 7AG, 7AH, 12A, 12B and 12C;
   (150) 7A, 7B, 7C, 7AE, 7AF, 7AG, 7AH, 12A, 12B and 12C;
   (151) 7A, 7D, 7P, 7AB, 7W, 12A, 12B and 12C;
   (152) 7A, 7B, 7X, 7AB, 7W, 12A, 12B and 12C;
   (153) 7A, 7B, 7C, 7AE, 7W, 12A, 12B and 12C;
   (154) 7A, 7B, 7C, 7AE, 7V, 7AH; , 12A, 12B and 12C
   (155) 7A, 7J, 7R, 7AD, 7AH, 12D and 12C;
   (156) 7A, 7H, 7F, 7R, 7AD, 7AH, 12D and 12C;
   (157) 7A, 7H, 7Q, 7Z, 7AD, 7AH, 12D and 12C;
   (158) 7A, 7H, 7Q, 7AC, 7AG, 7AH, 12D and 12C;
   (159) 7A, 7D, 7I, 7R, 7AD, 7AH, 12D and 12C;
   (160) 7A, 7D, 7E, 7F, 7R, 7AD, 7AH, 12D and 12C;
   (161) 7A, 7D, 7E, 7Q, 7Z, 7AD, 7AH, 12D and 12C;
   (164) 7A, 7D, 7E, 7Q, 7AC, 7AG, 7AH, 12D and 12C;
   (163) 7A, 7D, 7P, 7N, 7AD, 7AH, 12D and 12C;
   (164) 7A, 7D, 7P, 7Y, 7Z, 7AD, 7AH, 12D and 12C;
   (165) 7A, 7D, 7P, 7Y, 7AC, 7AG, 7AH, 12D and 12C;
   (166) 7A, 7D, 7P, 7AB, 7V, 7AH, 12D and 12C;
   (167) 7A, 7D, 7P, 7AB, 7AF, 7AG, 7AH, 12D and 12C;
   (168) 7A, 7B, 7M, 7AD, 7AH, 12D and 12C;
   (169) 7A, 7B, 7L, 7Z, 7AD, 7AH, 12D and 12C;
   (170) 7A, 7B, 7L, 7AC, 7AG, 7AH, 12D and 12C;
   (171) 7A, 7B, 7X, 7Y, 7Z, 7AD, 7AH, 12D and 12C;
   (172) 7A, 7B, 7X, 7Y, 7AC, 7AG, 7AH, 12D and 12C;
   (173) 7A, 7B, 7X, 7AB, 7V, 7AH, 12D and 12C;
   (174) 7A, 7B, 7X, 7AB, 7AF, 7AG, 7AH, 12D and 12C;
   (175) 7A, 7B, 7C, 7U, 7AH, 12D and 12C;
   (176) 7A, 7B, 7C, 7T, 7AG, 7AH, 12D and 12C;
   (177) 7A, 7B, 7C, 7AE, 7AF, 7AG, 7AH, 12D and 12C;
   (178) 7A, 7D, 7P, 7AB, 7W, 12D and 12C;
   (179) 7A, 7B, 7X, 7AB, 7W, 12D and 12C;
   (180) 7A, 7B, 7C, 7AE, 7W, 12D and 12C;
   (181) 7A, 7B, 7C, 7AE, 7V, 7AH, 12D and 12C;
   (182) 71, 7R, 7AD, 7AH, 12A, 12B and 12C;
   (183) 7E, 7F, 7R, 7AD, 7AH, 12A, 12B and 12C;
   (184) 7E, 7Q, 7Z, 7AD, 7AH, 12A, 12B and 12C;
   (185) 7E, 7Q, 7AC, 7AG, 7AH, 12A, 12B and 12C;
   (186) 7P, 7N, 7AD, 7AH, 12A, 12B and 12C;
   (187) 7P, 7Y, 7Z, 7AD, 7AH, 12A, 12B and 12C;
   (188) 7P, 7Y, 7AC, 7AG, 7AH, 12A, 12B and 12C;
   (189) 7P, 7AB, 7V, 7AH, 12A, 12B and 12C;
   (190) 7P, 7AB, 7AF, 7AG, 7AH, 12A, 12B and 12C;
   (191) 7P, 7AB, 7W, 12A, 12B and 12C;
   (192) 71, 7R, 7AD, 7AH, 12D and 12C;
   (193) 7E, 7F, 7R, 7AD, 7AH, 12D and 12C;
   (194) 7E, 7Q, 7Z, 7AD, 7AH, 12D and 12C;
   (195) 7E, 7Q, 7AC, 7AG, 7AH, 12D and 12C;
   (196) 7P, 7N, 7AD, 7AH, 12D and 12C;
   (197) 7P, 7Y, 7Z, 7AD, 7AH, 12D and 12C;
   (198) 7P, 7Y, 7AC, 7AG, 7AH, 12D and 12C;
   (199) 7P, 7AB, 7V, 7AH, 12D and 12C;
   (200) 7P, 7AB, 7AF, 7AG, 7AH, 12D and 12C;
   (201) 7P, 7AB, 7W, 12D and 12C;
   (202) 7AS, 71, 7R, 7AD, 7AH, 12A, 12B and 12C;
   (203) 7AS, 7E, 7F, 7R, 7AD, 7AH, 12A, 12B and 12C;
   (204) 7AS, 7E, 7Q, 7Z, 7AD, 7AH, 12A, 12B and 12C;
   (205) 7AS, 7E, 7Q, 7AC, 7AG, 7AH, 12A, 12B and 12C;
   (206) 7AS, 7P, 7N, 7AD, 7AH, 12A, 12B and 12C;
   (207) 7AS, 7P, 7Y, 7Z, 7AD, 7AH, 12A, 12B and 12C;
   (208) 7AS, 7P, 7Y, 7AC, 7AG, 7AH, 12A, 12B and 12C;
   (209) 7AS, 7P, 7AB, 7V, 7AH, 12A, 12B and 12C;
   (210) 7AS, 7P, 7AB, 7AF, 7AG, 7AH, 12A, 12B and 12C;
   (211) 7AS, 7P, 7AB, 7W, 12A, 12B and 12C;
   (212) 7AS, 71, 7R, 7AD, 7AH, 12D and 12C;
   (213) 7AS, 7E, 7F, 7R, 7AD, 7AH, 12D and 12C;
   (214) 7AS, 7E, 7Q, 7Z, 7AD, 7AH, 12D and 12C;
   (215) 7AS, 7E, 7Q, 7AC, 7AG, 7AH, 12D and 12C;
   (216) 7AS, 7P, 7N, 7AD, 7AH, 12D and 12C;
   (217) 7AS, 7P, 7Y, 7Z, 7AD, 7AH, 12D and 12C;
   (218) 7AS, 7P, 7Y, 7AC, 7AG, 7AH, 12D and 12C;
   (219) 7AS, 7P, 7AB, 7V, 7AH, 12D and 12C;
   (220) 7AS, 7P, 7AB, 7AF, 7AG, 7AH, 12D and 12C; and
   (221) 7AS, 7P, 7AB, 7W, 12D and 12C,
   wherein 1B is a 5-aminopentanoate reductase, a 5-aminopent-2-enoate aminotransferase, a 5-aminopent-2-enoate dehydrogenase or 5-aminopent-2-enoate amine oxidase, wherein 1D is a 2-oxoadipate decarboxylase, wherein 1E is a glutarate semialdehyde reductase, wherein 1F is a 5-hydroxyvalerate reductase, wherein 1G is a 5-hydroxypent-2-enoate dehydratase, wherein 1I is a 5-aminopentanoate aminotransferase, a 5-aminopentanoate dehydrogenase or a 5-aminopentanoate amine oxidase, wherein 1J is a 5-aminopent-4-enoate deaminase, wherein 1K is a 5-hydroxypent-2-enoate reductase, wherein 1L is a 5-hydroxyvaleryl-CoA transferase or a 5-hydroxyvaleryl-CoA synthetase, wherein 1M is a 5-hydroxypentanoyl-CoA dehydrogenase, wherin 1N is a 5-hydroxypent-2-enoyl-CoA dehydratase, wherein 1O is a 2,4-pentadienoyl-CoA transferase, a 2,4-pentadienoyl-CoA synthetase or a 2,4-pentadienoyl-CoA hydrolase, wherein 1P is a 5-hydroxypent-2-enoyl-CoA transferase or a 5-hydroxypent-2-enoyl-CoA synthetase, wherein in 1Q is a 5-hydroxyvaleryl-CoA dehydratase/dehydrogenase, wherein 1S is a glutaryl-CoA reductase, wherein 1T is a 2,4-pentadienoate decarboxylase, wherein 1U is a 5-hydroxypent-2-enoate decarboxylase, wherein 1V is a 3-buten-1-ol dehydratase, wherein 1W is a 5-hydroxyvalerate decarboxylase, wherein 3A is a 3-oxopentanoyl-CoA thiolase or a 3-oxopentanoyl-CoA synthase, wherein 3B is a 3-oxopentanoyl-CoA reductase, wherein 3C is a 3-hydroxypentanoyl-CoA dehydratase, wherein 3D is a pent-2-enoyl-CoA isomerase, wherein 3E is a pent-3-enoyl-CoA dehydrogenase, wherein 3F is a 2,4-pentadienoyl-CoA hydrolase, a 2,4-pentadienoyl-CoA transferase or a 2,4-pentadienoyl-CoA synthetase, wherein 3G is a pent-2-enoyl-CoA dehydrogenase, wherein 5A is a 4-hydroxy-2-oxovalerate aldolase, wherein 5B is a 4-hydroxy-2-oxovalerate dehydratase, wherein 5C is a 2-oxopentenoate decarboxylase, wherein 5D is a 3-buten-1-al reductase, wherein 5E is a 3-buten-1-ol dehydratase, wherein 6A is a 2,4-pentadienoate reductase (acid reducing), wherein 6B is a penta-2,4-dienal decarbonylase, wherein 6C is a 2,4-pentadienoyl-CoA reductase (acid reducing), wherein 6D is a 2,4-pentadienoyl-CoA phosphotransferase, wherein 6E is a 2,4-pentadienoyl-phosphate reductase, wherein 6F is a 2,4-pentadienoyl-CoA hydrolase, a 2,4-pentadienoyl-CoA transferase or a 2,4-pentadienoyl-CoA synthetase, wherein 6H is a 2,4-pentadienoate kinase, wherein 7A is a 3-ketoacyl-ACP synthase, wherein 7B is an acetoacetyl-ACP reductase, wherein 7C is a 3-hydroxybutyryl-ACP dehydratase, wherein 7D is an acetoacetyl-CoA:ACP transferase, wherein 7E is an acetoacetyl-CoA hydrolase, an acetoacetyl-CoA transferase or an acetoacetyl-CoA synthetase, wherein 7F is an acetoacetate reductase (acid reducing), wherein 7H is an acetoacetyl-ACP thioesterase, wherein 71 is an acetoacetyl-CoA reductase (CoA-dependent, aldehyde forming), wherein 7J is an acetoacetyl-ACP reductase (aldehyde forming), wherein 7K is an acetoacetyl-CoA reductase (alcohol forming), wherein 7L is an 3-hydroxybutyryl-ACP thioesterase, wherein 7M is an 3-hydroxybutyryl-ACP reductase (aldehyde forming), wherein 7N is an 3-hydroxybutyryl-CoA reductase (aldehyde forming), wherein 7O is an 3-hydroxybutyryl-CoA reductase (alcohol forming), wherein 7P is an acetoacetyl-CoA reductase (ketone reducing), wherein 7Q is an acetoacetate reductase (ketone reducing), wherein 7R is a 3-oxobutyraldehyde reductase (ketone reducing), wherein 7T is a crotonyl-ACP thioesterase, wherein 7U is a crotonyl-ACP reductase (aldehyde forming), wherein 7V is a crotonyl-CoA reductase (aldehyde forming), wherein 7W is a crotonyl-CoA (alcohol forming), wherein 7X is a 3-hydroxybutyryl-CoA:ACP transferase, wherein 7Y is a 3-hydroxybutyryl-CoA hydrolase, a 3-hydroxybutyryl-CoA transferase or a 3-hydroxybutyryl-CoA synthetase, wherein 7Z is a 3-hydroxybutyrate reductase, wherein 7AB is a 3-hydroxybutyryl-CoA dehydratase, wherein 7AC is a 3-hydroxybutyrate dehydratase, wherein 7AD is a 3-hydroxybutyraldehyde dehydratase, wherein 7AE is a crotonyl-CoA:ACP transferase, wherein 7AF is a crotonyl-CoA hydrolase, a crotonyl-CoA transferase or a crotonyl-CoA synthetase, wherein 7AG is a crotonate reductase, wherein 7AH is a crotonaldehyde reductase, wherein 7AS is an acetoacetyl-CoA synthase, wherein 12A is a crotyl alcohol kinase, wherein 12B is a 2-butenyl-4-phosphate kinase, wherein 12C is a butadiene synthase, and wherein 12D is a crotyl alcohol diphosphokinase.
25. The non-naturally occurring microbial organism of item 24, wherein said microbial organism comprises two, three, four, five, six, seven, eight, nine, ten or eleven exogenous nucleic acids each encoding a butadiene pathway enzyme.
26. The non-naturally occurring microbial organism of item 25, wherein said microbial organism comprises exogenous nucleic acids encoding each of the enzymes of at least one of the pathways selected from (1)-(221).
27. The non-naturally occurring microbial organism of item 24, wherein said at least one exogenous nucleic acid is a heterologous nucleic acid.
28. The non-naturally occurring microbial organism of item 24, wherein said non-naturally occurring microbial organism is in a substantially anaerobic culture medium.
29. The non-naturally occurring microbial organism of item 24, wherein said non-naturally occuring microbial organism comprising a butadiene pathway selected from (43)-(79) further comprises a glutaryl-CoA pathway comprising at least one exogenous nucleic acid encoding a glutaryl-CoA pathway enzyme expressed in a sufficient amount to produce glutaryl-CoA, said glutaryl-CoA pathway comprising a pathway selected from:
   an acetoacetyl-CoA thiolase or acetoacetyl-CoA synthase; an acetoacetyl-CoA reductase; a 3-hydroxybutyryl-CoA dehydratase; and a glutaryl-CoA dehydrogenase; or
   a 2-aminoadipate aminotransferase, a 2-aminoadipate dehydrogenase or a 2-amininoadipate amine oxidase; and a 2-oxoadipate dehydrogenase, a 2-oxoadipate:ferridoxin oxidoreductase or a 2-oxoadipate formate lyase.
30. The non-naturally occurring microbial organism of item 24, wherein said non-naturally occuring microbial organism comprising a butadiene pathway selected from (80)-(116) further comprises a 5-aminopentanoate pathway comprising at least one exogenous nucleic acid encoding a 5-aminopentanoate pathway enzyme expressed in a sufficient amount to produce 5-aminopentanoate, said 5-aminopentanoate pathway comprising a 2-aminoadipate decarboxylase; or a 2-aminoadipate decarboxylase and a 2-aminoadipate aminotransferase, a 2-aminoadipate dehydrogenase or a 2-aminoadipate amine oxidase.
31. The non-naturally occurring microbial organism of item 24, wherein said non-naturally occuring microbial organism comprising a butadiene pathway selected from (1)-(42) further comprises a 2-oxoadipate pathway comprising an exogenous nucleic acid encoding a 2-oxoadipate pathway enzyme expressed in a sufficient amount to produce a 2-oxoadipate, said 2-oxoadipate pathway comprising a 2-aminoadipate aminotransferase, a 2-aminoadipate dehydrogenase or a 2-aminoadipate amine oxidase.
32. The non-naturally occurring microbial organism of item 24, further comprising:
   (i) a reductive TCA pathway comprising at least one exogenous nucleic acid encoding a reductive TCA pathway enzyme, wherein said at least one exogenous nucleic acid is selected from an ATP-citrate lyase, a citrate lyase, a citryl-CoA synthetase, a citryl-CoA lyase, a fumarate reductase, and an alpha-ketoglutarate:ferredoxin oxidoreductase;
   (ii) a reductive TCA pathway comprising at least one exogenous nucleic acid encoding a reductive TCA pathway enzyme, wherein said at least one exogenous nucleic acid is selected from a pyruvate:ferredoxin oxidoreductase, a phosphoenolpyruvate carboxylase, a phosphoenolpyruvate carboxykinase, a CO dehydrogenase, and an H₂ hydrogenase; or
   (iii) at least one exogenous nucleic acid encodes an enzyme selected from a CO dehydrogenase, an H₂ hydrogenase, and combinations thereof.
33. The non-naturally occurring microbial organism of item 32, wherein said microbial organism comprising (i) further comprises an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof.
34. The non-naturally occurring microbial organism of item 32, wherein said microbial organism comprising (ii) further comprises an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof.
35. The non-naturally occurring microbial orgnaism of item 32, wherein said microbial organism comprising (i) comprises three exogenous nucleic acids encoding an ATP-citrate lyase or a citrate lyase, a fumarate reductase, and an alpha-ketoglutarate:ferredoxin oxidoreductase;
   wherein said microbial organism comprising (ii) comprises four exogenous nucleic acids encoding a pyruvate:ferredoxin oxidoreductase; a phosphoenolpyruvate carboxylase or a phosphoenolpyruvate carboxykinase; a CO dehydrogenase; and an H₂ hydrogenase; or
   wherein said microbial organism comprising (iii) comprises two exogenous nucleic acids encoding a CO dehydrogenase and an H₂ hydrogenase.
36. A method for producing butadiene, comprising culturing the non-naturally occurring microbial organism of any one of items 24-35 under conditions and for a sufficient period of time to produce butadiene.
37. A non-naturally occurring microbial organism, comprising a microbial organism having a 3-buten-1-ol pathway comprising at least one exogenous nucleic acid encoding a 3-buten-1-ol pathway enzyme expressed in a sufficient amount to produce 3-buten-1-ol, wherein said 3-buten-1-ol pathway comprises a pathway selected from:
   (1) 1D, 1E, 1F and 1U;
   (2) 1D, 1I, 1B, 1C, 1K and 1U;
   (3) 1D, 1E, 1L, 1M, 1P and 1U;
   (4) 1D, 1E and 1W;
   (5) 1S, 1E, 1F and 1U;
   (6) 1S, 1I, 1B, 1C, 1K and 1U;
   (7) 1S, 1E, 1L, 1M, 1P and 1U;
   (8) 1S, 1E and 1W;
   (9) 1B, 1C, 1K and 1U;
   (10) 1I, 1E, 1F and 1U;
   (11) 1I, 1E, 1L, 1M, 1P and 1U;
   (12) 1I, 1E and 1W; and
   (13) 5A, 5B, 5C and 5D,
   wherein 1B is a 5-aminopentanoate reductase, wherein 1C is a 5-aminopent-2-enoate aminotransferase, a 5-aminopent-2-enoate dehydrogenase or an amine oxidase, wherein 1D is a 2-oxoadipate decarboxylase, wherein 1E is a glutarate semialdehyde reductase, wherein 1F is a 5-hydroxyvalerate dehydrogenase, wherein 1I is a 5-aminopentanoate aminotransferase, a 5-aminopentanoate dehydrogenase or a 5-aminopentanoate amine oxidase, wherein 1K is a 5-hydroxypent-2-enoate reductase, wherein 1L is a 5-hydroxyvaleryl-CoA transferase or a 5-hydroxyvaleryl-CoA synthetase, wherein 1M is a 5-hydroxypentanoyl-CoA dehydrogenase, wherein 1P is a 5-hydroxypent-2-enoyl-CoA transferase or a 5-hydroxypent-2-enoyl-CoA synthetase, wherein 1S is a glutaryl-CoA reductase, wherein 1U is a 5-hydroxypent-2-enoate decarboxylase, wherein 1W is a 5-hydroxyvalerate decarboxylase, wherein 5A is a 4-hydroxy-2-oxovalerate aldolase, wherein 5B is a 4-hydroxy-2-oxovalerate dehydratase, wherein 5C is a 2-oxopentenoate decarboxylase, wherein 5D is a 3-buten-1-al reductase.
38. The non-naturally occurring microbial organism of item 37, wherein said microbial organism comprises two, three, four, five or six exogenous nucleic acids each encoding a 3-buten-1-ol pathway enzyme.
39. The non-naturally occurring microbial organism of item 38, wherein said microbial organism comprises exogenous nucleic acids encoding each of the enzymes of at least one of the pathways selected from (1)-(13).
40. The non-naturally occurring microbial organism of item 37, wherein said at least one exogenous nucleic acid is a heterologous nucleic acid.
41. The non-naturally occurring microbial organism of item 37, wherein said non-naturally occurring microbial organism is in a substantially anaerobic culture medium.
42. The non-naturally occurring microbial organism of item 37, further comprising:
   (i) a reductive TCA pathway comprising at least one exogenous nucleic acid encoding a reductive TCA pathway enzyme, wherein said at least one exogenous nucleic acid is selected from an ATP-citrate lyase, citrate lyase, a fumarate reductase, and an alpha-ketoglutarate:ferredoxin oxidoreductase;
   (ii) a reductive TCA pathway comprising at least one exogenous nucleic acid encoding a reductive TCA pathway enzyme, wherein said at least one exogenous nucleic acid is selected from a pyruvate:ferredoxin oxidoreductase, a phosphoenolpyruvate carboxylase, a phosphoenolpyruvate carboxykinase, a CO dehydrogenase, and an H₂ hydrogenase; or
   (iii) at least one exogenous nucleic acid encodes an enzyme selected from a CO dehydrogenase, an H₂ hydrogenase, and combinations thereof.
43. The non-naturally occurring microbial organism of item 42, wherein said microbial organism comprising (i) further comprises an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacctylasc, an acetyl-CoA synthetase, an NAD(P)H:ferredoxin oxidoreductase, ferredoxin, and combinations thereof.
44. The non-naturally occurring microbial organism of item 42, wherein said microbial organism comprising (ii) further comprises an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof.
45. The non-naturally occurring microbial orgnaism of item 42, wherein said microbial organism comprising (i) comprises three exogenous nucleic acids encoding an ATP-citrate lyase or a citrate lyase, a fumarate reductase, and an alpha-ketoglutarate:ferredoxin oxidoreductase;
   wherein said microbial organism comprising (ii) comprises four exogenous nucleic acids encoding a pyruvate:ferredoxin oxidoreductase; a phosphoenolpyruvate carboxylase or a phosphoenolpyruvate carboxykinase; a CO dehydrogenase; and an H₂ hydrogenase; or
   wherein said microbial organism comprising (iii) comprises two exogenous nucleic acids encoding a CO dehydrogenase and an H₂ hydrogenase.
46. A method for producing 3-buten-1-ol, comprising culturing the non-naturally occurring microbial organism of any one of items 37-45 under conditions and for a sufficient period of time to produce 3-buten-1-ol.
47. A method for producing butadiene, comprising culturing the non-naturally occurring microbial organism of any one of items 37-45 under conditions and for a sufficient to produce 3-buten-1-ol, and chemically dehydrating said 3-buten-1-ol to produce butadiene.
48. A non-naturally occurring microbial organism, comprising a microbial organism having a crotyl alcohol pathway comprising at least one exogenous nucleic acid encoding a crotyl alcohol pathway enzyme expressed in a sufficient amount to produce crotyl alcohol, wherein said crotyl alcohol pathway comprises a pathway selected from:
   (1) 7A, 7J, 7R, 7AD and 7AH;
   (2) 7A, 7H, 7F, 7R, 7AD and 7AH;
   (3) 7A, 7H, 7Q, 7Z, 7AD and 7AH;
   (4) 7A, 7H, 7Q, 7AC, 7AG and 7AH;
   (5) 7A, 7D, 71, 7R, 7AD and 7AH;
   (6) 7A, 7D, 7E, 7F, 7R, 7AD and 7AH;
   (7) 7A, 7D, 7E, 7Q, 7Z, 7AD and 7AH;
   (8) 7A, 7D, 7E, 7Q, 7AC, 7AG and 7AH;
   (9) 7A, 7D, 7P, 7N, 7AD and 7AH;
   (10) 7A, 7D, 7P, 7Y, 7Z, 7AD and 7AH;
   (11) 7A, 7D, 7P, 7Y, 7AC, 7AG and 7AH;
   (12) 7A, 7D, 7P, 7AB, 7V and 7AH;
   (13) 7A, 7D, 7P, 7AB, 7AF, 7AG and 7AH;
   (14) 7A, 7B, 7M, 7AD and 7AH;
   (15) 7A, 7B, 7L, 7Z, 7AD and 7AH;
   (16) 7A, 7B, 7L, 7AC, 7AG and 7AH;
   (17) 7A, 7B, 7X, 7Y, 7Z, 7AD and 7AH;
   (18) 7A, 7B, 7X, 7Y, 7AC, 7AG and 7AH;
   (19) 7A, 7B, 7X, 7AB, 7V and 7AH;
   (20) 7A, 7B, 7X, 7AB, 7AF, 7AG and 7AH;
   (21) 7A, 7B, 7C, 7U and 7AH;
   (22) 7A, 7B, 7C, 7T, 7AG and 7AH;
   (23) 7A, 7B, 7C, 7AE, 7AF, 7AG and 7AH;
   (24) 7A, 7D, 7P, 7AB and 7W;
   (25) 7A, 7B, 7X, 7AB and 7W;
   (26) 7A, 7B, 7C, 7AE and 7W;
   (27) 7A, 7B, 7C, 7AE, 7V and 7AH;
   (28) 71, 7R, 7AD and 7AH;
   (29) 7E, 7F, 7R, 7AD and 7AH;
   (30) 7E, 7Q, 7Z, 7AD and 7AH;
   (31) 7E, 7Q, 7AC, 7AG and 7AH;
   (32) 7P, 7N, 7AD and 7AH;
   (33) 7P, 7Y, 7Z, 7AD and 7AH;
   (34) 7P, 7Y, 7AC, 7AG and 7AH;
   (35) 7P, 7AB, 7V and 7AH;
   (36) 7P, 7AB, 7AF, 7AG and 7AH;
   (37) 7P, 7AB and 7W;
   (38) 7AS, 71, 7R, 7AD and 7AH;
   (39) 7AS, 7E, 7F, 7R, 7AD and 7AH;
   (40) 7AS, 7E, 7Q, 7Z, 7AD and 7AH;
   (41) 7AS, 7E, 7Q, 7AC, 7AG and 7AH;
   (42) 7AS, 7P, 7N, 7AD and 7AH;
   (43) 7AS, 7P, 7Y, 7Z, 7AD and 7AH;
   (44) 7AS, 7P, 7Y, 7AC, 7AG and 7AH;
   (45) 7AS, 7P, 7AB, 7V and 7AH;
   (46) 7AS, 7P, 7AB, 7AF, 7AG and 7AH; and
   (47) 7AS, 7P, 7AB and 7W,
   wherein 7A is a 3-ketoacyl-ACP synthase, wherein 7B is an acetoacetyl-ACP reductase, wherein 7C is a 3-hydroxybutyryl-ACP dehydratase, wherein 7D is an acetoacetyl-CoA:ACP transferase, wherein 7E is an acetoacetyl-CoA hydrolase, an acetoacetyl-CoA transferase or an acetoacetyl-CoA synthetase, wherein 7F is an acetoacetate reductase (acid reducing), wherein 7H is an acetoacetyl-ACP thioesterase, wherein 71 is an acetoacetyl-CoA reductase (CoA-dependent, aldehyde forming), wherein 7J is an acetoacetyl-ACP reductase (aldehyde forming), wherein 7K is an acetoacetyl-CoA reductase (alcohol forming), wherein 7L is an 3-hydroxybutyryl-ACP thioesterase, wherein 7M is an 3-hydroxybutyryl-ACP reductase (aldehyde forming), wherein 7N is an 3-hydroxybutyryl-CoA reductase (aldehyde forming), wherein 7O is an 3-hydroxybutyryl-CoA reductase (alcohol forming), wherein 7P is an acetoacetyl-CoA reductase (ketone reducing), wherein 7Q is an acetoacetate reductase (ketone reducing), wherein 7R is a 3-oxobutyraldehyde reductase (ketone reducing), wherein 7T is a crotonyl-ACP thioesterase, wherein 7U is a crotonyl-ACP reductase (aldehyde forming), wherein 7V is a crotonyl-CoA reductase (aldehyde forming), wherein 7W is a crotonyl-CoA (alcohol forming), wherein 7X is a 3-hydroxybutyryl-CoA:ACP transferase, wherein 7Y is a 3-hydroxybutyryl-CoA hydrolase, a 3-hydroxybutyryl-CoA transferase or a 3-hydroxybutyryl-CoA synthetase, wherein 7Z is a 3-hydroxybutyrate reductase, wherein 7AB is a 3-hydroxybutyryl-CoA dehydratase, wherein 7AC is a 3-hydroxybutyrate dehydratase, wherein 7AD is a 3-hydroxybutyraldehyde dehydratase, wherein 7AE is a crotonyl-CoA:ACP transferase, wherein 7AF is a crotonyl-CoA hydrolase, a crotonyl-CoA transferase or a crotonyl-CoA synthetase, wherein 7AG is a crotonate reductase, wherein 7AH is a crotonaldehyde reductase and wherein 7AS is an acetoacetyl-CoA synthase.
49. The non-naturally occurring microbial organism of item 1, wherein said microbial organism comprises two, three, four, five, six or seven exogenous nucleic acids each encoding a crotyl alcohol pathway enzyme.
50. The non-naturally occurring microbial organism of item 1A, wherein said microbial organism comprises exogenous nucleic acids encoding each of the enzymes of at least one of the pathways selected from (1)-(47).
51. The non-naturally occurring microbial organism of item 1, wherein said at least one exogenous nucleic acid is a heterologous nucleic acid.
52. The non-naturally occurring microbial organism of item 1, wherein said non-naturally occurring microbial organism is in a substantially anaerobic culture medium.
53. The non-naturally occurring microbial organism of item 48, further comprising:
   (i) a reductive TCA pathway comprising at least one exogenous nucleic acid encoding a reductive TCA pathway enzyme, wherein said at least one exogenous nucleic acid is selected from an ATP-citrate lyase, a citrate lyase, a citryl-CoA synthetase, a citryl-CoA lyase, a fumarate reductase, and an alpha-ketoglutarate:ferredoxin oxidoreductase;
   (ii) a reductive TCA pathway comprising at least one exogenous nucleic acid encoding a reductive TCA pathway enzyme, wherein said at least one exogenous nucleic acid is selected from a pyruvate:ferredoxin oxidoreductase, a phosphoenolpyruvate carboxylase, a phosphoenolpyruvate carboxykinase, a CO dehydrogenase, and an H₂ hydrogenase; or
   (iii) at least one exogenous nucleic acid encodes an enzyme selected from a CO dehydrogenase, an H₂ hydrogenase, and combinations thereof.
54. The non-naturally occurring microbial organism of item 53, wherein said microbial organism comprising (i) further comprises an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof.
55. The non-naturally occurring microbial organism of item 53, wherein said microbial organism comprising (ii) further comprises an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof.
56. The non-naturally occurring microbial orgnaism of item 53, wherein said microbial organism comprising (i) comprises three exogenous nucleic acids encoding an ATP-citrate lyase or a citrate lyase, a fumarate reductase, and an alpha-ketoglutarate:ferredoxin oxidoreductase;
   wherein said microbial organism comprising (ii) comprises four exogenous nucleic acids encoding a pyruvate:ferredoxin oxidoreductase; a phosphoenolpyruvate carboxylase or a phosphoenolpyruvate carboxykinase; a CO dehydrogenase; and an H₂ hydrogenase; or
   wherein said microbial organism comprising (iii) comprises two exogenous nucleic acids encoding a CO dehydrogenase and an H₂ hydrogenase.
57. A method for producing crotyl alcohol, comprising culturing the non-naturally occurring microbial organism of any one of items 48-56 under conditions and for a sufficient period of time to produce crotyl alcohol.
58. A non-naturally occurring microbial organism, comprising a microbial organism having a propylene pathway comprising at least one exogenous nucleic acid encoding a propylene pathway enzyme expressed in a sufficient amount to produce propylene, wherein said propylene pathway comprises a pathway selected from:
   (1) 7A, 7J, 7R, 7AD and 7AO;
   (2) 7A, 7H, 7F, 7R, 7AD and 7AO;
   (3) 7A, 7D, 71, 7R, 7AD and 7AO;
   (4) 7A, 7D, 7E, 7F, 7R, 7AD and 7AO;
   (5) 7A, 7H, 7Q, 7Z, 7AD and 7AO;
   (6) 7A, 7D, 7E, 7Q, 7AD and 7AO;
   (7) 7A, 7D, 7P, 7Y, 7Z, 7AD and 7AO;
   (8) 7A, 7D, 7P, 7N, 7AD and 7AO;
   (9) 7A, 7B, 7X, 7N, 7AD and 7AO;
   (10) 7A, 7B, 7X, 7Y, 7Z, 7AD and 7AO;
   (11) 7A, 7H, 7Q, 7V, 7AG and 7AO;
   (12) 7A, 7D, 7E, 7Q, 7AC, 7AG and 7AO;
   (13) 7A, 7D, 7P, 7Y, 7AC, 7AG and 7AO;
   (14) 7A, 7D, 7P, 7AB, 7AF, 7AG and 7AO;
   (15) 7A, 7P, 7AB, 7V and 7AO;
   (16) 7A, 7B, 7M, 7AD and 7AO;
   (17) 7A, 7B, 7L, 7Z, 7AD and 7AO;
   (18) 7A, 7B, 7X, 7N, 7AD and 7AO;
   (19) 7A, 7B, 7X, 7Y, 7Z, 7AD and 7AO;
   (20) 7A, 7B, 7C, 7U and 7AO;
   (21) 7A, 7B, 7C, 7T, 7AG and 7AO;
   (22) 7A, 7B, 7C, 7AE, 7V and 7AO;
   (23) 7A, 7B, 7C, 7AE, 7AF, 7AG and 7AO;
   (24) 7A, 7H, 7Q and 7AR;
   (25) 7A, 7D, 7E, 7Q and 7AR;
   (26) 7A, 7D, 7P, 7Y and 7AR;
   (27) 7A, 7B, 7X, 7Y and 7AR;
   (28) 7A, 7B, 7L and 7AR;
   (29) 7A, 7H, 7Q, 7AC and 7AQ;
   (30) 7A, 7D, 7E, 7Q, 7AC and 7AQ;
   (31) 7A, 7D, 7P, 7Y, 7AC and 7AQ;
   (32) 7A, 7D, 7P, 7AB, 7AF and 7AQ;
   (33) 7A, 7B, 7L, 7AC and 7AQ;
   (34) 7A, 7B, 7X, 7Y, 7AC and 7AQ;
   (35) 7A, 7B, 7X, 7AB, 7AF and 7AQ;
   (36) 7A, 7B, 7C, 7AE, 7AF and 7AQ;
   (37) 7A, 7B, 7C, 7T and 7AQ;
   (38) 7A, 7H, 7Q, 7AC, 7AN and 7AK
   (39) 7A, 7D, 7E, 7Q, 7AC, 7AN and 7AK;
   (40) 7A, 7D, 7P, 7Y, 7AC, 7AN and 7AK;
   (41) 7A, 7D, 7P, 7AB, 7AF, 7AN and 7AK;
   (42) 7A, 7D, 7P, 7AB, 7AM, 7AJ and 7AK;
   (43) 7A, 7B, 7L, 7AC, 7AN and 7AK;
   (44) 7A, 7B, 7X, 7Y, 7AC, 7AN and 7AK;
   (45) 7A, 7B, 7X, 7AB, 7AF, 7AN and 7AK;
   (46) 7A, 7B, 7X, 7AB, 7AM, 7AJ and 7AK;
   (47) 7A, 7B, 7C, 7T, 7AN and 7AK;
   (48) 7A, 7B, 7C, 7AE, 7AF, 7AN and 7AK;
   (49) 7A, 7B, 7C, 7AE, 7AM, 7AJ and 7AK;
   (50) 7A, 7B, 7C, 7AL, 7AP and 7AK;
   (51) 7A, 7B, 7C, 7AL, 7AI, 7AJ and 7AK;
   (52) 7A, 7B, 7X, 7AB, 7V and 7AO;
   (53) 7A 7B, 7L, 7AC, 7AG and 7AO;
   (54) 7A, 7B, 7X, 7Y, 7AC, 7AC, 7AG and 7AO;
   (55) 7A, 7B, 7X, 7AB, 7AF, 7AG and 7AO;
   (56) 7A, 7H, 7Q, 7AC, 7AG and 7AO;
   (57) 71, 7R, 7AD and 7AO;
   (58) 7E, 7F, 7R, 7AD and 7AO;
   (59) 7E, 7Q, 7Z, 7AD and 7AO;
   (60) 7P, 7Y, 7Z, 7AD and 7AO;
   (61) 7P, 7N, 7AD and 7AO;
   (62) 7E, 7Q, 7AC, 7AG and 7AO;
   (63) 7P, 7Y, 7AC, 7AG and 7AO;
   (64) 7P, 7AB, 7AF, 7AG and 7AO;
   (65) 7P, 7AB, 7V and 7AO;
   (66) 7E, 7Q and 7AR;
   (67) 7P, 7Y and 7AR;
   (68) 7E, 7Q, 7AC and 7AQ;
   (69) 7P, 7Y, 7AC and 7AQ;
   (70) 7P, 7AB, 7AF and 7AQ;
   (71) 7E, 7Q, 7AC, 7AN and 7AK;
   (72) 7P, 7Y, 7AC, 7AN and 7AK;
   (73) 7P, 7AB, 7AF, 7AN and 7AK;
   (74) 7P, 7AB, 7AM, 7AJ and 7AK;
   (75) 7AS, 71, 7R, 7AD and 7AO;
   (76) 7AS, 7E, 7F, 7R, 7AD and 7AO;
   (77) 7AS, 7E, 7Q, 7AD and 7AO;
   (78) 7AS, 7P, 7Y, 7Z, 7AD and 7AO;
   (79) 7AS, 7P, 7N, 7AD and 7AO;
   (80) 7AS, 7E, 7Q, 7AC, 7AG and 7AO;
   (81) 7AS, 7P, 7Y, 7AC, 7AG and 7AO;
   (82) 7AS, 7P, 7AB, 7AF, 7AG and 7AO;
   (83) 7AS, 7E, 7Q and 7AR;
   (84) 7AS, 7P, 7Y and 7AR;
   (85) 7AS, 7E, 7Q, 7AC and 7AQ;
   (86) 7AS, 7P, 7Y, 7AC and 7AQ;
   (87) 7AS, 7P, 7AB, 7AF and 7AQ;
   (88) 7AS, 7E, 7Q, 7AC, 7AN and 7AK;
   (89) 7AS, 7P, 7Y, 7AC, 7AN and 7AK;
   (90) 7AS, 7P, 7AB, 7AF, 7AN and 7AK; and
   (91) 7AS, 7P, 7AB, 7AM, 7AJ and 7AK,
   wherein 7A is a 3-ketoacyl-ACP synthase, wherein 7B is an acetoacetyl-ACP reductase, wherein 7C is a 3-hydroxybutyryl-ACP dehydratase, wherein 7D is an acetoacetyl-CoA:ACP transferase, wherein 7E is an acetoacetyl-CoA hydrolase, an acetoacetyl-CoA transferase or an acetoacetyl-CoA synthetase, wherein 7F is an acetoacetate reductase (acid reducing), wherein 7H is an acetoacetyl-ACP thioesterase, wherein 71 is an acetoacetyl-CoA reductase (CoA-dependent, aldehyde forming), wherein 7J is an acetoacetyl-ACP reductase (aldehyde forming), wherein 7L is a 3-hydroxybutyryl-ACP thioesterase, wherein 7M is a 3-hydroxybutyryl-ACP reductase (aldehyde forming), wherein 7N is a 3-hydroxybutyryl-CoA reductase (aldehyde forming), wherein 7P is an acetoacetyl-CoA reductase (ketone reducing), wherein 7Q is an acetoacetate reductase (ketone reducing), wherein 7R is a 3-oxobutyraldehyde reductase (ketone reducing), wherein 7S is a 4-hydroxy-2-butanone reductase, wherein 7T is a crotonyl-ACP thioesterase, wherein 7U is a crotonyl-ACP reductase (aldehyde forming), wherein 7V is a crotonyl-CoA reductase (aldehyde forming), wherein 7X is a 3-hydroxybutyryl-CoA:ACP transferase, wherein 7Y is a 3-hydroxybutyryl-CoA hydrolase, a 3-hydroxybutyryl-CoA transferase or a 3-hydroxybutyryl-CoA synthetase, wherein 7Z is a 3-hydroxybutyrate reductase, wherein 7AB is a 3-hydroxybutyryl-CoA dehydratase, wherein 7AC is a 3-hydroxybutyrate dehydratase, wherein 7AD is a 3-hydroxybutyraldehyde dehydratase, wherein 7AE is a crotonyl-CoA:ACP transferase, wherein 7AF is a crotonyl-CoA hydrolase, a crotonyl-CoA transferase or a crotonyl-CoA synthetase, wherein 7AG is a crotonate reductase, wherein 7AI is a butryl-CoA:ACP transferase, wherein 7AJ is a butyryl-CoA transferase, a butyryl-CoA hydrolase or a butyryl-CoA synthetase, wherein 7AK is a butyrate decarboxylase, wherein 7AL is a crotonyl-ACP reductase, wherein 7AM is a crotonyl-CoA reductase, wherein 7AN is a crotonate reductase, wherein 7AO is a crotonaldehyde decarbonylase, wherein 7AP is a butyryl-ACP thioesterase, wherein 7AQ is a crotonate decarboxylase, wherein 7AR is a 3-hydroxybutyrate decarboxylase and wherein 7AS is an acetoacetyl-CoA synthase.
59. The non-naturally occurring microbial organism of item 58, wherein said microbial organism comprises two, three, four, five, six, seven or eight exogenous nucleic acids each encoding a propylene pathway enzyme.
60. The non-naturally occurring microbial organism of item 59, wherein said microbial organism comprises exogenous nucleic acids encoding each of the enzymes of at least one of the pathways selected from (1)-(91).
61. The non-naturally occurring microbial organism of item 58, wherein said at least one exogenous nucleic acid is a heterologous nucleic acid.
62. The non-naturally occurring microbial organism of item 58, wherein said non-naturally occurring microbial organism is in a substantially anaerobic culture medium.
63. The non-naturally occurring microbial organism of item 58, further comprising:
   (i) a reductive TCA pathway comprising at least one exogenous nucleic acid encoding a reductive TCA pathway enzyme, wherein said at least one exogenous nucleic acid is selected from an ATP-citrate lyase, a citrate lyase, a citryl-CoA synthetase, a citryl-CoA lyase, a fumarate reductase, and an alpha-ketoglutarate:ferredoxin oxidoreductase;
   (ii) a reductive TCA pathway comprising at least one exogenous nucleic acid encoding a reductive TCA pathway enzyme, wherein said at least one exogenous nucleic acid is selected from a pyruvate:ferredoxin oxidoreductase, a phosphoenolpyruvate carboxylase, a phosphoenolpyruvate carboxykinase, a CO dehydrogenase, and an H₂ hydrogenase; or
   (iii) at least one exogenous nucleic acid encodes an enzyme selected from a CO dehydrogenase, an H₂ hydrogenase, and combinations thereof.
64. The non-naturally occurring microbial organism of item 63 [[62]], wherein said microbial organism comprising (i) further comprises an exogenous nucleic acid encoding an enzyme selected from a pyruvate:ferredoxin oxidoreductase, an aconitase, an isocitrate dehydrogenase, a succinyl-CoA synthetase, a succinyl-CoA transferase, a fumarase, a malate dehydrogenase, an acetate kinase, a phosphotransacetylase, an acetyl-CoA synthetase, an NAD(P)H:ferredoxin oxidoreductase, ferredoxin, and combinations thereof.
65. The non-naturally occurring microbial organism of item 63 [[62]], wherein said microbial organism comprising (ii) further comprises an exogenous nucleic acid encoding an enzyme selected from an aconitase, an isocitrate dehydrogenase, a succinyl-CoA synthetase, a succinyl-CoA transferase, a fumarase, a malate dehydrogenase, and combinations thereof.
66. The non-naturally occurring microbial orgnaism of item 63 [[62]], wherein said microbial organism comprising (i) comprises three exogenous nucleic acids encoding an ATP-citrate lyase or a citrate lyase, a fumarate reductase, and an alpha-ketoglutarate: ferredoxin oxidoreductase;
   wherein said microbial organism comprising (ii) comprises four exogenous nucleic acids encoding a pyruvate:ferredoxin oxidoreductase; a phosphoenolpyruvate carboxylase or a phosphoenolpyruvate carboxykinase; a CO dehydrogenase; and an H₂ hydrogenase; or
   wherein said microbial organism comprising (iii) comprises two exogenous nucleic acids encoding a CO dehydrogenase and an H₂ hydrogenase.
67. A method for producing propylene, comprising culturing the non-naturally occurring microbial organism of any one of items 58-66 under conditions and for a sufficient period of time to produce propylene.
68. A process for the production of butadiene comprising:
   (a) culturing by fermentation in a sufficient amount of nutrients and media the non-naturally occurring microbial organism of any one of items 48-56 to produce crotyl alcohol; and
   (b) converting crotyl alcohol produced by culturing said non-naturally occurring microbial organism to butadiene.
69. The process of item 68, wherein step (b) is performed by chemical dehydration in the presence of a catalyst.

## Claims

1. A non-naturally occurring microbial organism, comprising a microbial organism having a 1,3-butanediol pathway comprising exogenous nucleic acids each encoding a 1,3-butanediol pathway enzyme expressed in a sufficient amount to produce 1,3-butanediol, wherein said 1,3-butanediol pathway comprises a pathway selected from:
(6) 7A, 7D, 7E, 7F, 7G and 7S;
(7) 7A, 7D, 7I, 7G and 7S;
(8) 7A, 7D, 7K and 7S;
(9) 7A, 7H, 7F, 7G and 7S;
(10) 7A, 7J, 7G and 7S;
(11) 7A, 7J, 7R and 7AA;
(12) 7A, 7H, 7F, 7R and 7AA;
(13) 7A, 7H, 7Q, 7Z and 7AA;
(14) 7A, 7D, 71, 7R and 7AA;
(15) 7A, 7D, 7E, 7F, 7R and 7AA;
(16) 7A, 7D, 7E, 7Q, 7Z and 7AA;
(17) 7A, 7D, 7P, 7N and 7AA;
(18) 7A, 7D, 7P, 7Y, 7Z and 7AA;
(19) 7A, 7B, 7M and 7AA;
(20) 7A, 7B, 7L, 7Z and 7AA;
(21) 7A, 7B, 7X, 7N and 7AA;
(22) 7A, 7B, 7X, 7Y, 7Z and 7AA;
(23) 7A, 7D, 7P and 7O;
(24) 7A, 7B, 7X and 7O;
(25) 7A, 7D, 7E, 7F, 7R and 7AA; and
(26) 7A, 7D, 7E, 7F, 7G and 7S
wherein 7A is a 3-ketoacyl-ACP synthase, wherein 7B is an acetoacetyl-ACP reductase, wherein 7D is an acetoacetyl-CoA:ACP transferase, wherein 7E is an acetoacetyl-CoA hydrolase, acetoacetyl-CoA transferase or acetoacetyl-CoA synthetase catalyzing conversion of acetoacetyl-CoA to acetoacetate, wherein 7F is an acetoacetate reductase (acid reducing) catalyzing conversion of acetoacetate to 3-oxobuytraldehyde, wherein 7G is a 3-oxobutyraldehyde reductase (aldehyde reducing) catalyzing conversion of 3-oxobutyraldehyde to 4-hydroxy-2-butanone, wherein 7H is an acetoacetyl-ACP thioesterase, wherein 71 is an acetoacetyl-CoA reductase (CoA-dependent, aldehyde forming) catalyzing conversion of acetoacetyl-CoA to 3-oxobuytraldehyde, wherein 7J is an acetoacetyl-ACP reductase (aldehyde forming), wherein 7K is an acetoacetyl-CoA reductase (alcohol forming) catalyzing conversion of acetoacetyl-CoA to 4-hydroxy-2-butanone, wherein 7L is a 3-hydroxybutyryl-ACP thioesterase, wherein 7M is a 3-hydroxybutyryl-ACP reductase (aldehyde forming), wherein 7N is a 3-hydroxybutyryl-CoA reductase (aldehyde forming) catalyzing conversion of 3-hydroxybutyryl-CoA to 3-hydroxybutyraldehyde, wherein 7O is a 3-hydroxybutyryl-CoA reductase (alcohol forming) catalyzing conversion of 3-hydroxybutyryl-CoA to 1,3-butanediol, wherein 7P is an acetoacetyl-CoA reductase (ketone reducing) catalyzing conversion of acetoacetyl-CoA to 3-hydroxybutyryl-CoA, wherein 7Q is an acetoacetate reductase (ketone reducing) catalyzing conversion of acetoacetate to 3-hydroxybutyrate, wherein 7R is a 3-oxobutyraldehyde reductase (ketone reducing) catalyzing conversion of 3-oxobutyraldehyde to 3-hydroxybutyraldehyde, wherein 7S is a 4-hydroxy-2-butanone reductase catalyzing conversion of 4-hydroxy-2-butanone to 1,3-butanediol, wherein 7X is a 3-hydroxybutyryl-CoA:ACP transferase, wherein 7Y is a 3-hydroxybutyryl-CoA hydrolase, a 3-hydroxybutyryl-CoA transferase or a 3-hydroxybutyryl-CoA synthetase catalyzing conversion of 3-hydroxybutyryl-CoA to 3-hydroxybutyrate, wherein 7Z is a 3-hydroxybutyrate reductase catalyzing conversion of 3-hydroxybutyrate to 3-hydroxybutyraldehdye, and wherein 7AA is a 3-hydroxybutyraldehyde reductase catalyzing conversion of 3-hydroxybutyraldehyde to 1,3-butanediol.

2. The non-naturally occurring microbial organism of claim 1, wherein said 1,3-butanediol pathway comprises a pathway selected from:
(6) 7A, 7D, 7E, 7F, 7G and 7S;
(7) 7A, 7D, 71, 7G and 7S;
(8) 7A, 7D, 7K and 7S;
(9) 7A, 7H, 7F, 7G and 7S;
(10) 7A, 7J, 7G and 7S; and
(26) 7A, 7D, 7E, 7F, 7G and 7S

3. The non-naturally occurring microbial organism of claim 1, wherein said 1,3-butanediol pathway comprises a pathway selected from:
(11) 7A, 7J, 7R and 7AA;
(12) 7A, 7H, 7F, 7R and 7AA;
(13) 7A, 7H, 7Q, 7Z and 7AA;
(14) 7A, 7D, 71, 7R and 7AA;
(15) 7A, 7D, 7E, 7F, 7R and 7AA;
(16) 7A, 7D, 7E, 7Q, 7Z and 7AA;
(17) 7A, 7D, 7P, 7N and 7AA;
(18) 7A, 7D, 7P, 7Y, 7Z and 7AA;
(19) 7A, 7B, 7M and 7AA;
(20) 7A, 7B, 7L, 7Z and 7AA;
(21) 7A, 7B, 7X, 7N and 7AA;
(22) 7A, 7B, 7X, 7Y, 7Z and 7AA; and
(25) 7A, 7D, 7E, 7F, 7R and 7AA

4. The non-naturally occurring microbial organism of claim 1, wherein said 1,3-butanediol pathway comprises a pathway selected from:
(23) 7A, 7D, 7P and 7O; and
(24) 7A, 7B, 7X and 7O

5. The non-naturally occurring microbial organism of any one of claims 1-4, wherein at least one, two, three, four, five or six of said exogenous nucleic acids are heterologous nucleic acids.

6. The non-naturally occurring microbial organism of any one of claims 1-5, further comprising:
(i) a reductive TCA pathway comprising at least one exogenous nucleic acid encoding a reductive TCA pathway enzyme, wherein said at least one exogenous nucleic acid is selected from an ATP-citrate lyase, a citrate lyase, a citryl-CoA synthetase, a citryl-CoA lyase, a fumarate reductase, and an alpha-ketoglutarate:ferredoxin oxidoreductase;
(ii) a reductive TCA pathway comprising at least one exogenous nucleic acid encoding a reductive TCA pathway enzyme, wherein said at least one exogenous nucleic acid is selected from a pyruvate:ferredoxin oxidoreductase, a phosphoenolpyruvate carboxylase, a phosphoenolpyruvate carboxykinase, a CO dehydrogenase, and an H2 hydrogenase; or
(iii) at least one exogenous nucleic acid encodes an enzyme selected from a CO dehydrogenase, an H₂ hydrogenase, and combinations thereof.

7. The non-naturally occurring microbial organism of any one of claims 1-6, wherein said non-naturally occurring microorganism is a species of bacteria, yeast, or fungus.

8. The non-naturally occurring microbial organism of claim 7, wherein said bacteria is selected from the group consisting of *Escherichia coli, Klebsiella oxytoca, Anaerobiospirillum succiniciproducens, Actinobacillus succinogenes*, *Mannheimia succiniciproducens, Rhizobium etli, Bacillus subtilis, Corynebacterium glutamicum, Cupriavidus necator, Gluconobacter oxydans, Zymomonas mobilis, Lactococcus lactis, Lactobacillus plantarum, Streptomyces coelicolor*, *Clostridium acetobutylicum*, *Pseudomonas fluorescens*, and *Pseudomonoas putida.*

9. The non-naturally occurring microbial organism of claim 8, wherein said yeast or fungus is selected from the group consisting of *Saccharomyces cerevisiae, Schizosaccharomyces pombe, Kluyveromyces lactis, Kluyveromyces marxianus, Aspergillus terreus, Aspergillus niger, Pichia pastoris, Rhizopus arrhizus, Rhiobus oryzae, Yarrowia lipolytica,* and *Candida albicans.*

10. A method for producing 1,3-butanediol, comprising culturing the non-naturally occurring microbial organism of any one of claims 1-9 under conditions and for a sufficient period of time to produce 1,3-butanediol.

11. The method of claim 10, wherein said method further comprises separating the 1,3-butanediol from other components in the culture.

12. The method of claim 11, wherein the separating comprises extraction, continuous liquid-liquid extraction, pervaporation, membrane filtration, membrane separation, reverse osmosis, electrodialysis, distillation, crystallization, centrifugation, extractive filtration, ion exchange chromatography, size exclusion chromatography, adsorption chromatography, and/or ultrafiltration.

13. The method of claim 12, wherein the separating comprises distillation.
